# EUROPEAN PATENT SPECIFICATION

(11) **EP 2 655 650 B1**
(45) Date of publication and mention of the grant of the patent: **20.12.2017**
(21) Application number: 11811536.9
(22) Date of filing: 20.12.2011
(51) Int. Cl.: C12P 17/06, C12P 17/16, C12N 9/88, C12N 15/70, C12N 9/00

(54) **ENZYMATIC SYNTHESIS OF ACTIVE PHARMACEUTICAL INGREDIENT AND INTERMEDIATES THEREOF**
ENZYMATISCHE SYNTHESE EINES PHARMAZEUTISCHEN WIRKSTOFFS UND ZWISCHENPRODUKTE DAVON
SYNTHÈSE ENZYMATIQUE D'UN PRINCIPE ACTIF PHARMACEUTIQUE ET DE SES INTERMÉDIAIRES

(30) Priority: 20.12.2010 EP 10015774; 31.05.2011 EP 11168227
(43) Date of publication of application: 30.10.2013
(73) Proprietor: LEK Pharmaceuticals d.d., 1526 Ljubljana (SI)
(72) Inventor: MRAK, Peter, 1526 Ljubljana (SI); ZOHAR, Tadeja, 1526 Ljubljana (SI); OSLAJ, Matej, 1526 Ljubljana (SI); KOPITAR, Gregor, 1526 Ljubljana (SI)
(74) Representative: Prüfer & Partner mbB Patentanwälte · Rechtsanwälte
(86) International application number: PCT/EP2011/073412
(87) International publication number: WO 2012/095244

(56) References cited:
- WO-A1-2007/068498
- WO-A1-2013/068917
- WO-A2-2009/092702
- COZIER GYLES E ET AL: "Characterization of the membrane quinoprotein glucose dehydrogenase from Escherichia coli and characterization of a site-directed mutant in which histidine-262 has been changed to tyrosine", BIOCHEMICAL JOURNAL, THE BIOCHEMICAL SOCIETY, LONDON, GB, vol. 340, no. 3, 15 June 1999 (1999-06-15) , pages 639-647, XP002253111, ISSN: 0264-6021, DOI: 10.1042/0264-6021:3400639 cited in the application
- S. M. SOUTHALL ET AL: "Soluble aldose sugar dehydrogenase from Escherichia coli: A highly exposed active site conferring broad substrate specificity", JOURNAL OF BIOLOGICAL CHEMISTRY, vol. 281, no. 41, 1 January 2006 (2006-01-01), pages 30650-30659, XP055028730, ISSN: 0021-9258, DOI: 10.1074/jbc.M601783200 cited in the application
- ARJEN J. J. OLSTHOORN ET AL: "On the Mechanism and Specificity of Soluble, Quinoprotein Glucose Dehydrogenase in the Oxidation of Aldose Sugars", BIOCHEMISTRY, vol. 37, no. 39, 1 September 1998 (1998-09-01), pages 13854-13861, XP055028519, ISSN: 0006-2960, DOI: 10.1021/bi9808868 cited in the application
- CLINE ET AL: "Enzymatic characterization and comparison of three sugar dehydrogenases from a pseudomonad.", JOURNAL OF BIOLOGICAL CHEMISTRY, vol. 240, no. 11, 1 November 1965 (1965-11-01), pages 4493-4497, XP055028572, ISSN: 0021-9258 cited in the application
- ASADA Y ET AL: "Biochemical and structural characterization of a short-chain dehydrogenase/reductase of Thermus thermophilus HB8", CHEMICO-BIOLOGICAL INTERACTIONS, ELSEVIER SCIENCE IRLAND, IR, vol. 178, no. 1-3, 16 March 2009 (2009-03-16), pages 117-126, XP025909722, ISSN: 0009-2797, DOI: 10.1016/J.CBI.2008.09.018 [retrieved on 2008-09-24]
- MOLINARI F: "Oxidations with isolated and cell-bound dehydrogenases and oxidases", CURRENT ORGANIC CHEMISTRY, vol. 10, no. 11, July 2006 (2006-07), pages 1247-1263, XP008152350, ISSN: 1385-2728, DOI: 10.2174/138527206777698048
- JOHANNIS A. DUINE: "Quinoproteins: enzymes containing the quinonoid cofactor pyrroloquinoline quinone, topaquinone or tryptophan-tryptophan quinone", EUROPEAN JOURNAL OF BIOCHEMISTRY, vol. 200, no. 2, 1 September 1991 (1991-09-01), pages 271-284, XP055028699, ISSN: 0014-2956, DOI: 10.1111/j.1432-1033.1991.tb16183.x cited in the application
- BUCHERT J: "A XYLOSE-OXIDIZING MEMBRANE-BOUND ALDOSE DEHYDROGENASE OF GLUCONOBACTER OXYDANS ATCC 621", JOURNAL OF BIOTECHNOLOGY, ELSEVIER SCIENCE PUBLISHERS, AMSTERDAM, NL, vol. 18, no. 1/02, 1 January 1991 (1991-01-01), pages 103-114, XP009027385, ISSN: 0168-1656, DOI: 10.1016/0168-1656(91)90239-R
- SMOLANDER M ET AL: "Large-scale applicable purification and characterization of a membrane-bound PQQ-dependent aldose dehydrogenase", JOURNAL OF BIOTECHNOLOGY, ELSEVIER SCIENCE PUBLISHERS, AMSTERDAM, NL, vol. 29, no. 3, 1 June 1993 (1993-06-01), pages 287-297, XP023794612, ISSN: 0168-1656, DOI: 10.1016/0168-1656(93)90060-Z [retrieved on 1993-06-01]
- MICHAEL WOLBERG ET AL: "Chemoenzymatic synthesis of the chiral side-chain of statins: application of an alcohol dehydrogenase catalysed ketone reduction on a large scale", BIOPROCESS AND BIOSYSTEMS ENGINEERING, SPRINGER, BERLIN, DE, vol. 31, no. 3, 21 February 2008 (2008-02-21), pages 183-191, XP019590888, ISSN: 1615-7605
- ANNA BARIOTAKI ET AL: "Enzymatic Reductions for the Regio- and Stereoselective Synthesis of Hydroxy-keto Esters and Dihydroxy Esters", ORGANIC LETTERS, vol. 14, no. 7, 6 April 2012 (2012-04-06), pages 1792-1795, XP055028569, ISSN: 1523-7060, DOI: 10.1021/ol3003833
- Lutz Hilterhaus ET AL: "Oxidation by dehydrogenases (38.3)" In: "Enzyme catalysis in organic synthesis", 1 January 2012 (2012-01-01), Wiley-VCH, Weinheim, Germany, XP055028575, ISBN: 978-3-527-32547-4 vol. 3, pages 1591-1594,
- MANZONI M ET AL: "Biosynthesis and biotechnological production of statins by filamentous fungi and application of these cholesterol-lowering drugs", APPLIED MICROBIOLOGY AND BIOTECHNOLOGY, SPRINGER VERLAG, BERLIN, DE, vol. 58, no. 5, 1 April 2002 (2002-04-01), pages 555-564, XP002505368, ISSN: 0175-7598, DOI: 10.1007/S00253-002-0932-9

### SEQUENCE LISTING

<110> Lek Pharmaceuticals d.d.
<120> Enzymatic synthesis of active pharmaceutical ingredient and
   intermediates thereof
<130> Enzymatic synthesis of active pharmaceutical ingredient and
   intermediates thereof
<150> EP10015774.2
   <151> 2010-12-20
<150> EP11168227.4
   <151> 2011-05-31
<160> 82
<170> PatentIn version 3.5
<210> 1
   <211> 1134
   <212> DNA
   <213> Artificial
<220>
   <223> GDH 01; Sequence based on nucleotide sequence encoding aldose
   sugar dehydrogenase YliI (originates from Escherichia coli)
<400> 1
   gcgccatatg catcgacaat cctttttcct tgtgcccctt atttgtcttt cttccgctct      60
   ctgggcggct cctgcaacgg taaatgtcga agtactgcaa gacaaactcg accatccctg     120
   ggcactggcc tttttacccg ataatcacgg tatgttaatc actctgcgcg gcggcgagtt     180
   gcgtcactgg caagcaggaa aaggattatc tgcgccgctt tccggagttc cggacgtttg     240
   ggcgcacggg cagggcggcc tgctggacgt ggttttagcg cctgattttg ctcagtctcg     300
   ccgcatctgg ttaagttatt ccgaagttgg cgatgatggc aaagccggaa ctgctgtggg     360
   ttatggccgc ttaagtgatg atctctcaaa agtgaccgac ttccgcaccg tcttccgcca     420
   gatgccaaaa ctgtctaccg gcaaccattt tggcgggcgg ctggtattcg acggtaaagg     480
   ttatcttttt attgctctgg gcgaaaacaa tcagcgcccg acggcgcagg atctggataa     540
   attacagggc aaactggtgc gtctgaccga ccagggcgaa atcccggatg ataatccttt     600
   tataaaggaa tccggtgtgc gcgccgagat ctggtcttat ggcattcgta atccgcaagg     660
   aatggcgatg aatccgtgga gtaatgcact gtggctgaat gaacatggcc cgcgcggtgg     720
   tgatgaaatt aatatcccgc aaaaaggcaa aaactacggc tggccgctgg caacctgggg     780
   aatcaactat tcaggcttta agataccgga agcgaaaggg gagatcgtcg ccgggaccga     840
   gcaacctgtt ttttactgga aagattcgcc cgctgtgagc ggcatggcct tctataacag     900
   cgataaattc ccccagtggc agcaaaaatt atttattggc gcgctgaaag ataaagatgt     960
   cattgtgatg agcgtcaacg gcgacaaagt gacagaagat ggccgtattt taacggacag    1020
   agggcagcga attcgtgatg ttcgcactgg acccgacggt tatttatacg ttctcaccga    1080
   cgagtccagt ggggaattac ttaaagttag cccacgcaat tagcctgagc gcgc          1134
<210> 2
   <211> 371
   <212> PRT
   <213> Escherichia coli
<400> 2
   Met His Arg Gln Ser Phe Phe Leu Val Pro Leu Ile Cys Leu Ser Ser
   1               5                   10                  15
   Ala Leu Trp Ala Ala Pro Ala Thr Val Asn Val Glu Val Leu Gln Asp
               20                  25                  30
   Lys Leu Asp His Pro Trp Ala Leu Ala Phe Leu Pro Asp Asn His Gly
           35                  40                  45
   Met Leu Ile Thr Leu Arg Gly Gly Glu Leu Arg His Trp Gln Ala Gly
       50                  55                  60
   Lys Gly Leu Ser Ala Pro Leu Ser Gly Val Pro Asp Val Trp Ala His
   65                  70                  75                  80
   Gly Gln Gly Gly Leu Leu Asp Val Val Leu Ala Pro Asp Phe Ala Gln
                   85                  90                  95
   Ser Arg Arg Ile Trp Leu Ser Tyr Ser Glu Val Gly Asp Asp Gly Lys
               100                 105                 110
   Ala Gly Thr Ala Val Gly Tyr Gly Arg Leu Ser Asp Asp Leu Ser Lys
           115                 120                 125
   Val Thr Asp Phe Arg Thr Val Phe Arg Gln Met Pro Lys Leu Ser Thr
       130                 135                 140
   Gly Asn His Phe Gly Gly Arg Leu Val Phe Asp Gly Lys Gly Tyr Leu
   145                 150                 155                 160
   Phe Ile Ala Leu Gly Glu Asn Asn Gln Arg Pro Thr Ala Gln Asp Leu
                   165                 170                 175
   Asp Lys Leu Gln Gly Lys Leu Val Arg Leu Thr Asp Gln Gly Glu Ile
               180                 185                 190
   Pro Asp Asp Asn Pro Phe Ile Lys Glu Ser Gly Val Arg Ala Glu Ile
           195                 200                 205
   Trp Ser Tyr Gly Ile Arg Asn Pro Gln Gly Met Ala Met Asn Pro Trp
       210                 215                 220
   Ser Asn Ala Leu Trp Leu Asn Glu His Gly Pro Arg Gly Gly Asp Glu
   225 230 235 240
   Ile Asn Ile Pro Gln Lys Gly Lys Asn Tyr Gly Trp Pro Leu Ala Thr
   245 250 255
   Trp Gly Ile Asn Tyr Ser Gly Phe Lys Ile Pro Glu Ala Lys Gly Glu
   260 265 270
   Ile Val Ala Gly Thr Glu Gln Pro Val Phe Tyr Trp Lys Asp Ser Pro
   275 280 285
   Ala Val Ser Gly Met Ala Phe Tyr Asn Ser Asp Lys Phe Pro Gln Trp
   290 295 300
   Gln Gln Lys Leu Phe Ile Gly Ala Leu Lys Asp Lys Asp Val Ile Val
   305 310 315 320
   Met Ser Val Asn Gly Asp Lys Val Thr Glu Asp Gly Arg Ile Leu Thr
   325 330 335
   Asp Arg Gly Gln Arg Ile Arg Asp Val Arg Thr Gly Pro Asp Gly Tyr
   340 345 350
   Leu Tyr Val Leu Thr Asp Glu Ser Ser Gly Glu Leu Leu Lys Val Ser
   355 360 365
   Pro Arg Asn
   370
<210> 3
   <211> 2416
   <212> DNA
   <213> Artificial
<220>
   <223> GDH 02; Sequence based on nucleotide sequence encoding membrane bound glucose dehydrogenase Gcd (originates from Escherichia coli)
<400> 3
   gcgccatatg gcaattaaca atacaggctc gcgacgatta ctcgtcacgc taacagccct 60
   ttttgcagcg ctttgcgggc tgtatctact cattggcgga ggctggctgg tcgcgattgg 120
   cggctcctgg tactacccta tcgctggcct tgtgatgctc ggcgtcgcct ggatgctgtg 180
   gcgcagtaaa cgcgccgcgc tttggctata cgcagccctg ctgctcggca ccatgatttg 240
   gggcgtctgg gaagttggtt tcgacttctg ggcgctgact ccgcgcagcg acattctggt 300
   cttcttcggc atctggctga tcctgccgtt tgtctggcgt cgcctggtca ttcctgccag 360
   cggcgcagtt gccgcactgg tggtcgcact gctgattagc ggtggtatcc tgacctgggc 420
   cggatttaac gatccgcagg agatcaacgg caccttaagc gccgatgcca cacctgctga 480
   agctatctcc cccgtagccg atcaggactg gcctgcctat ggtcgtaatc aggaaggtca 540
   acgcttttcg ccgctgaaac aaattaacgc cgataacgtc cataatctga aagaagcctg 600
   ggtgttccgt actggcgatg tgaagcagcc gaacgatccg ggtgaaatca ccaatgaagt 660
   gacgccgatt aaagtgggcg acacccttta cctgtgtacc gctcaccagc gcctgtttgc 720
   gcttgatgcc gccagcggca aagagaaatg gcattacgat cctgagctga aaaccaacga 780
   gtctttccag cacgtaacct gccgtggtgt ctcttatcat gaagccaaag cagaaaccgc 840
   ttcgccggaa gtgatggcgg attgcccgcg tcgtatcatt cttccggtca atgatggtcg 900
   actgattgcg attaacgctg aaaacggcaa actgtgcgaa accttcgcca ataaaggcgt 960
   gctcaatctg caaagcaata tgccagacac caaaccgggt ctgtatgaac cgacttcgcc 1020
   accgattatc accgataaaa ccatcgtgat ggccggttca gttaccgata acttctcaac 1080
   ccgcgaaacg tctggcgtga tccgtggttt tgatgtcaac accggggagc tgctgtgggc 1140
   ttttgatccc ggcgcgaaag atccgaacgc aatcccgtct gacgaacaca cctttacctt 1200
   taactcgcca aactcctggg caccagcggc ctatgacgcg aagctggatc tggtctatct 1260
   gccgatgggc gtgaccacgc cggatatctg gggcggtaac cgcacaccgg aacaggaacg 1320
   ttatgccagc tcgattctgg cgctgaatgc cactaccggg aaactggcgt ggagctacca 1380
   gaccgttcac cacgacctgt gggacatgga tcttccggca cagccgacgc tggcggacat 1440
   caccgttaat ggtcagaaag tgccagttat ttacgctccg gcgaaaaccg gcaacatttt 1500
   tgtgctcgat cgtcgtaatg gcgaactggt ggttccggca ccggaaaaac cggttcccca 1560
   aggtgcagcg aaaggcgatt acgtaacccc aactcaaccg ttttctgaac tgagcttccg 1620
   tccgacgaaa gatttgagcg gtgcggatat gtggggagcc accatgtttg accaactggt 1680
   gtgccgcgtg atgttccacc agatgcgcta tgaaggcatt ttcaccccgc catctgaaca 1740
   gggtacgctg gtcttcccgg gtaacctggg gatgttcgaa tggggcggga tttccgttga 1800
   tccaaatcgt gaagtggcga ttgccaaccc aatggcactg ccgtttgttt cgaaactgat 1860
   cccgcgtggt cctggcaacc cgatggagca gccgaaagat gccaaaggca cgggtacgga 1920
   atccggcatt cagccacagt acggtgtacc gtatggtgtc acgctcaacc cgttcctctc 1980
   accatttggt ctgccatgta aacagccagc atggggttat atctcggcgc tggatctgaa 2040
   aactaatgaa gtggtgtgga agaaacgtat tggtacgccg caggacagta tgccgttccc 2100
   gatgccggtt ccggtgccgt tcaatatggg tatgccgatg ctgggcgggc caatctccac 2160
   ggcgggtaac gtgctgttta tcgccgctac ggcagataac tacctgcgcg cttacaacat 2220
   gagcaacggt gaaaaactgt ggcagggtcg tttaccagcg ggtggtcagg ctacgccaat 2280
   gacctatgaa gtgaatggta agcagtatgt ggtgatctcc gcaggcggtc acggttcatt 2340
   tggtacgaag atgggcgact atattgtggc ttatgcgctg ccggatgatg tgaagtaaga 2400
   cttgcgctga gcgcgc 2416
<210> 4
   <211> 796
   <212> PRT
   <213> Escherichia coli
<400> 4
   Met Ala Ile Asn Asn Thr Gly Ser Arg Arg Leu Leu Val Thr Leu Thr
   1               5                   10                  15
   Ala Leu Phe Ala Ala Leu Cys Gly Leu Tyr Leu Leu Ile Gly Gly Gly
               20                  25                  30
   Trp Leu Val Ala Ile Gly Gly Ser Trp Tyr Tyr Pro Ile Ala Gly Leu
           35                  40                  45
   Val Met Leu Gly Val Ala Trp Met Leu Trp Arg Ser Lys Arg Ala Ala
       50                  55                  60
   Leu Trp Leu Tyr Ala Ala Leu Leu Leu Gly Thr Met Ile Trp Gly Val
   65                  70                  75                  80
   Trp Glu Val Gly Phe Asp Phe Trp Ala Leu Thr Pro Arg Ser Asp Ile
                   85                  90                  95
   Leu Val Phe Phe Gly Ile Trp Leu Ile Leu Pro Phe Val Trp Arg Arg
               100                 105                 110
   Leu Val Ile Pro Ala Ser Gly Ala Val Ala Ala Leu Val Val Ala Leu
           115                 120                 125
   Leu Ile Ser Gly Gly Ile Leu Thr Trp Ala Gly Phe Asn Asp Pro Gln
       130                 135                 140
   Glu Ile Asn Gly Thr Leu Ser Ala Asp Ala Thr Pro Ala Glu Ala Ile
   145                 150                 155                 160
   Ser Pro Val Ala Asp Gln Asp Trp Pro Ala Tyr Gly Arg Asn Gln Glu
                   165                 170                 175
   Gly Gln Arg Phe Ser Pro Leu Lys Gln Ile Asn Ala Asp Asn Val His
               180                 185                 190
   Asn Leu Lys Glu Ala Trp Val Phe Arg Thr Gly Asp Val Lys Gln Pro
           195                 200                 205
   Asn Asp Pro Gly Glu Ile Thr Asn Glu Val Thr Pro Ile Lys Val Gly
       210                 215                 220
   Asp Thr Leu Tyr Leu Cys Thr Ala His Gln Arg Leu Phe Ala Leu Asp
   225                 230                 235                 240
   Ala Ala Ser Gly Lys Glu Lys Trp His Tyr Asp Pro Glu Leu Lys Thr
                   245                 250                 255
   Asn Glu Ser Phe Gln His Val Thr Cys Arg Gly Val Ser Tyr His Glu
               260                 265                 270
   Ala Lys Ala Glu Thr Ala Ser Pro Glu Val Met Ala Asp Cys Pro Arg
           275                 280                 285
   Arg Ile Ile Leu Pro Val Asn Asp Gly Arg Leu Ile Ala Ile Asn Ala
       290                 295                 300
   Glu Asn Gly Lys Leu Cys Glu Thr Phe Ala Asn Lys Gly Val Leu Asn
   305                 310                 315                 320
   Leu Gln Ser Asn Met Pro Asp Thr Lys Pro Gly Leu Tyr Glu Pro Thr
                   325                 330                 335
   Ser Pro Pro Ile Ile Thr Asp Lys Thr Ile Val Met Ala Gly Ser Val
               340                 345                 350
   Thr Asp Asn Phe Ser Thr Arg Glu Thr Ser Gly Val Ile Arg Gly Phe
           355                 360                 365
   Asp Val Asn Thr Gly Glu Leu Leu Trp Ala Phe Asp Pro Gly Ala Lys
       370                 375                 380
   Asp Pro Asn Ala Ile Pro Ser Asp Glu His Thr Phe Thr Phe Asn Ser
   385                 390                 395                 400
   Pro Asn Ser Trp Ala Pro Ala Ala Tyr Asp Ala Lys Leu Asp Leu Val
                   405                 410                 415
   Tyr Leu Pro Met Gly Val Thr Thr Pro Asp Ile Trp Gly Gly Asn Arg
               420                 425                 430
   Thr Pro Glu Gln Glu Arg Tyr Ala Ser Ser Ile Leu Ala Leu Asn Ala
           435                 440                 445
   Thr Thr Gly Lys Leu Ala Trp Ser Tyr Gln Thr Val His His Asp Leu
       450                 455                 460
   Trp Asp Met Asp Leu Pro Ala Gln Pro Thr Leu Ala Asp Ile Thr Val
   465 470 475 480
   Asn Gly Gln Lys Val Pro Val Ile Tyr Ala Pro Ala Lys Thr Gly Asn
   485 490 495
   Ile Phe Val Leu Asp Arg Arg Asn Gly Glu Leu Val Val Pro Ala Pro
   500 505 510
   Glu Lys Pro Val Pro Gln Gly Ala Ala Lys Gly Asp Tyr Val Thr Pro
   515 520 525
   Thr Gln Pro Phe Ser Glu Leu Ser Phe Arg Pro Thr Lys Asp Leu Ser
   530 535 540
   Gly Ala Asp Met Trp Gly Ala Thr Met Phe Asp Gln Leu Val Cys Arg
   545 550 555 560
   Val Met Phe His Gln Met Arg Tyr Glu Gly Ile Phe Thr Pro Pro Ser
   565 570 575
   Glu Gln Gly Thr Leu Val Phe Pro Gly Asn Leu Gly Met Phe Glu Trp
   580 585 590
   Gly Gly Ile Ser Val Asp Pro Asn Arg Glu Val Ala Ile Ala Asn Pro
   595 600 605
   Met Ala Leu Pro Phe Val Ser Lys Leu Ile Pro Arg Gly Pro Gly Asn
   610 615 620
   Pro Met Glu Gln Pro Lys Asp Ala Lys Gly Thr Gly Thr Glu Ser Gly
   625 630 635 640
   Ile Gln Pro Gln Tyr Gly Val Pro Tyr Gly Val Thr Leu Asn Pro Phe
   645 650 655
   Leu Ser Pro Phe Gly Leu Pro Cys Lys Gln Pro Ala Trp Gly Tyr Ile
   660 665 670
   Ser Ala Leu Asp Leu Lys Thr Asn Glu Val Val Trp Lys Lys Arg Ile
   675 680 685
   Gly Thr Pro Gln Asp Ser Met Pro Phe Pro Met Pro Val Pro Val Pro
   690 695 700
   Phe Asn Met Gly Met Pro Met Leu Gly Gly Pro Ile Ser Thr Ala Gly
   705 710 715 720
   Asn Val Leu Phe Ile Ala Ala Thr Ala Asp Asn Tyr Leu Arg Ala Tyr
   725 730 735
   Asn Met Ser Asn Gly Glu Lys Leu Trp Gln Gly Arg Leu Pro Ala Gly
               740                 745                 750
   Gly Gln Ala Thr Pro Met Thr Tyr Glu Val Asn Gly Lys Gln Tyr Val
           755                 760                 765
   Val Ile Ser Ala Gly Gly His Gly Ser Phe Gly Thr Lys Met Gly Asp
       770                 775                 780
   Tyr Ile Val Ala Tyr Ala Leu Pro Asp Asp Val Lys
   785                 790                 795
<210> 5
   <211> 1450
   <212> DNA
   <213> Artificial
<220>
   <223> GDH 03; Nucleotide sequence based on sequence encoding gene gdhB (originates from Acinetobacter calcoaceticus)
<400> 5
   atggacaaac acctgctggc gaaaatcgcg ctgctgtctg cggttcagct ggttaccctg 60
   tctgcgttcg cggacgttcc gctgaccccg tctcagttcg cgaaagcgaa atctgaaaac 120
   ttcgacaaaa aagttatcct gtctaacctg aacaaaccgc acgcgctgct gtggggtccg 180
   gacaaccaga tctggctgac cgaacgtgcg accggtaaaa tcctgcgtgt taacccggaa 240
   tctggttctg ttaaaaccgt tttccaggtt ccggaaatcg ttaacgacgc ggacggtcag 300
   aacggtctgc tgggtttcgc gttccacccg gacttcaaaa acaacccgta catctacatc 360
   tctggtacct tcaaaaaccc gaaatctacc gacaaagaac tgccgaacca gaccatcatc 420
   cgtcgttaca cctacaacaa atctaccgac accctggaaa aaccggttga cctgctggcg 480
   ggtctgccgt cttctaaaga ccaccagtct ggtcgtctgg ttatcggtcc ggaccagaaa 540
   atctactaca ccatcggtga ccagggtcgt aaccagctgg cgtacctgtt cctgccgaac 600
   caggcgcagc acaccccgac ccagcaggaa ctgaacggta aagactacca cacctacatg 660
   ggtaaagttc tgcgtctgaa cctggacggt tctatcccga aagacaaccc gtctttcaac 720
   ggtgttgttt ctcacatcta caccctgggt caccgtaacc cgcagggtct ggcgttcacc 780
   ccgaacggta aactgctgca gtctgaacag ggtccgaact ctgacgacga aatcaacctg 840
   atcgttaaag gtggtaacta cggttggccg aacgttgcgg gttacaaaga cgactctggt 900
   tacgcgtacg cgaactactc tgcggcggcg aacaaatcta tcaaagacct ggcgcagaac 960
   ggtgttaaag ttgcggcggg tgttccggtt accaaagaat ctgaatggac cggtaaaaac 1020
   ttcgttccgc cgctgaaaac cctgtacacc gttcaggaca cctacaacta caacgacccg 1080
   acctgcggtg aaatgaccta catctgctgg ccgaccgttg cgccgtcttc tgcgtacgtt 1140
   tacaaaggtg gtaaaaaagc gatcaccggt tgggaaaaca ccctgctggt tccgtctctg 1200
   aaacgtggtg ttatcttccg tatcaaactg gacccgacct actctaccac ctacgacgac 1260
   gcggttccga tgttcaaatc taacaaccgt taccgtgacg ttatcgcgtc tccggacggt 1320
   aacgttctgt acgttctgac cgacaccgcg ggtaacgttc agaaagacga cggttctgtt 1380
   accaacaccc tggaaaaccc gggttctctg atcaaattca cctacaaagc gaaataagct 1440
   cagcaagctt 1450
<210> 6
   <211> 478
   <212> PRT
   <213> Artificial
<220>
   <223> GDH 03; Amino acid sequence of protein based on GdhB protein (originates from Acinetobacter calcoaceticus)
<400> 6
   Met Asp Lys His Leu Leu Ala Lys Ile Ala Leu Leu Ser Ala Val Gln
   1               5                   10                  15
   Leu Val Thr Leu Ser Ala Phe Ala Asp Val Pro Leu Thr Pro Ser Gln
               20                  25                  30
   Phe Ala Lys Ala Lys Ser Glu Asn Phe Asp Lys Lys Val Ile Leu Ser
           35                  40                  45
   Asn Leu Asn Lys Pro His Ala Leu Leu Trp Gly Pro Asp Asn Gln Ile
       50                  55                  60
   Trp Leu Thr Glu Arg Ala Thr Gly Lys Ile Leu Arg Val Asn Pro Glu
   65                  70                  75                  80
   Ser Gly Ser Val Lys Thr Val Phe Gln Val Pro Glu Ile Val Asn Asp
                   85                  90                  95
   Ala Asp Gly Gln Asn Gly Leu Leu Gly Phe Ala Phe His Pro Asp Phe
               100                 105                 110
   Lys Asn Asn Pro Tyr Ile Tyr Ile Ser Gly Thr Phe Lys Asn Pro Lys
           115                 120                 125
   Ser Thr Asp Lys Glu Leu Pro Asn Gln Thr Ile Ile Arg Arg Tyr Thr
       130                 135                 140
   Tyr Asn Lys Ser Thr Asp Thr Leu Glu Lys Pro Val Asp Leu Leu Ala
   145                 150                 155                 160
   Gly Leu Pro Ser Ser Lys Asp His Gln Ser Gly Arg Leu Val Ile Gly
                   165                 170                 175
   Pro Asp Gln Lys Ile Tyr Tyr Thr Ile Gly Asp Gln Gly Arg Asn Gln
               180                 185                 190
   Leu Ala Tyr Leu Phe Leu Pro Asn Gln Ala Gln His Thr Pro Thr Gln
           195                 200                 205
   Gln Glu Leu Asn Gly Lys Asp Tyr His Thr Tyr Met Gly Lys Val Leu
       210                 215                 220
   Arg Leu Asn Leu Asp Gly Ser Ile Pro Lys Asp Asn Pro Ser Phe Asn
   225                 230                 235                 240
   Gly Val Val Ser His Ile Tyr Thr Leu Gly His Arg Asn Pro Gln Gly
                   245                 250                 255
   Leu Ala Phe Thr Pro Asn Gly Lys Leu Leu Gln Ser Glu Gln Gly Pro
               260                 265                 270
   Asn Ser Asp Asp Glu Ile Asn Leu Ile Val Lys Gly Gly Asn Tyr Gly
           275                 280                 285
   Trp Pro Asn Val Ala Gly Tyr Lys Asp Asp Ser Gly Tyr Ala Tyr Ala
       290                 295                 300
   Asn Tyr Ser Ala Ala Ala Asn Lys Ser Ile Lys Asp Leu Ala Gln Asn
   305                 310                 315                 320
   Gly Val Lys Val Ala Ala Gly Val Pro Val Thr Lys Glu Ser Glu Trp
                   325                 330                 335
   Thr Gly Lys Asn Phe Val Pro Pro Leu Lys Thr Leu Tyr Thr Val Gln
               340                 345                 350
   Asp Thr Tyr Asn Tyr Asn Asp Pro Thr Cys Gly Glu Met Thr Tyr Ile
           355                 360                 365
   Cys Trp Pro Thr Val Ala Pro Ser Ser Ala Tyr Val Tyr Lys Gly Gly
       370                 375                 380
   Lys Lys Ala Ile Thr Gly Trp Glu Asn Thr Leu Leu Val Pro Ser Leu
   385                 390                 395                 400
   Lys Arg Gly Val Ile Phe Arg Ile Lys Leu Asp Pro Thr Tyr Ser Thr
                   405                 410                 415
   Thr Tyr Asp Asp Ala Val Pro Met Phe Lys Ser Asn Asn Arg Tyr Arg
   420 425 430
   Asp Val Ile Ala Ser Pro Asp Gly Asn Val Leu Tyr Val Leu Thr Asp
   435 440 445
   Thr Ala Gly Asn Val Gln Lys Asp Asp Gly Ser Val Thr Asn Thr Leu
   450 455 460
   Glu Asn Pro Gly Ser Leu Ile Lys Phe Thr Tyr Lys Ala Lys
   465 470 475
<210> 7
   <211> 1450
   <212> DNA
   <213> Artificial
<220>
   <223> GDH 04; Nucleotide sequence based on sequence encoding gene gdhB originating from Acinetobacter calcoaceticus.
<400> 7
   atggacaaac acctgctggc gaaaatcgcg ctgctgtctg cggttcagct ggttaccctg 60
   tctgcgttcg cggacgttcc gctgaccccg tctcagttcg cgaaagcgaa atctgaaaac 120
   ttcgacaaaa aagttatcct gtctaacctg aacaaaccgc acgcgctgct gtggggtccg 180
   gacaaccaga tctggctgac cgaacgtgcg accggtaaaa tcctgcgtgt taacccggaa 240
   tctggttctg ttaaaaccgt tttccaggtt ccggaaatcg ttaacgacgc ggacggtcag 300
   aacggtctgc tgggtttcgc gttccacccg gacttcaaaa acaacccgta catctacatc 360
   tctggtacct tcaaaaaccc gaaatctacc gacaaagaac tgccgaacca gaccatcatc 420
   cgtcgttaca cctacaacaa atctaccgac accctggaaa aaccggttga cctgctggcg 480
   ggtctgccgt cttctaaaga ccaccagtct ggtcgtctgg ttatcggtcc ggaccagaaa 540
   atctactaca ccatcggtga ccagggtcgt aaccagctgg cgtacctgtt cctgccgaac 600
   caggcgcagc acaccccgac ccagcaggaa ctgaacggta aagactacca cacctacatg 660
   ggtaaagttc tgcgtctgaa cctggacggt aaaatcccga aagacaaccc gtctttcaac 720
   ggtgttgttt ctcacatcta caccctgggt caccgtaacc cgcagggtct ggcgttcacc 780
   ccgaacggta aactgctgca gtctgaacag ggtccgaact ctgacgacga aatcaacctg 840
   atcgttaaag gtggtaacta cggttggccg aacgttgcgg gttacaaaga cgactctggt 900
   tacgcgtacg cgaactactc tgcggcggcg aacaaatcta tcaaagacct ggcgcagaac 960
   ggtgttaaag ttgcggcggg tgttccggtt accaaagaat ctgaatggac cggtaaaaac 1020
   ttcgttccgc cgctgaaaac cctgtacacc gttcaggaca cctacaacta caacgacccg 1080
   acctgcggtg aaatgaccta catctgctgg ccgaccgttg cgccgtcttc tgcgtacgtt 1140
   tacaaaggtg gtaaaaaagc gatcaccggt tgggaaaaca ccctgctggt tccgtctctg 1200
   aaacgtggtg ttatcttccg tatcaaactg gacccgacct actctaccac ctacgacgac 1260
   gcggttccga tgttcaaatc taacaaccgt taccgtgacg ttatcgcgtc tccggacggt 1320
   aacgttctgt acgttctgac cgacaccgcg ggtaacgttc agaaagacga cggttctgtt 1380
   accaacaccc tggaaaaccc gggttctctg atcaaattca cctacaaagc gaaataagct 1440
   cagcaagctt 1450
<210> 8
   <211> 478
   <212> PRT
   <213> Artificial
<220>
   <223> GDH 04; Amino acid sequence of protein based on GdhB protein (originates from Acinetobacter calcoaceticus)
<400> 8
   Met Asp Lys His Leu Leu Ala Lys Ile Ala Leu Leu Ser Ala Val Gln
   1               5                   10                  15
   Leu Val Thr Leu Ser Ala Phe Ala Asp Val Pro Leu Thr Pro Ser Gln
               20                  25                  30
   Phe Ala Lys Ala Lys Ser Glu Asn Phe Asp Lys Lys Val Ile Leu Ser
           35                  40                  45
   Asn Leu Asn Lys Pro His Ala Leu Leu Trp Gly Pro Asp Asn Gln Ile
       50                  55                  60
   Trp Leu Thr Glu Arg Ala Thr Gly Lys Ile Leu Arg Val Asn Pro Glu
   65                  70                  75                  80
   Ser Gly Ser Val Lys Thr Val Phe Gln Val Pro Glu Ile Val Asn Asp
                   85                  90                  95
   Ala Asp Gly Gln Asn Gly Leu Leu Gly Phe Ala Phe His Pro Asp Phe
               100                 105                 110
   Lys Asn Asn Pro Tyr Ile Tyr Ile Ser Gly Thr Phe Lys Asn Pro Lys
           115                 120                 125
   Ser Thr Asp Lys Glu Leu Pro Asn Gln Thr Ile Ile Arg Arg Tyr Thr
       130                 135                 140
   Tyr Asn Lys Ser Thr Asp Thr Leu Glu Lys Pro Val Asp Leu Leu Ala
   145                 150                 155                 160
   Gly Leu Pro Ser Ser Lys Asp His Gln Ser Gly Arg Leu Val Ile Gly
                   165                 170                 175
   Pro Asp Gln Lys Ile Tyr Tyr Thr Ile Gly Asp Gln Gly Arg Asn Gln
               180                 185                 190
   Leu Ala Tyr Leu Phe Leu Pro Asn Gln Ala Gln His Thr Pro Thr Gln
           195                 200                 205
   Gln Glu Leu Asn Gly Lys Asp Tyr His Thr Tyr Met Gly Lys Val Leu
       210                 215                 220
   Arg Leu Asn Leu Asp Gly Lys Ile Pro Lys Asp Asn Pro Ser Phe Asn
   225                 230                 235                 240
   Gly Val Val Ser His Ile Tyr Thr Leu Gly His Arg Asn Pro Gln Gly
                   245                 250                 255
   Leu Ala Phe Thr Pro Asn Gly Lys Leu Leu Gln Ser Glu Gln Gly Pro
               260                 265                 270
   Asn Ser Asp Asp Glu Ile Asn Leu Ile Val Lys Gly Gly Asn Tyr Gly
           275                 280                 285
   Trp Pro Asn Val Ala Gly Tyr Lys Asp Asp Ser Gly Tyr Ala Tyr Ala
       290                 295                 300
   Asn Tyr Ser Ala Ala Ala Asn Lys Ser Ile Lys Asp Leu Ala Gln Asn
   305                 310                 315                 320
   Gly Val Lys Val Ala Ala Gly Val Pro Val Thr Lys Glu Ser Glu Trp
                   325                 330                 335
   Thr Gly Lys Asn Phe Val Pro Pro Leu Lys Thr Leu Tyr Thr Val Gln
               340                 345                 350
   Asp Thr Tyr Asn Tyr Asn Asp Pro Thr Cys Gly Glu Met Thr Tyr Ile
           355                 360                 365
   Cys Trp Pro Thr Val Ala Pro Ser Ser Ala Tyr Val Tyr Lys Gly Gly
       370                 375                 380
   Lys Lys Ala Ile Thr Gly Trp Glu Asn Thr Leu Leu Val Pro Ser Leu
   385                 390                 395                 400
   Lys Arg Gly Val Ile Phe Arg Ile Lys Leu Asp Pro Thr Tyr Ser Thr
                   405                 410                 415
   Thr Tyr Asp Asp Ala Val Pro Met Phe Lys Ser Asn Asn Arg Tyr Arg
               420                 425                 430
   Asp Val Ile Ala Ser Pro Asp Gly Asn Val Leu Tyr Val Leu Thr Asp
   435 440 445
   Thr Ala Gly Asn Val Gln Lys Asp Asp Gly Ser Val Thr Asn Thr Leu
   450 455 460
   Glu Asn Pro Gly Ser Leu Ile Lys Phe Thr Tyr Lys Ala Lys
   465 470 475
<210> 9
   <211> 799
   <212> DNA
   <213> Artificial
<220>
   <223> GDH 05; Nucleotide sequence based on gene deoC (originates from Escherichia coli)
<400> 9
   ccggcatatg actgatctga aagcaagcag cctgcgtgca ctgaaattga tggacctgac      60
   caccctgaat gacgacgaca ccgacgagaa agtgatcgcc ctgtgtcatc aggccaaaac    120
   tccggtcggc aataccgccg ctatctgtat ctatcctcgc tttatcccga ttgctcgcaa     180
   aactctgaaa gagcagggca ccccggaaat ccgtatcgct acggtaacca acttcccaca     240
   cggtaacgac gacatcgaca tcgcgctggc agaaacccgt gcggcaatcg cctacggtgc     300
   tgatgaagtt gacgttgtgt tcccgtaccg cgcgctgatg gcgggtaacg agcaggttgg     360
   ttttgacctg gtgaaagcct gtaaagaggc ttgcgcggca gcgaatgtac tgctgaaagt     420
   gatcatcgaa accggcgaac tgaaagacga agcgctgatc cgtaaagcgt ctgaaatctc     480
   catcaaagcg ggtgcggact tcatcaaaac ctctaccggt aaagtggctg tgaacgcgac     540
   gccggaaagc gcgcgcatca tgatggaagt gatccgtgat atgggcgtag aaaaaaccgt     600
   tggtttcaaa ccggcgggcg gcgtgcgtac tgcggaagat gcgcagaaat atctcgccat     660
   tgcagatgaa ctgttcggtg ctgactgggc agatgcgcgt cactaccgct ttggcgcttc     720
   cagcctgctg gcaagcctgc tgaaagcgct gggtcacggc gacggtaaga gcgccagcag     780
   ctactaatga gctgagcgg                                                   799
<210> 10
   <211> 259
   <212> PRT
   <213> Escherichia coli
<400> 10
   Met Thr Asp Leu Lys Ala Ser Ser Leu Arg Ala Leu Lys Leu Met Asp
   1               5                   10                  15
   Leu Thr Thr Leu Asn Asp Asp Asp Thr Asp Glu Lys Val Ile Ala Leu
               20                  25                  30
   Cys His Gln Ala Lys Thr Pro Val Gly Asn Thr Ala Ala Ile Cys Ile
   35 40 45
   Tyr Pro Arg Phe Ile Pro Ile Ala Arg Lys Thr Leu Lys Glu Gln Gly
   50 55 60
   Thr Pro Glu Ile Arg Ile Ala Thr Val Thr Asn Phe Pro His Gly Asn
   65 70 75 80
   Asp Asp Ile Asp Ile Ala Leu Ala Glu Thr Arg Ala Ala Ile Ala Tyr
   85 90 95
   Gly Ala Asp Glu Val Asp Val Val Phe Pro Tyr Arg Ala Leu Met Ala
   100 105 110
   Gly Asn Glu Gln Val Gly Phe Asp Leu Val Lys Ala Cys Lys Glu Ala
   115 120 125
   Cys Ala Ala Ala Asn Val Leu Leu Lys Val Ile Ile Glu Thr Gly Glu
   130 135 140
   Leu Lys Asp Glu Ala Leu Ile Arg Lys Ala Ser Glu Ile Ser Ile Lys
   145 150 155 160
   Ala Gly Ala Asp Phe Ile Lys Thr Ser Thr Gly Lys Val Ala Val Asn
   165 170 175
   Ala Thr Pro Glu Ser Ala Arg Ile Met Met Glu Val Ile Arg Asp Met
   180 185 190
   Gly Val Glu Lys Thr Val Gly Phe Lys Pro Ala Gly Gly Val Arg Thr
   195 200 205
   Ala Glu Asp Ala Gln Lys Tyr Leu Ala Ile Ala Asp Glu Leu Phe Gly
   210 215 220
   Ala Asp Trp Ala Asp Ala Arg His Tyr Arg Phe Gly Ala Ser Ser Leu
   225 230 235 240
   Leu Ala Ser Leu Leu Lys Ala Leu Gly His Gly Asp Gly Lys Ser Ala
   245 250 255
   Ser Ser Tyr
<210> 11
   <211> 3362
   <212> DNA
   <213> Artificial
<220>
   <223> Nucleotide sequence based on the pqqABCDE operon (originates from Gluconobacter oxydans)
<400> 11
   cgcgggtacc tcgcatctgc cggttatcgg agaccaagga gaaaaatcat ggcctggaac 60
   acaccgaaag ttaccgaaat cccgctgggc gcagaaatca actcgtatgt ctgcggcgag 120
   aagaaataag ccgctttccc ggggacccgt ccttgaggaa taatggcacg gccgctcccc 180
   catggagcgg ccgttttcgt tcatgggtgc tctgtggtgc cccagtcaga cggtttgtga 240
   aaaaatgatt gatgtcatcg tgcttggcgc ggcggcaggg ggcggttttc cgcagtggaa 300
   ctccgcagca cccggctgtg tggccgcccg cacgcgacag ggcgcgaaag cccggaccca 360
   ggcctccctt gccgtcagtg ccgacggaaa gcgctggttc attctcaacg cctcgcccga 420
   tctgcggcag cagatcatcg atacgccggc cctgcatcat cagggcagcc tgcgtggaac 480
   gcccattcag ggcgtcgtcc tgacctgcgg cgagatcgac gccataaccg ggcttctgac 540
   cctgcgtgag cgtgagcctt ttaccctgat gggcagcgac tcgacccttc agcagcttgc 600
   ggacaatccg atcttcggtg cgctcgatcc ggaaatcgtc ccacgtgttc cgctcattct 660
   cgatgaagcc acgtccctga tgaacaagga cgggattccg tccggtcttt tgctcacggc 720
   cttcgccgtt ccgggcaagg cgccgcttta cgcggaagcc gcagggtcac gcccggacga 780
   gacgctgggc ctttccatta cggatggatg caagacgatg ctcttcattc ccggctgtgc 840
   gcagatcacg tcggaaatcg tggaacgggt agcggcagcc gatctcgtgt tctttgacgg 900
   gacactgtgg cgggatgacg aaatgatccg cgccgggttg agcccgaaga gcggacagcg 960
   gatgggacat gtgtccgtga atgatgccgg gggaccggtc gaatgtttca cgacatgcga 1020
   aaaaccccgt aaagtgttga ttcatatcaa caactccaat ccaattctgt tcgaagacag 1080
   ccccgaacgc aaagacgtcg aacgcgccgg atggacggtt gcggaagacg gcatgacttt 1140
   cagactggac acaccatgac gctcctcaca cctgaccagc ttgaagcaca gcttcgccag 1200
   atcggggccg agcggtatca caaccggcac ccgttccatc gcaagctgca tgacggcaag 1260
   ctggacaagg cacaggttca ggcttgggcg ctgaaccgct attattatca ggcccgcatc 1320
   ccggcgaagg atgcgacgct tctcgcacgt ctgccgacgg ccgaactgcg ccgcgaatgg 1380
   cgtcgccgga tcgaggacca tgacggcacg gagcccggaa cgggcggtgt tgcgcgctgg 1440
   ctgatgctga cggatggtct ggggctggac cgggattatg tggaaagcct cgatggtctg 1500
   cttccagcca cgcgcttctc ggtcgatgcc tatgtgaact tcgtgcggga ccagtcgatt 1560
   ctggcggcca ttgcgtcgtc gctgacggaa ctgttttcgc ccacgatcat cagcgagcgc 1620
   gtctcgggga tgctgcggca ctacgacttt gtgtcggaaa agacgctggc ctatttcacg 1680
   ccgcgcctga cgcaggcccc gcgggattcc gatttcgcgc tggcctatgt ccgcgaaaag 1740
   gcccgcacgc cggagcagca gaaagaagtc ctgggagcgc tggagttcaa gtgctccgtg 1800
   ctgtggacga tgctggatgc gctcgactac gcctatgtgg aaggccacat tccgccgggg 1860
   gctttcgttc catgacggag gccccgcatg tcgtggcgga ggggacggtt ctctcctttg 1920
   cccgggggca tcgtctccag cacgatcgtg tgcgggacgt gtggatcgtg caggcgcctg 1980
   aaaaagcatt tgtagttgag ggcgccgcgc cgcatattct gcggctgctg gatgggaagc 2040
   gcagcgtcgg cgagatcatc cagcagcttg caatcgagtt ttccgccccg cgtgaggtca 2100
   ttgcgaaaga tgtcctcgcg cttctttctg aactgacaga aaagaacgtc ctgcacacat 2160
   gacactccct tcgccgccga tgagccttct ggctgaactg acgcatcgat gcccgctttc 2220
   ctgcccctac tgctccaatc cgcttgaact cgaacgcaag gcggcagaac tcgacacggc 2280
   cacctggact gccgtactgg agcaggcggc cgagcttggg gtgctccagg ttcatttctc 2340
   tggcggcgag cctatggcgc ggcctgatct ggtcgaactg gtctccgtcg cacggagact 2400
   caacctgtat tccaacttga tcacgtccgg cgtgttgctg gacgaaccga aactggaagc 2460
   tctcgacagg gcggggctgg atcacatcca gctctctttc caagacgtga cggaggcggg 2520
   agccgagcgt atcggcggtc tcaagggagc gcaggcccgc aaggttgcgg cggcgcggct 2580
   catccgcgcg tccggcattc cgatgacgct caattttgtg gtgcacaggg aaaatgtcgc 2640
   ccgtatcccc gagatgttcg ccctggcgcg ggaactcgga gcggggcggg tggagatcgc 2700
   gcatacccag tattatggct gggggctgaa aaaccgtgag gcgcttcttc ccagccggga 2760
   tcagctggag gaatccacac gcgccgtgga agcggagcgc gctaagggtg gtttgtccgt 2820
   tgattatgtg acgccggact atcatgcaga ccggcccaag ccctgcatgg ggggatgggg 2880
   ccagcgtttc gtgaatgtca caccttcggg ccgggtcctg ccgtgtcatg cagccgaaat 2940
   cattccggat gtcgcattcc cgaatgtgca ggatgtgacc ctgtccgaaa tctggaacat 3000
   ctcaccgctg ttcaacatgt tccgcgggac ggactggatg ccggagccct gccgctcctg 3060
   cgagcgcaag gagcgtgact ggggcgggtg tcgctgtcag gcgatggcgc tgacggggaa 3120
   tgccgcgaat accgatcccg tatgcagtct ctccccctat cacgatcggg tggagcaggc 3180
   cgtcgagaac aacatgcagc cagaaagcac gttgttctac aggcgttata cgtaatcgcc 3240
   gtaaatattg actttgctaa gatggagaaa ggatgtgccg ttcaggacaa tcttgatata 3300
   tcgagcaata atatctgttt ctatctgaag aggttctcca aactctccgc ccggatccgc 3360
   gc 3362
<210> 12
   <211> 26
   <212> PRT
   <213> Gluconobacter oxydans
<400> 12
   Met Ala Trp Asn Thr Pro Lys Val Thr Glu Ile Pro Leu Gly Ala Glu
   1 5 10 15
   Ile Asn Ser Tyr Val Cys Gly Glu Lys Lys
   20 25
<210> 13
   <211> 302
   <212> PRT
   <213> Gluconobacter oxydans
<400> 13
   Met Ile Asp Val Ile Val Leu Gly Ala Ala Ala Gly Gly Gly Phe Pro
   1               5                   10                  15
   Gln Trp Asn Ser Ala Ala Pro Gly Cys Val Ala Ala Arg Thr Arg Gln
               20                  25                  30
   Gly Ala Lys Ala Arg Thr Gln Ala Ser Leu Ala Val Ser Ala Asp Gly
           35                  40                  45
   Lys Arg Trp Phe Ile Leu Asn Ala Ser Pro Asp Leu Arg Gln Gln Ile
       50                  55                  60
   Ile Asp Thr Pro His His Gln Gly Ser Leu Arg Gly Thr Pro Ile Gln
   65                  70                  75                  80
   Gly Val Val Leu Thr Cys Gly Glu Ile Asp Ala Ile Thr Gly Leu Leu
                   85                  90                  95
   Thr Leu Arg Glu Arg Glu Pro Phe Thr Leu Met Gly Ser Asp Ser Thr
               100                 105                 110
   Leu Gln Gln Leu Ala Asp Asn Pro Ile Phe Gly Ala Leu Asp Pro Glu
           115                 120                 125
   Ile Val Pro Arg Val Pro Leu Ile Leu Asp Glu Ala Thr Ser Leu Met
       130                 135                 140
   Asn Lys Asp Gly Ile Pro Ser Gly Leu Leu Leu Thr Ala Phe Ala Val
   145                 150                 155                 160
   Pro Gly Lys Ala Pro Leu Tyr Ala Glu Ala Ala Gly Ser Arg Pro Asp
                   165                 170                 175
   Glu Thr Leu Gly Leu Ser Ile Thr Asp Gly Cys Lys Thr Met Leu Phe
               180                 185                 190
   Ile Pro Gly Cys Ala Gln Ile Thr Ser Glu Ile Val Glu Arg Val Ala
           195                 200                 205
   Ala Ala Asp Leu Val Phe Phe Asp Gly Thr Leu Trp Arg Asp Asp Glu
   210 215 220
   Met Ile Arg Ala Gly Leu Ser Pro Lys Ser Gly Gln Arg Met Gly His
   225 230 235 240
   Val Ser Val Asn Asp Ala Gly Gly Pro Val Glu Cys Phe Thr Thr Cys
   245 250 255
   Glu Lys Pro Arg Lys Val Leu Ile His Ile Asn Asn Ser Asn Pro Ile
   260 265 270
   Leu Phe Glu Asp Ser Pro Glu Arg Lys Asp Val Glu Arg Ala Gly Trp
   275 280 285
   Thr Val Ala Glu Asp Gly Met Thr Phe Arg Leu Asp Thr Pro
   290 295 300
<210> 14
   <211> 239
   <212> PRT
   <213> Gluconobacter oxydans
<400> 14
   Met Thr Leu Leu Thr Pro Asp Gln Leu Glu Ala Gln Leu Arg Gln Ile
   1               5                   10                  15
   Gly Ala Glu Arg Tyr His Asn Arg His Pro Phe His Arg Lys Leu His
               20                  25                  30
   Asp Gly Lys Leu Asp Lys Ala Gln Val Gln Ala Trp Ala Leu Asn Arg
           35                  40                  45
   Tyr Tyr Tyr Gln Ala Arg Ile Pro Ala Lys Asp Ala Thr Leu Leu Ala
       50                  55                  60
   Arg Leu Pro Thr Ala Glu Leu Arg Arg Glu Trp Arg Arg Arg Ile Glu
   65                  70                  75                  80
   Asp His Asp Gly Thr Glu Pro Gly Thr Gly Gly Val Ala Arg Trp Leu
                   85                  90                  95
   Met Leu Thr Asp Gly Leu Gly Leu Asp Arg Asp Tyr Val Glu Ser Leu
               100                 105                 110
   Asp Gly Leu Leu Pro Ala Thr Arg Phe Ser Val Asp Ala Tyr Val Asn
           115                 120                 125
   Phe Val Arg Asp Gln Ser Ile Leu Ala Ala Ile Ala Ser Ser Leu Thr
       130                 135                 140
   Glu Leu Phe Ser Pro Thr Ile Ile Ser Glu Arg Val Ser Gly Met Leu
   145                 150                 155                 160
   Arg His Tyr Asp Phe Val Ser Glu Lys Thr Leu Ala Tyr Phe Thr Pro
                   165                 170                 175
   Arg Leu Thr Gln Ala Pro Arg Asp Ser Asp Phe Ala Leu Ala Tyr Val
               180                 185                 190
   Arg Glu Lys Ala Arg Thr Pro Glu Gln Gln Lys Glu Val Leu Gly Ala
           195                 200                 205
   Leu Glu Phe Lys Cys Ser Val Leu Trp Thr Met Leu Asp Ala Leu Asp
       210                 215                 220
   Tyr Ala Tyr Val Glu Gly His Ile Pro Pro Gly Ala Phe Val Pro
   225                 230                 235
<210> 15
   <211> 96
   <212> PRT
   <213> Gluconobacter oxydans
<400> 15
   Met Thr Glu Ala Pro His Val Val Ala Glu Gly Thr Val Leu Ser Phe
   1 5 10 15
   Ala Arg Gly His Arg Leu Gln His Asp Arg Val Arg Asp Val Trp Ile
   20 25 30
   Val Gln Ala Pro Glu Lys Ala Phe Val Val Glu Gly Ala Ala Pro His
   35 40 45
   Ile Leu Arg Leu Leu Asp Gly Lys Arg Ser Val Gly Glu Ile Ile Gln
   50 55 60
   Gln Leu Ala Ile Glu Phe Ser Ala Pro Arg Glu Val Ile Ala Lys Asp
   65 70 75 80
   Val Leu Ala Leu Leu Ser Glu Leu Thr Glu Lys Asn Val Leu His Thr
   85 90 95
<210> 16
   <211> 358
   <212> PRT
   <213> Gluconobacter oxydans
<400> 16
   Met Thr Leu Pro Ser Pro Pro Met Ser Leu Leu Ala Glu Leu Thr His
   1               5                   10                  15
   Arg Cys Pro Leu Ser Cys Pro Tyr Cys Ser Asn Pro Leu Glu Leu Glu
               20                  25                  30
   Arg Lys Ala Ala Glu Leu Asp Thr Ala Thr Trp Thr Ala Val Leu Glu
           35                  40                  45
   Gln Ala Ala Glu Leu Gly Val Leu Gln Val His Phe Ser Gly Gly Glu
       50                  55                  60
   Pro Met Ala Arg Pro Asp Leu Val Glu Leu Val Ser Val Ala Arg Arg
   65                  70                  75                  80
   Leu Asn Leu Tyr Ser Asn Leu Ile Thr Ser Gly Val Leu Leu Asp Glu
                   85                  90                  95
   Pro Lys Leu Glu Ala Leu Asp Arg Ala Gly Leu Asp His Ile Gln Leu
               100                 105                 110
   Ser Phe Gln Asp Val Thr Glu Ala Gly Ala Glu Arg Ile Gly Gly Leu
           115                 120                 125
   Lys Gly Ala Gln Ala Arg Lys Val Ala Ala Ala Arg Leu Ile Arg Ala
       130                 135                 140
   Ser Gly Ile Pro Met Thr Leu Asn Phe Val Val His Arg Glu Asn Val
   145                 150                 155                 160
   Ala Arg Ile Pro Glu Met Phe Ala Leu Ala Arg Glu Leu Gly Ala Gly
                   165                 170                 175
   Arg Val Glu Ile Ala His Thr Gln Tyr Tyr Gly Trp Gly Leu Lys Asn
               180                 185                 190
   Arg Glu Ala Leu Leu Pro Ser Arg Asp Gln Leu Glu Glu Ser Thr Arg
           195                 200                 205
   Ala Val Glu Ala Glu Arg Ala Lys Gly Gly Leu Ser Val Asp Tyr Val
       210                 215                 220
   Thr Pro Asp Tyr His Ala Asp Arg Pro Lys Pro Cys Met Gly Gly Trp
   225                 230                 235                 240
   Gly Gln Arg Phe Val Asn Val Thr Pro Ser Gly Arg Val Leu Pro Cys
                   245                 250                 255
   His Ala Ala Glu Ile Ile Pro Asp Val Ala Phe Pro Asn Val Gln Asp
               260                 265                 270
   Val Thr Leu Ser Glu Ile Trp Asn Ile Ser Pro Leu Phe Asn Met Phe
   275 280 285
   Arg Gly Thr Asp Trp Met Pro Glu Pro Cys Arg Ser Cys Glu Arg Lys
   290 295 300
   Glu Arg Asp Trp Gly Gly Cys Arg Cys Gln Ala Met Ala Leu Thr Gly
   305 310 315 320
   Asn Ala Ala Asn Thr Asp Pro Val Cys Ser Leu Ser Pro Tyr His Asp
   325 330 335
   Arg Val Glu Gln Ala Val Glu Asn Asn Met Gln Pro Glu Ser Thr Leu
   340 345 350
   Phe Tyr Arg Arg Tyr Thr
   355
<210> 17
   <211> 4279
   <212> DNA
   <213> Artificial
<220>
   <223> Nucleotide sequence based on the pqqABCDE operon (originates from Kluyvera intermedia)
<400> 17
   gcgcggtacc aagcttcgct gccgcaaaac agcagatgtt ccagcagatg tgccaaaccc 60
   ggccagcgat ccgcttcatc cagactgccg gcctgcacgc gcatcagcgc cgccgcctca 120
   cgcgcgtcgg gctgatgata aagatggcag cgcaggccgt tggcgagcgt gagctggcgc 180
   gcgtgcatca ggtgacgcgc tgcttaaaag atcagttcgg agttgctgcg attgcggaac 240
   tgcagctgct ggattttgat gtcgctgcag ttggcttcac gtcgtgcttc gaggatcttg 300
   ccgtgatgcg gcgatttgct gcacaccgga tcggtgttat cggcatcgcc ggtcagcata 360
   aaggcctgac agcgacagcc gccaaagtct ttcgcttttt catcgcagga gcgacagggt 420
   tccggcatcc agtcaaagcc gcgatagcgg ttaaaaccaa acgagttgta ccagatatcg 480
   tccagcgtgc gctccagcac cgacgggaac gcaaccggca gctgacgcgc gctgtggcac 540
   ggcaacgcgg tgcagtcggc gtgacgctga ggaagatcga tccccatccg cccatgcagg 600
   gtttcggccg ctcttcgtag taatccggcg tcacgaacag caggattggt gagattgccg 660
   ctggcgccca tgcgctgacg ataatcggcc accaccgctt cggcgttggc gatctgctcg 720
   cgcgtcggca gcaatccttc acgattgagt tgcgcccagc catagaattg gcaagtcgcc 780
   agctcgacat cgtcggcatc cagttcgata cagagatcga tgattttgtc gatctggtcg 840
   atattgtggc gatgcagcac gaaattcagc accatcggat agccgtgcgc tttcaccgct 900
   ttcgccatct ccagcttctg ctggaaggcc ttttttgacc cggccagcgc cgcgttcagc 960
   gtttcatcgc tggcctggaa gctgatctgg atatgatcga gtccggcgtc ggcaaaggcg 1020
   tcgagttttt tcgccgtcag gccgatgccg gaggtgatca ggttggtgta gaaaccgaga 1080
   tcgcgcgcgg cgcgaatcaa ctccggcagg tctttacggg ttagcggttc gccgccggaa 1140
   aaaccgagct gcacgctgcc catggcgcgc gcctgacgaa acacctcaat ccactgttcg 1200
   gtggtcagct ctttcttctg ctgggaaaaa tccagcggat tggagcaata tggacactgc 1260
   agcgggcagc gataggtcag ttctgccagc agccacagcg gtggcgtcac gcttttactc 1320
   gggttcacga cactgtatcc atttttgttc aatggcggat tgcaggaact ctttgacgtc 1380
   gtcgccgaca ccgcccgcgt ccgggaaacg cgcatccagc gtggcaacga tcgccgccac 1440
   atcctgtttg ccatccacca gttcgaggat cgccacggcg gtctcgttca gtttggccat 1500
   gccttctgga tagagcacca catggctatc ctgcgccgct tcccactgca tgcggtagcc 1560
   gcgacggaaa gccggaatca cattatcttt catgttgtta taccagtctt gtgctgtgcc 1620
   aggacacctg atcggtgacc gttatctttc atgttgttat accagtcttg tgctgtgcca 1680
   ggacacctga tcggtgaccg tatgataagg cggacgttgc agcgcatacg ccatggtcat 1740
   ggcatcgagc atggtccaga gaatatcgag tttaaactgc aggatctcca gcatgcggtt 1800
   ctgctgttcg gcgcgggtaa acacctcaag cgccagcgcc agcccatgct ccacatcgcg 1860
   gttcgcctgg ctcaaacggc tgcggaagta gaaatagcct tcctctttga tccacggata 1920
   gtgctgcggc cagctgtcga gacgcgactg atgaatctgc ggcgcaaaca gctcggtgag 1980
   cgagctacag gccgcttcct gccagttagc acggcgcgcg aagttcacgt aggcgtccac 2040
   cgcaaaacgc acgcccggca gcaccagctt ctctgacagc aacacgtcac gttccagccc 2100
   caccgcctca cccaactgca gccaggcttc aatcccgcct tcgtgaccgt tgctgccgtc 2160
   gtgatcgagg atgcgctgca cccatttacg ccgcgtcgcc gcatccgggc aattcgccat 2220
   gatcgccgca tctttcagcg gaatgttggt ctgataatag aagcggttcg ccacccagcc 2280
   ctgaatctgc tcgcgcgtcg cctcgccatt gtgcatggca atgtgatacg ggtgatgtat 2340
   gtggtagaac gcgcctttat cgcgcagcgc ctgctcaaac gcggcgggcg ataatgcttc 2400
   agtgatgatc atgatgcaaa ccgcgcagcg cctgctcaaa cgcggcgggc gataatgctt 2460
   cagtgatgat catgatgcaa actcctgcag atgaatcgcc atgccatccc aactcacttc 2520
   aatgccctgc tgcgtcaaat aggcgcgctg cggggattgc tcattaagga tcggattggt 2580
   gttgttaatg tgaatcaaaa tcttgcgttt ggccggcagc gcagccagca acgccattaa 2640
   accttgttct tcggccagcg ctaagtggcc catcgctttg ccagtatttt tgccaacgcc 2700
   ggtggcgagc agctcgtcgt cctgccacac cgtgccatca ataacaggca atcggctctt 2760
   tgcagccacg gcatggatca ccgcatccgg ctcgccgagt cccggcgcgt agagcagcgt 2820
   ttggccattg ccggtggttt catgaacagc gccacgttgt ggtccggcag ggcgatcgcg 2880
   atacggcgaa tacggcggcg cgttgctgag aatcggaatc gcggtgaact gcaaactgtg 2940
   gcagacatcg acgcggaacg gctccagcgg cgttaacgca tgatgctgca agccgccatt 3000
   ccagtgctgc aacatggtga aaatcgggaa accgctggtg agatcgccat gcacttccgg 3060
   cgtgcaccac acctgatgcg gacagccttc gcgcaggctc aacaagccag tggtgtgatc 3120
   gatttggctg tcggtgagaa tgatgctgcc aatcgccgtg ccgcgcagca cacccttttt 3180
   gatcagttct ggcgtgtggg caatttgctg gctgatgtcc ggcgaggcgt tgcacagcac 3240
   ccaatcttca ccgttgtcgc tgacaatgat tgaagattgg gtgcgcgcct gcgcctgaat 3300
   actgccgtta cgcaggccgc tgcaattggc gcagttacag ttccactgcg ggaagccgcc 3360
   gcccgccgct gaaccgagga ctttaataaa catgaacgtt gaccaaagag agaaaacaaa 3420
   atgcccgcac gaacggcggg cagaagtctt agcggttgga gatgtacaga gtcacttcca 3480
   agcccagacg caggtcaata aacgcaggtt ttttccacat aatggtcttc ctcataaaac 3540
   aaaaacgcag agaaatctct gctgaagagg attgcggctg tgacttagat cacatcgcaa 3600
   tccgtacgcg ccgcaggatg ttgcgccaat gtagcgagca gatcaaccct gattcggtaa 3660
   ggaatggtgc taatcctgcc aaatttgttg caacacgcgc tgccagttgc cccatgcgag 3720
   ctgctcaagt gctgtttgat cgtagtcgaa cgaacgcaac aacgcataaa gatgcgggta 3780
   atccgctcac gtctttcaag cgcctcaggc acgctgatgc catcaaaatc ggagcccagc 3840
   gccacatgat cgcggcccat tatcgcgatc aggtgctcaa catgtttaac aatttcgatc 3900
   aatgcggtgt cgctgtcgcg ttttccgtcg ctgcgcagaa aagcgttgcc aaaattcacg 3960
   cccaccacgc cgccgctgtc gcggatggcg cgcagctgat cgtcggtgag attgcgcggc 4020
   tgcgcgcaga gcgcgtgcgc attggagtgc gtcgccacca gcggcgcggt tgacaggcgc 4080
   gcggtgtccc agaaggcttt ctcgttcatg tgcgacatat cgatcagcat gcgatggcga 4140
   ttggcagcgg cgatcagctg ctcaccttgt tcggtcaagc ctggcccgct gtccggtgag 4200
   ttggggaatg atccgctgac gccttcgcca aagcgattgg gcagattcca gaacggcccg 4260
   atgctgcgtg gatccgcgc 4279
<210> 18
   <211> 23
   <212> PRT
   <213> Kluyvera intermedia
<400> 18
   Met Trp Lys Lys Pro Ala Phe Ile Asp Leu Arg Leu Gly Leu Glu Val
   1 5 10 15
   Thr Leu Tyr Ile Ser Asn Arg
   20
<210> 19
   <211> 307
   <212> PRT
   <213> Kluyvera intermedia
<400> 19
   Met Phe Ile Lys Val Leu Gly Ser Ala Ala Gly Gly Gly Phe Pro Gln
   1               5                   10                  15
   Trp Asn Cys Asn Cys Ala Asn Cys Ser Gly Leu Arg Asn Gly Ser Ile
               20                  25                  30
   Gln Ala Gln Ala Arg Thr Gln Ser Ser Ile Ile Val Ser Asp Asn Gly
           35                  40                  45
   Glu Asp Trp Val Leu Cys Asn Ala Ser Pro Asp Ile Ser Gln Gln Ile
       50                  55                  60
   Ala His Thr Pro Glu Leu Ile Lys Lys Gly Val Leu Arg Gly Thr Ala
   65                  70                  75                  80
   Ile Gly Ser Ile Ile Leu Thr Asp Ser Gln Ile Asp His Thr Thr Gly
                   85                  90                  95
   Leu Leu Ser Leu Arg Glu Gly Cys Pro His Gln Val Trp Cys Thr Pro
               100                 105                 110
   Glu Val His Gly Asp Leu Thr Ser Gly Phe Pro Ile Phe Thr Met Leu
           115                 120                 125
   Gln His Trp Asn Gly Gly Leu Gln His His Ala Leu Thr Pro Leu Glu
       130                 135                 140
   Pro Phe Arg Val Asp Val Cys His Ser Leu Gln Phe Thr Ala Ile Pro
   145                 150                 155                 160
   Ile Leu Ser Asn Ala Pro Pro Tyr Ser Pro Tyr Arg Asp Arg Pro Ala
                   165                 170                 175
   Gly Pro Gln Arg Gly Ala Val His Glu Thr Thr Gly Asn Gly Gln Thr
               180                 185                 190
   Leu Leu Tyr Ala Pro Gly Leu Gly Glu Pro Asp Ala Val Ile His Ala
           195                 200                 205
   Val Ala Ala Lys Ser Arg Leu Pro Val Ile Asp Gly Thr Val Trp Gln
       210                 215                 220
   Asp Asp Glu Leu Leu Ala Thr Gly Val Gly Lys Asn Thr Gly Lys Ala
   225 230 235 240
   Met Gly His Leu Ala Leu Ala Glu Glu Gln Gly Leu Met Ala Leu Leu
   245 250 255
   Ala Ala Leu Pro Ala Lys Arg Lys Ile Leu Ile His Ile Asn Asn Thr
   260 265 270
   Asn Pro Ile Leu Asn Glu Gln Ser Pro Gln Arg Ala Tyr Leu Thr Gln
   275 280 285
   Gln Gly Ile Glu Val Ser Trp Asp Gly Met Ala Ile His Leu Gln Glu
   290 295 300
   Phe Ala Ser
   305
<210> 20
   <211> 251
   <212> PRT
   <213> Kluyvera intermedia
<400> 20
   Met Ile Ile Thr Glu Ala Leu Ser Pro Ala Ala Phe Glu Gln Ala Leu
   1 5 10 15
   Arg Asp Lys Gly Ala Phe Tyr His Ile His His Pro Tyr His Ile Ala
   20 25 30
   Met His Asn Gly Glu Ala Thr Arg Glu Gln Ile Gln Gly Trp Val Ala
   35 40 45
   Asn Arg Phe Tyr Tyr Gln Thr Asn Ile Pro Leu Lys Asp Ala Ala Ile
   50 55 60
   Met Ala Asn Cys Pro Asp Ala Ala Thr Arg Arg Lys Trp Val Gln Arg
   65 70 75 80
   Ile Leu Asp His Asp Gly Ser Asn Gly His Glu Gly Gly Ile Glu Ala
   85 90 95
   Trp Leu Gln Leu Gly Glu Ala Val Gly Leu Glu Arg Asp Val Leu Leu
   100 105 110
   Ser Glu Lys Leu Val Leu Pro Gly Val Arg Phe Ala Val Asp Ala Tyr
   115 120 125
   Val Asn Phe Ala Arg Arg Ala Asn Trp Gln Glu Ala Ala Cys Ser Ser
   130 135 140
   Leu Thr Glu Leu Phe Ala Pro Gln Ile His Gln Ser Arg Leu Asp Ser
   145                 150                 155                 160
   Trp Pro Gln His Tyr Pro Trp Ile Lys Glu Glu Gly Tyr Phe Tyr Phe
                   165                 170                 175
   Arg Ser Arg Leu Ser Gln Ala Asn Arg Asp Val Glu His Gly Leu Ala
               180                 185                 190
   Leu Ala Leu Glu Val Phe Thr Arg Ala Glu Gln Gln Asn Arg Met Leu
           195                 200                 205
   Glu Ile Leu Gln Phe Lys Leu Asp Ile Leu Trp Thr Met Leu Asp Ala
       210                 215                 220
   Met Thr Met Ala Tyr Ala Leu Gln Arg Pro Pro Tyr His Thr Val Thr
   225                 230                 235                 240
   Asp Gln Val Ser Trp His Ser Thr Arg Leu Val
                   245                 250
<210> 21
   <211> 92
   <212> PRT
   <213> Kluyvera intermedia
<400> 21
   Met Lys Asp Asn Val Ile Pro Ala Phe Arg Arg Gly Tyr Arg Met Gln
   1               5                   10                  15
   Trp Glu Ala Ala Gln Asp Ser His Val Val Leu Tyr Pro Glu Gly Met
               20                  25                  30
   Ala Lys Leu Asn Glu Thr Ala Val Ala Ile Leu Glu Leu Val Asp Gly
           35                  40                  45
   Lys Gln Asp Val Ala Ala Ile Val Ala Thr Leu Asp Ala Arg Phe Pro
       50                  55                  60
   Asp Ala Gly Gly Val Gly Asp Asp Val Lys Glu Phe Leu Gln Ser Ala
   65                  70                  75                  80
   Ile Glu Gln Lys Trp Ile Gln Cys Arg Glu Pro Glu
                   85                  90
<210> 22
   <211> 374
   <212> PRT
   <213> Kluyvera intermedia
<400> 22
   Val Asn Pro Ser Lys Ser Val Thr Pro Pro Leu Trp Leu Leu Ala Glu
   1               5                   10                  15
   Leu Thr Tyr Arg Cys Pro Leu Gln Cys Pro Tyr Cys Ser Asn Pro Leu
               20                  25                  30
   Asp Phe Ser Gln Gln Lys Lys Glu Leu Thr Thr Glu Gln Trp Ile Glu
           35                  40                  45
   Val Phe Arg Gln Ala Arg Ala Met Gly Ser Val Gln Leu Gly Phe Ser
       50                  55                  60
   Gly Gly Glu Pro Leu Thr Arg Lys Asp Leu Pro Glu Leu Ile Arg Ala
   65                  70                  75                  80
   Ala Arg Asp Leu Gly Phe Tyr Thr Asn Leu Ile Thr Ser Gly Ile Gly
                   85                  90                  95
   Leu Thr Ala Lys Lys Leu Asp Ala Phe Ala Asp Ala Gly Leu Asp His
               100                 105                 110
   Ile Gln Ile Ser Phe Gln Ala Ser Asp Glu Thr Leu Asn Ala Ala Leu
           115                 120                 125
   Ala Gly Ser Lys Lys Ala Phe Gln Gln Lys Leu Glu Met Ala Lys Ala
       130                 135                 140
   Val Lys Ala His Gly Tyr Pro Met Val Leu Asn Phe Val Leu His Arg
   145                 150                 155                 160
   His Asn Ile Asp Gln Ile Asp Lys Ile Ile Asp Leu Cys Ile Glu Leu
                   165                 170                 175
   Asp Ala Asp Asp Val Glu Leu Ala Thr Cys Gln Phe Tyr Gly Trp Ala
               180                 185                 190
   Gln Leu Asn Arg Glu Gly Leu Leu Pro Thr Arg Glu Gln Ile Ala Asn
           195                 200                 205
   Ala Glu Ala Val Val Ala Asp Tyr Arg Gln Arg Met Gly Ala Ser Gly
       210                 215                 220
   Asn Leu Thr Asn Pro Ala Val Arg Asp Ala Gly Leu Leu Arg Arg Ala
   225                 230                 235                 240
   Ala Glu Thr Leu His Gly Arg Met Gly Ile Asp Leu Pro Gln Arg His
                   245                 250                 255
   Ala Asp Cys Thr Ala Leu Pro Cys His Ser Ala Arg Gln Leu Pro Val
               260                 265                 270
   Ala Phe Pro Ser Val Leu Glu Arg Thr Leu Asp Asp Ile Trp Tyr Asn
           275                 280                 285
   Ser Phe Gly Phe Asn Arg Tyr Arg Gly Phe Asp Trp Met Pro Glu Pro
       290                 295                 300
   Cys Arg Ser Cys Asp Glu Lys Ala Lys Asp Phe Gly Gly Cys Arg Cys
   305                 310                 315                 320
   Gln Ala Phe Met Leu Thr Gly Asp Ala Asp Asn Thr Asp Pro Val Cys
                   325                 330                 335
   Ser Lys Ser Pro His His Gly Lys Ile Leu Glu Ala Arg Arg Glu Ala
               340                 345                 350
   Asn Cys Ser Asp Ile Lys Ile Gln Gln Leu Gln Phe Arg Asn Arg Ser
           355                 360                 365
   Asn Ser Glu Leu Ile Phe
       370
<210> 23
   <211> 1116
   <212> DNA
   <213> Escherichia coli
<400> 23
   atgcatcgac aatccttttt ccttgtgccc cttatttgtc tttcttccgc tctctgggcg 60
   gctcctgcaa cggtaaatgt cgaagtactg caagacaaac tcgaccatcc ctgggcactg 120
   gcctttttac ccgataatca cggtatgtta atcactctgc gcggcggcga gttgcgtcac 180
   tggcaagcag gaaaaggatt atctgcgccg ctttccggag ttccggacgt ttgggcgcac 240
   gggcagggcg gcctgctgga cgtggtttta gcgcctgatt ttgctcagtc tcgccgcatc 300
   tggttaagtt attccgaagt tggcgatgat ggcaaagccg gaactgctgt gggttatggc 360
   cgcttaagtg atgatctctc aaaagtgacc gacttccgca ccgtctttcg ccagatgcca 420
   aaactgtcta ccggcaacca ttttggcggg cggctggtat tcgacggtaa aggttatctt 480
   tttattgctc tgggcgaaaa caatcagcgc ccgacggcgc aggatctgga taaattacag 540
   ggcaaactgg tgcgtctgac cgaccagggc gaaatcccgg atgataatcc ttttataaag 600
   gaatccggtg cgcgcgccga gatctggtct tatggcattc gtaatccgca aggaatggcg 660
   atgaatccgt ggagtaatgc actgtggctg aatgaacatg gcccgcgcgg tggtgatgaa 720
   attaatatcc cgcaaaaagg caaaaactac ggctggccgc tggcaacctg gggaatcaac 780
   tattcaggct ttaagatacc ggaagcgaaa ggggagatcg tcgccgggac cgagcaacct 840
   gttttttact ggaaagattc gcccgctgtg agcggcatgg ccttctataa cagcgataaa 900
   ttcccccagt ggcagcaaaa attatttatt ggcgcgctga aagataaaga tgtcattgtg 960
   atgagcgtca acggcgacaa agtgacagaa gatggccgta ttttaacgga cagagggcag 1020
   cgaattcgtg atgttcgcac tggacccgac ggttatttat acgttctcac cgacgagtcc 1080
   agtggggaat tacttaaagt tagcccacgc aattag 1116
<210> 24
   <211> 371
   <212> PRT
   <213> Escherichia coli
<400> 24
   Met His Arg Gln Ser Phe Phe Leu Val Pro Leu Ile Cys Leu Ser Ser
   1               5                   10                  15
   Ala Leu Trp Ala Ala Pro Ala Thr Val Asn Val Glu Val Leu Gln Asp
               20                  25                  30
   Lys Leu Asp His Pro Trp Ala Leu Ala Phe Leu Pro Asp Asn His Gly
           35                  40                  45
   Met Leu Ile Thr Leu Arg Gly Gly Glu Leu Arg His Trp Gln Ala Gly
       50                  55                  60
   Lys Gly Leu Ser Ala Pro Leu Ser Gly Val Pro Asp Val Trp Ala His
   65                  70                  75                  80
   Gly Gln Gly Gly Leu Leu Asp Val Val Leu Ala Pro Asp Phe Ala Gln
                   85                  90                  95
   Ser Arg Arg Ile Trp Leu Ser Tyr Ser Glu Val Gly Asp Asp Gly Lys
               100                 105                 110
   Ala Gly Thr Ala Val Gly Tyr Gly Arg Leu Ser Asp Asp Leu Ser Lys
           115                 120                 125
   Val Thr Asp Phe Arg Thr Val Phe Arg Gln Met Pro Lys Leu Ser Thr
       130                 135                 140
   Gly Asn His Phe Gly Gly Arg Leu Val Phe Asp Gly Lys Gly Tyr Leu
   145                 150                 155                 160
   Phe Ile Ala Leu Gly Glu Asn Asn Gln Arg Pro Thr Ala Gln Asp Leu
                   165                 170                 175
   Asp Lys Leu Gln Gly Lys Leu Val Arg Leu Thr Asp Gln Gly Glu Ile
   180 185 190
   Pro Asp Asp Asn Pro Phe Ile Lys Glu Ser Gly Ala Arg Ala Glu Ile
   195 200 205
   Trp Ser Tyr Gly Ile Arg Asn Pro Gln Gly Met Ala Met Asn Pro Trp
   210 215 220
   Ser Asn Ala Leu Trp Leu Asn Glu His Gly Pro Arg Gly Gly Asp Glu
   225 230 235 240
   Ile Asn Ile Pro Gln Lys Gly Lys Asn Tyr Gly Trp Pro Leu Ala Thr
   245 250 255
   Trp Gly Ile Asn Tyr Ser Gly Phe Lys Ile Pro Glu Ala Lys Gly Glu
   260 265 270
   Ile Val Ala Gly Thr Glu Gln Pro Val Phe Tyr Trp Lys Asp Ser Pro
   275 280 285
   Ala Val Ser Gly Met Ala Phe Tyr Asn Ser Asp Lys Phe Pro Gln Trp
   290 295 300
   Gln Gln Lys Leu Phe Ile Gly Ala Leu Lys Asp Lys Asp Val Ile Val
   305 310 315 320
   Met Ser Val Asn Gly Asp Lys Val Thr Glu Asp Gly Arg Ile Leu Thr
   325 330 335
   Asp Arg Gly Gln Arg Ile Arg Asp Val Arg Thr Gly Pro Asp Gly Tyr
   340 345 350
   Leu Tyr Val Leu Thr Asp Glu Ser Ser Gly Glu Leu Leu Lys Val Ser
   355 360 365
   Pro Arg Asn
   370
<210> 25
   <211> 2391
   <212> DNA
   <213> Escherichia coli
<400> 25
   ttacttcaca tcatccggca gcgcataagc cacaatatag tcgcccatct tcgtaccaaa        60
   tgaaccgtga ccgcctgcgg agatcaccac atactgctta ccattcactt cataggtcat       120
   tggcgtagcc tgaccacccg ctggtaaacg accctgccac agtttttcac cgttgctcat       180
   gttgtaagcg cgcaggtagt tatctgccgt agcggcgata aacagcacgt tacccgccgt       240
   ggagattggc ccgcccagca tcggcatacc catattgaac ggcaccggaa ccggcatcgg 300
   gaacggcata ctgtcctgcg gcgtaccaat acgtttcttc cacaccactt cattagtttt 360
   cagatccagc gccgagatat aaccccatgc tggctgttta catggcagac caaatggtga 420
   gaggaacggg ttgagcgtga caccatacgg tacaccgtac tgtggctgaa tgccggattc 480
   cgtacccgtg cctttggcat ctttcggctg ctccatcggg ttgccaggac cacgcgggat 540
   cagtttcgaa acaaacggca gtgccattgg gttggcaatc gccacttcac gatttggatc 600
   aacggaaatc ccgccccatt cgaacatccc caggttaccc gggaagacca gcgtaccctg 660
   ttcagatggc ggggtgaaaa tgccttcata gcgcatctgg tggaacatca cgcggcacac 720
   cagttggtca aacatggtgg ctccccacat atccgcaccg ctcaaatctt tcgtcggacg 780
   gaagctcagt tcagaaaacg gttgagttgg ggttacgtaa tcgcctttcg ctgcaccttg 840
   gggaaccggt ttttccggtg ccggaaccac cagttcgcca ttacgacgat cgagcacaaa 900
   aatgttgccg gttttcgccg gagcgtaaat aactggcact ttctgaccat taacggtgat 960
   gtccgccagc gtcggctgtg ccggaagatc catgtcccac aggtcgtggt gaacggtctg 1020
   gtagctccac gccagtttcc cggtagtggc attcagcgcc agaatcgagc tggcataacg 1080
   ttcctgttcc ggtgtgcggt taccgcccca gatatccggc gtggtcacgc ccatcggcag 1140
   atagaccaga tccagcttcg cgtcataggc cgctggtgcc caggagtttg gcgagttaaa 1200
   ggtaaaggtg tgttcgtcag acgggattgc gttcggatct ttcgcgccgg gatcaaaagc 1260
   ccacagcagc tccccggtgt tgacatcaaa accacggatc acgccagacg tttcgcgggt 1320
   tgagaagtta tcggtaactg aaccggccat cacgatggtt ttatcggtga taatcggtgg 1380
   cgaagtcggt tcatacagac ccggtttggt gtctggcata ttgctttgca gattgagcac 1440
   gcctttattg gcgaaggttt cgcacagttt gccgttttca gcgttaatcg caatcagtcg 1500
   accatcattg accggaagaa tgatacgacg cgggcaatcc gccatcactt ccggcgaagc 1560
   ggtttctgct ttggcttcat gataagagac accacggcag gttacgtgct ggaaagactc 1620
   gttggttttc agctcaggat cgtaatgcca tttctctttg ccgctggcgg catcaagcgc 1680
   aaacaggcgc tggtgagcgg tacacaggta aagggtgtcg cccactttaa tcggcgtcac 1740
   ttcattggtg atttcacccg gatcgttcgg ctgcttcaca tcgccagtac ggaacaccca 1800
   ggcttctttc agattatgga cgttatcggc gttaatttgt ttcagcggcg aaaagcgttg 1860
   accttcctga ttacgaccat aggcaggcca gtcctgatcg gctacggggg agatagcttc 1920
   agcaggtgtg gcatcggcgc ttaaggtgcc gttgatctcc tgcggatcgt taaatccggc 1980
   ccaggtcagg ataccaccgc taatcagcag tgcgaccacc agtgcggcaa ctgcgccgct 2040
   ggcaggaatg accaggcgac gccagacaaa cggcaggatc agccagatgc cgaagaagac 2100
   cagaatgtcg ctgcgcggag tcagcgccca gaagtcgaaa ccaacttccc agacgcccca 2160
   aatcatggtg ccgagcagca gggctgcgta tagccaaagc gcggcgcgtt tactgcgcca 2220
   cagcatccag gcgacgccga gcatcacaag gccagcgata gggtagtacc aggagccgcc     2280
   aatcgcgacc agccagcctc cgccaatgag tagatacagc ccgcaaagcg ctgcaaaaag     2340
   ggctgttagc gtgacgagta atcgtcgcga gcctgtattg ttaattgcca t              2391
<210> 26
   <211> 796
   <212> PRT
   <213> Escherichia coli
<400> 26
   Met Ala Ile Asn Asn Thr Gly Ser Arg Arg Leu Leu Val Thr Leu Thr
   1               5                   10                  15
   Ala Leu Phe Ala Ala Leu Cys Gly Leu Tyr Leu Leu Ile Gly Gly Gly
               20                  25                  30
   Trp Leu Val Ala Ile Gly Gly Ser Trp Tyr Tyr Pro Ile Ala Gly Leu
           35                  40                  45
   Val Met Leu Gly Val Ala Trp Met Leu Trp Arg Ser Lys Arg Ala Ala
       50                  55                  60
   Leu Trp Leu Tyr Ala Ala Leu Leu Leu Gly Thr Met Ile Trp Gly Val
   65                  70                  75                  80
   Trp Glu Val Gly Phe Asp Phe Trp Ala Leu Thr Pro Arg Ser Asp Ile
                   85                  90                  95
   Leu Val Phe Phe Gly Ile Trp Leu Ile Leu Pro Phe Val Trp Arg Arg
               100                 105                 110
   Leu Val Ile Pro Ala Ser Gly Ala Val Ala Ala Leu Val Val Ala Leu
           115                 120                 125
   Leu Ile Ser Gly Gly Ile Leu Thr Trp Ala Gly Phe Asn Asp Pro Gln
       130                 135                 140
   Glu Ile Asn Gly Thr Leu Ser Ala Asp Ala Thr Pro Ala Glu Ala Ile
   145                 150                 155                 160
   Ser Pro Val Ala Asp Gln Asp Trp Pro Ala Tyr Gly Arg Asn Gln Glu
                   165                 170                 175
   Gly Gln Arg Phe Ser Pro Leu Lys Gln Ile Asn Ala Asp Asn Val His
               180                 185                 190
   Asn Leu Lys Glu Ala Trp Val Phe Arg Thr Gly Asp Val Lys Gln Pro
           195                 200                 205
   Asn Asp Pro Gly Glu Ile Thr Asn Glu Val Thr Pro Ile Lys Val Gly
       210                 215                 220
   Asp Thr Leu Tyr Leu Cys Thr Ala His Gln Arg Leu Phe Ala Leu Asp
   225                 230                 235                 240
   Ala Ala Ser Gly Lys Glu Lys Trp His Tyr Asp Pro Glu Leu Lys Thr
                   245                 250                 255
   Asn Glu Ser Phe Gln His Val Thr Cys Arg Gly Val Ser Tyr His Glu
               260                 265                 270
   Ala Lys Ala Glu Thr Ala Ser Pro Glu Val Met Ala Asp Cys Pro Arg
           275                 280                 285
   Arg Ile Ile Leu Pro Val Asn Asp Gly Arg Leu Ile Ala Ile Asn Ala
       290                 295                 300
   Glu Asn Gly Lys Leu Cys Glu Thr Phe Ala Asn Lys Gly Val Leu Asn
   305                 310                 315                 320
   Leu Gln Ser Asn Met Pro Asp Thr Lys Pro Gly Leu Tyr Glu Pro Thr
                   325                 330                 335
   Ser Pro Pro Ile Ile Thr Asp Lys Thr Ile Val Met Ala Gly Ser Val
               340                 345                 350
   Thr Asp Asn Phe Ser Thr Arg Glu Thr Ser Gly Val Ile Arg Gly Phe
           355                 360                 365
   Asp Val Asn Thr Gly Glu Leu Leu Trp Ala Phe Asp Pro Gly Ala Lys
       370                 375                 380
   Asp Pro Asn Ala Ile Pro Ser Asp Glu His Thr Phe Thr Phe Asn Ser
   385                 390                 395                 400
   Pro Asn Ser Trp Ala Pro Ala Ala Tyr Asp Ala Lys Leu Asp Leu Val
                   405                 410                 415
   Tyr Leu Pro Met Gly Val Thr Thr Pro Asp Ile Trp Gly Gly Asn Arg
               420                 425                 430
   Thr Pro Glu Gln Glu Arg Tyr Ala Ser Ser Ile Leu Ala Leu Asn Ala
           435                 440                 445
   Thr Thr Gly Lys Leu Ala Trp Ser Tyr Gln Thr Val His His Asp Leu
       450                 455                 460
   Trp Asp Met Asp Leu Pro Ala Gln Pro Thr Leu Ala Asp Ile Thr Val
   465 470 475 480
   Asn Gly Gln Lys Val Pro Val Ile Tyr Ala Pro Ala Lys Thr Gly Asn
   485 490 495
   Ile Phe Val Leu Asp Arg Arg Asn Gly Glu Leu Val Val Pro Ala Pro
   500 505 510
   Glu Lys Pro Val Pro Gln Gly Ala Ala Lys Gly Asp Tyr Val Thr Pro
   515 520 525
   Thr Gln Pro Phe Ser Glu Leu Ser Phe Arg Pro Thr Lys Asp Leu Ser
   530 535 540
   Gly Ala Asp Met Trp Gly Ala Thr Met Phe Asp Gln Leu Val Cys Arg
   545 550 555 560
   Val Met Phe His Gln Met Arg Tyr Glu Gly Ile Phe Thr Pro Pro Ser
   565 570 575
   Glu Gln Gly Thr Leu Val Phe Pro Gly Asn Leu Gly Met Phe Glu Trp
   580 585 590
   Gly Gly Ile Ser Val Asp Pro Asn Arg Glu Val Ala Ile Ala Asn Pro
   595 600 605
   Met Ala Leu Pro Phe Val Ser Lys Leu Ile Pro Arg Gly Pro Gly Asn
   610 615 620
   Pro Met Glu Gln Pro Lys Asp Ala Lys Gly Thr Gly Thr Glu Ser Gly
   625 630 635 640
   Ile Gln Pro Gln Tyr Gly Val Pro Tyr Gly Val Thr Leu Asn Pro Phe
   645 650 655
   Leu Ser Pro Phe Gly Leu Pro Cys Lys Gln Pro Ala Trp Gly Tyr Ile
   660 665 670
   Ser Ala Leu Asp Leu Lys Thr Asn Glu Val Val Trp Lys Lys Arg Ile
   675 680 685
   Gly Thr Pro Gln Asp Ser Met Pro Phe Pro Met Pro Val Pro Val Pro
   690 695 700
   Phe Asn Met Gly Met Pro Met Leu Gly Gly Pro Ile Ser Thr Ala Gly
   705 710 715 720
   Asn Val Leu Phe Ile Ala Ala Thr Ala Asp Asn Tyr Leu Arg Ala Tyr
                   725                 730                 735
   Asn Met Ser Asn Gly Glu Lys Leu Trp Gln Gly Arg Leu Pro Ala Gly
               740                 745                 750
   Gly Gln Ala Thr Pro Met Thr Tyr Glu Val Asn Gly Lys Gln Tyr Val
           755                 760                 765
   Val Ile Ser Ala Gly Gly His Gly Ser Phe Gly Thr Lys Met Gly Asp
       770                 775                 780
   Tyr Ile Val Ala Tyr Ala Leu Pro Asp Asp Val Lys
   785                 790                 795
<210> 27
   <211> 2427
   <212> DNA
   <213> Gluconobacter oxydans
<400> 27
   atgagcacaa tttcccggcc caggctctgg gccctgataa cggccgcggt attcgcgctt 60
   tgcggcgcga tccttaccgt tggcggcgca tgggtcgctg ccatcggcgg ccctctttat 120
   tatgtcatcc ttggcctggc acttctcgcc acggctttcc tctcattccg gcgcaatccg 180
   gctgccctct atctgttcgc agtcgtcgtc ttcggaacgg tcatctggga actcaccgtt 240
   gtcggtctcg acatctgggc cctgatcccg cgctcggaca tcgtcatcat cctcggcatc 300
   tggctgctgc tgccgttcgt ctcgcgcgcc agatcggtgg cacgcggacg accgtcctgc 360
   cgctcgccgg ccgttggcgt tgcggttctg gccctgttcg ccagcctctt caccgacccg 420
   catgacatca gcggcgaact gccgacgcag atcgcaaacg cctcccccgc cgacccggac 480
   aacgttccgg ccagcgagtg gcacgcttat ggtcgtacgc aggccggtga ccgctggtcc 540
   ccgctgaacc agatcaatgc gacgaacgtc agcaacctca aggtcgcatg gcatatccac 600
   accaaggata tgatgaactc caacgacccg ggcgaagcga cgaacgaagc gacgccgatc 660
   gagttcaaca acacgcttta tatgtgctca ctgcatcaga agctgtttgc ggttgatggt 720
   gccaccggca acgtcaagtg ggtctacgat ccgaagctcc agatcaaccc tggcttccag 780
   catctgacct gccgtggcgt cagcttccac gaaacgccgg ccaatgccat ggattccgat 840
   ggcaatcctg ctccgacgga ctgcgccaag gactccatcc tgccggtcaa tgatggccgt 900
   ctggttgaag tcgatgccga cacgggcaag acctgctccg gcttcggcaa caatggcgag 960
   atcgacctgc gcgttccgaa ccagccttac acgacgcctg gccagtacga gccgacgtcc 1020
   ccgccggtca tcacagacaa gctgatcatc gccaacagcg ccatcaccga taacggttcg 1080
   gtcaagcagg cttcgggcgc cacgcaggca ttcgacgtct acaccggcaa gcgcgtctgg 1140
   gtgttcgatg cgtccaaccc ggatccgaac cagcttccgg atgagagcca ccctgtcttc 1200
   cacccgaact cgccaaactc ctggatcgtg tcgtcctacg acgccaacct gaacctcgtg 1260
   tacatcccga tgggcgtggg gactcccgac cagtggggcg gtgaccgcac gaaggattcc 1320
   gagcgtttcg ctccgggtat cgttgcgctg aacgccgata cgggcaagct cgcctggttc 1380
   taccagaccg ttcatcacga tctgtgggac atggagcttc cgtcccagcc gagcctcgtg 1440
   gatgtgacac agaaggacgg cacgcttgtt ccggccatct acgctccgac caagaccggc 1500
   gacattttcg tcctcgaccg tcgtaccggc aaggaaatcg tcccggctcc ggaaaccccg 1560
   gttccccagg gtgctgctcc gggtgaccac accagcccga cccagccgat gtcgcagctg 1620
   accctgcgtc cgaagaaccc gctgaacgac tccgatatct ggggcggcac gatcttcgac 1680
   cagatgttct gcagcatcta tttccacacc ctccgctacg aaggcccctt cacgccgccg 1740
   tcgctcaagg gctcgctcat cttcccgggt gatctgggaa tgttcgaatg gggtggtctg 1800
   gccgtcgatc cgcagcgtca ggtggctttc gccaacccga tttccctgcc gttcgtctct 1860
   cagcttgttc cccgcggacc gggcaacccg ctctggcctg aagaaaatgc caagggcacg 1920
   ggtggtgaaa ccggcctgca gcacaactat ggcatcccgt atgccgtcaa cctgcatccg 1980
   ttcctggatc cggtgctgct gccgttcggc atcaagatgc cgtgccgcac gccgccctgg 2040
   ggctatgtcg ccggtattga cctgaagacc aacaaggtcg tctggcagca ccgcaacggc 2100
   accctgcgtg actcgatgta tggcagctcc ctgccgatcc cgctgccgcc gatcaagatc 2160
   ggtgtcccga gcctcggtgg cccgctctcc acggctggca atctcggctt cctgacggcg 2220
   tccatggatt actacatccg tgcgtacaac ctgacgacgg gcaaggtgct gtggcaggac 2280
   cgtctgccgg ctggtgctca ggcaacgccg atcacctatg ccatcaacgg caagcagtac 2340
   atcgtgacct atgcaggcgg acacaactcg ttcccgaccc gcatgggcga cgacatcatc 2400
   gcctacgccc tgcccgatca gaaatga 2427
<210> 28
   <211> 808
   <212> PRT
   <213> Gluconobacter oxydans
<400> 28
   Met Ser Thr Thr Ser Arg Pro Gly Leu Trp Ala Leu Ile Thr Ala Ala
   1               5                   10                  15
   Val Phe Ala Leu Cys Gly Ala Ile Leu Thr Val Gly Gly Ala Trp Val
               20                  25                  30
   Ala Ala Ile Gly Gly Pro Leu Tyr Tyr Val Ile Leu Gly Leu Ala Leu
           35                  40                  45
   Leu Ala Thr Ala Phe Leu Ser Phe Arg Arg Asn Pro Ala Ala Leu Tyr
       50                  55                  60
   Leu Phe Ala Val Val Val Phe Gly Thr Val Ile Trp Glu Leu Thr Val
   65                  70                  75                  80
   Val Gly Leu Asp Ile Trp Ala Leu Ile Pro Arg Ser Asp Ile Val Ile
                   85                  90                  95
   Ile Leu Gly Ile Trp Leu Leu Leu Pro Phe Val Ser Arg Gln Ile Gly
               100                 105                 110
   Gly Thr Arg Thr Thr Val Leu Pro Leu Ala Gly Ala Val Gly Val Ala
           115                 120                 125
   Val Leu Ala Leu Phe Ala Ser Leu Phe Thr Asp Pro His Asp Ile Ser
       130                 135                 140
   Gly Asp Leu Pro Thr Gln Ile Ala Asn Ala Ser Pro Ala Asp Pro Asp
   145                 150                 155                 160
   Asn Val Pro Ala Ser Glu Trp His Ala Tyr Gly Arg Thr Gln Ala Gly
                   165                 170                 175
   Asp Arg Trp Ser Pro Leu Asn Gln Ile Asn Ala Ser Asn Val Ser Asn
               180                 185                 190
   Leu Lys Val Ala Trp His Ile His Thr Lys Asp Met Met Asn Ser Asn
           195                 200                 205
   Asp Pro Gly Glu Ala Thr Asn Glu Ala Thr Pro Ile Glu Phe Asn Asn
       210                 215                 220
   Thr Leu Tyr Met Cys Ser Leu His Gln Lys Leu Phe Ala Val Asp Gly
   225                 230                 235                 240
   Ala Thr Gly Asn Val Lys Trp Val Tyr Asp Pro Lys Leu Gln Ile Asn
                   245                 250                 255
   Pro Gly Phe Gln His Leu Thr Cys Arg Gly Val Ser Phe His Glu Thr
               260                 265                 270
   Pro Ala Asn Ala Thr Asp Ser Asp Gly Asn Pro Ala Pro Thr Asp Cys
           275                 280                 285
   Ala Lys Arg Ile Ile Leu Pro Val Asn Asp Gly Arg Leu Val Glu Val
       290                 295                 300
   Asp Ala Asp Thr Gly Lys Thr Cys Ser Gly Phe Gly Asn Asn Gly Glu
   305                 310                 315                 320
   Ile Asp Leu Arg Val Pro Asn Gln Pro Tyr Thr Thr Pro Gly Gln Tyr
                   325                 330                 335
   Glu Pro Thr Ser Pro Pro Val Ile Thr Asp Lys Leu Ile Ile Ala Asn
               340                 345                 350
   Ser Ala Ile Thr Asp Asn Gly Ser Val Lys Gln Ala Ser Gly Ala Thr
           355                 360                 365
   Gln Ala Phe Asp Val Tyr Thr Gly Lys Arg Val Trp Val Phe Asp Ala
       370                 375                 380
   Ser Asn Pro Asp Pro Asn Gln Leu Pro Asp Asp Ser His Pro Val Phe
   385                 390                 395                 400
   His Pro Asn Ser Pro Asn Ser Trp Ile Val Ser Ser Tyr Asp Arg Asn
                   405                 410                 415
   Leu Asn Leu Val Tyr Ile Pro Met Gly Val Gly Thr Pro Asp Gln Trp
               420                 425                 430
   Gly Gly Asp Arg Thr Lys Asp Ser Glu Arg Phe Ala Pro Gly Ile Val
           435                 440                 445
   Ala Leu Asn Ala Asp Thr Gly Lys Leu Ala Trp Phe Tyr Gln Thr Val
       450                 455                 460
   His His Asp Leu Trp Asp Met Asp Val Pro Ser Gln Pro Ser Leu Val
   465                 470                 475                 480
   Asp Val Thr Gln Lys Asp Gly Thr Leu Val Pro Ala Ile Tyr Ala Pro
                   485                 490                 495
   Thr Lys Thr Gly Asp Ile Phe Val Leu Asp Arg Arg Thr Gly Lys Glu
               500                 505                 510
   Ile Val Pro Ala Pro Glu Thr Pro Val Pro Gln Gly Ala Ala Pro Gly
           515                 520                 525
   Asp His Thr Ser Pro Thr Gln Pro Met Ser Gln Leu Thr Leu Arg Pro
       530                 535                 540
   Lys Asn Pro Leu Asn Asp Ser Asp Ile Trp Gly Gly Thr Ile Phe Asp
   545                 550                 555                 560
   Gln Met Phe Cys Ser Ile Tyr Phe His Thr Leu Arg Tyr Glu Gly Pro
                   565                 570                 575
   Phe Thr Pro Pro Ser Leu Lys Gly Ser Leu Ile Phe Pro Gly Asp Leu
               580                 585                 590
   Gly Met Phe Glu Trp Gly Gly Leu Ala Val Asp Pro Gln Arg Gln Val
           595                 600                 605
   Ala Phe Ala Asn Pro Ile Ser Leu Pro Phe Val Ser Gln Leu Val Pro
       610                 615                 620
   Arg Gly Pro Gly Asn Pro Leu Trp Pro Glu Lys Asp Ala Lys Gly Thr
   625                 630                 635                 640
   Gly Gly Glu Thr Gly Leu Gln His Asn Tyr Gly Ile Pro Tyr Ala Val
                   645                 650                 655
   Asn Leu His Pro Phe Leu Asp Pro Val Leu Leu Pro Phe Gly Ile Lys
               660                 665                 670
   Met Pro Cys Arg Thr Pro Pro Trp Gly Tyr Val Ala Gly Ile Asp Leu
           675                 680                 685
   Lys Thr Asn Lys Val Val Trp Gln His Arg Asn Gly Thr Leu Arg Asp
       690                 695                 700
   Ser Met Tyr Gly Ser Ser Leu Pro Ile Pro Leu Pro Pro Ile Lys Ile
   705                 710                 715                 720
   Gly Val Pro Ser Leu Gly Gly Pro Leu Ser Thr Ala Gly Asn Leu Gly
                   725                 730                 735
   Phe Leu Thr Ala Ser Met Asp Tyr Tyr Ile Arg Ala Tyr Asn Leu Thr
               740                 745                 750
   Thr Gly Lys Val Leu Trp Gln Asp Arg Leu Pro Ala Gly Ala Gln Ala
           755                 760                 765
   Thr Pro Ile Thr Tyr Ala Ile Asn Gly Lys Gln Tyr Ile Val Thr Tyr
       770                 775                 780
   Ala Gly Gly His Asn Ser Phe Pro Thr Arg Met Gly Asp Asp Ile Ile
   785                 790                 795                 800
   Ala Tyr Ala Leu Pro Asp Gln Lys
                   805
<210> 29
   <211> 2406
   <212> DNA
   <213> Acinetobacter calcoaceticus
<400> 29
   atgaatcaac ctacttcaag atcaggttta acgactttta ccgtaattat tattgggtta      60
   ctggcgttat tcctgttaat tggaggtatt tggctcgcta cactaggcgg ttcaatttac     120
   tacattatag ctggagtatt actcctcatt gttgcatggc aactctacaa gcgtgcttct     180
   actgctttgt ggttttatgc tgcattaatg ctaggtacca ttatctggag tgtctgggaa     240
   gttggaacag acttttgggc gcttgcacca cgtttagata ttttaggtat tcttggttta     300
   tggttattgg ttccggctgt aactcgtgga atcaacaacc ttggatcaag taaagttgcc     360
   ttatcttcaa ctttagcaat tgcaatcgtg ttgatggttt attctatctt caatgatccg     420
   caagaaatta atggtgaaat caaaacacct caaccagaaa cagctcaagc cgtgcctggt     480
   gttgctgaaa gtgattggcc agcttatggt cgtactcaag caggcgtgcg ttattctcca     540
   ttgaaacaga tcaatgatca aaacgtaaaa gacttgaaag ttgcttggac tttacgtact     600
   ggcgatctta agacagataa cgactctggc gaaacgacta atcaggttac accgattaaa     660
   atcggtaata acatgtttat ctgtacagct caccagcagt taattgctat tgatcctgct     720
   acaggtaaag aaaaatggcg ttttgatccg aaacttaaaa cggataaatc gttccagcat     780
   ctaacttgtc gtggtgtgat gtactacgat gcaaacaata caactgagtt tgcaacgagt     840
   cttcaaagca agaaatctag ctctacacaa tgtccacgta aggtatttgt accagtcaac     900
   gatggccgtt tagtggctgt aaatgctgac actggtaaag catgtactga ctttggtcaa     960
   aatggtcaag tgaacttaca agagttcatg ccatatgctt atccaggcgg ttataacccg    1020
   acatctcctg gtatcgtgac tggttcaact gttgttattg ctggttctgt aacagataac    1080
   tactcaaata aagagccatc tggtgtgatt cgtggatacg acgttaacac tggtaaactt    1140
   ctttgggtat ttgacactgg cgcagcggat ccaaatgcaa tgccgggcga aggtactact    1200
   tttgttcaca actcaccaaa tgcgtgggca cctttagcat acgatgccaa acttgacatc    1260
   gtttatgtac caacaggtgt aggtacacca gacatctggg gtggtgaccg taccgagctg    1320
   aaagagcgtt atgcaaactc aatgttagcg attaatgctt ctactggtaa attagtgtgg    1380
   aacttccaga caacccatca cgatttatgg gatatggatg taccatcaca accatcttta    1440
   gctgatatca aaaacaaagc tggccaaact gttcctgcaa tctatgtatt gacgaaaaca    1500
   ggtaatgcct ttgtgcttga tcgccgtaat ggtcaaccga ttgttcctgt aactgagaaa    1560
   ccagttccac aaacagttaa acgtggacca caaactaaag gtgagttcta ttcaaaaact    1620
   cagccattct ctgacttgaa cttggcgcca caagataaat tgactgataa agacatgtgg    1680
   ggtgccacta tgcttgatca gctcatgtgt cgtgtatctt tcaaacgtct aaattacgat    1740
   ggtatttata cgccaccatc tgaaaacggt actttagttt tccctggtaa cttaggtgta    1800
   tttgaatggg gcggtatgtc agttaaccct gatcgtcagg ttgctgtaat gaacccgatt    1860
   ggtctgccat tcgtcagtcg tttaattcct gctgatccaa accgtgcaca aactgcaaaa    1920
   ggtgcgggaa ctgagcaggg cgtacaaccg atgtacggcg taccatatgg tgttgaaatt      1980
   agcgcattct tatctccgct tggtttacct tgtaaacaac cggcttgggg ctatgtagct      2040
   ggcgttgatt tgaaaactca tgaagtggta tggaaaaaac gtattggtac aatccgtgac      2100
   agtttaccga acttgttcca gttgccagct gtgaaaattg gtgtgcctgg tttaggtggt      2160
   tcaatttcta ctgccggtaa tgtcatgttt gttggtgcaa ctcaagataa ctacttacgt      2220
   gcgtttaacg ttactaacgg taagaaactt tgggaagcgc gtttaccagc aggtggacaa      2280
   gcaacaccaa tgacttatga aatcaatggt aagcaatatg ttgtaatcat ggctggtggt      2340
   catggttcat ttggtacgaa aatgggcgac tatttagtgg cttatgcctt accagataac      2400
   aaataa                                                                  2406
<210> 30
   <211> 801
   <212> PRT
   <213> Acinetobacter calcoaceticus
<400> 30
   Met Asn Gln Pro Thr Ser Arg Ser Gly Leu Thr Thr Phe Thr Val Ile
   1               5                   10                  15
   Ile Ile Gly Leu Leu Ala Leu Phe Leu Leu Ile Gly Gly Ile Trp Leu
               20                  25                  30
   Ala Thr Leu Gly Gly Ser Ile Tyr Tyr Ile Ile Ala Gly Val Leu Leu
           35                  40                  45
   Leu Ile Val Ala Trp Gln Leu Tyr Lys Arg Ala Ser Thr Ala Leu Trp
       50                  55                  60
   Phe Tyr Ala Ala Leu Met Leu Gly Thr Ile Ile Trp Ser Val Trp Glu
   65                  70                  75                  80
   Val Gly Thr Asp Phe Trp Ala Leu Ala Pro Arg Leu Asp Ile Leu Gly
                   85                  90                  95
   Ile Leu Gly Leu Trp Leu Leu Val Pro Ala Val Thr Arg Gly Ile Asn
               100                 105                 110
   Asn Leu Gly Ser Ser Lys Val Ala Leu Ser Ser Thr Leu Ala Ile Ala
           115                 120                 125
   Ile Val Leu Met Val Tyr Ser Ile Phe Asn Asp Pro Gln Glu Ile Asn
       130                 135                 140
   Gly Glu Ile Lys Thr Pro Gln Pro Glu Thr Ala Gln Ala Val Pro Gly
   145                 150                 155                 160
   Val Ala Glu Ser Asp Trp Pro Ala Tyr Gly Arg Thr Gln Ala Gly Val
   165 170 175
   Arg Tyr Ser Pro Leu Lys Gln Ile Asn Asp Gln Asn Val Lys Asp Leu
   180 185 190
   Lys Val Ala Trp Thr Leu Arg Thr Gly Asp Leu Lys Thr Asp Asn Asp
   195 200 205
   Ser Gly Glu Thr Thr Asn Gln Val Thr Pro Ile Lys Ile Gly Asn Asn
   210 215 220
   Met Phe Ile Cys Thr Ala His Gln Gln Leu Ile Ala Ile Asp Pro Ala
   225 230 235 240
   Thr Gly Lys Glu Lys Trp Arg Phe Asp Pro Lys Leu Lys Thr Asp Lys
   245 250 255
   Ser Phe Gln His Leu Thr Cys Arg Gly Val Met Tyr Tyr Asp Ala Asn
   260 265 270
   Asn Thr Thr Glu Phe Ala Thr Ser Leu Gln Ser Lys Lys Ser Ser Ser
   275 280 285
   Thr Gln Cys Pro Arg Lys Val Phe Val Pro Val Asn Asp Gly Arg Leu
   290 295 300
   Val Ala Val Asn Ala Asp Thr Gly Lys Ala Cys Thr Asp Phe Gly Gln
   305 310 315 320
   Asn Gly Gln Val Asn Leu Gln Glu Phe Met Pro Tyr Ala Tyr Pro Gly
   325 330 335
   Gly Tyr Asn Pro Thr Ser Pro Gly Ile Val Thr Gly Ser Thr Val Val
   340 345 350
   Ile Ala Gly Ser Val Thr Asp Asn Tyr Ser Asn Lys Glu Pro Ser Gly
   355 360 365
   Val Ile Arg Gly Tyr Asp Val Asn Thr Gly Lys Leu Leu Trp Val Phe
   370 375 380
   Asp Thr Gly Ala Ala Asp Pro Asn Ala Met Pro Gly Glu Gly Thr Thr
   385 390 395 400
   Phe Val His Asn Ser Pro Asn Ala Trp Ala Pro Leu Ala Tyr Asp Ala
   405 410 415
   Lys Leu Asp Ile Val Tyr Val Pro Thr Gly Val Gly Thr Pro Asp Ile
               420                 425                 430
   Trp Gly Gly Asp Arg Thr Glu Leu Lys Glu Arg Tyr Ala Asn Ser Met
           435                 440                 445
   Leu Ala Ile Asn Ala Ser Thr Gly Lys Leu Val Trp Asn Phe Gln Thr
       450                 455                 460
   Thr His His Asp Leu Trp Asp Met Asp Val Pro Ser Gln Pro Ser Leu
   465                 470                 475                 480
   Ala Asp Ile Lys Asn Lys Ala Gly Gln Thr Val Pro Ala Ile Tyr Val
                   485                 490                 495
   Leu Thr Lys Thr Gly Asn Ala Phe Val Leu Asp Arg Arg Asn Gly Gln
               500                 505                 510
   Pro Ile Val Pro Val Thr Glu Lys Pro Val Pro Gln Thr Val Lys Arg
           515                 520                 525
   Gly Pro Gln Thr Lys Gly Glu Phe Tyr Ser Lys Thr Gln Pro Phe Ser
       530                 535                 540
   Asp Leu Asn Leu Ala Pro Gln Asp Lys Leu Thr Asp Lys Asp Met Trp
   545                 550                 555                 560
   Gly Ala Thr Met Leu Asp Gln Leu Met Cys Arg Val Ser Phe Lys Arg
                   565                 570                 575
   Leu Asn Tyr Asp Gly Ile Tyr Thr Pro Pro Ser Glu Asn Gly Thr Leu
               580                 585                 590
   Val Phe Pro Gly Asn Leu Gly Val Phe Glu Trp Gly Gly Met Ser Val
           595                 600                 605
   Asn Pro Asp Arg Gln Val Ala Val Met Asn Pro Ile Gly Leu Pro Phe
       610                 615                 620
   Val Ser Arg Leu Ile Pro Ala Asp Pro Asn Arg Ala Gln Thr Ala Lys
   625                 630                 635                 640
   Gly Ala Gly Thr Glu Gln Gly Val Gln Pro Met Tyr Gly Val Pro Tyr
                   645                 650                 655
   Gly Val Glu Ile Ser Ala Phe Leu Ser Pro Leu Gly Leu Pro Cys Lys
               660                 665                 670
   Gln Pro Ala Trp Gly Tyr Val Ala Gly Val Asp Leu Lys Thr His Glu
   675 680 685
   Val Val Trp Lys Lys Arg Ile Gly Thr Ile Arg Asp Ser Leu Pro Asn
   690 695 700
   Leu Phe Gln Leu Pro Ala Val Lys Ile Gly Val Pro Gly Leu Gly Gly
   705 710 715 720
   Ser Ile Ser Thr Ala Gly Asn Val Met Phe Val Gly Ala Thr Gln Asp
   725 730 735
   Asn Tyr Leu Arg Ala Phe Asn Val Thr Asn Gly Lys Lys Leu Trp Glu
   740 745 750
   Ala Arg Leu Pro Ala Gly Gly Gln Ala Thr Pro Met Thr Tyr Glu Ile
   755 760 765
   Asn Gly Lys Gln Tyr Val Val Ile Met Ala Gly Gly His Gly Ser Phe
   770 775 780
   Gly Thr Lys Met Gly Asp Tyr Leu Val Ala Tyr Ala Leu Pro Asp Asn
   785 790 795 800
   Lys
<210> 31
   <211> 1437
   <212> DNA
   <213> Acinetobacter calcoaceticus
<400> 31
   atgaataaac atttattggc taaaattgct ttattaagcg ctgttcagct agttacactc 60
   tcagcatttg ctgatgttcc tctaactcca tctcaatttg ctaaagcgaa atcagagaac 120
   tttgacaaga aagttattct atctaatcta aataagccgc atgctttgtt atggggacca 180
   gataatcaaa tttggttaac tgagcgagca acaggtaaga ttctaagagt taatccagag 240
   tcgggtagtg taaaaacagt ttttcaggta ccagagattg tcaatgatgc tgatgggcag 300
   aatggtttat taggttttgc cttccatcct gattttaaaa ataatcctta tatctatatt 360
   tcaggtacat ttaaaaatcc gaaatctaca gataaagaat taccgaacca aacgattatt 420
   cgtcgttata cctataataa atcaacagat acgctcgaga agccagtcga tttattagca 480
   ggattacctt catcaaaaga ccatcagtca ggtcgtcttg tcattgggcc agatcaaaag 540
   atttattata cgattggtga ccaagggcgt aaccagcttg cttatttgtt cttgccaaat 600
   caagcacaac atacgccaac tcaacaagaa ctgaatggta aagactatca cacctatatg 660
   ggtaaagtac tacgcttaaa tcttgatgga agtattccaa aggataatcc aagttttaac 720
   ggggtggtta gccatattta tacacttgga catcgtaatc cgcagggctt agcattcact 780
   ccaaatggta aattattgca gtctgaacaa ggcccaaact ctgacgatga aattaacctc 840
   attgtcaaag gtggcaatta tggttggccg aatgtagcag gttataaaga tgatagtggc 900
   tatgcttatg caaattattc agcagcagcc aataagtcaa ttaaggattt agctcaaaat 960
   ggagtaaaag tagccgcagg ggtccctgtg acgaaagaat ctgaatggac tggtaaaaac 1020
   tttgtcccac cattaaaaac tttatatacc gttcaagata cctacaacta taacgatcca 1080
   acttgtggag agatgaccta catttgctgg ccaacagttg caccgtcatc tgcctatgtc 1140
   tataagggcg gtaaaaaagc aattactggt tgggaaaata cattattggt tccatcttta 1200
   aaacgtggtg tcattttccg tattaagtta gatccaactt atagcactac ttatgatgac 1260
   gctgtaccga tgtttaagag caacaaccgt tatcgtgatg tgattgcaag tccagatggg 1320
   aatgtcttat atgtattaac tgatactgcc ggaaatgtcc aaaaagatga tggctcagta 1380
   acaaatacat tagaaaaccc aggatctctc attaagttca cctataaggc taagtaa 1437
<210> 32
   <211> 478
   <212> PRT
   <213> Acinetobacter calcoaceticus
<400> 32
   Met Asn Lys His Leu Leu Ala Lys Ile Ala Leu Leu Ser Ala Val Gln
   1               5                   10                  15
   Leu Val Thr Leu Ser Ala Phe Ala Asp Val Pro Leu Thr Pro Ser Gln
               20                  25                  30
   Phe Ala Lys Ala Lys Ser Glu Asn Phe Asp Lys Lys Val Ile Leu Ser
           35                  40                  45
   Asn Leu Asn Lys Pro His Ala Leu Leu Trp Gly Pro Asp Asn Gln Ile
       50                  55                  60
   Trp Leu Thr Glu Arg Ala Thr Gly Lys Ile Leu Arg Val Asn Pro Glu
   65                  70                  75                  80
   Ser Gly Ser Val Lys Thr Val Phe Gln Val Pro Glu Ile Val Asn Asp
                   85                  90                  95
   Ala Asp Gly Gln Asn Gly Leu Leu Gly Phe Ala Phe His Pro Asp Phe
               100                 105                 110
   Lys Asn Asn Pro Tyr Ile Tyr Ile Ser Gly Thr Phe Lys Asn Pro Lys
           115                 120                 125
   Ser Thr Asp Lys Glu Leu Pro Asn Gln Thr Ile Ile Arg Arg Tyr Thr
   130 135 140
   Tyr Asn Lys Ser Thr Asp Thr Leu Glu Lys Pro Val Asp Leu Leu Ala
   145 150 155 160
   Gly Leu Pro Ser Ser Lys Asp His Gln Ser Gly Arg Leu Val Ile Gly
   165 170 175
   Pro Asp Gln Lys Ile Tyr Tyr Thr Ile Gly Asp Gln Gly Arg Asn Gln
   180 185 190
   Leu Ala Tyr Leu Phe Leu Pro Asn Gln Ala Gln His Thr Pro Thr Gln
   195 200 205
   Gln Glu Leu Asn Gly Lys Asp Tyr His Thr Tyr Met Gly Lys Val Leu
   210 215 220
   Arg Leu Asn Leu Asp Gly Ser Ile Pro Lys Asp Asn Pro Ser Phe Asn
   225 230 235 240
   Gly Val Val Ser His Ile Tyr Thr Leu Gly His Arg Asn Pro Gln Gly
   245 250 255
   Leu Ala Phe Thr Pro Asn Gly Lys Leu Leu Gln Ser Glu Gln Gly Pro
   260 265 270
   Asn Ser Asp Asp Glu Ile Asn Leu Ile Val Lys Gly Gly Asn Tyr Gly
   275 280 285
   Trp Pro Asn Val Ala Gly Tyr Lys Asp Asp Ser Gly Tyr Ala Tyr Ala
   290 295 300
   Asn Tyr Ser Ala Ala Ala Asn Lys Ser Ile Lys Asp Leu Ala Gln Asn
   305 310 315 320
   Gly Val Lys Val Ala Ala Gly Val Pro Val Thr Lys Glu Ser Glu Trp
   325 330 335
   Thr Gly Lys Asn Phe Val Pro Pro Leu Lys Thr Leu Tyr Thr Val Gln
   340 345 350
   Asp Thr Tyr Asn Tyr Asn Asp Pro Thr Cys Gly Glu Met Thr Tyr Ile
   355 360 365
   Cys Trp Pro Thr Val Ala Pro Ser Ser Ala Tyr Val Tyr Lys Gly Gly
   370 375 380
   Lys Lys Ala Ile Thr Gly Trp Glu Asn Thr Leu Leu Val Pro Ser Leu
   385 390 395 400
   Lys Arg Gly Val Ile Phe Arg Ile Lys Leu Asp Pro Thr Tyr Ser Thr
   405 410 415
   Thr Tyr Asp Asp Ala Val Pro Met Phe Lys Ser Asn Asn Arg Tyr Arg
   420 425 430
   Asp Val Ile Ala Ser Pro Asp Gly Asn Val Leu Tyr Val Leu Thr Asp
   435 440 445
   Thr Ala Gly Asn Val Gln Lys Asp Asp Gly Ser Val Thr Asn Thr Leu
   450 455 460
   Glu Asn Pro Gly Ser Leu Ile Lys Phe Thr Tyr Lys Ala Lys
   465 470 475
<210> 33
   <211> 1059
   <212> DNA
   <213> Vibrio parahaemolyticus
<400> 33
   ttggctcttg gcgcgattgg agcagtgctc tcaacttccg tatacgcttg gcaagccgag 60
   cttgttgcgt ctggcctcaa ggtaccatgg gggatgagct ctattgacga caaccaattg 120
   cttgtcacac aacgccacgg cgaaataggt gtggttaacc ttgatgatgg cttctatcgt 180
   actgtcgcta caccggcaca ggtgagtgct gttggtcagg gaggcttgct tgatgttgcc 240
   aagtctcctt tcgaagacaa tacgttctat tttacctatt caaagcaagt ggaagatgca 300
   tacgaaacgg cattagcttc cgctcaatat caacaaggta agctgattgg atggaaagag 360
   cttctcgtga ctcagtctgg atctgatacg ggcaggcatt ttggtagccg aatcaccttt 420
   gacgaacgct acgtgtatat gtctgtcggc gatagaggcg ttcgcgacaa cgggcaaaat 480
   cagagaacac acgctggctc gatcctgcgc ctattaccaa acgggctagc cccagaagac 540
   aacccgttta ccaaagtaaa ctcagcgtta aacgagatat ggagctatgg tcaccgcaat 600
   cctcaaggac tcttttacga ccgcagcact caacagctat ggtctatcga gcatggtccg 660
   cgcggcggag atgaaatcaa cttgattgtc aaaggagaaa actatggctg gcctgttacc 720
   tcccacggca aagagtattg ggggccaatc tcagttggtg aagccaaaac cctgccgggt 780
   attcaaacac cgaagaaagt gtatgtgcct tctatcgcac ctagctctct cattctttac 840
   agaggggata agtacccatc cctaaatggc aaactgatat ctggcgcatt gaagctcacc 900
   cacctcaatg tcgtcacact cgatgagcaa gcaaatattg ttgctgaaga gcggctacta 960
   gaatccttag gagagcgaat acgagatctg gaagtaacac cagtaggaga gattctgttt 1020
   agcactgaca gtgggaatat ctaccgtctc gtggagtaa 1059
<210> 34
   <211> 352
   <212> PRT
   <213> Vibrio parahaemolyticus
<400> 34
   Met Ala Leu Gly Ala Ile Gly Ala Val Leu Ser Thr Ser Val Tyr Ala
   1 5 10 15
   Trp Gln Ala Glu Leu Val Ala Ser Gly Leu Lys Val Pro Trp Gly Met
   20 25 30
   Ser Ser Ile Asp Asp Asn Gln Leu Leu Val Thr Gln Arg His Gly Glu
   35 40 45
   Ile Gly Val Val Asn Leu Asp Asp Gly Phe Tyr Arg Thr Val Ala Thr
   50 55 60
   Pro Ala Gln Val Ser Ala Val Gly Gln Gly Gly Leu Leu Asp Val Ala
   65 70 75 80
   Lys Ser Pro Phe Glu Asp Asn Thr Phe Tyr Phe Thr Tyr Ser Lys Gln
   85 90 95
   Val Glu Asp Ala Tyr Glu Thr Ala Leu Ala Ser Ala Gln Tyr Gln Gln
   100 105 110
   Gly Lys Leu Ile Gly Trp Lys Glu Leu Leu Val Thr Gln Ser Gly Ser
   115 120 125
   Asp Thr Gly Arg His Phe Gly Ser Arg Ile Thr Phe Asp Glu Arg Tyr
   130 135 140
   Val Tyr Met Ser Val Gly Asp Arg Gly Val Arg Asp Asn Gly Gln Asn
   145 150 155 160
   Gln Arg Thr His Ala Gly Ser Ile Leu Arg Leu Leu Pro Asn Gly Leu
   165 170 175
   Ala Pro Glu Asp Asn Pro Phe Thr Lys Val Asn Ser Ala Leu Asn Glu
   180 185 190
   Ile Trp Ser Tyr Gly His Arg Asn Pro Gln Gly Leu Phe Tyr Asp Arg
   195 200 205
   Ser Thr Gln Gln Leu Trp Ser Ile Glu His Gly Pro Arg Gly Gly Asp
   210 215 220
   Glu Ile Asn Leu Ile Val Lys Gly Glu Asn Tyr Gly Trp Pro Val Thr
   225 230 235 240
   Ser His Gly Lys Glu Tyr Trp Gly Pro Ile Ser Val Gly Glu Ala Lys
                   245                 250                 255
   Thr Leu Pro Gly Ile Gln Thr Pro Lys Lys Val Tyr Val Pro Ser Ile
               260                 265                 270
   Ala Pro Ser Ser Leu Ile Leu Tyr Arg Gly Asp Lys Tyr Pro Ser Leu
           275                 280                 285
   Asn Gly Lys Leu Ile Ser Gly Ala Leu Lys Leu Thr His Leu Asn Val
       290                 295                 300
   Val Thr Leu Asp Glu Gln Ala Asn Ile Val Ala Glu Glu Arg Leu Leu
   305                 310                 315                 320
   Glu Ser Leu Gly Glu Arg Ile Arg Asp Leu Glu Val Thr Pro Val Gly
                   325                 330                 335
   Glu Ile Leu Phe Ser Thr Asp Ser Gly Asn Ile Tyr Arg Leu Val Glu
               340                 345                 350
<210> 35
   <211> 2339
   <212> DNA
   <213> Rhizobium leguminosarum
<400> 35
   tcacggcaac gtataggcga tcacataatc gccgggcgtg gtgccgaccg agccgtggcc     60
   gccggcgacc atgacgacat attgcttgtt gtcatcggtc atataggtca tcggcgtcgc    120
   ctggccgccg gcgggaagcc gtccctgcca gagctcccgg ccactcgtca cgtcataggc    180
   gcgcaggtag ttgtcgaccg cggcccccag gaaggcgacg ccgcccttgg tcagcatcgg    240
   cccgccgatg ccgggcacgc ccaccttgaa gggcaggggc aatggcgtca tgtcgtggac    300
   ggtgccgttc ttgtgcatat aggcgatctt gccggtgcgc aggtcgacgc cggcgacata    360
   accccatggc ggcgcctggc aggggatttg gagcagcccg aggaagggac ccatgaagac    420
   gccgtagggc gcgccgtcat tgcggttgag cccctgttcg ctgcccttct cgtcctggcc    480
   tctcggagga atgtcggcgg ccggcaccag gcgcgaggta aaggcgagat aggtcggcat    540
   gccgaacatg atctgccgct ccggatccac cgccaccgag ccccagttga aggtgccgaa    600
   attgccggga tagacgatcg tccctttcag cgaaggcggc gtaaagcggc cctcatagtg    660
   gtagcggtgg aagtcgatgc ggcaggccat ctgatcgaac agcgatacgc cccacatgtc    720
   tctttcctgg agcggctttg gcgagaaggt gaggtccgag atcggctgcg tcggcgaggt    780
   atgatcgccc gagaccgcgc cgcctggcgc tggtatttcc ttgatcggga tgatcggctc    840
   gccgctgcgc cggtcgagca catagatatc gccctgtttg gtcgggccga ccaaggccgg    900
   aaccacggtg ccgtcttgct tcgtcaggtc gatcagcgcc ggctgggccg gcacgtccat      960
   gtcccagaga tcgtgatgca ctgtctggcg cacccagcgc aactggccgg tggcgatatc     1020
   gagcgcgacg atagaggagg agaatttctc gacattgtcg ctgcggccga tgccgatctg     1080
   gtcgggtact tggttgccga gcgggatgta gaccatgccg agcgcctcgt cgacgctgaa     1140
   gaccgaccaa ctgttcggcg agttggtcgt ataggtctgg ccctcggtga tcggcgtcgt     1200
   cacatcggga ttgccggaat cccaattcca gaccagggcg ccggtattga tgtcgaaggc     1260
   gcggatgacg ccggattgct cctcagtcga ataattgtcg ttcaccgccc cgccgacgat     1320
   gattttgccc gccaccgcga ccggcggcga agtggaataa tagtatccgg ccgggttgaa     1380
   ccgcatgccg gtttccaaat gcagcacgcc ctggtcggca aagctggtgc agaccttccc     1440
   gtccgccgca tcgagcgcga tcagccgggc gtctgaggtc ggcaggtaga cgcgctcggc     1500
   gcagggctgg ccggcggcaa cggttggatc ggcataatag gtgacgccgc ggcaggtctg     1560
   atgctgccgg tcggggttca tgcccgagtt ggcgtcgtat ttccatttct ccttgccggt     1620
   cttggcgtcg agcgcgatcg cccagttatg cggcgtgcag agatagagcg tgtccttcac     1680
   cttcagcggc gtcacctgat aggtcgtctc gccgacatcg tccggccgct tgacgtcgcc     1740
   ggtctggtat cgccacgctt ccttgagggt ggagacgttt tcggcggtga tctggtcgag     1800
   tggcgaatag cgctggccga agggcgtgcg gccgtattga tgccattcgc cgtccgggac     1860
   gctgccgccg aaggcgggat tggcggcgac cgtatccttg ggcagctcgc cggcgagatc     1920
   atgggggtca gtcgtcatcg aatagagggc gacaaggatg gcgagaatga caggcgcggc     1980
   gagcggccag ggtttgcggg cataggtgat gcccgttggg ctgcgcaggc caagcggccg     2040
   gcggatccat ggcgtcagca gccagagccc gagcaggatg atcatgccgc cgcggggccc     2100
   aagctgccac cagtcgaagc cgacctccca aatcgcccag gcgagtgctg cgacgacgag     2160
   caccgcatga cccagagcgc caccgccttg cgcatcagca gcagcccggc ggtaatcagg     2220
   aacatcagcc cggcgaacag atagaagacg ctgccgccga gcgtgacgag ccagagcccg     2280
   ccgccgctga gagtgagccc gacgatgatg aagaagatgg aggtgacgat gatcgccat      2339
<210> 36
   <211> 779
   <212> PRT
   <213> Rhizobium leguminosarum
<400> 36
   Met Ala Ile Ile Val Thr Ser Ile Phe Phe Ile Ile Val Gly Leu Thr
   1               5                   10                  15
   Leu Ser Gly Gly Gly Leu Trp Leu Val Thr Leu Gly Gly Ser Val Phe
               20                  25                  30
   Tyr Leu Phe Ala Gly Leu Met Phe Leu Ile Thr Ala Gly Leu Leu Leu
   35 40 45
   Met Arg Lys Ala Val Ala Leu Trp Val Tyr Ala Val Leu Val Val Ala
   50 55 60
   Ala Leu Ala Trp Ala Ile Trp Glu Val Gly Phe Asp Trp Trp Gln Leu
   65 70 75 80
   Gly Pro Arg Gly Gly Met Ile Ile Leu Leu Gly Leu Trp Leu Leu Thr
   85 90 95
   Pro Trp Ile Arg Arg Pro Leu Gly Leu Arg Ser Pro Thr Gly Ile Thr
   100 105 110
   Tyr Ala Ala Asn Pro Trp Pro Leu Ala Ala Pro Val Ile Leu Ala Ile
   115 120 125
   Leu Val Ala Leu Tyr Ser Met Thr Thr Asp Pro His Asp Leu Ala Gly
   130 135 140
   Glu Leu Pro Lys Asp Thr Val Ala Ala Asn Pro Ala Phe Gly Gly Ser
   145 150 155 160
   Val Pro Asp Gly Glu Trp His Gln Tyr Gly Arg Thr Pro Phe Gly Gln
   165 170 175
   Arg Tyr Ser Pro Leu Asp Gln Ile Thr Ala Glu Asn Val Ser Thr Leu
   180 185 190
   Lys Glu Ala Trp Arg Tyr Gln Thr Gly Asp Val Lys Arg Pro Asp Asp
   195 200 205
   Val Gly Glu Thr Thr Tyr Gln Val Thr Pro Leu Lys Val Lys Asp Thr
   210 215 220
   Leu Tyr Leu Cys Thr Pro His Asn Trp Ala Ile Ala Leu Asp Ala Lys
   225 230 235 240
   Thr Gly Lys Glu Lys Trp Lys Tyr Asp Ala Asn Ser Gly Met Asn Pro
   245 250 255
   Asp Arg Gln His Gln Thr Cys Arg Gly Val Thr Tyr Tyr Ala Asp Pro
   260 265 270
   Thr Val Ala Ala Gly Gln Pro Cys Ala Glu Arg Val Tyr Leu Pro Thr
   275 280 285
   Ser Asp Ala Arg Leu Ile Ala Leu Asp Ala Ala Asp Gly Lys Val Cys
   290 295 300
   Thr Ser Phe Ala Asp Gln Gly Val Leu His Leu Glu Thr Gly Met Arg
   305 310 315 320
   Phe Asn Pro Ala Gly Tyr Tyr Tyr Ser Thr Ser Pro Pro Val Ala Val
   325 330 335
   Ala Gly Lys Ile Ile Val Gly Gly Ala Val Asn Asp Asn Tyr Ser Thr
   340 345 350
   Glu Glu Gln Ser Gly Val Ile Arg Ala Phe Asp Ile Asn Thr Gly Ala
   355 360 365
   Leu Val Trp Asn Trp Asp Ser Gly Asn Pro Asp Val Thr Thr Pro Ile
   370 375 380
   Thr Glu Gly Gln Thr Tyr Thr Thr Asn Ser Pro Asn Ser Trp Ser Val
   385 390 395 400
   Phe Ser Val Asp Glu Ala Leu Gly Met Val Tyr Ile Pro Leu Gly Asn
   405 410 415
   Gln Val Pro Asp Gln Ile Gly Ile Gly Arg Ser Asp Asn Val Glu Lys
   420 425 430
   Phe Ser Ser Ser Ile Val Ala Leu Asp Ile Ala Thr Gly Gln Leu Arg
   435 440 445
   Trp Val Arg Gln Thr Val His His Asp Leu Trp Asp Met Asp Val Pro
   450 455 460
   Ala Gln Pro Ala Leu Ile Asp Leu Thr Lys Gln Asp Gly Thr Val Val
   465 470 475 480
   Pro Ala Leu Val Gly Pro Thr Lys Gln Gly Asp Ile Tyr Val Leu Asp
   485 490 495
   Arg Arg Ser Gly Glu Pro Ile Ile Pro Ile Lys Glu Ile Pro Ala Pro
   500 505 510
   Gly Gly Ala Val Ser Gly Asp His Thr Ser Pro Thr Gln Pro Ile Ser
   515 520 525
   Asp Leu Thr Phe Ser Pro Lys Pro Leu Gln Glu Arg Asp Met Trp Gly
   530 535 540
   Val Ser Leu Phe Asp Gln Met Ala Cys Arg Ile Asp Phe His Arg Tyr
   545 550 555 560
   His Tyr Glu Gly Arg Phe Thr Pro Pro Ser Leu Lys Gly Thr Ile Val
   565 570 575
   Tyr Pro Gly Asn Phe Gly Thr Phe Asn Trp Gly Ser Val Ala Val Asp
   580 585 590
   Pro Glu Arg Gln Ile Met Phe Gly Met Pro Thr Tyr Leu Ala Phe Thr
   595 600 605
   Ser Arg Leu Val Pro Ala Ala Asp Ile Pro Pro Arg Gly Gln Asp Glu
   610 615 620
   Lys Gly Ser Glu Gln Gly Leu Asn Arg Asn Asp Gly Ala Pro Tyr Gly
   625 630 635 640
   Val Phe Met Gly Pro Phe Leu Gly Leu Leu Gln Ile Pro Cys Gln Ala
   645 650 655
   Pro Pro Trp Gly Tyr Val Ala Gly Val Asp Leu Arg Thr Gly Lys Ile
   660 665 670
   Ala Tyr Met His Lys Asn Gly Thr Val His Asp Met Thr Pro Leu Pro
   675 680 685
   Leu Pro Phe Lys Val Gly Val Pro Gly Ile Gly Gly Pro Met Leu Thr
   690 695 700
   Lys Gly Gly Val Ala Phe Leu Gly Ala Ala Val Asp Asn Tyr Leu Arg
   705 710 715 720
   Ala Tyr Asp Val Thr Ser Gly Arg Glu Leu Trp Gln Gly Arg Leu Pro
   725 730 735
   Ala Gly Gly Gln Ala Thr Pro Met Thr Tyr Met Thr Asp Asp Asn Lys
   740 745 750
   Gln Tyr Val Val Met Val Ala Gly Gly His Gly Ser Val Gly Thr Thr
   755 760 765
   Pro Gly Asp Tyr Val Ile Ala Tyr Thr Leu Pro
   770 775
<210> 37
   <211> 2244
   <212> DNA
   <213> Salmonella enterica
<400> 37
   ttatttcgcg tcgtcaggca aagcataggc cacgatatag tcgcccatct tcgtaccaaa 60
   cgaaccgtgg cctccggcag aaacgaccac gtactgctta ccgtttacct cataggtcat 120
   cggcgttgcc tggcctccgg cgggtaaacg tccctgccac agtttttccc cgttgctcat 180
   gttataagcg cgcaggtagt tgtcggcggt cgcggcaatg aacagcacat taccggcggt 240
   tgagattggc ccgcccagca ttggcatccc catgttgaac ggcaccggaa ccggcatcgg 300
   gaacggcata ctgtcgcgcg gcgtccctat acgtttcttc cacacgattt cattggtctt 360
   cagatccaac gcggaaatat agccccaggc cggttgttta cacggcaggc caaacggcga 420
   aaggaacgga ttcagcgtga cgccgaatgg tacgccgtac tgtggctgaa tacccgcttc 480
   tgtaccagta cctttcgcgt ctttcggcgg ctccatcgga ttacccgggc cgcgtgggat 540
   cagcttcgag acaaacggca gcgccatcgg gttagcgata gcgacctgac ggtctggatc 600
   gacggaaata ccgccccatt caaacatccc caggttgcca gggaatacca atgtcccctg 660
   ctcggacggc ggggtgaaga taccttcata gcgcaactgg tggaacatga cgcggcacac 720
   cagttggtcg aacatggtcg cgccccacat atccgcgccg gagagatctt ttttcgggcg 780
   gaaggttaaa tcagagaacg gctgagtttt ggcgacgtaa tcgccttttg ccgcgccctg 840
   cgggaccggt tttccggggg cgggcacgac cagctcgcca ttacgtctgt ccagcacaaa 900
   gatattaccg gttttcgctg gcgcatagat aaccggaacg gtagtaccgt caacggtaat 960
   atccgccagc gtcggctgtg ccggcaggtc catatcccac agatcgtgat ggacggtctg 1020
   ataactccag gccagtttcc cggtggtggc attcagcgcc aggatggagc tggcataacg 1080
   ttcctgctcc ggcgtgcggt taccgcccca gatatccggc gtggtcacgc ccatcggcag 1140
   atagaccaga tccagtttcg catcataggc ggcaggcgcc caggagttcg gcgagttaaa 1200
   ggtaaaagcg tgctcatcgg ctggaatcgc gttcggatct ttcgcccccg gatcgaaggc 1260
   ccacatgagt ttaccgctat taacatcaaa accacggata acgccggacg tttcgcgggt 1320
   agagaagtta tccgtgaccg aaccggcaat gacaattgtt ttatcggtga taatcggcgg 1380
   cgacgtcggt tcatacagtc ccggcgtggt atccggcata ttggtttgca gattcaggac 1440
   gcctttgttg gcgaaggttt cacacagctt gccggtttcg gcattcaccg cgaaaaggcg 1500
   accgtcgttg accgggagga taatacggcg cggacagtcg gcgatgacct ccggcgaggc 1560
   ggtatccgct ttggcctcat gataagagac gccgcggcag gtcacatgct ggaaagacga 1620
   atcggtcttc aactgaggat caaaatgcca cttctcttta ccgctggcgg cgtccagcgc 1680
   gaacagacgc tggtgagctg tacacagata aagcgtatcg ccgactttaa tcggggtcac 1740
   ttcgttggtg atttcgcccg gatcgttggg ttgtttcaga tcgccggtgc ggaagaccca 1800
   cgcttctttc agttggtgaa cgttatccgc agtgatctgt ttcagcggcg aatagcgttg 1860
   gccttcctga ttacgtccat aggccggcca gtcttcgtcg gcgatcgacg agcttgttgc 1920
   ggcgggcgtg gcgtcggcgc gaagcgtacc gttgatctcc tgcgggtcgt tgaagccagc 1980
   ccaggtcaga atgccgccgc taatcagcag cgccacgacc agcgccgcca ctgcgccgct      2040
   ggaaggaacc accagccgat gccagacaaa cggcaatatt agccagatgc cgaaaaacac      2100
   cagaatatcg ctacgcggcg tgagcgccca gaagtcgaac ccgacttccc acacgcccca      2160
   aatcattgtc gccagcagca gggcggcata cagccacagt gcggcacgtt tactgcgcca      2220
   tagcaatccg gccacaacca gcat                                            2244
<210> 38
   <211> 747
   <212> PRT
   <213> Salmonella enterica
<400> 38
   Met Leu Val Val Ala Gly Leu Leu Trp Arg Ser Lys Arg Ala Ala Leu
   1               5                   10                  15
   Trp Leu Tyr Ala Ala Leu Leu Leu Ala Thr Met Ile Trp Gly Val Trp
               20                  25                  30
   Glu Val Gly Phe Asp Phe Trp Ala Leu Thr Pro Arg Ser Asp Ile Leu
           35                  40                  45
   Val Phe Phe Gly Ile Trp Leu Ile Leu Pro Phe Val Trp His Arg Leu
       50                  55                  60
   Val Val Pro Ser Ser Gly Ala Val Ala Ala Leu Val Val Ala Leu Leu
   65                  70                  75                  80
   Ile Ser Gly Gly Ile Leu Thr Trp Ala Gly Phe Asn Asp Pro Gln Glu
                   85                  90                  95
   Ile Asn Gly Thr Leu Arg Ala Asp Ala Thr Pro Ala Ala Thr Ser Ser
               100                 105                 110
   Ser Ile Ala Asp Glu Asp Trp Pro Ala Tyr Gly Arg Asn Gln Glu Gly
           115                 120                 125
   Gln Arg Tyr Ser Pro Leu Lys Gln Ile Thr Ala Asp Asn Val His Gln
       130                 135                 140
   Leu Lys Glu Ala Trp Val Phe Arg Thr Gly Asp Leu Lys Gln Pro Asn
   145                 150                 155                 160
   Asp Pro Gly Glu Ile Thr Asn Glu Val Thr Pro Ile Lys Val Gly Asp
                   165                 170                 175
   Thr Leu Tyr Leu Cys Thr Ala His Gln Arg Leu Phe Ala Leu Asp Ala
               180                 185                 190
   Ala Ser Gly Lys Glu Lys Trp His Phe Asp Pro Gln Leu Lys Thr Asp
   195 200 205
   Ser Ser Phe Gln His Val Thr Cys Arg Gly Val Ser Tyr His Glu Ala
   210 215 220
   Lys Ala Asp Thr Ala Ser Pro Glu Val Ile Ala Asp Cys Pro Arg Arg
   225 230 235 240
   Ile Ile Leu Pro Val Asn Asp Gly Arg Leu Phe Ala Val Asn Ala Glu
   245 250 255
   Thr Gly Lys Leu Cys Glu Thr Phe Ala Asn Lys Gly Val Leu Asn Leu
   260 265 270
   Gln Thr Asn Met Pro Asp Thr Thr Pro Gly Leu Tyr Glu Pro Thr Ser
   275 280 285
   Pro Pro Ile Ile Thr Asp Lys Thr Ile Val Ile Ala Gly Ser Val Thr
   290 295 300
   Asp Asn Phe Ser Thr Arg Glu Thr Ser Gly Val Ile Arg Gly Phe Asp
   305 310 315 320
   Val Asn Ser Gly Lys Leu Met Trp Ala Phe Asp Pro Gly Ala Lys Asp
   325 330 335
   Pro Asn Ala Ile Pro Ala Asp Glu His Ala Phe Thr Phe Asn Ser Pro
   340 345 350
   Asn Ser Trp Ala Pro Ala Ala Tyr Asp Ala Lys Leu Asp Leu Val Tyr
   355 360 365
   Leu Pro Met Gly Val Thr Thr Pro Asp Ile Trp Gly Gly Asn Arg Thr
   370 375 380
   Pro Glu Gln Glu Arg Tyr Ala Ser Ser Ile Leu Ala Leu Asn Ala Thr
   385 390 395 400
   Thr Gly Lys Leu Ala Trp Ser Tyr Gln Thr Val His His Asp Leu Trp
   405 410 415
   Asp Met Asp Leu Pro Ala Gln Pro Thr Leu Ala Asp Ile Thr Val Asp
   420 425 430
   Gly Thr Thr Val Pro Val Ile Tyr Ala Pro Ala Lys Thr Gly Asn Ile
   435 440 445
   Phe Val Leu Asp Arg Arg Asn Gly Glu Leu Val Val Pro Ala Pro Glu
       450                 455                 460
   Lys Pro Val Pro Gln Gly Ala Ala Lys Gly Asp Tyr Val Ala Lys Thr
   465                 470                 475                 480
   Gln Pro Phe Ser Asp Leu Thr Phe Arg Pro Lys Lys Asp Leu Ser Gly
                   485                 490                 495
   Ala Asp Met Trp Gly Ala Thr Met Phe Asp Gln Leu Val Cys Arg Val
               500                 505                 510
   Met Phe His Gln Leu Arg Tyr Glu Gly Ile Phe Thr Pro Pro Ser Glu
           515                 520                 525
   Gln Gly Thr Leu Val Phe Pro Gly Asn Leu Gly Met Phe Glu Trp Gly
       530                 535                 540
   Gly Ile Ser Val Asp Pro Asp Arg Gln Val Ala Ile Ala Asn Pro Met
   545                 550                 555                 560
   Ala Leu Pro Phe Val Ser Lys Leu Ile Pro Arg Gly Pro Gly Asn Pro
                   565                 570                 575
   Met Glu Pro Pro Lys Asp Ala Lys Gly Thr Gly Thr Glu Ala Gly Ile
               580                 585                 590
   Gln Pro Gln Tyr Gly Val Pro Phe Gly Val Thr Leu Asn Pro Phe Leu
           595                 600                 605
   Ser Pro Phe Gly Leu Pro Cys Lys Gln Pro Ala Trp Gly Tyr Ile Ser
       610                 615                 620
   Ala Leu Asp Leu Lys Thr Asn Glu Ile Val Trp Lys Lys Arg Ile Gly
   625                 630                 635                 640
   Thr Pro Arg Asp Ser Met Pro Phe Pro Met Pro Val Pro Val Pro Phe
                   645                 650                 655
   Asn Met Gly Met Pro Met Leu Gly Gly Pro Ile Ser Thr Ala Gly Asn
               660                 665                 670
   Val Leu Phe Ile Ala Ala Thr Ala Asp Asn Tyr Leu Arg Ala Tyr Asn
           675                 680                 685
   Met Ser Asn Gly Glu Lys Leu Trp Gln Gly Arg Leu Pro Ala Gly Gly
       690                 695                 700
   Gln Ala Thr Pro Met Thr Tyr Glu Val Asn Gly Lys Gln Tyr Val Val
   705 710 715 720
   Val Ser Ala Gly Gly His Gly Ser Phe Gly Thr Lys Met Gly Asp Tyr
   725 730 735
   Ile Val Ala Tyr Ala Leu Pro Asp Asp Ala Lys
   740 745
<210> 39
   <211> 2046
   <212> DNA
   <213> Solibacter usitatus
<400> 39
   atgatccgat cgctagcgtt cgccctcgcc tccgttcgac tgcttgccgc ctcggattgg      60
   ccggggtacg gtggcggccc cgcggggatt cgtcactcgc ccttgaagca gatcaatcgc     120
   tccaacgtgt cgcacctcaa ggtcgcgtgg acgtacgaca ccaaggatgg gccgggcgat     180
   ccacagacgc agccgatcgt ggtcgatggc gttctctatg gcgtaactcc gacccacaaa     240
   gccatcgcgc taaacggcgc caccggaaag ctgctgtgga ccttcgactc cggcattcgc     300
   ggacgtggtc ccaatcgctc ggtagtctgg tggtcgccgc gaacggagcg gcggctcttc     360
   gtcgcggtgc agagctttat ctacgcgctc gacccagcca ccgggaagcc catggcggat     420
   ttcggcgagc agggccgcat cgatctccgc cagggcctgg ggcgcgatgc cgccaagcag     480
   tcctacgtcc tgaccagccc gggaattatt tatcgcgatc tgttgatcgt gggcggccgc     540
   ctgccggaag cgctgccggc gccgcccggc gacatccgcg cctatgatgt ccgcaccggc     600
   aaacagcgct ggaccttcca cacgattccg catccgggcg agcccggcta cgagacctgg     660
   ccctccgatg cctggaccta ctccggcggc gccaacaact gggccggcat ggcgctcgac     720
   gagaagcgtg gcatcgtcta cgtccccacc ggctcagccg ccagcgattt ttatggcgcc     780
   gaccgcctgg gcgacaacct ctacgccaac tgcctgctgg cgctgaacgc cgccaccggc     840
   gagcgcgtct ggcacttcca ggcggtgcat catgacatct gggatcgtga ctttccctcc     900
   ccgcccgcgc tggtcaccgt ccggcacgaa ggccgcacgg tcgatgccgt tgtgcagacc     960
   tccaagcagg gctgggtcta cctgttcaat cgcgccaccg gcaagccgtt gttccccatc    1020
   gagagccggc cctatcccgt cagcgagctg ccgggcgaac gtgctgccgc cgaacagtcg    1080
   ctcccgacca ggccggccgc ctttgcgcgc caggctctga ctgaagacgt gctcacccgc    1140
   cgcaccgatg caacccacgc gtgggcgctt gccgagttcc acaagctgcg cagcgccgga    1200
   cagttcctgc ccttcggggc agggaatgag accgtcattt ttccaggctt cgatggcggc    1260
   gccgagtggg gcggtccggc cttcgatccc tccacgggat tgctgtacgt caacgccaat    1320
   gaaatggcct ggagggcggg cctcaaggaa tcccagccgg aaaactcagc ccggcaactg    1380
   tatgtggcgc agtgcggcac ctgccatggc gacgacctca agggctcgcc tccgcagttc    1440
   ccggcgttga acgaccttgc cggaaagcgc tcggcccagg aaatcggcgc aatcattcgc    1500
   gagggcggcg ggcgcatgcc tgctttcccc cggcttcgtc ccgacgacat caccgcgctc    1560
   agcgattatc tgctccgcgg ccgcagcaag gagctggaga gcaccgcctc actggatcgc    1620
   gaaccgcgat ttcgttttac cggctatcac aagttcgtcg atcccgatgg ctatccggca    1680
   atcgcgccgc cgtggggcac gctcaacgcc atcaacctca ataccggcga atacgcgtgg    1740
   cgcatcccgc tcggcgaata tcccgacctc gcggccaaaa acacgggatc ggaaaactac    1800
   ggcggcccca tcgtgaccgc cggcggcctc gtgtttatcg cggccaccaa ctatgaccgc    1860
   aagttccgag ccttcgacaa ggccaccggc gaactgctct ggcagactac gctcccgatg    1920
   gccggcaacg ccacacccat cacctatgaa atcgccggcc gccagtacgt cgtgatctac    1980
   gcgaccggcg gcaagtccgg caagtggggt ccttcgggcg gaatctacat ggcgttctca    2040
   ctgtaa                                                               2046
<210> 40
   <211> 681
   <212> PRT
   <213> Solibacter usitatus
<400> 40
   Met Ile Arg Ser Leu Ala Phe Ala Leu Ala Ser Val Arg Leu Leu Ala
   1 5 10 15
   Ala Ser Asp Trp Pro Gly Tyr Gly Gly Gly Pro Ala Gly Ile Arg His
   20 25 30
   Ser Pro Leu Lys Gln Ile Asn Arg Ser Asn Val Ser His Leu Lys Val
   35 40 45
   Ala Trp Thr Tyr Asp Thr Lys Asp Gly Pro Gly Asp Pro Gln Thr Gln
   50 55 60
   Pro Ile Val Val Asp Gly Val Leu Tyr Gly Val Thr Pro Thr His Lys
   65 70 75 80
   Ala Ile Ala Leu Asn Gly Ala Thr Gly Lys Leu Leu Trp Thr Phe Asp
   85 90 95
   Ser Gly Ile Arg Gly Arg Gly Pro Asn Arg Ser Val Val Trp Trp Ser
   100 105 110
   Pro Arg Thr Glu Arg Arg Leu Phe Val Ala Val Gln Ser Phe Ile Tyr
   115 120 125
   Ala Leu Asp Pro Ala Thr Gly Lys Pro Met Ala Asp Phe Gly Glu Gln
   130 135 140
   Gly Arg Ile Asp Leu Arg Gln Gly Leu Gly Arg Asp Ala Ala Lys Gln
   145 150 155 160
   Ser Tyr Val Leu Thr Ser Pro Gly Ile Ile Tyr Arg Asp Leu Leu Ile
   165 170 175
   Val Gly Gly Arg Leu Pro Glu Ala Leu Pro Ala Pro Pro Gly Asp Ile
   180 185 190
   Arg Ala Tyr Asp Val Arg Thr Gly Lys Gln Arg Trp Thr Phe His Thr
   195 200 205
   Ile Pro His Pro Gly Glu Pro Gly Tyr Glu Thr Trp Pro Ser Asp Ala
   210 215 220
   Trp Thr Tyr Ser Gly Gly Ala Asn Asn Trp Ala Gly Met Ala Leu Asp
   225 230 235 240
   Glu Lys Arg Gly Ile Val Tyr Val Pro Thr Gly Ser Ala Ala Ser Asp
   245 250 255
   Phe Tyr Gly Ala Asp Arg Leu Gly Asp Asn Leu Tyr Ala Asn Cys Leu
   260 265 270
   Leu Ala Leu Asn Ala Ala Thr Gly Glu Arg Val Trp His Phe Gln Ala
   275 280 285
   Val His His Asp Ile Trp Asp Arg Asp Phe Pro Ser Pro Pro Ala Leu
   290 295 300
   Val Thr Val Arg His Glu Gly Arg Thr Val Asp Ala Val Val Gln Thr
   305 310 315 320
   Ser Lys Gln Gly Trp Val Tyr Leu Phe Asn Arg Ala Thr Gly Lys Pro
   325 330 335
   Leu Phe Pro Ile Glu Ser Arg Pro Tyr Pro Val Ser Glu Leu Pro Gly
   340 345 350
   Glu Arg Ala Ala Ala Glu Gln Ser Leu Pro Thr Arg Pro Ala Ala Phe
   355 360 365
   Ala Arg Gln Ala Leu Thr Glu Asp Val Leu Thr Arg Arg Thr Asp Ala
   370 375 380
   Thr His Ala Trp Ala Leu Ala Glu Phe His Lys Leu Arg Ser Ala Gly
   385 390 395 400
   Gln Phe Leu Pro Phe Gly Ala Gly Asn Glu Thr Val Ile Phe Pro Gly
                   405                 410                 415
   Phe Asp Gly Gly Ala Glu Trp Gly Gly Pro Ala Phe Asp Pro Ser Thr
               420                 425                 430
   Gly Leu Leu Tyr Val Asn Ala Asn Glu Met Ala Trp Arg Ala Gly Leu
           435                 440                 445
   Lys Glu Ser Gln Pro Glu Asn Ser Ala Arg Gln Leu Tyr Val Ala Gln
       450                 455                 460
   Cys Gly Thr Cys His Gly Asp Asp Leu Lys Gly Ser Pro Pro Gln Phe
   465                 470                 475                 480
   Pro Ala Leu Asn Asp Leu Ala Gly Lys Arg Ser Ala Gln Glu Ile Gly
                   485                 490                 495
   Ala Ile Ile Arg Glu Gly Gly Gly Arg Met Pro Ala Phe Pro Arg Leu
               500                 505                 510
   Arg Pro Asp Asp Ile Thr Ala Leu Ser Asp Tyr Leu Leu Arg Gly Arg
           515                 520                 525
   Ser Lys Glu Leu Glu Ser Thr Ala Ser Leu Asp Arg Glu Pro Arg Phe
       530                 535                 540
   Arg Phe Thr Gly Tyr His Lys Phe Val Asp Pro Asp Gly Tyr Pro Ala
   545                 550                 555                 560
   Ile Ala Pro Pro Trp Gly Thr Leu Asn Ala Ile Asn Leu Asn Thr Gly
                   565                 570                 575
   Glu Tyr Ala Trp Arg Ile Pro Leu Gly Glu Tyr Pro Asp Leu Ala Ala
               580                 585                 590
   Lys Asn Thr Gly Ser Glu Asn Tyr Gly Gly Pro Ile Val Thr Ala Gly
           595                 600                 605
   Gly Leu Val Phe Ile Ala Ala Thr Asn Tyr Asp Arg Lys Phe Arg Ala
       610                 615                 620
   Phe Asp Lys Ala Thr Gly Glu Leu Leu Trp Gln Thr Thr Leu Pro Met
   625                 630                 635                 640
   Ala Gly Asn Ala Thr Pro Ile Thr Tyr Glu Ile Ala Gly Arg Gln Tyr
                   645                 650                 655
   Val Val Ile Tyr Ala Thr Gly Gly Lys Ser Gly Lys Trp Gly Pro Ser
   660 665 670
   Gly Gly Ile Tyr Met Ala Phe Ser Leu
   675 680
<210> 41
   <211> 2169
   <212> DNA
   <213> Klebsiella pneumoniae
<400> 41
   atggcaactg gcaacgcgcc gcgcggattc ccccggatcc tgcagtggct tctcgccgga 60
   ctgatgctca tcatcggtct ggctgtgggt attcttgggg caaaactggc cctcgtcggc 120
   ggtacactgt acttcgcgct gatgggcgtg gtcatggtca tcgcggcggt gttgattttc 180
   cgcaaccgcc gcggcggtat tctgttatac gccgtggcgt ttatcgcctc ggtgatctgg 240
   gcgattagtg acgctggctg gaactactgg ccgctcttct cgcgcctgtt tgcgctcggc 300
   gtactggcct tcctggccgc cctggtctgg cctttcctcg ccagcccacc ggcgaaaaaa 360
   ggcccggcct atggcgtcgc cgccgttctc gccgtggcgc tggcggtgag ctttggctgg 420
   atgttcaaat ccgcaccgct ggtcagcgcg actgaagcgg taccagtcaa gcccgtcgcg 480
   ccaggcgaac agcagaaaaa ctgggcgcac tggggtaaca ccacccacgg cgaccgtttc 540
   gccgccctcg accaaatcaa caaacaaaac gtcaaccagc tgcaggttgc ctgggtagcg 600
   catactggcg atatcccaca gagcaacggc tcgggcgcag aagatcagaa caccccgctg 660
   cagattggcg atactctcta tgtctgtacc ccttacagta aagtgctggc gctggatgtg 720
   gacagcggaa aagagaaatg gcgctatgac tccaaatcct cttcacccaa ctggcagcgc 780
   tgccgcggct tagggtatta cgaagatagc caggcgcaaa ccgcgctagc gtcaggcacg 840
   cagccggccg cctgttcccg tcgtctgttc ctgccgacca tcgacgcccg cctgatcgcc 900
   attgatgccg ataccggcaa actgtgcgaa aacttcggcg atggcggtat tgtcgacctc 960
   agcgtcggca tgggtgaagt gaaagcgggt tactatcagc agacctccac cccgctggtg 1020
   gcgggcaatg tggtggtggt gggcggccgc gtcgcggata actactccac tggcgaaccg 1080
   ccgggcgtgg tccgcgcgtt tgatgtccac accggcaaac tggcatgggc gtgggatccg 1140
   ggcaatccag cgctgaccgg tgttccgccg gaaggccaga cctacacgcg cggtacgcca 1200
   aacgtctggt cggcgatgtc ctacgacgcg aagctgaatc tgatctatct gcccaccggc 1260
   aacgccacgc cagatttctt tggcggcgaa cgtaccgcgc tggacgataa atacagctcc 1320
   tctatcgtgg ctgtggatgc caccaccggc caggtgcgct ggcacttcca gaccacacac 1380
   cacgatcggt gggactttga cctgccgtct cagccgctgc tgtacgatct gcctgacggc 1440
   aagggcggca ccaccccggt gctggtgcag accagcaagc agggcatgat ctttatgctc 1500
   aaccgcgaga ccggcgagcc ggtggccaaa gtggaagagc gtccggtgcc ggcgggcaat 1560
   gtcaaaggtg aacgctactc gccgacgcaa ccttactcgg tagggatgcc gatgatcggc 1620
   aaccagacgt tgaccgagtc cgatatgtgg ggagcgacac cgatcgacct gctgctgtgc 1680
   cgtattcagt tcaaagagat gcgccatcag ggcgtcttta ccccgccagg tgaagaccgt 1740
   tccctgcagt tcccgggctc gctcggcggc atgaactggg gcagtgtttc gctggatcca 1800
   aacaacagcc tgatgtttgt caacgatatg cgtctgggcc tggccaacta tatggtgccg 1860
   cgcgcgaagg tggcgaaaga cgccagcggg atcgagatgg gcatcgtgcc gatggagggc 1920
   acgccgtttg gcgcgatacg cgaacgtttc ctgtcgccgc tgggcattcc gtgccagaag 1980
   ccgccgttcg gcaccatgtc cgcggtagat ctgaaaaccg ggaaactggt gtggcaggtg 2040
   ccggttggca ccgtagaaga caccgggccg ctgggcatac gtatgcacat gccaattcca 2100
   atcggcatgc cgacccttgg cgcatcgcta gcaacgcagt ccggactgct gttcttcgcc 2160
   ggcacctag 2169
<210> 42
   <211> 722
   <212> PRT
   <213> Klebsiella pneumoniae
<400> 42
   Met Ala Thr Gly Asn Ala Pro Arg Gly Phe Pro Arg Ile Leu Gln Trp
   1               5                   10                  15
   Leu Leu Ala Gly Leu Met Leu Ile Ile Gly Leu Ala Val Gly Ile Leu
               20                  25                  30
   Gly Ala Lys Leu Ala Leu Val Gly Gly Thr Leu Tyr Phe Ala Leu Met
           35                  40                  45
   Gly Val Val Met Val Ile Ala Ala Val Leu Ile Phe Arg Asn Arg Arg
       50                  55                  60
   Gly Gly Ile Leu Leu Tyr Ala Val Ala Phe Ile Ala Ser Val Ile Trp
   65                  70                  75                  80
   Ala Ile Ser Asp Ala Gly Trp Asn Tyr Trp Pro Leu Phe Ser Arg Leu
                   85                  90                  95
   Phe Ala Leu Gly Val Leu Ala Phe Leu Ala Ala Leu Val Trp Pro Phe
               100                 105                 110
   Leu Ala Ser Pro Pro Ala Lys Lys Gly Pro Ala Tyr Gly Val Ala Ala
           115                 120                 125
   Val Leu Ala Val Ala Leu Ala Val Ser Phe Gly Trp Met Phe Lys Ser
       130                 135                 140
   Ala Pro Leu Val Ser Ala Thr Glu Ala Val Pro Val Lys Pro Val Ala
   145                 150                 155                 160
   Pro Gly Glu Gln Gln Lys Asn Trp Ala His Trp Gly Asn Thr Thr His
                   165                 170                 175
   Gly Asp Arg Phe Ala Ala Leu Asp Gln Ile Asn Lys Gln Asn Val Asn
               180                 185                 190
   Gln Leu Gln Val Ala Trp Val Ala His Thr Gly Asp Ile Pro Gln Ser
           195                 200                 205
   Asn Gly Ser Gly Ala Glu Asp Gln Asn Thr Pro Leu Gln Ile Gly Asp
       210                 215                 220
   Thr Leu Tyr Val Cys Thr Pro Tyr Ser Lys Val Leu Ala Leu Asp Val
   225                 230                 235                 240
   Asp Ser Gly Lys Glu Lys Trp Arg Tyr Asp Ser Lys Ser Ser Ser Pro
                   245                 250                 255
   Asn Trp Gln Arg Cys Arg Gly Leu Gly Tyr Tyr Glu Asp Ser Gln Ala
               260                 265                 270
   Gln Thr Ala Leu Ala Ser Gly Thr Gln Pro Ala Ala Cys Ser Arg Arg
           275                 280                 285
   Leu Phe Leu Pro Thr Ile Asp Ala Arg Leu Ile Ala Ile Asp Ala Asp
       290                 295                 300
   Thr Gly Lys Leu Cys Glu Asn Phe Gly Asp Gly Gly Ile Val Asp Leu
   305                 310                 315                 320
   Ser Val Gly Met Gly Glu Val Lys Ala Gly Tyr Tyr Gln Gln Thr Ser
                   325                 330                 335
   Thr Pro Leu Val Ala Gly Asn Val Val Val Val Gly Gly Arg Val Ala
               340                 345                 350
   Asp Asn Tyr Ser Thr Gly Glu Pro Pro Gly Val Val Arg Ala Phe Asp
           355                 360                 365
   Val His Thr Gly Lys Leu Ala Trp Ala Trp Asp Pro Gly Asn Pro Ala
       370                 375                 380
   Leu Thr Gly Val Pro Pro Glu Gly Gln Thr Tyr Thr Arg Gly Thr Pro
   385                 390                 395                 400
   Asn Val Trp Ser Ala Met Ser Tyr Asp Ala Lys Leu Asn Leu Ile Tyr
   405 410 415
   Leu Pro Thr Gly Asn Ala Thr Pro Asp Phe Phe Gly Gly Glu Arg Thr
   420 425 430
   Ala Leu Asp Asp Lys Tyr Ser Ser Ser Ile Val Ala Val Asp Ala Thr
   435 440 445
   Thr Gly Gln Val Arg Trp His Phe Gln Thr Thr His His Asp Arg Trp
   450 455 460
   Asp Phe Asp Leu Pro Ser Gln Pro Leu Leu Tyr Asp Leu Pro Asp Gly
   465 470 475 480
   Lys Gly Gly Thr Thr Pro Val Leu Val Gln Thr Ser Lys Gln Gly Met
   485 490 495
   Ile Phe Met Leu Asn Arg Glu Thr Gly Glu Pro Val Ala Lys Val Glu
   500 505 510
   Glu Arg Pro Val Pro Ala Gly Asn Val Lys Gly Glu Arg Tyr Ser Pro
   515 520 525
   Thr Gln Pro Tyr Ser Val Gly Met Pro Met Ile Gly Asn Gln Thr Leu
   530 535 540
   Thr Glu Ser Asp Met Trp Gly Ala Thr Pro Ile Asp Leu Leu Leu Cys
   545 550 555 560
   Arg Ile Gln Phe Lys Glu Met Arg His Gln Gly Val Phe Thr Pro Pro
   565 570 575
   Gly Glu Asp Arg Ser Leu Gln Phe Pro Gly Ser Leu Gly Gly Met Asn
   580 585 590
   Trp Gly Ser Val Ser Leu Asp Pro Asn Asn Ser Leu Met Phe Val Asn
   595 600 605
   Asp Met Arg Leu Gly Leu Ala Asn Tyr Met Val Pro Arg Ala Lys Val
   610 615 620
   Ala Lys Asp Ala Ser Gly Ile Glu Met Gly Ile Val Pro Met Glu Gly
   625 630 635 640
   Thr Pro Phe Gly Ala Ile Arg Glu Arg Phe Leu Ser Pro Leu Gly Ile
   645 650 655
   Pro Cys Gln Lys Pro Pro Phe Gly Thr Met Ser Ala Val Asp Leu Lys
   660 665 670
   Thr Gly Lys Leu Val Trp Gln Val Pro Val Gly Thr Val Glu Asp Thr
   675 680 685
   Gly Pro Leu Gly Ile Arg Met His Met Pro Ile Pro Ile Gly Met Pro
   690 695 700
   Thr Leu Gly Ala Ser Leu Ala Thr Gln Ser Gly Leu Leu Phe Phe Ala
   705 710 715 720
   Gly Thr
<210> 43
   <211> 2346
   <212> DNA
   <213> Rhodobacter sphaeroides
<400> 43
   ttgaccgcta ccctgaccgc catcgtcctt gccctcgccg ggctgggcct cctcatcccg 60
   ggcgcctggc tggccgttct gggcgggagc tggttctacc tcgtggccgg agccctcatg 120
   ctgctgacgg catggcaggt gttccgccgc tcctcctccg ccgaatggat ctatggcggc 180
   ctcatcctcg gcgcgctcgt ctggtccgtc tgggaagtgg gcttcgactg gtgggaactc 240
   attccccgcg gcggcatcat cgcgcttctg ggcatctggc tctacctgcc cttcatgcgg 300
   cggcagctgc tgaccgagga cggccgcccc gcgcccggcc tgcccttggc cctgcccctt 360
   ctggcggcca tcggggccgc catctggtca tggaccggag acgaagcggg acatgcaggc 420
   tcgctgccga cggaagtggt cagcgccgag cccgatctcg gcccctccgc gcctcccggc 480
   gagtggcacc agtacggccg cacgcagtac gggcagcgct acagcccgct cgaacagatc 540
   aacatccaga atgtcgccga gctcgagcag gtctggcagt atcagaccgg cgatgtgaag 600
   ctgccgcagg acgtgaccga gaccacctat caggtcacgc cgctgaaggt cgccgaccgt 660
   ctctacatct gcaccccgca cgatctggcc atcgcgctcg atgcggcgac cggcaaggaa 720
   gcctggcgct tcgacgcgcg ctccgggctc gagtccgacc ggcagcacca gacctgccgc 780
   ggcgtgacct actggcggga tccggcacgc gccgagggag agctctgcgc cgagcgggtc 840
   tatctgccga cggccgatgc gcggctgatc gcgctcgacg caaagtccgg cgcggtctgc 900
   accttcttcg ccgacgaggg tacgctccat ctcgagaacg ggatgcccta caccccggca 960
   ggcttctact attccacctc cccgcccgtg gcggtcggag gccggatcat catcggcggc 1020
   gcggtcaacg acaatttctc ggtctattcg cagtcgggcg tgatccgggc cttcgatgcc 1080
   aacaccgggg cgctcctgtg gaactgggac agcgccaacc ccgacaagac cgcccccatc 1140
   gactggatga acggcgagac ctataccgca aattcgccca actcctggtc ggtcttctcg 1200
   gtcgatgagg aacgcggcct cgtctacatc ccgctcggca accaggtccc cgaccagctg 1260
   ggcttcaacc gctcgcccgc cgtcgaggaa cattcctcct cggtcgtggc gctcgacgtg 1320
   gcgacgggtc agaaggcctg ggtgttccag accgtgcacc atgacctgtg ggacatggac 1380
   gtgcccgccc agcccgttct gatcgatctc gacatcgacg gtcagacggt gcccgcgctc 1440
   gtgcagccga cgaagcaggg cgacatctat gtgctcaacc gcgagacggg cgagccgatc 1500
   ctgcccgtca ccgaagagcc cgccccgcag gagggcgctc tgcgcgaaga gacccctgcc 1560
   ccgacgcagc ccacctcggc gctgagcttc aaacccgagg cgctgcgcga gaaggacatg 1620
   tggggcgtca cgctctacga ccagctcgcc tgccggatcc agtatcaccg gttgaactac 1680
   gagggccgct acacgccccc gtcgctgaac ggcaccatcg tctatccggg caatttcggc 1740
   accttcaact ggggctccgt cgccgtcgat cccgagcggc aggtgatgtt cggcatgccc 1800
   acctacctgc ccttcacgag ccagctggtt ccggccgagg acatcccgcc gcccggcgca 1860
   gatcagaagg ccagcgaaca gggcctgaac cgcaacgagg gcgcgcccta cggcgtcatc 1920
   atggggccct tcctcgggcc gctcggcgtg ccctgctcgg cgccgccgtg gggcttcgtc 1980
   gcgggggcgg acctccgcac cggagagatc gcctacatgc accgcaacgg caccgtgcgc 2040
   gacatgacgc ccctgccgct gcccttcaag gtgggcgtgc cggggatcgg cgggccgatc 2100
   gtcacgcgcg gcggcgtggc cttcctcgga gcggcggtcg acgactacct gcgcgcctac 2160
   gatgtgacca cgggcgatca gctctggcag gcgcggctgc ccgcgggcgg ccagtcgacg 2220
   ccgatgacct acgagcagga cggccggcag ttcgtggtga tcgtggcggg cgggcacggc 2280
   tcggtcggca cgaaaccggg cgactatgtg atcgcctacg ccctgcccga cggctcggag 2340
   ggttga 2346
<210> 44
   <211> 781
   <212> PRT
   <213> Rhodobacter sphaeroides
<400> 44
   Met Thr Ala Thr Leu Thr Ala Ile Val Leu Ala Leu Ala Gly Leu Gly
   1 5 10 15
   Leu Leu Ile Pro Gly Ala Trp Leu Ala Val Leu Gly Gly Ser Trp Phe
   20 25 30
   Tyr Leu Val Ala Gly Ala Leu Met Leu Leu Thr Ala Trp Gln Val Phe
   35 40 45
   Arg Arg Ser Ser Ser Ala Glu Trp Ile Tyr Gly Gly Leu Ile Leu Gly
   50 55 60
   Ala Leu Val Trp Ser Val Trp Glu Val Gly Phe Asp Trp Trp Glu Leu
   65 70 75 80
   Ile Pro Arg Gly Gly Ile Ile Ala Leu Leu Gly Ile Trp Leu Tyr Leu
   85 90 95
   Pro Phe Met Arg Arg Gln Leu Leu Thr Glu Asp Gly Arg Pro Ala Pro
   100 105 110
   Gly Leu Pro Leu Ala Leu Pro Leu Leu Ala Ala Ile Gly Ala Ala Ile
   115 120 125
   Trp Ser Trp Thr Gly Asp Glu Ala Gly His Ala Gly Ser Leu Pro Thr
   130 135 140
   Glu Val Val Ser Ala Glu Pro Asp Leu Gly Pro Ser Ala Pro Pro Gly
   145 150 155 160
   Glu Trp His Gln Tyr Gly Arg Thr Gln Tyr Gly Gln Arg Tyr Ser Pro
   165 170 175
   Leu Glu Gln Ile Asn Ile Gln Asn Val Ala Glu Leu Glu Gln Val Trp
   180 185 190
   Gln Tyr Gln Thr Gly Asp Val Lys Leu Pro Gln Asp Val Thr Glu Thr
   195 200 205
   Thr Tyr Gln Val Thr Pro Leu Lys Val Ala Asp Arg Leu Tyr Ile Cys
   210 215 220
   Thr Pro His Asp Leu Ala Ile Ala Leu Asp Ala Ala Thr Gly Lys Glu
   225 230 235 240
   Ala Trp Arg Phe Asp Ala Arg Ser Gly Leu Glu Ser Asp Arg Gln His
   245 250 255
   Gln Thr Cys Arg Gly Val Thr Tyr Trp Arg Asp Pro Ala Arg Ala Glu
   260 265 270
   Gly Glu Leu Cys Ala Glu Arg Val Tyr Leu Pro Thr Ala Asp Ala Arg
   275 280 285
   Leu Ile Ala Leu Asp Ala Lys Ser Gly Ala Val Cys Thr Phe Phe Ala
   290 295 300
   Asp Glu Gly Thr Leu His Leu Glu Asn Gly Met Pro Tyr Thr Pro Ala
   305 310 315 320
   Gly Phe Tyr Tyr Ser Thr Ser Pro Pro Val Ala Val Gly Gly Arg Ile
   325 330 335
   Ile Ile Gly Gly Ala Val Asn Asp Asn Phe Ser Val Tyr Ser Gln Ser
               340                 345                 350
   Gly Val Ile Arg Ala Phe Asp Ala Asn Thr Gly Ala Leu Leu Trp Asn
           355                 360                 365
   Trp Asp Ser Ala Asn Pro Asp Lys Thr Ala Pro Ile Asp Trp Met Asn
       370                 375                 380
   Gly Glu Thr Tyr Thr Ala Asn Ser Pro Asn Ser Trp Ser Val Phe Ser
   385                 390                 395                 400
   Val Asp Glu Glu Arg Gly Leu Val Tyr Ile Pro Leu Gly Asn Gln Val
                   405                 410                 415
   Pro Asp Gln Leu Gly Phe Asn Arg Ser Pro Ala Val Glu Glu His Ser
               420                 425                 430
   Ser Ser Val Val Ala Leu Asp Val Ala Thr Gly Gln Lys Ala Trp Val
           435                 440                 445
   Phe Gln Thr Val His His Asp Leu Trp Asp Met Asp Val Pro Ala Gln
       450                 455                 460
   Pro Val Leu Ile Asp Leu Asp Ile Asp Gly Gln Thr Val Pro Ala Leu
   465                 470                 475                 480
   Val Gln Pro Thr Lys Gln Gly Asp Ile Tyr Val Leu Asn Arg Glu Thr
                   485                 490                 495
   Gly Glu Pro Ile Leu Pro Val Thr Glu Glu Pro Ala Pro Gln Glu Gly
               500                 505                 510
   Ala Leu Arg Glu Glu Thr Pro Ala Pro Thr Gln Pro Thr Ser Ala Leu
           515                 520                 525
   Ser Phe Lys Pro Glu Ala Leu Arg Glu Lys Asp Met Trp Gly Val Thr
       530                 535                 540
   Leu Tyr Asp Gln Leu Ala Cys Arg Ile Gln Tyr His Arg Leu Asn Tyr
   545                 550                 555                 560
   Glu Gly Arg Tyr Thr Pro Pro Ser Leu Asn Gly Thr Ile Val Tyr Pro
                   565                 570                 575
   Gly Asn Phe Gly Thr Phe Asn Trp Gly Ser Val Ala Val Asp Pro Glu
               580                 585                 590
   Arg Gln Val Met Phe Gly Met Pro Thr Tyr Leu Pro Phe Thr Ser Gln
   595 600 605
   Leu Val Pro Ala Glu Asp Ile Pro Pro Pro Gly Ala Asp Gln Lys Ala
   610 615 620
   Ser Glu Gln Gly Leu Asn Arg Asn Glu Gly Ala Pro Tyr Gly Val Ile
   625 630 635 640
   Met Gly Pro Phe Leu Gly Pro Leu Gly Val Pro Cys Ser Ala Pro Pro
   645 650 655
   Trp Gly Phe Val Ala Gly Ala Asp Leu Arg Thr Gly Glu Ile Ala Tyr
   660 665 670
   Met His Arg Asn Gly Thr Val Arg Asp Met Thr Pro Leu Pro Leu Pro
   675 680 685
   Phe Lys Val Gly Val Pro Gly Ile Gly Gly Pro Ile Val Thr Arg Gly
   690 695 700
   Gly Val Ala Phe Leu Gly Ala Ala Val Asp Asp Tyr Leu Arg Ala Tyr
   705 710 715 720
   Asp Val Thr Thr Gly Asp Gln Leu Trp Gln Ala Arg Leu Pro Ala Gly
   725 730 735
   Gly Gln Ser Thr Pro Met Thr Tyr Glu Gln Asp Gly Arg Gln Phe Val
   740 745 750
   Val Ile Val Ala Gly Gly His Gly Ser Val Gly Thr Lys Pro Gly Asp
   755 760 765
   Tyr Val Ile Ala Tyr Ala Leu Pro Asp Gly Ser Glu Gly
   770 775 780
<210> 45
   <211> 2391
   <212> DNA
   <213> Enterobacter sp.
<400> 45
   atggctgaaa caaaaactca acagtcgcgt ctactggtaa ctttaacagc agcgttcgca 60
   gcgttttgcg cactctatct gttgatcggt ggcgtctggc tggtcgcatt aggcggctcc 120
   tggtattacc cgattgcggg tctggttatg gttggcgtaa ccgttctgct cttaaaaggt 180
   aaacgatccg cactgtggct ctacgctgca ctgcttcttg tcacaatgtt ttggggagtc 240
   tgggaagttg gcttcgactt ctgggcgctg acaccgcgca gcgacatcct ggtcttcttt 300
   gggatctggc tgatcctgcc gttcgtctgg cgtagcctga gggttccttc cagcggtgca 360
   gttgcggcgc tggtggtttc cctgctgatt accggcggca tgctaacctg ggctgggttt 420
   aacgatccgc aggaagtgaa aggcacgctg agcgcagatt ccacacccgc tgcggcgatc 480
   tctgacgttg cagacggtga ctggccggct tatggtcgca accaggaagg ccaacgttat 540
   tctccgctga agcaaatcaa cgcggataac gttaaaaacc tgaaggaagc ctgggtgttc 600
   cgcaccggcg accttaagca gccaaacgac ccgggcgaat tgaccaacga agtgacgcca 660
   atcaaagtcg gcaacatgct ttatctgtgt actgcacacc agcgtctgtt tgcgcttgat 720
   gcggcgaccg gtaaagagaa atggcacttc gacccgcagt tgaattctaa tccgtcgttc 780
   cagcacatta cctgccgtgg cgtgtcctat cacgaagccc gcgcggataa tgccagcccg 840
   gaagtggttg ctgattgccc gcgtcgcatc atgctgccgg tcaacgatgg tcgactgttc 900
   gccatcaacg ccgaaaccgg caaactgtgc gaaacctttg gcaacaaagg cattctgaat 960
   ctgcagacca atatgccgga taccacgccg ggtctgtacg aaccgacttc cccgccgatt 1020
   atcaccgata aaacaattgt gattgccggt tcggtcacgg ataacttctc gactcgcgaa 1080
   acgtccggcg ttatccgtgg tttcgacgta aacacgggca aactgctgtg ggccttcgat 1140
   ccgggcgcga aagatcctaa cgcgattccg tcggatgaac acaccttcac cttcaactcg 1200
   ccaaactcat gggcgcctgc ggcgtatgac gcgaagctag acctggtgta cctgccaatg 1260
   ggtgtgacca cgccagatat ctggggcgga aaccgcacgc cagagcaaga gcgttacgcc 1320
   agcgcgattg tggcgctgaa tgcgacgacc gggaaactgg cctggagcta tcagaccgta 1380
   caccacgacc tgtgggatat ggatatgcca tcccagccga cgctggcgga tatcaccgtt 1440
   aacggtaaaa ccgttccagt gatttatgcc ccggcgaaaa ccggcaacat ctttgtactc 1500
   gaccgtacca acggcaaact ggtggttccg gcaccggaaa aaccggttcc gcaaggtgca 1560
   gccaaaggcg actatgtctc taaaacccag cctttctcgg acctgagctt ccgtccaaag 1620
   aaagacctga ccggtgccga tatgtggggc gccaccatgt tcgaccagct ggtgtgccgc 1680
   gtgatgttcc atcagctgcg ctatgaaggc atcttcacgc cgccgtctga gcagggcacg 1740
   ctggtcttcc cgggtaatct ggggatgttc gaatggggcg gtatctcggt tgatcctaac 1800
   cgtcaggtgg cgattgcaaa cccgatggcg ttgccgttcg tctctaagct tatccctcgc 1860
   ggcccgggta acccgatgga gcagccgaaa gatgcaaaag gtagcggtac agaagcgggc 1920
   attcagccgc agtacggcgt accgtttggc gtgacgctga accccttcct gtcgccgttt 1980
   ggcctgccgt gtaaacagcc tgcatggggt tatatttctg cgctggatct gaaaaccaat 2040
   caggttgtgt ggaaaaaacg tattggtacg ccacaggaca gcatgccgtt cccgatgccg 2100
   attcccgtgc cgttcaatat ggggatgcca atgctgggtg gtccgatttc caccgccggt 2160
   aacgtcctgt tcatcgcggc gaccgcagat aactacctgc gcgcgtacaa catgagcaac 2220
   ggtgaaaagc tgtggcaagg ccgtctgcct gcgggtgggc aagcgacgcc gatgacctat 2280
   gaagtggatg gcaagcagta tgttgttatc tctgcgggcg gtcacggttc gtttggtacg 2340
   aagatgggcg actatattgt cgcgtatgct ctgcctgacg atgtgaaata a 2391
<210> 46
   <211> 796
   <212> PRT
   <213> Enterobacter sp.
<400> 46
   Met Ala Glu Thr Lys Thr Gln Gln Ser Arg Leu Leu Val Thr Leu Thr
   1               5                   10                  15
   Ala Ala Phe Ala Ala Phe Cys Ala Leu Tyr Leu Leu Ile Gly Gly Val
               20                  25                  30
   Trp Leu Val Ala Leu Gly Gly Ser Trp Tyr Tyr Pro Ile Ala Gly Leu
           35                  40                  45
   Val Met Val Gly Val Thr Val Leu Leu Leu Lys Gly Lys Arg Ser Ala
       50                  55                  60
   Leu Trp Leu Tyr Ala Ala Leu Leu Leu Val Thr Met Phe Trp Gly Val
   65                  70                  75                  80
   Trp Glu Val Gly Phe Asp Phe Trp Ala Leu Thr Pro Arg Ser Asp Ile
                   85                  90                  95
   Leu Val Phe Phe Gly Ile Trp Leu Ile Leu Pro Phe Val Trp Arg Ser
               100                 105                 110
   Leu Arg Val Pro Ser Ser Gly Ala Val Ala Ala Leu Val Val Ser Leu
           115                 120                 125
   Leu Ile Thr Gly Gly Met Leu Thr Trp Ala Gly Phe Asn Asp Pro Gln
       130                 135                 140
   Glu Val Lys Gly Thr Leu Ser Ala Asp Ser Thr Pro Ala Ala Ala Ile
   145                 150                 155                 160
   Ser Asp Val Ala Asp Gly Asp Trp Pro Ala Tyr Gly Arg Asn Gln Glu
                   165                 170                 175
   Gly Gln Arg Tyr Ser Pro Leu Lys Gln Ile Asn Ala Asp Asn Val Lys
               180                 185                 190
   Asn Leu Lys Glu Ala Trp Val Phe Arg Thr Gly Asp Leu Lys Gln Pro
           195                 200                 205
   Asn Asp Pro Gly Glu Leu Thr Asn Glu Val Thr Pro Ile Lys Val Gly
       210                 215                 220
   Asn Met Leu Tyr Leu Cys Thr Ala His Gln Arg Leu Phe Ala Leu Asp
   225                 230                 235                 240
   Ala Ala Thr Gly Lys Glu Lys Trp His Phe Asp Pro Gln Leu Asn Ser
                   245                 250                 255
   Asn Pro Ser Phe Gln His Ile Thr Cys Arg Gly Val Ser Tyr His Glu
               260                 265                 270
   Ala Arg Ala Asp Asn Ala Ser Pro Glu Val Val Ala Asp Cys Pro Arg
           275                 280                 285
   Arg Ile Met Leu Pro Val Asn Asp Gly Arg Leu Phe Ala Ile Asn Ala
       290                 295                 300
   Glu Thr Gly Lys Leu Cys Glu Thr Phe Gly Asn Lys Gly Ile Leu Asn
   305                 310                 315                 320
   Leu Gln Thr Asn Met Pro Asp Thr Thr Pro Gly Leu Tyr Glu Pro Thr
                   325                 330                 335
   Ser Pro Pro Ile Ile Thr Asp Lys Thr Ile Val Ile Ala Gly Ser Val
               340                 345                 350
   Thr Asp Asn Phe Ser Thr Arg Glu Thr Ser Gly Val Ile Arg Gly Phe
           355                 360                 365
   Asp Val Asn Thr Gly Lys Leu Leu Trp Ala Phe Asp Pro Gly Ala Lys
       370                 375                 380
   Asp Pro Asn Ala Ile Pro Ser Asp Glu His Thr Phe Thr Phe Asn Ser
   385                 390                 395                 400
   Pro Asn Ser Trp Ala Pro Ala Ala Tyr Asp Ala Lys Leu Asp Leu Val
                   405                 410                 415
   Tyr Leu Pro Met Gly Val Thr Thr Pro Asp Ile Trp Gly Gly Asn Arg
               420                 425                 430
   Thr Pro Glu Gln Glu Arg Tyr Ala Ser Ala Ile Val Ala Leu Asn Ala
           435                 440                 445
   Thr Thr Gly Lys Leu Ala Trp Ser Tyr Gln Thr Val His His Asp Leu
       450                 455                 460
   Trp Asp Met Asp Met Pro Ser Gln Pro Thr Leu Ala Asp Ile Thr Val
   465 470 475 480
   Asn Gly Lys Thr Val Pro Val Ile Tyr Ala Pro Ala Lys Thr Gly Asn
   485 490 495
   Ile Phe Val Leu Asp Arg Thr Asn Gly Lys Leu Val Val Pro Ala Pro
   500 505 510
   Glu Lys Pro Val Pro Gln Gly Ala Ala Lys Gly Asp Tyr Val Ser Lys
   515 520 525
   Thr Gln Pro Phe Ser Asp Leu Ser Phe Arg Pro Lys Lys Asp Leu Thr
   530 535 540
   Gly Ala Asp Met Trp Gly Ala Thr Met Phe Asp Gln Leu Val Cys Arg
   545 550 555 560
   Val Met Phe His Gln Leu Arg Tyr Glu Gly Ile Phe Thr Pro Pro Ser
   565 570 575
   Glu Gln Gly Thr Leu Val Phe Pro Gly Asn Leu Gly Met Phe Glu Trp
   580 585 590
   Gly Gly Ile Ser Val Asp Pro Asn Arg Gln Val Ala Ile Ala Asn Pro
   595 600 605
   Met Ala Leu Pro Phe Val Ser Lys Leu Ile Pro Arg Gly Pro Gly Asn
   610 615 620
   Pro Met Glu Gln Pro Lys Asp Ala Lys Gly Ser Gly Thr Glu Ala Gly
   625 630 635 640
   Ile Gln Pro Gln Tyr Gly Val Pro Phe Gly Val Thr Leu Asn Pro Phe
   645 650 655
   Leu Ser Pro Phe Gly Leu Pro Cys Lys Gln Pro Ala Trp Gly Tyr Ile
   660 665 670
   Ser Ala Leu Asp Leu Lys Thr Asn Gln Val Val Trp Lys Lys Arg Ile
   675 680 685
   Gly Thr Pro Gln Asp Ser Met Pro Phe Pro Met Pro Ile Pro Val Pro
   690 695 700
   Phe Asn Met Gly Met Pro Met Leu Gly Gly Pro Ile Ser Thr Ala Gly
   705 710 715 720
   Asn Val Leu Phe Ile Ala Ala Thr Ala Asp Asn Tyr Leu Arg Ala Tyr
   725 730 735
   Asn Met Ser Asn Gly Glu Lys Leu Trp Gln Gly Arg Leu Pro Ala Gly
   740 745 750
   Gly Gln Ala Thr Pro Met Thr Tyr Glu Val Asp Gly Lys Gln Tyr Val
   755 760 765
   Val Ile Ser Ala Gly Gly His Gly Ser Phe Gly Thr Lys Met Gly Asp
   770 775 780
   Tyr Ile Val Ala Tyr Ala Leu Pro Asp Asp Val Lys
   785 790 795
<210> 47
   <211> 1431
   <212> DNA
   <213> Bacillus cereus
<400> 47
   ttgacaaaag ttaaagttag tttacgaccc attgttcata atataaattt accaaccgta      60
   ttgaaaacaa caatacttcc aggcgaatcg accgaaagac tatttatcgc aacccaatta     120
   ggagagattt tttacatagg gaatggagtt ataaagatat ttttagatat tcgtcaccta     180
   attattaaat taggtacatt tgaagaaggt gtttctagta gcggttatga tgaacgcgga     240
   ttgcttggac ttgcgttcca tccacaattt tatcaaaatg ggttatttta tcttcattat     300
   tcagtagctg gaactcaagg gccaggtgca ttttctgagc aatttaaacc gaatccttgt     360
   gaccctaaaa cgctaaattt aaagtgggtt aatagaaata cgcaatatga tcacattgat     420
   acagtagaag aatggacttt acaatctaat ggtcaagctc aaaagcgacg aacattactg     480
   aatgtaagaa gaccattttt taatcataac ggagtcaata gtttaaactt ttcacctgag     540
   actggaaaac ttgtttttac aaatggagat ggcggatcag gttatgatcc atttaattta     600
   agccaagatg atttagaaat agcaggtaaa ataattgaaa tcgatgtaag taaaaataca     660
   tttataaata accctcctgt agttacacgc ttcgatgaac ttcctttatc tatacaagaa     720
   acacttacag taattgcaaa aggagtacgt aatataaccg gcatttcatt tcaaaggttt     780
   tataatcaat atatcaaata cgccggaaat gtcggacaag atattgtaga gtctattttt     840
   tcatttgttc aatataaacc tataccggtt acagaacttg ttcaaatgca ttttatgagg     900
   ttaactccca atcaagatgg ggttatcaat tttgggtggc gaggatggga aggggattta     960
   cctacttctt ttatacgaca ttgttctgag aatcagactt tggatgagag aacaatggtt    1020
   tattatgatg aaacaataca aacttcagtc aagcgtattc tgcctctact tagttatttt    1080
   cataaagatt ctagaacaga taagtttgga ggaacttcac ttacaggagt tcagccatat    1140
   atgggaaatg caattccaaa tttaacaggt agcgtagtgt ttactgacct tgctaagaaa    1200
   gaagattctc gaccgccagt taaaggtgtt ttagcctata ctagagcagg tacagacggt    1260
   aaacatgctg actttcatgc tattgaaacc aattatgatt ttggtacgca agcagcttat    1320
   tatatgagtt taggaacaaa tttgaatcaa actaaattat atttaggagt ttacagttct    1380
   atgaaagtaa ctgattttaa taaaggtacc atttttgaaa ttattccctg a             1431
<210> 48
   <211> 476
   <212> PRT
   <213> Bacillus cereus
<400> 48
   Met Thr Lys Val Lys Val Ser Leu Arg Pro Ile Val His Asn Ile Asn
   1               5                   10                  15
   Leu Pro Thr Val Leu Lys Thr Thr Ile Leu Pro Gly Glu Ser Thr Glu
               20                  25                  30
   Arg Leu Phe Ile Ala Thr Gln Leu Gly Glu Ile Phe Tyr Ile Gly Asn
           35                  40                  45
   Gly Val Ile Lys Ile Phe Leu Asp Ile Arg His Leu Ile Ile Lys Leu
       50                  55                  60
   Gly Thr Phe Glu Glu Gly Val Ser Ser Ser Gly Tyr Asp Glu Arg Gly
   65                  70                  75                  80
   Leu Leu Gly Leu Ala Phe His Pro Gln Phe Tyr Gln Asn Gly Leu Phe
                   85                  90                  95
   Tyr Leu His Tyr Ser Val Ala Gly Thr Gln Gly Pro Gly Ala Phe Ser
               100                 105                 110
   Glu Gln Phe Lys Pro Asn Pro Cys Asp Pro Lys Thr Leu Asn Leu Lys
           115                 120                 125
   Trp Val Asn Arg Asn Thr Gln Tyr Asp His Ile Asp Thr Val Glu Glu
       130                 135                 140
   Trp Thr Leu Gln Ser Asn Gly Gln Ala Gln Lys Arg Arg Thr Leu Leu
   145                 150                 155                 160
   Asn Val Arg Arg Pro Phe Phe Asn His Asn Gly Val Asn Ser Leu Asn
                   165                 170                 175
   Phe Ser Pro Glu Thr Gly Lys Leu Val Phe Thr Asn Gly Asp Gly Gly
               180                 185                 190
   Ser Gly Tyr Asp Pro Phe Asn Leu Ser Gln Asp Asp Leu Glu Ile Ala
           195                 200                 205
   Gly Lys Ile Ile Glu Ile Asp Val Ser Lys Asn Thr Phe Ile Asn Asn
       210                 215                 220
   Pro Pro Val Val Thr Arg Phe Asp Glu Leu Pro Leu Ser Ile Gln Glu
   225                 230                 235                 240
   Thr Leu Thr Val Ile Ala Lys Gly Val Arg Asn Ile Thr Gly Ile Ser
                   245                 250                 255
   Phe Gln Arg Phe Tyr Asn Gln Tyr Ile Lys Tyr Ala Gly Asn Val Gly
               260                 265                 270
   Gln Asp Ile Val Glu Ser Ile Phe Ser Phe Val Gln Tyr Lys Pro Ile
           275                 280                 285
   Pro Val Thr Glu Leu Val Gln Met His Phe Met Arg Leu Thr Pro Asn
       290                 295                 300
   Gln Asp Gly Val Ile Asn Phe Gly Trp Arg Gly Trp Glu Gly Asp Leu
   305                 310                 315                 320
   Pro Thr Ser Phe Ile Arg His Cys Ser Glu Asn Gln Thr Leu Asp Glu
                   325                 330                 335
   Arg Thr Met Val Tyr Tyr Asp Glu Thr Ile Gln Thr Ser Val Lys Arg
               340                 345                 350
   Ile Leu Pro Leu Leu Ser Tyr Phe His Lys Asp Ser Arg Thr Asp Lys
           355                 360                 365
   Phe Gly Gly Thr Ser Leu Thr Gly Val Gln Pro Tyr Met Gly Asn Ala
       370                 375                 380
   Ile Pro Asn Leu Thr Gly Ser Val Val Phe Thr Asp Leu Ala Lys Lys
   385                 390                 395                 400
   Glu Asp Ser Arg Pro Pro Val Lys Gly Val Leu Ala Tyr Thr Arg Ala
                   405                 410                 415
   Gly Thr Asp Gly Lys His Ala Asp Phe His Ala Ile Glu Thr Asn Tyr
               420                 425                 430
   Asp Phe Gly Thr Gln Ala Ala Tyr Tyr Met Ser Leu Gly Thr Asn Leu
           435                 440                 445
   Asn Gln Thr Lys Leu Tyr Leu Gly Val Tyr Ser Ser Met Lys Val Thr
       450                 455                 460
   Asp Phe Asn Lys Gly Thr Ile Phe Glu Ile Ile Pro
   465 470 475
<210> 49
   <211> 1176
   <212> DNA
   <213> Micromonospora sp.
<400> 49
   gtgagcccgc gtcttgcgta cccccgtgtc agccgcctcc ggacggcgct ggcggcgtcc 60
   tgcgcggcgt tgctcctggg cgccgccggc tgctccctcg gcgagcccga gccggacccg 120
   gccggcgcac cgcccaacct gcccacaccg tccggcaccg cgagtcccgg cggggccggt 180
   cagcaggttg tcgccaccgt gctggccaag gggctggagg tgccgtgggg catcgcgttc 240
   ctgcccgacg gcggggcgct ggtcaccgag cgggacaagg gacggatcct ccaggtcggc 300
   ccggagtccg gcccggacgg gctggccgtc cggccggtcc agaccgtgcc ggacgtggcc 360
   gccggcggcg agggcggcct gctgggcatc gcggtctccc ccgcgtacgc caaggaccgc 420
   acggtcttcg tctactacac ggccgaggac gacaaccgga tcgcgaagat gcagctcggg 480
   caggcgccga agccgatcct gaccggcatc ccgaagtccg gcacgcacaa cggcggcggg 540
   ctcggcttcg gcccggacgg ccacctctac gcgagcaccg gcgacgccgg gcgcaccgag 600
   aacgcccagg acgccaagag cctcggcggc aagatcctcc ggatcacgcc ggacggcaag 660
   cctgcgccgg gcaaccccac cgccggatcg ccggtctggt cgacgggcca ccgcaacgtc 720
   cagggcttcg cctggacgcc ggacaagaag atgtacgcgg tggagttcgg ccagaacacc 780
   tgggacgaga tcaaccagat caacaagggc gggaactacg gctggccccg ggtcgagggg 840
   cgcggcgacg acaagcgcta cgtgaacccg atcacacagt ggcccaccgg tgacgcctcc 900
   tgttccggcc tggcggcggc ggaccgtctg ctcgtcgccg cctgcctgcg cggccagcga 960
   ctctggctgg tggagctggc cggcaacggc acagtcctcg gtcagccgcg cgacctgctc 1020
   aacggccggt acggtcggct acgggccgtc gcggcggcgc cggacggctc gctctgggtg 1080
   agcacgtcga accgggacgg gcggggtacg cccaccgccg aggacgaccg cctcctgcgg 1140
   ctcgtcttcg ccgacggggg cgccggccgg agctga 1176
<210> 50
   <211> 391
   <212> PRT
   <213> Micromonospora sp.
<400> 50
   Met Ser Pro Arg Leu Ala Tyr Pro Arg Val Ser Arg Leu Arg Thr Ala
   1               5                   10                  15
   Leu Ala Ala Ser Cys Ala Ala Leu Leu Leu Gly Ala Ala Gly Cys Ser
               20                  25                  30
   Leu Gly Glu Pro Glu Pro Asp Pro Ala Gly Ala Pro Pro Asn Leu Pro
           35                  40                  45
   Thr Pro Ser Gly Thr Ala Ser Pro Gly Gly Ala Gly Gln Gln Val Val
       50                  55                  60
   Ala Thr Val Leu Ala Lys Gly Leu Glu Val Pro Trp Gly Ile Ala Phe
   65                  70                  75                  80
   Leu Pro Asp Gly Gly Ala Leu Val Thr Glu Arg Asp Lys Gly Arg Ile
                   85                  90                  95
   Leu Gln Val Gly Pro Glu Ser Gly Pro Asp Gly Leu Ala Val Arg Pro
               100                 105                 110
   Val Gln Thr Val Pro Asp Val Ala Ala Gly Gly Glu Gly Gly Leu Leu
           115                 120                 125
   Gly Ile Ala Val Ser Pro Ala Tyr Ala Lys Asp Arg Thr Val Phe Val
       130                 135                 140
   Tyr Tyr Thr Ala Glu Asp Asp Asn Arg Ile Ala Lys Met Gln Leu Gly
   145                 150                 155                 160
   Gln Ala Pro Lys Pro Ile Leu Thr Gly Ile Pro Lys Ser Gly Thr His
                   165                 170                 175
   Asn Gly Gly Gly Leu Gly Phe Gly Pro Asp Gly His Leu Tyr Ala Ser
               180                 185                 190
   Thr Gly Asp Ala Gly Arg Thr Glu Asn Ala Gln Asp Ala Lys Ser Leu
           195                 200                 205
   Gly Gly Lys Ile Leu Arg Ile Thr Pro Asp Gly Lys Pro Ala Pro Gly
       210                 215                 220
   Asn Pro Thr Ala Gly Ser Pro Val Trp Ser Thr Gly His Arg Asn Val
   225                 230                 235                 240
   Gln Gly Phe Ala Trp Thr Pro Asp Lys Lys Met Tyr Ala Val Glu Phe
                   245                 250                 255
   Gly Gln Asn Thr Trp Asp Glu Ile Asn Gln Ile Asn Lys Gly Gly Asn
               260                 265                 270
   Tyr Gly Trp Pro Arg Val Glu Gly Arg Gly Asp Asp Lys Arg Tyr Val
           275                 280                 285
   Asn Pro Ile Thr Gln Trp Pro Thr Gly Asp Ala Ser Cys Ser Gly Leu
   290 295 300
   Ala Ala Ala Asp Arg Leu Leu Val Ala Ala Cys Leu Arg Gly Gln Arg
   305 310 315 320
   Leu Trp Leu Val Glu Leu Ala Gly Asn Gly Thr Val Leu Gly Gln Pro
   325 330 335
   Arg Asp Leu Leu Asn Gly Arg Tyr Gly Arg Leu Arg Ala Val Ala Ala
   340 345 350
   Ala Pro Asp Gly Ser Leu Trp Val Ser Thr Ser Asn Arg Asp Gly Arg
   355 360 365
   Gly Thr Pro Thr Ala Glu Asp Asp Arg Leu Leu Arg Leu Val Phe Ala
   370 375 380
   Asp Gly Gly Ala Gly Arg Ser
   385 390
<210> 51
   <211> 2475
   <212> DNA
   <213> Xanthomonas campestris
<400> 51
   atgactgatc aatcctctaa acgcgggctc ggcacttggc tgttggtggc ctatgccgtg    60
   gtgctcgccg tgcttggtgc ggcgctcgcc tatgaaggcg ggcgcctggt ggccgttggt   120
   ggctcctggt actacgtgct ggccggtatc gccgtgctgg tggccggtgt gctgctggca   180
   ctgggcaaac gcgctggcct gtggctgttt ggcgctacgt tggcggccac catcgtgtgg   240
   gcgctgtggg aagtgggcct ggatggttgg ggcctgatcc cacgtctggc ctggatctcg   300
   gtgctcggcc tggtgctgtt gccgttctgg ggcgtggcgc ggcggcgcat gcagccgttg   360
   tccgggctcg gctatgccgt ggtcaccggc gtgctgccgg tgctgggcgc agcgctgatc   420
   ctgtggccgc tgctggtgcc gcgcaatgtg gaactggccg atgcatccaa gcagccagcc   480
   gatgcggcaa cgccgttcag ccgtggcagc gtgcccagcc cggacgggaa cgtggcggcc   540
   aaccacgatg ccagtaactg gaccgcctac gccggttcca acctgtccaa ccattacacc   600
   cccggcgcgc agatcacgcc ggagaacgtc aagggcctca aggtggcctg ggaattccat   660
   accggcgatc tcaagccgaa ggattccaag ctgggctatg cgttccagaa caccccgctc   720
   aaggtcggcg acctgctcta catctgcacc ccgacccaga aggtgatcgc ggtggaggcg   780
   gccaacggca aggagcgctg gcgcttcgac ccgcagacca atccgaaggc gatggccggc   840
   gtggccgcca ccacctgccg tggtgtgtcg tactaccagg cgccggaagg cactgccgag   900
   tgcccgacgc ggatcttctg gccgatggtc gatggccgcc tgggtgcgct ggatgcgcag 960
   accggcaagc tctgcgccag cttcggtaac aacggctatg tggacctcaa tgccggcacc 1020
   ggcaacacca agccgggctt cgtcggcccg acctcgccgc cggtggtcat gcgcggtgtg 1080
   gtgatccagc ccaccgggca ggtgcgcgac ggtcaggaac gcgatgcgcc gtccggcgtg 1140
   gtgcgcggct tcgacgcgct caccggccag ctgcgctggg cctgggatct gggcaacccg 1200
   gccatcaccg ccgaaccgcc ggccggccag acctataccc gctccacccc gaacgtgtgg 1260
   tcgctgatgg ccgccgatga cgagctgggg ctggtgtatc tgcccaccgg caacgctgcc 1320
   ggcgacttct tcggcaaggg ccgcaccccg caggaagagg aatacaccgc ctcgctggtg 1380
   gccgtggatg cggcaaccgg caaggagcgc tggcacttcc gtaccgtcaa tcacgatctg 1440
   tgggactacg acatcggccc gcagccgaac ctggttgatt ggccggttgc cggcggtggc 1500
   acccgccccg ccgtgatcca ggccaccaag tccggccagg tgttcgtgct cgaccgggcc 1560
   accggccagc cgatcatgcc ggtcaagcag attgcggtac cgcagggcac cgatcatggc 1620
   gactggaccg catccaccca gccggtgtcg ccgggcatgc ccaacaccgt gggtgcgccc 1680
   agccgtgact acgagaccat cgttgaatct gacgcgtggg gcatgacccc gttcgaccag 1740
   ctcgcttgcc gcatcgagtt caagaagctg cggtacgaag gcatgttcac cccgccaagc 1800
   ctgcagggct cgctgtcgtt caccggcaac catggcggca tcaactgggg cggcgtgtcg 1860
   gtcgatctgc agcgcggcat catggtgatg aacagcaatc gcctgcccta taccgagcac 1920
   gtctacccgc gcacggtgat gaacgagctg ggcgtggtgt cggtgttcaa cggcagcagc 1980
   aagaccaagg gctacatggc gcaggaaggc ctggcctatg gcgcgcgcaa ggagccgtgg 2040
   atgtcgccgc tcaacacgcc gtgcgtggca ccgccgtggg gctatatctc cggcgtggat 2100
   ctgcgcacgc agcaggtgat ctggcgccgt ccgctgggca ccggttacga ccagggccca 2160
   atgggcatcc cgtccaagac caagttcgag atcggcaccc cgaacaacag cggctcgctg 2220
   gccactgccg gcggcgtcac cttcatcggt gccagcctgg acaacttcat ccgcggcttt 2280
   gacacccgca ccggcaagca ggtctgggag acccgcgtgc ctgcgggccc gcaggccgcg 2340
   ccgctgagct acaccatcga tggcaagcag tacatcgttg ccgcggtggg tggccatgac 2400
   cgcatggaaa ccaagtccgg cgacagcgtc attgcctggg cactgcccga cgacgcggcc 2460
   gccagcgcca aataa 2475
<210> 52
   <211> 824
   <212> PRT
   <213> Xanthomonas campestris
<400> 52
   Met Thr Asp Gln Ser Ser Lys Arg Gly Leu Gly Thr Trp Leu Leu Val
   1               5                   10                  15
   Ala Tyr Ala Val Val Leu Ala Val Leu Gly Ala Ala Leu Ala Tyr Glu
               20                  25                  30
   Gly Gly Arg Leu Val Ala Val Gly Gly Ser Trp Tyr Tyr Val Leu Ala
           35                  40                  45
   Gly Ile Ala Val Leu Val Ala Gly Val Leu Leu Ala Leu Gly Lys Arg
       50                  55                  60
   Ala Gly Leu Trp Leu Phe Gly Ala Thr Leu Ala Ala Thr Ile Val Trp
   65                  70                  75                  80
   Ala Leu Trp Glu Val Gly Leu Asp Gly Trp Gly Leu Ile Pro Arg Leu
                   85                  90                  95
   Ala Trp Ile Ser Val Leu Gly Leu Val Leu Leu Pro Phe Trp Gly Val
               100                 105                 110
   Ala Arg Arg Arg Met Gln Pro Leu Ser Gly Leu Gly Tyr Ala Val Val
           115                 120                 125
   Thr Gly Val Leu Pro Val Leu Gly Ala Ala Leu Ile Leu Trp Pro Leu
       130                 135                 140
   Leu Val Pro Arg Asn Val Glu Leu Ala Asp Ala Ser Lys Gln Pro Ala
   145                 150                 155                 160
   Asp Ala Ala Thr Pro Phe Ser Arg Gly Ser Val Pro Ser Pro Asp Gly
                   165                 170                 175
   Asn Val Ala Ala Asn His Asp Ala Ser Asn Trp Thr Ala Tyr Ala Gly
               180                 185                 190
   Ser Asn Leu Ser Asn His Tyr Thr Pro Gly Ala Gln Ile Thr Pro Glu
           195                 200                 205
   Asn Val Lys Gly Leu Lys Val Ala Trp Glu Phe His Thr Gly Asp Leu
       210                 215                 220
   Lys Pro Lys Asp Ser Lys Leu Gly Tyr Ala Phe Gln Asn Thr Pro Leu
   225                 230                 235                 240
   Lys Val Gly Asp Leu Leu Tyr Ile Cys Thr Pro Thr Gln Lys Val Ile
                   245                 250                 255
   Ala Val Glu Ala Ala Asn Gly Lys Glu Arg Trp Arg Phe Asp Pro Gln
               260                 265                 270
   Thr Asn Pro Lys Ala Met Ala Gly Val Ala Ala Thr Thr Cys Arg Gly
   275 280 285
   Val Ser Tyr Tyr Gln Ala Pro Glu Gly Thr Ala Glu Cys Pro Thr Arg
   290 295 300
   Ile Phe Trp Pro Met Val Asp Gly Arg Leu Gly Ala Leu Asp Ala Gln
   305 310 315 320
   Thr Gly Lys Leu Cys Ala Ser Phe Gly Asn Asn Gly Tyr Val Asp Leu
   325 330 335
   Asn Ala Gly Thr Gly Asn Thr Lys Pro Gly Phe Val Gly Pro Thr Ser
   340 345 350
   Pro Pro Val Val Met Arg Gly Val Val Ile Gln Pro Thr Gly Gln Val
   355 360 365
   Arg Asp Gly Gln Glu Arg Asp Ala Pro Ser Gly Val Val Arg Gly Phe
   370 375 380
   Asp Ala Leu Thr Gly Gln Leu Arg Trp Ala Trp Asp Leu Gly Asn Pro
   385 390 395 400
   Ala Ile Thr Ala Glu Pro Pro Ala Gly Gln Thr Tyr Thr Arg Ser Thr
   405 410 415
   Pro Asn Val Trp Ser Leu Met Ala Ala Asp Asp Glu Leu Gly Leu Val
   420 425 430
   Tyr Leu Pro Thr Gly Asn Ala Ala Gly Asp Phe Phe Gly Lys Gly Arg
   435 440 445
   Thr Pro Gln Glu Glu Glu Tyr Thr Ala Ser Leu Val Ala Val Asp Ala
   450 455 460
   Ala Thr Gly Lys Glu Arg Trp His Phe Arg Thr Val Asn His Asp Leu
   465 470 475 480
   Trp Asp Tyr Asp Ile Gly Pro Gln Pro Asn Leu Val Asp Trp Pro Val
   485 490 495
   Ala Gly Gly Gly Thr Arg Pro Ala Val Ile Gln Ala Thr Lys Ser Gly
   500 505 510
   Gln Val Phe Val Leu Asp Arg Ala Thr Gly Gln Pro Ile Met Pro Val
   515 520 525
   Lys Gln Ile Ala Val Pro Gln Gly Thr Asp His Gly Asp Trp Thr Ala
   530 535 540
   Ser Thr Gln Pro Val Ser Pro Gly Met Pro Asn Thr Val Gly Ala Pro
   545 550 555 560
   Ser Arg Asp Tyr Glu Thr Ile Val Glu Ser Asp Ala Trp Gly Met Thr
   565 570 575
   Pro Phe Asp Gln Leu Ala Cys Arg Ile Glu Phe Lys Lys Leu Arg Tyr
   580 585 590
   Glu Gly Met Phe Thr Pro Pro Ser Leu Gln Gly Ser Leu Ser Phe Thr
   595 600 605
   Gly Asn His Gly Gly Ile Asn Trp Gly Gly Val Ser Val Asp Leu Gln
   610 615 620
   Arg Gly Ile Met Val Met Asn Ser Asn Arg Leu Pro Tyr Thr Glu His
   625 630 635 640
   Val Tyr Pro Arg Thr Val Met Asn Glu Leu Gly Val Val Ser Val Phe
   645 650 655
   Asn Gly Ser Ser Lys Thr Lys Gly Tyr Met Ala Gln Glu Gly Leu Ala
   660 665 670
   Tyr Gly Ala Arg Lys Glu Pro Trp Met Ser Pro Leu Asn Thr Pro Cys
   675 680 685
   Val Ala Pro Pro Trp Gly Tyr Ile Ser Gly Val Asp Leu Arg Thr Gln
   690 695 700
   Gln Val Ile Trp Arg Arg Pro Leu Gly Thr Gly Tyr Asp Gln Gly Pro
   705 710 715 720
   Met Gly Ile Pro Ser Lys Thr Lys Phe Glu Ile Gly Thr Pro Asn Asn
   725 730 735
   Ser Gly Ser Leu Ala Thr Ala Gly Gly Val Thr Phe Ile Gly Ala Ser
   740 745 750
   Leu Asp Asn Phe Ile Arg Gly Phe Asp Thr Arg Thr Gly Lys Gln Val
   755 760 765
   Trp Glu Thr Arg Val Pro Ala Gly Pro Gln Ala Ala Pro Leu Ser Tyr
   770 775 780
   Thr Ile Asp Gly Lys Gln Tyr Ile Val Ala Ala Val Gly Gly His Asp
   785 790 795 800
   Arg Met Glu Thr Lys Ser Gly Asp Ser Val Ile Ala Trp Ala Leu Pro
   805 810 815
   Asp Asp Ala Ala Ala Ser Ala Lys
   820
<210> 53
   <211> 2412
   <212> DNA
   <213> Pseudomonas fluorescens
<400> 53
   atgagcactg aaggtgcttc gagtcgaagc cgtcttctgc cgaacctgct cggcattctg 60
   cttctgctga tgggcctggc catgctggcc gggggaatca agctgagtac gctcggcggc 120
   tcgctgtatt acctgctggc cggtatcggc atcacgctga ccggcattct gatgctgatg 180
   cgccgtcgcg cagcattggg cctgtacgcc atcgtgctgt tcgccagcac tgtctgggcg 240
   ctgtgggaag ttggtctgga ctggtggcaa ctggtgccgc gtctggcgct gtggtttgtc 300
   ctcggtttcg tgatgctgct gccgtggttc cgtcgtccgc tgctgctcgc aggccctgcg 360
   ccgatgggca ccggcggttt gaccgtggct gtgattctgg ccggcgtcac tgccctggcc 420
   agcctgttca cccacccggg cgaaaccttc ggcgaactgg gtcgcgacac cgccgacacc 480
   accagcaccg ccccgcaaat gccggacggc gactggcagg cctacggccg caccgaattc 540
   ggtgaccgct actcgccact gaaacagatc accccggcca acgtcggcaa gctgcaagaa 600
   gcctggcgca tccagaccgg cgacctgccg actgccgacg acccggtcga gctgaccaac 660
   gaaaacaccc cgctgaaagc caacggcatg ctttacgcct gcactgcgca cagcaaagtg 720
   ctggcactgg acccggacac cggcaaggaa ctatggcgct tcgatccgca gatcaaaagc 780
   ccggaaggct tcaagggctt cgcccacatg acctgccgtg gcgtgtcgta ctacgacgaa 840
   gccgcgtacg ccaagtctga aaacgcggcg tccaccgtga tctccgaggc cggcaaagcc 900
   gtcgcccagg cctgcccgcg tcgcctgtac ctgcctaccg ccgatgcccg cctgatcgca 960
   ctgaacgccg acaccggcaa gatctgcgaa ggcttcggca ccaacggtgt ggttgacctg 1020
   acccagggca tcggcccgtt caccgctggc ggttactact ccacctctcc tgccgcgatc 1080
   acccgtgatc tggtgatcat gggcggtcac gtcaccgaca acgaatcgac caacgagccg 1140
   tccggcgtga tccgcgccta cgacgtgcgc gacggccatc tggtctggaa ctgggacagc 1200
   aacaacccgg acgccaccga acccttggcc ccgggccaga cctacagccg caactcggcc 1260
   aacatgtggt cgctggccag cgtcgatgaa aaactcggca tggtctatct gccattgggc 1320
   aaccagacgc ctgaccagtg gggcggcgat cgcacccccg gtgccgagaa attcagcgcc 1380
   ggcctggtcg cgctggacct ggcgaccggc aaggtgcgct ggaactacca gttcacccac 1440
   cacgacctgt gggacatgga cgtcggcagc cagccaaccc tgctggacat gaaaaccgcc 1500
   gacggcatca agcctgcgct gatcgccccg accaagcagg gcagcctgta cgtcctcgac 1560
   cgtcgtgacg gcacgccgat catcccgatc cgcgagatcc cggtgccgca aggcgccgtg 1620
   aaaggcgacc acaccgcccc gacccaggcc cgttcggacc tgaacctgct ggccccggaa 1680
   ctgaccgaaa aagccatgtg gggcgccagc ccgttcgacc agatgttgtg ccgcatccag 1740
   ttcaaggaac tgcgctacga aggccaatac acgcctccgt cggaacaggg cagcctgatc 1800
   tacccgggta acgtcggcgt gttcaactgg ggcggtgttt ctgtcgaccc ggttcgccag 1860
   atgctgttca ccagcccgaa ctacatggct ttcgtctcca agatgatccc acgtgccgac 1920
   gtacccgccg acagcaagcg cgaaagcgaa acttccggca tccagaaaaa caccggtgcg 1980
   ccgtatgccg tgaccatgca cccgttcatg tcgccactgg gcgtaccttg ccaggccccg 2040
   gcctggggct acgtcgccgg tatcgacctg accaccggca aagtcgtctg gaaacgcaaa 2100
   aacggtacca gccgcgacag ctcgccaatc ccgatcggct tcaccctggg cgtgccaagc 2160
   atgggcggct cgatcgtcac tgccggcggc gtcggcttcc tcagcggcac gctggaccag 2220
   tacctgcgcg cctacgacgt gaacaccggt aaagaactgt ggaaatcgcg tctgccggcc 2280
   ggcggccagg cgaccccaat gacctacacc ggcaaggacg gcaagcaata cgtcctgctc 2340
   gtggtgggtg gtcacggctc gctgggcacc aagatgggtg actatgtgat tgcctacaaa 2400
   ctgccggaat aa 2412
<210> 54
   <211> 803
   <212> PRT
   <213> Pseudomonas fluorescens
<400> 54
   Met Ser Thr Glu Gly Ala Ser Ser Arg Ser Arg Leu Leu Pro Asn Leu
   1               5                   10                  15
   Leu Gly Ile Leu Leu Leu Leu Met Gly Leu Ala Met Leu Ala Gly Gly
               20                  25                  30
   Ile Lys Leu Ser Thr Leu Gly Gly Ser Leu Tyr Tyr Leu Leu Ala Gly
           35                  40                  45
   Ile Gly Ile Thr Leu Thr Gly Ile Leu Met Leu Met Arg Arg Arg Ala
       50                  55                  60
   Ala Leu Gly Leu Tyr Ala Ile Val Leu Phe Ala Ser Thr Val Trp Ala
   65                  70                  75                  80
   Leu Trp Glu Val Gly Leu Asp Trp Trp Gln Leu Val Pro Arg Leu Ala
                   85                  90                  95
   Leu Trp Phe Val Leu Gly Phe Val Met Leu Leu Pro Trp Phe Arg Arg
   100 105 110
   Pro Leu Leu Leu Ala Gly Pro Ala Pro Met Gly Thr Gly Gly Leu Thr
   115 120 125
   Val Ala Val Ile Leu Ala Gly Val Thr Ala Leu Ala Ser Leu Phe Thr
   130 135 140
   His Pro Gly Glu Thr Phe Gly Glu Leu Gly Arg Asp Thr Ala Asp Thr
   145 150 155 160
   Thr Ser Thr Ala Pro Gln Met Pro Asp Gly Asp Trp Gln Ala Tyr Gly
   165 170 175
   Arg Thr Glu Phe Gly Asp Arg Tyr Ser Pro Leu Lys Gln Ile Thr Pro
   180 185 190
   Ala Asn Val Gly Lys Leu Gln Glu Ala Trp Arg Ile Gln Thr Gly Asp
   195 200 205
   Leu Pro Thr Ala Asp Asp Pro Val Glu Leu Thr Asn Glu Asn Thr Pro
   210 215 220
   Leu Lys Ala Asn Gly Met Leu Tyr Ala Cys Thr Ala His Ser Lys Val
   225 230 235 240
   Leu Ala Leu Asp Pro Asp Thr Gly Lys Glu Leu Trp Arg Phe Asp Pro
   245 250 255
   Gln Ile Lys Ser Pro Glu Gly Phe Lys Gly Phe Ala His Met Thr Cys
   260 265 270
   Arg Gly Val Ser Tyr Tyr Asp Glu Ala Ala Tyr Ala Lys Ser Glu Asn
   275 280 285
   Ala Ala Ser Thr Val Ile Ser Glu Ala Gly Lys Ala Val Ala Gln Ala
   290 295 300
   Cys Pro Arg Arg Leu Tyr Leu Pro Thr Ala Asp Ala Arg Leu Ile Ala
   305 310 315 320
   Leu Asn Ala Asp Thr Gly Lys Ile Cys Glu Gly Phe Gly Thr Asn Gly
   325 330 335
   Val Val Asp Leu Thr Gln Gly Ile Gly Pro Phe Thr Ala Gly Gly Tyr
   340 345 350
   Tyr Ser Thr Ser Pro Ala Ala Ile Thr Arg Asp Leu Val Ile Met Gly
   355 360 365
   Gly His Val Thr Asp Asn Glu Ser Thr Asn Glu Pro Ser Gly Val Ile
   370 375 380
   Arg Ala Tyr Asp Val Arg Asp Gly His Leu Val Trp Asn Trp Asp Ser
   385 390 395 400
   Asn Asn Pro Asp Ala Thr Glu Pro Leu Ala Pro Gly Gln Thr Tyr Ser
   405 410 415
   Arg Asn Ser Ala Asn Met Trp Ser Leu Ala Ser Val Asp Glu Lys Leu
   420 425 430
   Gly Met Val Tyr Leu Pro Leu Gly Asn Gln Thr Pro Asp Gln Trp Gly
   435 440 445
   Gly Asp Arg Thr Pro Gly Ala Glu Lys Phe Ser Ala Gly Leu Val Ala
   450 455 460
   Leu Asp Leu Ala Thr Gly Lys Val Arg Trp Asn Tyr Gln Phe Thr His
   465 470 475 480
   His Asp Leu Trp Asp Met Asp Val Gly Ser Gln Pro Thr Leu Leu Asp
   485 490 495
   Met Lys Thr Ala Asp Gly Ile Lys Pro Ala Leu Ile Ala Pro Thr Lys
   500 505 510
   Gln Gly Ser Leu Tyr Val Leu Asp Arg Arg Asp Gly Thr Pro Ile Ile
   515 520 525
   Pro Ile Arg Glu Ile Pro Val Pro Gln Gly Ala Val Lys Gly Asp His
   530 535 540
   Thr Ala Pro Thr Gln Ala Arg Ser Asp Leu Asn Leu Leu Ala Pro Glu
   545 550 555 560
   Leu Thr Glu Lys Ala Met Trp Gly Ala Ser Pro Phe Asp Gln Met Leu
   565 570 575
   Cys Arg Ile Gln Phe Lys Glu Leu Arg Tyr Glu Gly Gln Tyr Thr Pro
   580 585 590
   Pro Ser Glu Gln Gly Ser Leu Ile Tyr Pro Gly Asn Val Gly Val Phe
   595 600 605
   Asn Trp Gly Gly Val Ser Val Asp Pro Val Arg Gln Met Leu Phe Thr
   610 615 620
   Ser Pro Asn Tyr Met Ala Phe Val Ser Lys Met Ile Pro Arg Ala Asp
   625 630 635 640
   Val Pro Ala Asp Ser Lys Arg Glu Ser Glu Thr Ser Gly Ile Gln Lys
   645 650 655
   Asn Thr Gly Ala Pro Tyr Ala Val Thr Met His Pro Phe Met Ser Pro
   660 665 670
   Leu Gly Val Pro Cys Gln Ala Pro Ala Trp Gly Tyr Val Ala Gly Ile
   675 680 685
   Asp Leu Thr Thr Gly Lys Val Val Trp Lys Arg Lys Asn Gly Thr Ser
   690 695 700
   Arg Asp Ser Ser Pro Ile Pro Ile Gly Phe Thr Leu Gly Val Pro Ser
   705 710 715 720
   Met Gly Gly Ser Ile Val Thr Ala Gly Gly Val Gly Phe Leu Ser Gly
   725 730 735
   Thr Leu Asp Gln Tyr Leu Arg Ala Tyr Asp Val Asn Thr Gly Lys Glu
   740 745 750
   Leu Trp Lys Ser Arg Leu Pro Ala Gly Gly Gln Ala Thr Pro Met Thr
   755 760 765
   Tyr Thr Gly Lys Asp Gly Lys Gln Tyr Val Leu Leu Val Val Gly Gly
   770 775 780
   His Gly Ser Leu Gly Thr Lys Met Gly Asp Tyr Val Ile Ala Tyr Lys
   785 790 795 800
   Leu Pro Glu
<210> 55
   <211> 3525
   <212> DNA
   <213> Rhodopirellula baltica
<400> 55
   atgaggtctc ttatcagcac tatgtcgtcg ccaattgctt tcccctctcg ctcgttttcc 60
   cgttctttgc gtcggtcgcg gcgtccgtct tccttcattc gctccgcgat cgggctcggc 120
   ctcatcgtcg ctgcgatgcc ggtctgtgcc gttctcagtg gtaccgattc gatcggctcg 180
   gtgagtgccg acgagccagc tgccgcggcg gtgacgaata ccaaacccga agcgctggaa 240
   ccggaaatcg ccgaggcgtc cgaagaagcg gcccaggcga tggcgggatt caagatccct 300
   gaaggctggg aaatcaaact tttcgcggcc gagccccaag tcgccaacat cgtcgcgttt 360
   ggcgtggatt cgaagggccg agtctacgtt tgcgaaagct accggcagaa tcgcggcgtg 420
   acggacaacc gaggtcacga cgacgaatgg ttgctggctg atttgtcggc cgagacagtt 480
   caagaccgaa ttgattacca caaacggttg ctcggtgaag cggcgatcac ttacgcacaa 540
   catgacgatc ggatccgccg gttaacggac accgacggcg atggtgtcgc cgatgagtcc 600
   accgtcgtgg ccgatggctt caacggtttg gaagagggga ccggcgcggg agttttgatc 660
   aacggttcgg atatctatta cacctgcatt cccaaactgt ggaaattgac cgacgcggat 720
   gacgacggcg tcgcggaaga ttcgcaagtg ttgtcggatg ggtatggcgt ccgagtcgct 780
   ttccgtggtc acgacatgca cgggttgatc cgaggctatg acgggcgttt gtacttctcg 840
   atcggagacc gtggttacca cgtgacgact cccgaaggaa agctgttgtc caacccagcc 900
   gtcggcgctg tgttccgttg cgaaatggac ggcagccagt tggaagttta ttgcaacggt 960
   ttgcggaacc cgcaggagtt ggccttcaac gacatcggcg actggttcac cgtcgacaac 1020
   aactccgaca gtggcgacaa agctcgtttg gttcatctgt tggaaggtgg cgacacgggt 1080
   tggcggatgc actatcagta cttgcctgat cgcggacctt tcaatcgttt gaagatttgg 1140
   gagccgcacc acaacgaaca accggctcac cttgttccgc cgatcatcaa cttcaccgac 1200
   ggaccatcgg gactcgcctt ttatccgggc accggattcg gggaactact cgacaatcaa 1260
   tttctgattt gtgatttccg tggcggccca tccaatagcg gcatccggtc gttctcggtg 1320
   gatcccgatg gggcctttta caaaatgggc gccgatgacc agccgatttg gaacattctc 1380
   gcgaccgacg ttgcgttcac tccttcgggt gagcttttgg tcagtgactg ggtcgatggt 1440
   tgggacggac ttggcaaagg gcgcttgtac aagctgagtg acccggcaca gcaagacacc 1500
   gatgttgtga cggaagtcaa ggaactgctc agtggtgatt tcacctccgc atcggtggag 1560
   gatctgaccg agcaacttcg ccacatcgat cgtcgagtgc gtttgaatgc tcagtgggaa 1620
   ctcgccagcc gcggtgaaac gaaacccctg atcgacatcg cgtcagcgac tgacttggat 1680
   gctcgtttgc gtttacacgg catttgggga tgcgagcacg cggtgcgttt ggatggttcg 1740
   aagcaagcgg atgtgttggc cgccaatcgc ggttggttga ccgattccga cccggtcatt 1800
   cgtgccgccg cctgtgcttt ggccggcgat cggaacgatg ccgaatcagt cgcgaagatc 1860
   agcgagttgt tggctgacga atcgcctcgc gtgaagtact tcgccgcgat gtcattgtcc 1920
   gaattgttga cttcgcaaac tggcaacgct tctgtcaatc agcaagcgat cggcagtgtc 1980
   ttgcagattt tggcgaagaa cgacaacaac gatcctgccc tccgacacgc gtgcacgttt 2040
   ttcctgcgac gtgttgcatc ggaagacttg ttggcgggat tggcgacgca cagcagtgtt 2100
   cccgttcgcc gagccgccat cgccgctctt cgtggtcaag gcagcgagaa ggtgacggca 2160
   tttttgtccg acgccagttc actggtcttg gcggaagccg cgatggcaat tcacgaccga 2220
   ccaattccgg ctggcgtgga cgagttggcg aagctgattt cggtcgcgga tttgccgctg 2280
   aattcggagg cgttgttgcg tcgcgtgttg acggccaatt accgaattgg cacggctgac 2340
   tccgctgcgg ctctagcgtc cttcgctgcg tcgggtgaaa agccgacgtg ggctcgaatc 2400
   gaagcgatcg acatgttggc caattgggcc actccggaac cgcgtgaccg agtcacgaac 2460
   gaatatcgac ctttggaaag tcgtcctgaa atcgtcgctc gcgaagcgtt ggccaagcac 2520
   atcgaagtct tgatgatcac cgaccaaacg gttcgcgaaa aagcgatcga tgttggatcc 2580
   aaacttggga tcaagaaaat cgctccgcag ttgattgcac gcaccaatga cgctcaatcg 2640
   cgacccgcat cacgagcgtc ggcattgacg gcgttggctc gtttgcaacc cgctcaagcg 2700
   gtccagatcg ctcgggcgat cgatatcgag caaccgactc agttggtcac cgccgcactc 2760
   tccgttctgg gcaagcacga tggccccgcg tccgttgatc gcttcattgc cgcgacggcg 2820
   accgagagcc aactcgttcg aggattggct tgggacttgt tggccaacgt ggagtcgaaa 2880
   gaggccctcg agcacatcat caaaggtgtg aacgcttacc tcgaaaatga tcttccggcc 2940
   gatgttcagt tgaacctggt cgaagccgcc gcgaaacgtt tgcctgaaga ctggaacgcg 3000
   aagatcgccg ctcatcgaga gcaattggcg acgacggaac cattggcgaa gtggatggat 3060
   tcgttgcacg gtggtgatcc cgacaaggga gcgaagctgt tctttggcaa gaccgaactt 3120
   tcgtgtgtgc gttgccacaa agtggatcgc gcgggtggtg aagttggtcc ggtcctgaca 3180
   acgattggca aaacgcgaga ccgtcgcacg ttgctggaag cgatcgctct gccggatgca 3240
   aagatcgcag aaggcttcga gacggctgta atcgcggacg aagatggaca ggtcttcacc 3300
   gggattgtgg gagcggagac cgacgatgtg attgaactga tcgccgccga cgggtcacgg 3360
   tctcggatcg agaaggacta catcatcgct cggaagaaag gcaagtcatc gatgccggct 3420
   ggattgactg agcagatgac cgcccgcgaa ttgcgtgatt tggttgccta tttggcgagc 3480
   cttcaagtcg acccacgggc cggcgaggaa gaaggccacg aatga 3525
<210> 56
   <211> 1174
   <212> PRT
   <213> Rhodopirellula baltica
<400> 56
   Met Arg Ser Leu Ile Ser Thr Met Ser Ser Pro Ile Ala Phe Pro Ser
   1 5 10 15
   Arg Ser Phe Ser Arg Ser Leu Arg Arg Ser Arg Arg Pro Ser Ser Phe
   20 25 30
   Ile Arg Ser Ala Ile Gly Leu Gly Leu Ile Val Ala Ala Met Pro Val
   35 40 45
   Cys Ala Val Leu Ser Gly Thr Asp Ser Ile Gly Ser Val Ser Ala Asp
   50 55 60
   Glu Pro Ala Ala Ala Ala Val Thr Asn Thr Lys Pro Glu Ala Leu Glu
   65 70 75 80
   Pro Glu Ile Ala Glu Ala Ser Glu Glu Ala Ala Gln Ala Met Ala Gly
   85 90 95
   Phe Lys Ile Pro Glu Gly Trp Glu Ile Lys Leu Phe Ala Ala Glu Pro
   100 105 110
   Gln Val Ala Asn Ile Val Ala Phe Gly Val Asp Ser Lys Gly Arg Val
   115 120 125
   Tyr Val Cys Glu Ser Tyr Arg Gln Asn Arg Gly Val Thr Asp Asn Arg
   130 135 140
   Gly His Asp Asp Glu Trp Leu Leu Ala Asp Leu Ser Ala Glu Thr Val
   145 150 155 160
   Gln Asp Arg Ile Asp Tyr His Lys Arg Leu Leu Gly Glu Ala Ala Ile
   165 170 175
   Thr Tyr Ala Gln His Asp Asp Arg Ile Arg Arg Leu Thr Asp Thr Asp
   180 185 190
   Gly Asp Gly Val Ala Asp Glu Ser Thr Val Val Ala Asp Gly Phe Asn
   195 200 205
   Gly Leu Glu Glu Gly Thr Gly Ala Gly Val Leu Ile Asn Gly Ser Asp
   210 215 220
   Ile Tyr Tyr Thr Cys Ile Pro Lys Leu Trp Lys Leu Thr Asp Ala Asp
   225 230 235 240
   Asp Asp Gly Val Ala Glu Asp Ser Gln Val Leu Ser Asp Gly Tyr Gly
   245 250 255
   Val Arg Val Ala Phe Arg Gly His Asp Met His Gly Leu Ile Arg Gly
   260 265 270
   Tyr Asp Gly Arg Leu Tyr Phe Ser Ile Gly Asp Arg Gly Tyr His Val
   275 280 285
   Thr Thr Pro Glu Gly Lys Leu Leu Ser Asn Pro Ala Val Gly Ala Val
   290 295 300
   Phe Arg Cys Glu Met Asp Gly Ser Gln Leu Glu Val Tyr Cys Asn Gly
   305 310 315 320
   Leu Arg Asn Pro Gln Glu Leu Ala Phe Asn Asp Ile Gly Asp Trp Phe
   325 330 335
   Thr Val Asp Asn Asn Ser Asp Ser Gly Asp Lys Ala Arg Leu Val His
   340 345 350
   Leu Leu Glu Gly Gly Asp Thr Gly Trp Arg Met His Tyr Gln Tyr Leu
   355 360 365
   Pro Asp Arg Gly Pro Phe Asn Arg Leu Lys Ile Trp Glu Pro His His
   370 375 380
   Asn Glu Gln Pro Ala His Leu Val Pro Pro Ile Ile Asn Phe Thr Asp
   385 390 395 400
   Gly Pro Ser Gly Leu Ala Phe Tyr Pro Gly Thr Gly Phe Gly Glu Leu
   405 410 415
   Leu Asp Asn Gln Phe Leu Ile Cys Asp Phe Arg Gly Gly Pro Ser Asn
   420 425 430
   Ser Gly Ile Arg Ser Phe Ser Val Asp Pro Asp Gly Ala Phe Tyr Lys
   435 440 445
   Met Gly Ala Asp Asp Gln Pro Ile Trp Asn Ile Leu Ala Thr Asp Val
   450 455 460
   Ala Phe Thr Pro Ser Gly Glu Leu Leu Val Ser Asp Trp Val Asp Gly
   465 470 475 480
   Trp Asp Gly Leu Gly Lys Gly Arg Leu Tyr Lys Leu Ser Asp Pro Ala
   485 490 495
   Gln Gln Asp Thr Asp Val Val Thr Glu Val Lys Glu Leu Leu Ser Gly
   500 505 510
   Asp Phe Thr Ser Ala Ser Val Glu Asp Leu Thr Glu Gln Leu Arg His
   515 520 525
   Ile Asp Arg Arg Val Arg Leu Asn Ala Gln Trp Glu Leu Ala Ser Arg
   530 535 540
   Gly Glu Thr Lys Pro Leu Ile Asp Ile Ala Ser Ala Thr Asp Leu Asp
   545 550 555 560
   Ala Arg Leu Arg Leu His Gly Ile Trp Gly Cys Glu His Ala Val Arg
   565 570 575
   Leu Asp Gly Ser Lys Gln Ala Asp Val Leu Ala Ala Asn Arg Gly Trp
               580                 585                 590
   Leu Thr Asp Ser Asp Pro Val Ile Arg Ala Ala Ala Cys Ala Leu Ala
           595                 600                 605
   Gly Asp Arg Asn Asp Ala Glu Ser Val Ala Lys Ile Ser Glu Leu Leu
       610                 615                 620
   Ala Asp Glu Ser Pro Arg Val Lys Tyr Phe Ala Ala Met Ser Leu Ser
   625                 630                 635                 640
   Glu Leu Leu Thr Ser Gln Thr Gly Asn Ala Ser Val Asn Gln Gln Ala
                   645                 650                 655
   Ile Gly Ser Val Leu Gln Ile Leu Ala Lys Asn Asp Asn Asn Asp Pro
               660                 665                 670
   Ala Leu Arg His Ala Cys Thr Phe Phe Leu Arg Arg Val Ala Ser Glu
           675                 680                 685
   Asp Leu Leu Ala Gly Leu Ala Thr His Ser Ser Val Pro Val Arg Arg
       690                 695                 700
   Ala Ala Ile Ala Ala Leu Arg Gly Gln Gly Ser Glu Lys Val Thr Ala
   705                 710                 715                 720
   Phe Leu Ser Asp Ala Ser Ser Leu Val Leu Ala Glu Ala Ala Met Ala
                   725                 730                 735
   Ile His Asp Arg Pro Ile Pro Ala Gly Val Asp Glu Leu Ala Lys Leu
               740                 745                 750
   Ile Ser Val Ala Asp Leu Pro Leu Asn Ser Glu Ala Leu Leu Arg Arg
           755                 760                 765
   Val Leu Thr Ala Asn Tyr Arg Ile Gly Thr Ala Asp Ser Ala Ala Ala
       770                 775                 780
   Leu Ala Ser Phe Ala Ala Ser Gly Glu Lys Pro Thr Trp Ala Arg Ile
   785                 790                 795                 800
   Glu Ala Ile Asp Met Leu Ala Asn Trp Ala Thr Pro Glu Pro Arg Asp
                   805                 810                 815
   Arg Val Thr Asn Glu Tyr Arg Pro Leu Glu Ser Arg Pro Glu Ile Val
               820                 825                 830
   Ala Arg Glu Ala Leu Ala Lys His Ile Glu Val Leu Met Ile Thr Asp
   835 840 845
   Gln Thr Val Arg Glu Lys Ala Ile Asp Val Gly Ser Lys Leu Gly Ile
   850 855 860
   Lys Lys Ile Ala Pro Gln Leu Ile Ala Arg Thr Asn Asp Ala Gln Ser
   865 870 875 880
   Arg Pro Ala Ser Arg Ala Ser Ala Leu Thr Ala Leu Ala Arg Leu Gln
   885 890 895
   Pro Ala Gln Ala Val Gln Ile Ala Arg Ala Ile Asp Ile Glu Gln Pro
   900 905 910
   Thr Gln Leu Val Thr Ala Ala Leu Ser Val Leu Gly Lys His Asp Gly
   915 920 925
   Pro Ala Ser Val Asp Arg Phe Ile Ala Ala Thr Ala Thr Glu Ser Gln
   930 935 940
   Leu Val Arg Gly Leu Ala Trp Asp Leu Leu Ala Asn Val Glu Ser Lys
   945 950 955 960
   Glu Ala Leu Glu His Ile Ile Lys Gly Val Asn Ala Tyr Leu Glu Asn
   965 970 975
   Asp Leu Pro Ala Asp Val Gln Leu Asn Leu Val Glu Ala Ala Ala Lys
   980 985 990
   Arg Leu Pro Glu Asp Trp Asn Ala Lys Ile Ala Ala His Arg Glu Gln
   995 1000 1005
   Leu Ala Thr Thr Glu Pro Leu Ala Lys Trp Met Asp Ser Leu His
   1010 1015 1020
   Gly Gly Asp Pro Asp Lys Gly Ala Lys Leu Phe Phe Gly Lys Thr
   1025 1030 1035
   Glu Leu Ser Cys Val Arg Cys His Lys Val Asp Arg Ala Gly Gly
   1040 1045 1050
   Glu Val Gly Pro Val Leu Thr Thr Ile Gly Lys Thr Arg Asp Arg
   1055 1060 1065
   Arg Thr Leu Leu Glu Ala Ile Ala Leu Pro Asp Ala Lys Ile Ala
   1070 1075 1080
   Glu Gly Phe Glu Thr Ala Val Ile Ala Asp Glu Asp Gly Gln Val
   1085 1090 1095
   Phe Thr Gly Ile Val Gly Ala Glu Thr Asp Asp Val Ile Glu Leu
   1100 1105 1110
   Ile Ala Ala Asp Gly Ser Arg Ser Arg Ile Glu Lys Asp Tyr Ile
   1115 1120 1125
   Ile Ala Arg Lys Lys Gly Lys Ser Ser Met Pro Ala Gly Leu Thr
   1130 1135 1140
   Glu Gln Met Thr Ala Arg Glu Leu Arg Asp Leu Val Ala Tyr Leu
   1145 1150 1155
   Ala Ser Leu Gln Val Asp Pro Arg Ala Gly Glu Glu Glu Gly His
   1160 1165 1170
   Glu
<210> 57
   <211> 2382
   <212> DNA
   <213> Erwinia tasmaniensis
<400> 57
   atgcatagta aagcatcaag aatactgatc ctgctaacgg ttatattcgc cgcgctaagc 60
   ggcttatatc tgttaatcgg cggtatctgg ttggctaaac ttggcggttc tctctactac 120
   atcatcgctg gtgtggttct gttggtcacc gcattcctgc tgcaccgccg tcgcggttca 180
   gcgctgctgc tgtacgcgct gttcctgctg ggtacgaccg tctggtcgct gtgggaagtg 240
   ggctctgact tctgggcgct gactccgcgt ctggatatca ccttcttcct cggactgtgg 300
   ttggtattgc catttatctg gcgtgagctg aacggcacag ggtcattctc ccgcgtggcc 360
   ctttctgccg tgctggtctt tgtcgtcgcg gtcctcgcct attcaatatt taacgatccg 420
   caggaaatca acgggtcact gaatgctgaa caggcggaag cggtgccggc tgaagatggc 480
   gtcgcaccgg gcgactggcc ggcctatggc cgtacccagg gcgggacgcg ctactctcct 540
   ctgaagcaga taaatgataa aaacgtgggt gaactgcagg aagcctggac tttccagacc 600
   ggtgacctga aatccccaag cgatccgggt gagatcacca atgaggccac gccgatcaaa 660
   atcggcaatg cgctctacct gtgcaccgcg catcagcagc tgttcgcgct ggatgcggcc 720
   accggtaaga agaagtggat gttcgatccg aagctgaagc cgaatccaac cttccagcac 780
   gtcacctgtc gcggcgtttc ttattatcag accccgcagg cggctgcgac gccggccgga 840
   accgaaccgg ctctctgctc acgtcgtatt ctgctgccgg tcaatgacgg cagcatgtat 900
   gcgctggacg cagaaaccgg cgcgctgtgc gaacagtttg gtgacaaagg cagactgaac 960
   ctgcagagca acatgccgta tgcaaaagtg ggttcttacg agccgacttc accgccgatc 1020
   gtcacggcca ccagcattgt aatggcgggc gcggttaccg ataactactc cactaaacag 1080
   ccgtctggcg tggtgcgtgg ttttgacgtg aacaccggca aactgctgtg ggcattcgac 1140
   agcggagcga aagatccgaa tctgctgccg catgacgatc agaagtatac gccgaactca 1200
   cctaactcgt gggcaccggc agcctatgat gacaagctgg acctggtcta tctgccgatg 1260
   ggtgtgtcaa cgccggatat ctggggcggt catcgtaccc cggaaatgga gcgtttcgcc 1320
   aacggcgtgc tggcgctgaa tgccaccacc ggtaaactgg catggttcta tcagaccgtg 1380
   catcacgacc tgtgggatat ggacgtacct gcacagccga ctctggccga tatcgacgac 1440
   aaagacggac acaaggtgcc ggtgatttat atcccgacca aaaccggcga tatctttgtg 1500
   ctgaaccgca ccaacggcca gcccgtggtg ccagcgccgg aaatggtggt gccgggtggc 1560
   cctgctaagg gcgatcgtct ttcgccgacc cagccatact ctgaactgag cttccgtcca 1620
   aaagcccatc tggcgggtaa agacatgtgg ggtgcaacca tctacgatca gctggtctgc 1680
   cgcgttatgt tccaccagct gcgctatgaa ggtccgttca ctccgccatc cgagcagggt 1740
   acattggtat tcccaggcaa cctcggtatg tttgagtggg gcggtatcgc ggtggatggc 1800
   gatcgtcagg ttgccatcac taacccaatg gcgctgccgt tcgtttcccg cctgatccca 1860
   cgcgggccgg gcaatcccat tgagccggat gaaaatgata agggcggcac cggtagcgag 1920
   aaaggtattc agccgcagta cggtttgccg tacggcgtga cgctgaaccc attcctctct 1980
   ccgatcggct taccttgtaa gcagccttca tggggctata tttctgcggt tgatctgaag 2040
   accaacgaaa tcgtgtggaa aaagcgtatt ggcaccgtgc gcgacagctc accgcttccg 2100
   cttccgttta aaatgggcat gccaatgctg ggtgggccag ttgccaccgc gggcaacctg 2160
   ttctttattg cggcaacggc agataactac ctgcgtgcgt ttaacgtgac taacggcaaa 2220
   cagctgtggg aagcgcgcct gcccgcgggt ggtcaggcaa cgccaatgac gtatgaagtt 2280
   aacggcaagc agtacgtgct gatttttgcg ggtggacatg gctcgtttgg caccaagctg 2340
   ggcgactatg tgaaggccta tgcactgccg gacagcaagt aa 2382
<210> 58
   <211> 793
   <212> PRT
   <213> Erwinia tasmaniensis
<400> 58
   Met His Ser Lys Ala Ser Arg Ile Leu Ile Leu Leu Thr Val Ile Phe
   1               5                   10                  15
   Ala Ala Leu Ser Gly Leu Tyr Leu Leu Ile Gly Gly Ile Trp Leu Ala
               20                  25                  30
   Lys Leu Gly Gly Ser Leu Tyr Tyr Ile Ile Ala Gly Val Val Leu Leu
   35 40 45
   Val Thr Ala Phe Leu Leu His Arg Arg Arg Gly Ser Ala Leu Leu Leu
   50 55 60
   Tyr Ala Leu Phe Leu Leu Gly Thr Thr Val Trp Ser Leu Trp Glu Val
   65 70 75 80
   Gly Ser Asp Phe Trp Ala Leu Thr Pro Arg Leu Asp Ile Thr Phe Phe
   85 90 95
   Leu Gly Leu Trp Leu Val Leu Pro Phe Ile Trp Arg Glu Leu Asn Gly
   100 105 110
   Thr Gly Ser Phe Ser Arg Val Ala Leu Ser Ala Val Leu Val Phe Val
   115 120 125
   Val Ala Val Leu Ala Tyr Ser Ile Phe Asn Asp Pro Gln Glu Ile Asn
   130 135 140
   Gly Ser Leu Asn Ala Glu Gln Ala Glu Ala Val Pro Ala Glu Asp Gly
   145 150 155 160
   Val Ala Pro Gly Asp Trp Pro Ala Tyr Gly Arg Thr Gln Gly Gly Thr
   165 170 175
   Arg Tyr Ser Pro Leu Lys Gln Ile Asn Asp Lys Asn Val Gly Glu Leu
   180 185 190
   Gln Glu Ala Trp Thr Phe Gln Thr Gly Asp Leu Lys Ser Pro Ser Asp
   195 200 205
   Pro Gly Glu Ile Thr Asn Glu Ala Thr Pro Ile Lys Ile Gly Asn Ala
   210 215 220
   Leu Tyr Leu Cys Thr Ala His Gln Gln Leu Phe Ala Leu Asp Ala Ala
   225 230 235 240
   Thr Gly Lys Lys Lys Trp Met Phe Asp Pro Lys Leu Lys Pro Asn Pro
   245 250 255
   Thr Phe Gln His Val Thr Cys Arg Gly Val Ser Tyr Tyr Gln Thr Pro
   260 265 270
   Gln Ala Ala Ala Thr Pro Ala Gly Thr Glu Pro Ala Leu Cys Ser Arg
   275 280 285
   Arg Ile Leu Leu Pro Val Asn Asp Gly Ser Met Tyr Ala Leu Asp Ala
   290 295 300
   Glu Thr Gly Ala Leu Cys Glu Gln Phe Gly Asp Lys Gly Arg Leu Asn
   305 310 315 320
   Leu Gln Ser Asn Met Pro Tyr Ala Lys Val Gly Ser Tyr Glu Pro Thr
   325 330 335
   Ser Pro Pro Ile Val Thr Ala Thr Ser Ile Val Met Ala Gly Ala Val
   340 345 350
   Thr Asp Asn Tyr Ser Thr Lys Gln Pro Ser Gly Val Val Arg Gly Phe
   355 360 365
   Asp Val Asn Thr Gly Lys Leu Leu Trp Ala Phe Asp Ser Gly Ala Lys
   370 375 380
   Asp Pro Asn Leu Leu Pro His Asp Asp Gln Lys Tyr Thr Pro Asn Ser
   385 390 395 400
   Pro Asn Ser Trp Ala Pro Ala Ala Tyr Asp Asp Lys Leu Asp Leu Val
   405 410 415
   Tyr Leu Pro Met Gly Val Ser Thr Pro Asp Ile Trp Gly Gly His Arg
   420 425 430
   Thr Pro Glu Met Glu Arg Phe Ala Asn Gly Val Leu Ala Leu Asn Ala
   435 440 445
   Thr Thr Gly Lys Leu Ala Trp Phe Tyr Gln Thr Val His His Asp Leu
   450 455 460
   Trp Asp Met Asp Val Pro Ala Gln Pro Thr Leu Ala Asp Ile Asp Asp
   465 470 475 480
   Lys Asp Gly His Lys Val Pro Val Ile Tyr Ile Pro Thr Lys Thr Gly
   485 490 495
   Asp Ile Phe Val Leu Asn Arg Thr Asn Gly Gln Pro Val Val Pro Ala
   500 505 510
   Pro Glu Met Val Val Pro Gly Gly Pro Ala Lys Gly Asp Arg Leu Ser
   515 520 525
   Pro Thr Gln Pro Tyr Ser Glu Leu Ser Phe Arg Pro Lys Ala His Leu
   530 535 540
   Ala Gly Lys Asp Met Trp Gly Ala Thr Ile Tyr Asp Gln Leu Val Cys
   545 550 555 560
   Arg Val Met Phe His Gln Leu Arg Tyr Glu Gly Pro Phe Thr Pro Pro
   565 570 575
   Ser Glu Gln Gly Thr Leu Val Phe Pro Gly Asn Leu Gly Met Phe Glu
   580 585 590
   Trp Gly Gly Ile Ala Val Asp Gly Asp Arg Gln Val Ala Ile Thr Asn
   595 600 605
   Pro Met Ala Leu Pro Phe Val Ser Arg Leu Ile Pro Arg Gly Pro Gly
   610 615 620
   Asn Pro Ile Glu Pro Asp Glu Asn Asp Lys Gly Gly Thr Gly Ser Glu
   625 630 635 640
   Lys Gly Ile Gln Pro Gln Tyr Gly Leu Pro Tyr Gly Val Thr Leu Asn
   645 650 655
   Pro Phe Leu Ser Pro Ile Gly Leu Pro Cys Lys Gln Pro Ser Trp Gly
   660 665 670
   Tyr Ile Ser Ala Val Asp Leu Lys Thr Asn Glu Ile Val Trp Lys Lys
   675 680 685
   Arg Ile Gly Thr Val Arg Asp Ser Ser Pro Leu Pro Leu Pro Phe Lys
   690 695 700
   Met Gly Met Pro Met Leu Gly Gly Pro Val Ala Thr Ala Gly Asn Leu
   705 710 715 720
   Phe Phe Ile Ala Ala Thr Ala Asp Asn Tyr Leu Arg Ala Phe Asn Val
   725 730 735
   Thr Asn Gly Lys Gln Leu Trp Glu Ala Arg Leu Pro Ala Gly Gly Gln
   740 745 750
   Ala Thr Pro Met Thr Tyr Glu Val Asn Gly Lys Gln Tyr Val Leu Ile
   755 760 765
   Phe Ala Gly Gly His Gly Ser Phe Gly Thr Lys Leu Gly Asp Tyr Val
   770 775 780
   Lys Ala Tyr Ala Leu Pro Asp Ser Lys
   785 790
<210> 59
   <211> 2400
   <212> DNA
   <213> Burkholderia xenovorans
<400> 59
   atgcacagtt ccgcctcaat tcgcgtagcg catttgctta gcgttctgtt tgcctcattg 60
   tcgggggtgt acctgctggt cggcgggatc tggctagcgg cgaccggcgg atcgctgtac 120
   tacgttttcg ccggtatcgt catgctcgtt acggcggttc tgatttatcg gcgttcccca 180
   ctagcactcg gcctctacgc attactgctg ttgtgcacga tcgtgtggtc gctgtgggag 240
   gtcggcacgg atttctgggc tcttgccccg cgtctggacg tactggtcgt cttcggcatc 300
   tggctgctgt tgccgtatgt ctatcgcgcg ttcgagccgt ccgcaaaaac gcatggtctc 360
   gcgctcgggg ccacgttgat cgtgagcgtc gcagtgctgg tttttgcagt cttcaacgac 420
   ccacaggaaa tcaacggcac gctggcgagc gcgcctgccc aggcggcggc gacgcccgcc 480
   ggcgagcgcg tcgccgaggg cgactggccc gcctacggcc gtacccagca cggtacgcgc 540
   tactctccgc tcaagcagat caacgcggat aacgtcaagg atctgaaggt cgcgtggata 600
   ttccggaccg gcgacatgaa gcgctccacc gacccaggtg aaatcacgaa cgaagttacc 660
   ccgatcaagg tgggcgacat gctgtatctg tgctcgccgc accagatact gttcgcgctt 720
   gacgctgcca ccggccagga aaaatggcgc ttcgatccca agctgaagac cgatcccagt 780
   tttcagcatg tcacgtgccg tggggtgtcg taccatgatt cgaccgcttc gggcagtaac 840
   aacgacagcc cggaccaggc cccggctacg gcgcctgcaa tctgcgcgcg ccgcgtctac 900
   ctgcctgtca acaacgggca tctgtacgcg ctcgacgcgc agaccggcca gctatgcccg 960
   gacttcgcca atcacggcga cctcgacctg caagtgggcc agcccgtgac gaccgcgggt 1020
   caatatgaac ccacttcgcc gccggtcatc accgggaagg tgatcgtaat cgcaggctca 1080
   gtcgaggata actattcgac gcgggagccc tcgggagtga tccggggttt cgatgtcaac 1140
   accggcaaac tgttgtgggc gttcgatccg ggcgcgaaga acccggaagc ggtgctcggc 1200
   gcaggccagc attattcggt caactcgccg aactcctggg caccggctgt ttatgacgca 1260
   aagctcgatc ttgtctatct gccgatgggc gtaagcacgc ccgacatctg gggcggctac 1320
   cgcacgccgg agcaggaacg ctttgcgagc gggctgctcg cgctcaacgc gacgaccggc 1380
   aagctcgcct ggttttatca gacggtccac cacgacctgt gggacatgga cctgccggcg 1440
   cagccgacgc tcgccgatat cacggaccgg tcgggcaacc tcgtgccggc ggtctacgcc 1500
   ccggcaaaga cgggcaacat tttcgttctt gaccgccgca caggcacgcc gatcgttcct 1560
   gctccggaga agccggttcc gcaaggcgcg gcgcaaggcg atcacgtgtc gcccacccag 1620
   ccgttttcgg cgctgacctt ccggccggac aggaaactga ccggcgcgga catgtggggc 1680
   gcaacgatgt ttgaccagct cgtgtgccga gtgatgttcc agcgcctgaa ctatgacggc 1740
   acgtttaccc ctccgtcggt gaagggcacc ctggtttttc cgggtaacct cgggatgttc 1800
   gagtggggcg gcatagcagt ggataccgat cgccagatcg cgattgcgaa cccgatcgca 1860
   ctgccgttcg tgtccaggtt gatgccgcgt gggccgggca acccgatcga accggccgcc 1920
   ggcggcacgg gcggcagcgg taccgaatcc ggtatccagc cgcagtacgg cgtgcccttc 1980
   ggtgtgacgt tgaatgcctt catgtcgccg ctcggcctgc cgtgcaagca gccggcgtgg 2040
   ggctacatat ccgcggtcga tctgaagacc aaccagattg tctggaaaaa gcgcattggc 2100
   accgtgcgtg acagttcacc gctgccgctg ccgttcaaga tggggatgcc gatgctcggc 2160
   ggcccgatga caacggccgg cgacgtgttc ttcatcgggg ccaccgccga caactacatc 2220
   cgcgcgttcg acaccaacac cggcaagcag ttgtggcaag cacgtttgcc cgcgggtggc 2280
   caggcgacgc caatgaccta cgaggcgaaa gggaagcagt acgtggtcat cgcggccggc 2340
   ggccacggct cgttcggcac caggttgggc gactacgtga tggcttatgc gctgccttga 2400
<210> 60
   <211> 799
   <212> PRT
   <213> Burkholderia xenovorans
<400> 60
   Met His Ser Ser Ala Ser Ile Arg Val Ala His Leu Leu Ser Val Leu
   1 5 10 15
   Phe Ala Ser Leu Ser Gly Val Tyr Leu Leu Val Gly Gly Ile Trp Leu
   20 25 30
   Ala Ala Thr Gly Gly Ser Leu Tyr Tyr Val Phe Ala Gly Ile Val Met
   35 40 45
   Leu Val Thr Ala Val Leu Ile Tyr Arg Arg Ser Pro Leu Ala Leu Gly
   50 55 60
   Leu Tyr Ala Leu Leu Leu Leu Cys Thr Ile Val Trp Ser Leu Trp Glu
   65 70 75 80
   Val Gly Thr Asp Phe Trp Ala Leu Ala Pro Arg Leu Asp Val Leu Val
   85 90 95
   Val Phe Gly Ile Trp Leu Leu Leu Pro Tyr Val Tyr Arg Ala Phe Glu
   100 105 110
   Pro Ser Ala Lys Thr His Gly Leu Ala Leu Gly Ala Thr Leu Ile Val
   115 120 125
   Ser Val Ala Val Leu Val Phe Ala Val Phe Asn Asp Pro Gln Glu Ile
   130 135 140
   Asn Gly Thr Leu Ala Ser Ala Pro Ala Gln Ala Ala Ala Thr Pro Ala
   145 150 155 160
   Gly Glu Arg Val Ala Glu Gly Asp Trp Pro Ala Tyr Gly Arg Thr Gln
                   165                 170                 175
   His Gly Thr Arg Tyr Ser Pro Leu Lys Gln Ile Asn Ala Asp Asn Val
               180                 185                 190
   Lys Asp Leu Lys Val Ala Trp Ile Phe Arg Thr Gly Asp Met Lys Arg
           195                 200                 205
   Ser Thr Asp Pro Gly Glu Ile Thr Asn Glu Val Thr Pro Ile Lys Val
       210                 215                 220
   Gly Asp Met Leu Tyr Leu Cys Ser Pro His Gln Ile Leu Phe Ala Leu
   225                 230                 235                 240
   Asp Ala Ala Thr Gly Gln Glu Lys Trp Arg Phe Asp Pro Lys Leu Lys
                   245                 250                 255
   Thr Asp Pro Ser Phe Gln His Val Thr Cys Arg Gly Val Ser Tyr His
               260                 265                 270
   Asp Ser Thr Ala Ser Gly Ser Asn Asn Asp Ser Pro Asp Gln Ala Pro
           275                 280                 285
   Ala Thr Ala Pro Ala Ile Cys Ala Arg Arg Val Tyr Leu Pro Val Asn
       290                 295                 300
   Asn Gly His Leu Tyr Ala Leu Asp Ala Gln Thr Gly Gln Leu Cys Pro
   305                 310                 315                 320
   Asp Phe Ala Asn His Gly Asp Leu Asp Leu Gln Val Gly Gln Pro Val
                   325                 330                 335
   Thr Thr Ala Gly Gln Tyr Glu Pro Thr Ser Pro Pro Val Ile Thr Gly
               340                 345                 350
   Lys Val Ile Val Ile Ala Gly Ser Val Glu Asp Asn Tyr Ser Thr Arg
           355                 360                 365
   Glu Pro Ser Gly Val Ile Arg Gly Phe Asp Val Asn Thr Gly Lys Leu
       370                 375                 380
   Leu Trp Ala Phe Asp Pro Gly Ala Lys Asn Pro Glu Ala Val Leu Gly
   385                 390                 395                 400
   Ala Gly Gln His Tyr Ser Val Asn Ser Pro Asn Ser Trp Ala Pro Ala
                   405                 410                 415
   Val Tyr Asp Ala Lys Leu Asp Leu Val Tyr Leu Pro Met Gly Val Ser
   420 425 430
   Thr Pro Asp Ile Trp Gly Gly Tyr Arg Thr Pro Glu Gln Glu Arg Phe
   435 440 445
   Ala Ser Gly Leu Leu Ala Leu Asn Ala Thr Thr Gly Lys Leu Ala Trp
   450 455 460
   Phe Tyr Gln Thr Val His His Asp Leu Trp Asp Met Asp Leu Pro Ala
   465 470 475 480
   Gln Pro Thr Leu Ala Asp Ile Thr Asp Arg Ser Gly Asn Leu Val Pro
   485 490 495
   Ala Val Tyr Ala Pro Ala Lys Thr Gly Asn Ile Phe Val Leu Asp Arg
   500 505 510
   Arg Thr Gly Thr Pro Ile Val Pro Ala Pro Glu Lys Pro Val Pro Gln
   515 520 525
   Gly Ala Ala Gln Gly Asp His Val Ser Pro Thr Gln Pro Phe Ser Ala
   530 535 540
   Leu Thr Phe Arg Pro Asp Arg Lys Leu Thr Gly Ala Asp Met Trp Gly
   545 550 555 560
   Ala Thr Met Phe Asp Gln Leu Val Cys Arg Val Met Phe Gln Arg Leu
   565 570 575
   Asn Tyr Asp Gly Thr Phe Thr Pro Pro Ser Val Lys Gly Thr Leu Val
   580 585 590
   Phe Pro Gly Asn Leu Gly Met Phe Glu Trp Gly Gly Ile Ala Val Asp
   595 600 605
   Thr Asp Arg Gln Ile Ala Ile Ala Asn Pro Ile Ala Leu Pro Phe Val
   610 615 620
   Ser Arg Leu Met Pro Arg Gly Pro Gly Asn Pro Ile Glu Pro Ala Ala
   625 630 635 640
   Gly Gly Thr Gly Gly Ser Gly Thr Glu Ser Gly Ile Gln Pro Gln Tyr
   645 650 655
   Gly Val Pro Phe Gly Val Thr Leu Asn Ala Phe Met Ser Pro Leu Gly
   660 665 670
   Leu Pro Cys Lys Gln Pro Ala Trp Gly Tyr Ile Ser Ala Val Asp Leu
   675 680 685
   Lys Thr Asn Gln Ile Val Trp Lys Lys Arg Ile Gly Thr Val Arg Asp
   690 695 700
   Ser Ser Pro Leu Pro Leu Pro Phe Lys Met Gly Met Pro Met Leu Gly
   705 710 715 720
   Gly Pro Met Thr Thr Ala Gly Asp Val Phe Phe Ile Gly Ala Thr Ala
   725 730 735
   Asp Asn Tyr Ile Arg Ala Phe Asp Thr Asn Thr Gly Lys Gln Leu Trp
   740 745 750
   Gln Ala Arg Leu Pro Ala Gly Gly Gln Ala Thr Pro Met Thr Tyr Glu
   755 760 765
   Ala Lys Gly Lys Gln Tyr Val Val Ile Ala Ala Gly Gly His Gly Ser
   770 775 780
   Phe Gly Thr Arg Leu Gly Asp Tyr Val Met Ala Tyr Ala Leu Pro
   785 790 795
<210> 61
   <211> 2361
   <212> DNA
   <213> Pantoea citrea
<400> 61
<210> 62
   <211> 786
   <212> PRT
   <213> Pantoea citrea
<400> 62
   Met Ser Arg Val Ser Thr Ile Leu His Gly Leu Thr Arg Ile Phe Ser
   1               5                   10                  15
   Leu Phe Cys Ala Ala Trp Leu Leu Ile Gly Gly Val Trp Leu Leu Ser
               20                  25                  30
   Val Gly Gly Ser Ala Tyr Tyr Leu Ile Cys Gly Ile Ala Met Ala Ile
   35 40 45
   Phe Ser Ile Leu Leu Trp Lys Arg Arg Ser Ser Ala Phe Tyr Leu Tyr
   50 55 60
   Ser Leu Ile Leu Ile Leu Ser Ala Leu Trp Ala Trp Arg Glu Ala Gly
   65 70 75 80
   Thr Asp Phe Trp Asn Leu Val Pro Arg Leu Asp Ile Trp Ile Leu Phe
   85 90 95
   Gly Ile Trp Leu Ile Leu Pro Phe Ser Tyr Arg Arg Phe Asn Thr Ala
   100 105 110
   Gly Lys Lys Pro Leu Leu Ala Met Val Ile Gly Leu Gly Ile Asn Ala
   115 120 125
   Leu Leu Leu Leu Gly Ala Ser Leu His Asp Pro Gln Glu Ile Asn Gly
   130 135 140
   Val Leu Asn Val Ser Asp Lys Pro Pro Ala Glu Ser Ala Ala Ser Ala
   145 150 155 160
   Ala Asp Trp Pro Ala Tyr Gly Arg Thr Gln Glu Gly Val Arg Tyr Ser
   165 170 175
   Pro Leu Thr Gln Ile Asn Asp Lys Asn Val Gln Gln Leu Gln Val Ala
   180 185 190
   Trp Gln Phe His Thr Gly Asp His Lys Thr Ala Asn Asp Pro Gly Glu
   195 200 205
   Ile Thr Asn Glu Val Thr Pro Leu Lys Val Gly Asn Met Leu Tyr Leu
   210 215 220
   Cys Thr Pro His Gln Ile Leu Ile Ala Leu Asp Ala Ala Ser Gly Arg
   225 230 235 240
   Glu Lys Trp Arg Phe Asp Pro Gln Leu Lys Ser Asp Pro Thr Phe Gln
   245 250 255
   His Ile Thr Cys Arg Gly Val Ser Tyr His Glu Ile Lys Ser Val Gln
   260 265 270
   Gly Asp Ser Ser Ala Pro Ala Ala Cys Ser Arg Arg Ile Phe Leu Pro
   275 280 285
   Val Asp Asp Gly Arg Leu Phe Ala Val Asp Ala Leu Thr Gly Gln Arg
   290 295 300
   Cys Ser Asn Phe Ala Asn Asn Gly Glu Leu Asn Leu Gln His Leu Gln
   305 310 315 320
   Pro Asn Ala Tyr Pro Gly Gly Tyr Glu Pro Thr Ser Pro Pro Ile Ile
   325 330 335
   Thr Asp Lys Val Val Ile Ile Ala Gly Ser Val Thr Asp Asn Leu Ser
   340 345 350
   Thr Arg Glu Pro Ser Gly Val Ile Arg Gly Phe Asp Ile Asp Ser Gly
   355 360 365
   Lys Leu Leu Trp Val Phe Asp Pro Gly Ala Lys Asp Pro Asn Ala Val
   370 375 380
   Pro Ala Asp Gly Gln Thr Phe Val Ala Asn Ser Pro Asn Ser Trp Ala
   385 390 395 400
   Pro Ala Ala Tyr Asp Ala Gln Arg Asp Ile Ile Tyr Leu Pro Met Gly
   405 410 415
   Val Ser Thr Pro Asp Ile Trp Gly Gly Ala Val Thr Arg Cys Arg Asn
   420 425 430
   Val Leu Pro Ala Val Tyr Trp Leu Cys Met Pro Gln Leu Ala Ser Trp
   435 440 445
   His Gly Phe Thr Arg Arg Tyr Thr Met Gln Ser Phe Trp Asp Met Asp
   450 455 460
   Leu Pro Ser Gln Pro Thr Leu Ala Asp Ile Thr Asp Glu Gln Gly Lys
   465 470 475 480
   Thr Val Pro Val Val Tyr Val Pro Ala Lys Thr Gly Asn Ile Phe Val
   485 490 495
   Leu Asn Arg Asp Thr Gly Lys Pro Val Val Pro Ala Pro Glu Thr Pro
   500 505 510
   Val Pro Gln Gly Pro Ala Lys Gly Asp His Leu Ser Pro Thr Gln Pro
   515 520 525
   Phe Ser Glu Leu Thr Phe Arg Pro Lys Asn Lys Leu Gln Gly Arg Asp
   530 535 540
   Met Trp Gly Ala Thr Met Phe Asp Gln Leu Met Cys Arg Val Met Phe
   545 550 555 560
   His Lys Leu Arg Tyr Glu Gly Pro Phe Thr Pro Pro Ser Glu Gln Gly
   565 570 575
   Thr Leu Val Phe Pro Gly Asp Phe Gly Met Phe Glu Trp Gly Gly Ile
   580 585 590
   Ser Val Asn Thr Asp Gln Gln Phe Ala Ile Ala Asn Pro Met Ala Met
   595 600 605
   Pro Phe Ile Ser Lys Leu Ile Pro Arg Gly Pro Gly Asn Pro Ile Glu
   610 615 620
   Pro Gly Ala Asp Gly Ala Ala Gly Ser Gly Ser Glu Ser Gly Val Gln
   625 630 635 640
   His Met Tyr Gly Val Pro Tyr Gly Val Glu Leu Asn Pro Phe Leu Ser
   645 650 655
   Pro Leu Gly Leu Pro Cys Leu Gln Pro Ser Trp Gly Phe Val Ser Ala
   660 665 670
   Ile Asn Leu Arg Asn His Gln Ile Ile Trp Lys Lys Arg Ile Gly Thr
   675 680 685
   Val Arg Asp Ser Ala Pro Val Pro Leu Pro Phe Lys Met Gly Val Pro
   690 695 700
   Met Leu Gly Gly Pro Val Thr Thr Ala Gly Asn Ile Phe Phe Val Ala
   705 710 715 720
   Gly Thr Leu Asp Asn Tyr Leu Arg Ala Tyr Ser Val Arg Asp Gly Lys
   725 730 735
   Leu Leu Trp Gln Ala Arg Leu Pro Ala Gly Gly Gln Ala Thr Pro Met
   740 745 750
   Thr Tyr Glu Val Asp Gly Lys Gln Tyr Val Val Ile Met Ala Gly Gly
   755 760 765
   His Gly Ser Phe Gly Thr Arg Leu Gly Asp Ser Leu Ile Ala Tyr Lys
   770 775 780
   Leu Pro
   785
<210> 63
   <211> 5498
   <212> DNA
   <213> Klebsiella pneumoniae
<400> 63
   catgtggaag aaacctgctt ttatcgattt acgtctcggt ctggaagtga cgctgtacat      60
   ttctaaccgt taatcgcccc gcccgccgtt cgcgcgggca ccttcattca ttacccggtc     120
   cgtcttcatg ttcattaaag tcctcggctc cgccgccggc ggcggtttcc cgcaatggaa     180
   ctgcaactgc gccaactgtc agggtctgcg caacggcacc attcaggcca gtgcccgcac     240
   ccagtcgtcg atcatcgtca gcgataacgg caaagagtgg gtgctgtgca atgcctcgcc     300
   ggatatcagc cagcagattg cccatacccc cgagttaaat aaacccggcg tactgcgcgg     360
   gacgtctatc ggcggcatta ttctcaccga cagccagatc gaccacacca ccgggttgct     420
   gagcctgcgc gaaggctgcc cgcaccaggt gtggtgcacg ccggaggttc atgaggatct     480
   ctccaccggc ttcccggtgt ttaccatgct gcgacactgg aacggcggcc tggtgcatca     540
   tcccatcgcg ccgcagcagc cttttaccgt tgacgcctgc cctgatttgc agtttaccgc     600
   cgtgcctatc gccagcaacg cgccgcccta ttcgccgtat cgcgaccggc cgctgccagg     660
   ccataacgtg gcgctgttta tcgaataccg ccgcaacggg cagacgctgt tctatgcccc     720
   ggggctgggt gagccggatg aagcccttct gccgtggctg caaaaagcgg actgtctgct     780
   gatcgatggc accgtctggc aggatgacga gctgcaggcc gccggcgtcg ggcgcaatac     840
   cggccgtgat atgggacacc tggcgctcag cgatgagcac gggatgatgg ccctgctggc     900
   ctctctgccg gcaaaacgca aaattctcat tcatattaat aacaccaacc cgatccttaa     960
   cgagctgtct ccccagcgcc aggcgctaaa acaacagggg attgaagtga gctgggacgg    1020
   gatggcaatc acccttcagg ataccgcatg ctgatcaccg acacgctgtc gccgcaggcc    1080
   tttgaagagg ctctgcgggc taaaggcgcc ttctaccata ttcaccaccc ttaccacatc    1140
   gccatgcata acggcgaagc gacccgcgag caaattcagg gttgggtggc gaaccggttt    1200
   tattaccaga ccaccattcc gctgaaagac gcggcgatta tggctaactg cccggatgcg    1260
   cagacccggc gcaaatgggt gcagcggatc ctcgaccacg acggtagcca cggcgaagat    1320
   ggcgggattg aagcctggct gcggctgggg gaagcagtcg gtttgagccg cgacgacctg    1380
   ctcagcgagc gtcacgtgct gcccggcgtg cgcttcgcgg tggatgccta tcttaatttc    1440
   gctcgtcgcg cctgctggca ggaggcggcc tgcagctccc tgaccgagct gttcgcccca    1500
   cagatccatc agtcgcgcct cgacagctgg ccgcagcact atccgtggat caaagaggaa    1560
   ggctattttt acttccgcag tcgtctgagc caggctaacc gcgacgttga gcatggtctg    1620
   gcgctggcga agacctactg tgacagcgct gaaaaacaga accggatgct ggagatcctg    1680
   cagtttaagc tcgacatcct gtggtcgatg ctcgatgcca tgaccatggc ctacgctctg    1740
   caacgcccgc cctatcacac ggtcaccgac aaggcggcct ggcacacgac ccgactggtg    1800
   taatcatgca aaaaacgtcc atcgttgcct ttcgtcgcgg ctaccgactg cagtgggaag 1860
   ccgcccagga gagccatgtg atcctctatc cggagggaat ggctaaactc aatgagaccg 1920
   ccgcggcgat cctcgagctg gtcgatggcc ggcgcgacgt cgcggcgatt atcgccatgc 1980
   ttaacgaacg tttcccggaa gccggcggcg tcgatgacga cgtcgtcgag ttcctgcaga 2040
   tcgcctgtca acagaagtgg atcacctgcc gtgagccaga ataaacccgc cgtcaatccg 2100
   ccgctgtggc tgctggcgga gctgacctac cgctgcccgc tgcagtgtcc ctactgttcc 2160
   aatccgctgg acttcgcccg gcaggaaaag gagctgacca ccgaacaatg gatcgaggtc 2220
   tttcgccagg cgcgagcgat gggcagcgta cagctgggct tttccggcgg cgagccgctg 2280
   acccgtaaag atctgccgga gctgatccgc gccgcgcgcg acctcgggtt ctataccaac 2340
   ctgatcacct cgggaattgg gctaaccgag agcaaactcg acgccttcag cgaggccgga 2400
   ctggaccata tccagattag cttccaggcc agcgatgagg tgctcaacgc cgctcttgcc 2460
   ggcaataaaa aagccttcca gcagaagctg gcgatggcca gagcggtgaa agcgcgcgac 2520
   tacccgatgg tgctgaactt cgtcctccac cggcataaca tcgaccagct cgataaaatt 2580
   atcgagctgt gcattgagct ggaagccgat gacgtcgagc tcgccacctg ccagttttac 2640
   ggctgggcgt ttcttaatcg cgaggggtta ctgccgaccc gggaacagat cgcccgcgcc 2700
   gagcaggtgg tcgccgatta ccggcagaaa atggccgcca gcggtaacct caccaacctg 2760
   ctattcgtca ccccggacta ttacgaggaa cgcccgaaag gctgtatggg cggctgggga 2820
   tcgattttcc tcagcgtcac tccggaaggc actgcgttgc cgtgccacag cgcgcgccag 2880
   ctgccggtgg cgttcccgtc ggtgctggag cagagtctgg aatcgatctg gtatgactcg 2940
   ttcggcttca accgttatcg cgggtatgac tggatgccgg agccgtgccg ctcctgtgat 3000
   gaaaaagaga aagacttcgg cggctgccgc tgtcaggcct ttatgctgac cggcagcgcc 3060
   gataacgccg acccggtgtg cagcaaatcc ccacatcatc acaaaatcct tgaggcccgg 3120
   cgcgaagcgg cctgcagcga catcaaagtc agccagctgc agttccgcaa ccgtacccgc 3180
   tcgcagctta tctacaaaac ccgggaactg taatgacgct ggcgacccgc actgtcactc 3240
   tgccgggcgg cctgcaggct accctggttc atcagccgca ggccgatcgc gcggcggccc 3300
   tggtgcgggt tgccgccggc agccaccatg aaccgtcgtg cttccccggt ctggcgcacc 3360
   tgctggaaca cctgctgttt tacggcggtg agcgctaccg caatgatgaa cggctgatga 3420
   gctgggtgca gcgccaggca gggaatgtga atgcctccac cctgtcccgc cacagcgcgt 3480
   tctttttcga ggtcgccgcc gaggatctgg ctgacggcgt cgcgcgcctg caggagatgc 3540
   tgcaggcgcc gctgctgctc agggacgata ttcaacgcga agtcgcggtt atcgacgccg 3600
   aaaacggcct gatccaacag catgagttgt cgcgacggga agccgccgtg cgtcacgccg 3660
   ccatcgcgcc cgcggcgttt cgccgctttc aggtcggcga cgccgggtcg ctgggggagg 3720
   atttcctcgc gctacaggcg gccttacgtg actttcaccg cagccactac gtcgcccgcc 3780
   ggatgcaact ctggctgcag gggccgcagt cgctggaggt gctcggcgaa ctggcgaccc 3840
   gtttcgccac tgggcttgcc ccgggcgagg caccgccgcc agcgccgccg ctcagtctgg 3900
   gcgagccccc tcaactgcag ttggccgtct ccagccagcc cgcgctgtgg cgctgcccgc 3960
   tgatcgcctt aagtgacaat gtcacgttac tgcgcgagtt tttgctggat gaagcccccg 4020
   gtagcctgat ggccggcctg cgccagcgcg ggatggccga ggacgtggcg ctgaactggc 4080
   tgtatcagga tcagcacttc ggctggctgg cgctgatttt cgccagcgac cggccggaac 4140
   aggtcgaccg gcagataacc cactggttgc aggcgctaca gcagacgacg cctgagcagc 4200
   agcaacacta ctatcagctg tcccggcgcc gttttcaggc gctgtcgccc ctcgatcagc 4260
   tgcgccagcg ggcattcggc tttgcccccg gggcgccgcc caccgggttc gccgattttt 4320
   gcgccgccct gctggccgcc cccacggtca gcctggcctg ccagacgcag ccccccggag 4380
   caacggtagc cacccagggc tttagcctgc cgctcagccg ctggtcgcca cgtccggtct 4440
   ctgacccggc gctggcattc gctttttatc cgcaggccgc tggcgagctc gtggccgaaa 4500
   gcccggcgga agccgcgcca ctgcgtcacc tcccgtcacc gggagagccg ccgacgctcc 4560
   tgctgcgacc gcccttctac tgctcgccca cgccggccgg ggggctggcg cgcggggaac 4620
   agctgcgtcc attacttgcc gccctgcgcc atgccggggg acacggcgag tggcatctgt 4680
   tcgacggcag ctggcagctg atcctgcagt tgcctgcgtc cggccaatgg ccggaggcga 4740
   ttctgcaggc catcgtgcgg cagctcgcgc tcccggtcgc cccgctgccc ccaccgccgg 4800
   agagtattgc gatccgtcat ctcatggccc agctccccga acggctgggt acgtcagcgc 4860
   accaggaagg ttggctggcg gccctgattg gcggcagcgc ggaagatgcg cagtgggtag 4920
   cgcgtcagct gagccggctt accgtcccgg ttaatccgcc gatgcccgct ccggccacct 4980
   gccgcggcgg cgtcgagcgg ctggcttatc cccggggcga cacggcgcta ctggtctttc 5040
   ttccgctgcc ggaaggcgct tcattggcgg ccctgcgggt gctggcgcag ttctgcgagc 5100
   cgccgttttt ccagcgcctg cgggtggagc agcagatagg ctatgtggtg agctgccgct 5160
   atcagcgcgt tgccgatcgc gacggactgc tgatggcgct ccagtccccg gatcgccgcc 5220
   ccggggagct actccgctgc tgtaaaacct ttctgcgcca gctggccccc gtggatgagg 5280
   cgaccttcag gttgttacag cagcggctgg ccgctcaggc ccgcgcccgg gtagagccgc 5340
   aggtgcgcgc cctggacgcg ctgcgccagg agtataactt gccgggggtg acgccgcagg 5400
   cggctgacgc gctgcgcgtt gaagaggtgg tcgccctgtg gcatgagatg acccgccggc 5460
   gtcgtcgctg gcgggtgctg ttcacgacag ggagttaa 5498
<210> 64
   <211> 3473
   <212> DNA
   <213> Methylobacterium extorguens
<400> 64
   atgaagtggg ctgcccccat cgtttccgag atctgcgtcg gcatggaagt cacgagctac 60
   gagtcggccg agatcgacac cttcaactaa ggtgatttga gccgggttgg ggttgcaggc 120
   atcagcgggt tttcaccatg catgtcgtaa tcctgggctc ggctgcgggc ggcggcgttc 180
   ctcaatggaa ctgccgctgc tccatctgct ccctggcctg ggcgggcgat tcccgcgtca 240
   ggccgcgcac gcagtcgagc atcgcagtct ctcctgacgg ggaacgctgg ctcctgctga 300
   acgcctctcc cgatatccgt cagcagatcc aggccaatcc gcagatgcat ccgcgcgagg 360
   gcctgcgcca ctcgccgatc cacgcggtgc tgctgacgaa cggcgacgtc gatcacgttg 420
   cgggcctgct gaccctgcgc gagggccagc ccttcacgct ctacgcgaca cccggcatcc 480
   tggcctccgt ctccgacaac cgcgtcttcg acgtgatggc cgccgacgtg gtgaagcggc 540
   agacgatcgc cctcaacgag accttcgagc cggtgcccgg cctctcggtg acgctgttct 600
   ccgtccccgg caaggtgccg ctctggctgg aagacgcctc gatggagatc ggggcggaga 660
   ccgaaaccac ggtcggcacg atgatcgagg ccgggggcaa gcgcctcgcc tacatccccg 720
   gctgcgcccg ggtgacggag gatctcaaag cccgcatcgc cggcgcagac gcgctcctgt 780
   tcgacggcac ggtgctggag gacgacgaca tgatccgcgc cggtgtcggc accaagaccg 840
   gctggcgcat gggccatatc cagatgaacg gcgagaccgg ctcgatcgcg tctctcgccg 900
   atatcgagat cggccgacgg gtcttcgttc acatcaacaa caccaatccg gtcctgatcg 960
   aggattcgta cgagcgcgcg agcgtcgagg cgcgcggctg gaccgtcgcc catgacggcc 1020
   tgaccctcga tctctgatca ggctgatgtc ttgggaagag cccggtctgg aaatttagtg 1080
   ccggactgaa tatgttttgc acgatccaat cgtgcggcag cggccctgcc ccgatcggta 1140
   ccgggcccca tttaaaaata aatccaggaa acgcgactcg aagctcgggg gaaaccgaac 1200
   gccatgaccg cccaattccc gccgcccgtc ccggacaccg agcaacgcct gctgagccac 1260
   gaggagcttg aggcggcgct ccgcgatatc ggtgcacggc gctaccacaa cctccacccg 1320
   ttccaccggc tgctgcacga cggcaagctg tcgaaggatc aggtccgggc ctgggcgctc 1380
   aaccgctact attatcaggc gatgattccg gtgaaggatg cagcgctgct ggctcgcctg 1440
   ccggatgcgc agcttcgccg aatctggcgc cagcgcatcg tcgatcacga cggcgaccat 1500
   gagggcgacg gcggcatcga gcgttggctc aagcttgccg aaggcgtcgg cttcacccgc 1560
   gactacgtgc tctcgaccaa gggcatcctg tcggcgaccc gcttctcggt cgatgcctat 1620
   gtccacttcg tctccgagcg cagcctgctc gaagccatcg cctcctcgct gaccgagatg 1680
   ttctcgccga cgatcatctc cgagcgcgtc gccgggatgc tgaagaacta cgacttcatc 1740
   accaaggaca cgctggccta tttcgacaag cgcctgaccc aggccccgcg cgacgccgat 1800
   ttcgccctcg actacgtcaa gcggcacgcc accacgcctg agatgcagcg ggcggcgata 1860
   gatgcgttga cgttcaagtg caacgtgctc tggacgcaac tcgatgcgct ctacttcgcc 1920
   tatgtcgccc ccggcatggt gccgccggat gcttggcagc cgggcgaggg ccttgttgcc    1980
   gagacgaact ccgccgagga cagccccgcc gctgcggcca gccccgccgc gacgacagct    2040
   gaacccacgg ccttctcggg cagtgacgtg ccgcgcctgc cccgcggcgt gcgcctgcgc    2100
   ttcgacgagg tccgcaacaa gcacgtgctg ctcgcccccg agcgcacctt cgacctcgac    2160
   gacaacgccg tcgcggtcct caagctcgtc gatggccgga acacggtttc gcagatcgcc    2220
   cagattctgg gtcagaccta cgacgccgac ccggccatca tcgaagccga catcctcccg    2280
   atgctggccg gcctcgcgca aaaaagggtt ctggagcgat gaatgcaccg acacccgccc    2340
   cctcccccgt ggacgtcatt ccggcgccgg tgggtctgct cgccgagctg acgcaccgct    2400
   gcccgctgcg ctgcccatac tgctcgaacc cgctggagct cgaccggcgc tcggccgagc    2460
   tggacacgca gacgtggctg cgggtgctga cggaggcggc ggggctcggt gtgctgcacg    2520
   tccacctgtc gggcggtgaa ccgaccgccc gccccgacat cgtcgagatc acggccaaat    2580
   gcgccgaact cggcctgtac tcgaacctga tcacctccgg cgtcggcggt gccttagcga    2640
   agctcgacgc gctctacgac gtcggcctcg accacgtgca gctctccgtc caaggggtgg    2700
   acgcggccaa cgcggaaaag atcggcggcc ttaagaacgc gcagccgcag aagatgcaat    2760
   tcgctgcccg ggtcaccgaa ctcggcctgc cgctgacgct gaactcggtg atccaccgcg    2820
   gcaacatcca cgaggtgccg ggcttcatcg acctcgcggt caagctcggc gccaagcggc    2880
   tggaggtggc ccatacccag tattacggct gggcctatgt gaaccgcgcc gcgctgatgc    2940
   cggataagag ccaggtcgac gagtcgatcc gcatcgtcga ggccgcgcgc gagcgcctca    3000
   agggtcagct cgtcatcgac ctcgtggttc cggactacta cgccaagtac ccgaaggcct    3060
   gcgccggcgg ctggggccgc aagctgatga acgtgacgcc gcagggcaag gtgctgccct    3120
   gccacgccgc agaaaccatc cccggcctcg aattctggta cgtcaccgac cacgcgctcg    3180
   gcgagatctg gacgaagtcc ccggcctttg ccgcctatcg cggcacgtcc tggatgaagg    3240
   agccctgccg ctcctgcgac cggcgcgaga aggattgggg cgggtgccgc tgccaggcgc    3300
   tggcgctcac gggcgacgcg gccaacaccg atccggcctg ctccctttcg ccgctgcacg    3360
   cgaaaatgcg ggatcttgcc aaggaagagg ctgccgagac cccgcccgat tatatatacc    3420
   gcagcatcgg gacgaatgtg caaaacccgt tgagcgaaaa ggcacccctt tga           3473
<210> 65
   <211> 2738
   <212> DNA
   <213> Methylobacterium extorguens
<400> 65
   atgcacctct accggaaggc catcgcgccc gtgggaccgc agcgaggcct gaatcacgcc 60
   ggccgtgtgg acgccgcccc gttcgggcgc tccgaggcgg gcggcccgga ggtctccgcc 120
   ttcgtgctcg acaacgggct cgacgtggtg gtggtgcccg atcaccgggc gccggttgcc 180
   acgcacatgg tctggtaccg caacggctcg gccgatgatc cgatcggcca gtccggtatc 240
   gcccacttcc tcgaacacct gatgttcaag ggcaccgagc ggcacccggc cggcgccttc 300
   tcgaaagcgg tctcgtcgct cggcggccag gagaacgcct tcaccagcta cgattacacc 360
   gcctatttcc agcgcgtcgc ccgcgaccac ctctcgacga tgatggcctt cgaggccgac 420
   cggatgagcg gcctcgtgct cgacgacgcc gtggtggcgc ccgagcgcga cgtggtgctg 480
   gaggagcgcc ggatgcgggt cgagaccgat ccgtcggcgc agctctccga ggcgatgtcc 540
   gcctcgctgt tcgtgcacca tccctacggc atcccgatca tcggctggat gcacgagatc 600
   gaggagctga accgcaccca cgccatcgac tattacaagc gcttctacac ccctgagaac 660
   gcgatcctcg tggtggccgg cgacgtgacg ccggacgagg tgcggcgtct ggccgaggat 720
   acctacggcc gggtgacgcc gcagggcgcg cggccgctgc gcactcgccc gcgcgagccg 780
   gagccgcggg cgatgcgtcg gatcgcggtg gccgacccga aggtcgagca gccgaccctg 840
   cagcgcctct acctcacccc ctcctgcatg accgcccgcg acggcgaggg ctacgccctc 900
   gaactgctcg ccgaggtcgt cggcggcggc tcgacctcgt tcctctaccg caagctggtg 960
   ctggagatgg gcgtcgcggt gaatgccggc gcttggtaca tgggctcggc gatggatgac 1020
   acgcgctttg ccgtctacgc cgtgccggcc gagggcgtga ccctggaggc cctcgaagag 1080
   catatcgatc gcgtgctgcg ccgcgtcccc gaggcgctcg gtgcggaggc gatcgagcgg 1140
   gccaagatcc ggctcatggc cgagacggtc tattcctccg attcgcagag ctcgctcgcg 1200
   cgcatctacg gctcggcgct cgccatcggc gagaccgtcg aggaggtgcg ccgctggccg 1260
   gtcgagatcg aagcggtcac ccatgaccgg ctcgtcgcgg tcgccgcccg ctatctcgtg 1320
   cccgcccgct cggtgaccgg ctacctgacc aaggcgcgcg acccggacgt ggcgatcgcc 1380
   tgagacggcc ctgattcttc gcgccgccct cgatggcggc gccgtttgcg aaaagacgca 1440
   ttaaggaact tccgatgaat ctcgccgaga ccggcacccg caccgcgacg tcgccgaccc 1500
   aggcgttgcc gctcgcggcc gcccccggca tcgaggcttg gcacgtcgcc tcgccggtgg 1560
   tgccgatgat cgcgctctcc ttcaccttcg agggcggcgc ggcgcaggat gcggagggca 1620
   aggcgggcac cgcgcagatg atggcgcggc tgctcgacga gggcgcgggc gatctcgact 1680
   cggatgcctt ccaggaggcg ctcgcggccc gcgcgatcga gctgagcttc cacaccggcc 1740
   ccgattccat cggcggctcg ctcaagacgc tgctcacgca tgccgacgag gcgatccgtc 1800
   tgctggccct gtcgctggcc gagccgcgct tcgatcaacc ctcgatcgag cgcgtgcggg 1860
   cgcagatgat cgccagcctg cgctaccagc agaacgatcc cggcgtgctg gcctcccgcc 1920
   gctacttccg cgaggccttc ccggggcatg cctacggccg ctcctcgtcg ggcaccatcg 1980
   agaccctgtc ggcgatcacc cgagacgacc tcgtcgccct gcaccgggcc gtgatcggtc 2040
   gcggcagcct caaggtcgcg gctgtgggcg ccttcgacga ggcgacgatc accggcatga 2100
   tcgcccgcgc cttcggcgct ctgcccgagg ccggcccgct caaagccatt ccgccgaccg 2160
   cgatcaacga actcggccgc cgcatcgtcg tcgatctcga cgtgccgcaa tcggtgatcc    2220
   gcttcggcat gccgggcgtg gcgtggcgcg accccgactt catcccggcc tatgtgctga    2280
   accacatcct gggcggcggc gccttcacct cgcgcctgtt ccaggaagtg cgcgagaagc    2340
   gcggcctggc ctactcggtc ggcacctctc tgacctcgca ccgcgccgtc gccatgacct    2400
   ggggctacac cgccaccaag aacgagcgcg tcgtcgaggc cctcgacgtg atcggtgacg    2460
   agatccagcg cctcatcacc gacggcccct ccgacgagga gttgcagaag gccaaggact    2520
   atctcaccgg ctcctacgcg ctcggcttcg acacctcgac caagatcgcc aaccaattgg    2580
   tgcagatcgc cttcgagggg ctcggcatgg actacatcgc ccgccgcaac gacctcgtgg    2640
   cgagcgtgac ccaggccgac atccgccggg ccggcgcccg cacgctcggc gacggcaaga    2700
   tgctggtcgt cgccgcgggg cggcccacgg ggctgtag                            2738
<210> 66
   <211> 3194
   <212> DNA
   <213> Pseudomonas aeruginosa
<400> 66
   atgtggacca agcccagctt caccgacctg cgtctcggtt tcgaagtgac cctctacttc 60
   gccaaccgct gacccatccg gccccggtca tccggggcct ccctccagcc cgcggggcag 120
   acccatgcac atccgtattc tcggttcggc cgccggcggc ggctttcccc agtggaactg 180
   caactgccgc aactgtcgcg gggtccgcga cggcagcgta gcggcccagc cacgcacgca 240
   atcctccatc gccctgtcgg acgacggcga gcgctggatc ctctgcaacg cctcgcccga 300
   catccgtgtc cagatcgccg ccttcccggc cctgcagccg gcgcgtcggc cgcgcgatac 360
   ggcgatcggc gcgatcgtcc tgctcgacag ccagatcgac cacaccaccg gcctgctcag 420
   cctgcgcgaa ggctgcccgc acgaagtctg gtgcacgcag atggtccacc aggacctcag 480
   cgaaggcttt ccgctgttcc gcatgctttc ccactggaac ggcggcctgc gccaccggcc 540
   gatcgccctc gatggcgagc ctttcgccat cccggcctgt ccgcgcctgc gcttcaccgc 600
   gatccccctg cgcagcagcg cgccaccgta ttccccgcat cgcggcgacc cgcatccggg 660
   cgacaacatc ggcctgttcg tcgaggacct cgacagcgcc ggcacactgt tctacgcgcc 720
   gggcctcggc gaggtggacg aggcgctgct cgaatggatg cgccgcgccg actgcctgct 780
   ggtggacggc acgctctggc gcgacgacga gatgctggcc tgcgaggtcg gcgacaagct 840
   cggccggcag atgggccacc tggcgcagag cgggccgggc ggcatgctcg aggtactggc 900
   gaaggtgccg gccgcgcgca aggtgctcat ccatatcaac aacaccaatc ccatcctcga 960
   caccgcttcg gccgagcgcg ccgaactgga tgccagtggc atcgaagtgg cctgggatgg 1020
   catgcacatc cagctgtagg gggaccgaca tgagccgtgc cgccatggac cgcgccgagt 1080
   tcgaacgggc gctgcgcgac aaggggcgct actaccatat ccaccatccg ttccatgtcg 1140
   cgatgtacga gggccgtgcc agtcgcgaac agatccaggg ctgggtggcg aaccgcttct 1200
   actaccagct caacatcccg ctgaaggacg cggcgatcct ggccaactgt cccgaccgcg 1260
   aggtccgccg cgagtgggtc cagcgcatcc tcgaccacga cggcgccccc ggcgaggcgg 1320
   gaggcatcga ggcctggctg cgcctggcgg aggcggtggg cctggagcgc gagcaggtgc 1380
   tgtccgaaga acgggtgctg cccggcgtgc gcttcgccgt cgacgcctat gtcaacttcg 1440
   cccgtcgcgc cagctggcag gaggcggcga gcagttcgct gaccgaactc ttcgccccgc 1500
   agatccacca gtcgcggctg gacagctggc cgcgccacta tccgtggatc gaggcggccg 1560
   gctacgagta tttccgcagc cgcctggccc aggcccggcg tgacgtcgag cacggcctgc 1620
   ggatcaccct ggagcactat cggacccgcg aggcacagga acgcatgctg gagatcctgc 1680
   aattcaagct ggacgtgctg tggagcatgc tcgacgcgat gagcatggcc tacgagctgg 1740
   aacgtccgcc gtaccatacg gtgacccgtg agcgggtctg gcaccggggg ctggcgccat 1800
   gagcctgcca tcgctcgaca gcgtgccggt cctgcgccgg ggcttccgct tccagttcga 1860
   accggcccag gactgccatg tgctgctcta tcccgaaggg atggtcaagc tcaacgacag 1920
   cgccggggag atcctcaagc tggtcgatgg ccgccgcgac gtggcggcca tcgtcgcggc 1980
   gctgcgcgaa cgttttcccg aagttcccgg catagacgaa gacatcctga cgttcctcga 2040
   ggtggcccat gcgcaattct ggatcgagct gcagtgaaag cgtggggccg ccgctctggc 2100
   tgctggccga gctgacctac cgctgcccgt tgcagtgccc gtactgctcg aacccgctgg 2160
   aattcgcccg cgagggcgcc gagttgggca ccgcggaatg gatcgaggtg ttccgccagg 2220
   ctcgcgagct gggcgccgcc cagctcggtt tctccggcgg cgagccgctg ctgcgccagg 2280
   acctcgccga actgatcgag gcggggcgcg gcctgggctt ctacaccaac ctgatcacct 2340
   ccggcatcgg cctcgacgag gcacgcctgg cgcgcttcgc cgaggccggg ctggaccacg 2400
   tgcagatcag cttccaggcc gccgacgaag aggtgaacaa cctgctcgcc ggctcgcgca 2460
   aggccttcgc ccagaaactg gcgatggccc gcgcggtgaa agcccatggc tacccgatgg 2520
   tgctcaactt cgtcacccac cggcacaaca tcgacaacat cgagcggatc atccagctgt 2580
   gcatcgagct ggaggccgac tacgtggaac tggccacctg ccagttctac ggttgggccg 2640
   cgctgaaccg cgccgggttg ctgccgaccc gcgcccaact ggagcgcgcc gagcggatca 2700
   ccgccgaata ccgccagcgg ctggccgccg aaggcaatcc gtgcaagctg atcttcgtca 2760
   cccccgacta ctacgaggaa cggccgaagg cctgcatggg cggctgggcc agcgtgttcc 2820
   tcgacattac cccggacggc accgcgctgc cgtgccacag cgcgcggcaa ctgccgctga 2880
   agttccccaa cgtgcgcgag cacagcctgc gccacatctg gtacgagtcg ttcggcttca 2940
   accgctatcg cggcgacgcc tggatgcccg agccgtgccg ctcctgcgag gaaaaggagc 3000
   gtgaccacgg cggctgccgc tgccaggcgt tcctccttac cggcgacgcc gacgccaccg 3060
   acccggtctg cgccaagtcg gcccgccacg acctgatcct cgacgcccgg cgccaggccg     3120
   aggaggcgcc gctgggcctg gacgcgctga cctggcgcaa tcagcgcgcc tcgcgcctga     3180
   tctgcaaggc ctga                                                      3194
<210> 67
   <211> 2292
   <212> DNA
   <213> Pseudomonas aeruginosa
<400> 67
   gtgctgccca atggcctgcg cctgcacctg gcgcatgacc cggcggcatc gcgcgccgct 60
   gcctggctgc gggtggcggc gggcagccac gacgagccga ccgcgcatcc cggcctggca 120
   cacttcctcg agcacctgct gttcctcggc ggcgcggcgt ttcctggcga cgagcgtctc 180
   atgccgtggt tgcaggtgcg cggcggccag gtcaacgcca gcacccgggg caggagcacc 240
   gactatttct tcgaggtggc ggcggagcac cttggcgccg gtctggcacg cctgttcgac 300
   atgctcgtgc gaccgctgct ggatatcgac gcgcaacggc gcgagcgcga agtgctggaa 360
   gccgagtacc tggcgcgcgc ggccgacgag cagaccctga tcgatgcggc gctggccctc 420
   ggcctgcccg ccgggcatcc cctgcggcgt ttcgttgccg ggcgccgcga cagcctggcg 480
   ctggagaccg atgcgttcca gcgggcgctg cgcgaattcc atgccgccca ctatcacgcc 540
   ggcaattgcc agctatggct gcaagggccg caggcactgg acgagttgga gaggctggcg 600
   cggcgtgcct gcgccgacct gccgggtcgc tcgccaggcg cgagtccgtc gccgccgccg 660
   ttgtcaccct tcgccggcgc ggcgctagcg ctgcgcctgc cgggtccgcc gcgtctggtg 720
   ctgggctttg ccatcgacgc tttgcggggg gctgacgaac agaccctgct ggcattcgcc 780
   gaattgcttg gcgatcgctc gccgggcggg ttgctggcgg cactcggcga acagggcctg 840
   ggcgaatcgg tggcgctgcg ggtggtccat cgggacgcgc ggcaggcgct cctggcgctg 900
   accttcgaac tgttcgacgg cagcgcggcg gcggcgctgg aggagacctt tttcgactgg 960
   ctgcgcgccc tgcgcgacga tgccgcgagc ctgctggcgg cgcgccggcc gttgctggcg 1020
   gagcccactg cgccgctgga gcgactgcgc cagcgcgtgc ttggcctgcc agcggagatt 1080
   cgcccggcct gcctggatgc gctgcgcgcc gaacgctgcc tgcggttgca cctggacagc 1140
   gaactcgacg gcgccgaagc gcgctggtcg gcgggcttcc gcctgagcgt ggcgcccgtc 1200
   gccgcagcgc cgccgccgct ggcagcgcag cggcatgcct ggcgcttcga actgccgccg 1260
   ccgccgagca ccgccgccga gggcgcgctg ttcctgcgct ggcgctttcc cggcatatcc 1320
   gcacggtcac gcttcctggc gctgcgccag gcgttgcgtc cgctgtgcgg ccaggcgcgc 1380
   ctgcgggggg tggagatggg cctggaagcg ctcggcgaag actggacgct gagcctgctc 1440
   ggagcacgcg accgcctcga ggccgccgtg cgaccggccc tggcccggct cctcgcggcg 1500
   ccggccgact ggcgcgcaag cggcgagcgc ctgtcgttcg ccgagcggcg ccgcagcgcc 1560
   actggcctgc cgatccggca actgctggac gccttgcccg gcgtactcgg cgagccgctg 1620
   gcggaggtcg acgacgactg gcggcggacg cgctgggacg gcctggtcat gcaggccgcg 1680
   atgcctgatc cacgatgggt gcccgggcag gctaccggca agcgccttga gcctctgcca 1740
   gcgatgcccg gacggcatcg ccgcgagctg gccgtggacg gtgagtcggc gttgctgctg 1800
   ttctgtccgc tgcccgcgcc ggaggtgccg atggaggcgg cctggcgcct gctggcgcgc 1860
   ctgcacgagc cggccttcca gcggcgcctg cgcgacgagt tgcaactggg ctacgcgctg 1920
   ttctgcggct tccgtgaggt cggcgcacgg cgcggcctgc tgttcgccgc gcaatctccg 1980
   cgtgcctgcc cggaacgcct gctggagcac acggagatct tcctccagcg ttccgtcgag 2040
   gcgctcgcgc aactgccggc gcaacgcctg gccggcctgc gcgatgccct cgccgacgac 2100
   ctgcgccggg cgccggggac ttttgccgag cgggcgcgac acgcctgggc ggagcatctc 2160
   ggcggtggcc agggccgctc ccggctgctc gccgaggctg ctcgcggcct gggcgtcgac 2220
   gatctgcttg ccgcccaggc cgcgctgctg gaggcgcacg gcggctggtg ggtgctttcc 2280
   agccgacgct ga 2292
<210> 68
   <211> 3187
   <212> DNA
   <213> Gluconobacter oxydans
<400> 68
   atggcctgga acacaccgaa agttaccgaa atcccgctgg gcgcagaaat caactcgtat 60
   gtctgcggcg agaagaaata agccgctttc ccggggaccc gtccttgagg aataatggca 120
   cggccgctcc cccatggagc ggccgttttc gttcatgggt gctctgtggt gccccagtca 180
   gacggtttgt gaaaaaatga ttgatgtcat cgtgcttggc gcggcggcag ggggcggttt 240
   tccgcagtgg aactccgcag cacccggctg tgtggccgcc cgcacgcgac agggcgcgaa 300
   agcccggacc caggcctccc ttgccgtcag tgccgacgga aagcgctggt tcattctcaa 360
   cgcctcgccc gatctgcggc agcagatcat cgatacgccg gccctgcatc atcagggcag 420
   cctgcgtgga acgcccattc agggcgtcgt cctgacctgc ggcgagatcg acgccataac 480
   cgggcttctg accctgcgtg agcgtgagcc ttttaccctg atgggcagcg actcgaccct 540
   tcagcagctt gcggacaatc cgatcttcgg tgcgctcgat ccggaaatcg tcccacgtgt 600
   tccgctcatt ctcgatgaag ccacgtccct gatgaacaag gacgggattc cgtccggtct 660
   tttgctcacg gccttcgccg ttccgggcaa ggcgccgctt tacgcggaag ccgcagggtc 720
   acgcccggac gagacgctgg gcctttccat tacggatgga tgcaagacga tgctcttcat 780
   tcccggctgt gcgcagatca cgtcggaaat cgtggaacgg gtagcggcag ccgatctcgt 840
   gttctttgac gggacactgt ggcgggatga cgaaatgatc cgcgccgggt tgagcccgaa 900
   gagcggacag cggatgggac atgtgtccgt gaatgatgcc gggggaccgg tcgaatgttt 960
   cacgacatgc gaaaaacccc gtaaagtgtt gattcatatc aacaactcca atccaattct 1020
   gttcgaagac agccccgaac gcaaagacgt cgaacgcgcc ggatggacgg ttgcggaaga 1080
   cggcatgact ttcagactgg acacaccatg acgctcctca cacctgacca gcttgaagca 1140
   cagcttcgcc agatcggggc cgagcggtat cacaaccggc acccgttcca tcgcaagctg 1200
   catgacggca agctggacaa ggcacaggtt caggcttggg cgctgaaccg ctattattat 1260
   caggcccgca tcccggcgaa ggatgcgacg cttctcgcac gtctgccgac ggccgaactg 1320
   cgccgcgaat ggcgtcgccg gatcgaggac catgacggca cggagcccgg aacgggcggt 1380
   gttgcgcgct ggctgatgct gacggatggt ctggggctgg accgggatta tgtggaaagc 1440
   ctcgatggtc tgcttccagc cacgcgcttc tcggtcgatg cctatgtgaa cttcgtgcgg 1500
   gaccagtcga ttctggcggc cattgcgtcg tcgctgacgg aactgttttc gcccacgatc 1560
   atcagcgagc gcgtctcggg gatgctgcgg cactacgact ttgtgtcgga aaagacgctg 1620
   gcctatttca cgccgcgcct gacgcaggcc ccgcgggatt ccgatttcgc gctggcctat 1680
   gtccgcgaaa aggcccgcac gccggagcag cagaaagaag tcctgggagc gctggagttc 1740
   aagtgctccg tgctgtggac gatgctggat gcgctcgact acgcctatgt ggaaggccac 1800
   attccgccgg gggctttcgt tccatgacgg aggccccgca tgtcgtggcg gaggggacgg 1860
   ttctctcctt tgcccggggg catcgtctcc agcacgatcg tgtgcgggac gtgtggatcg 1920
   tgcaggcgcc tgaaaaagca tttgtagttg agggcgccgc gccgcatatt ctgcggctgc 1980
   tggatgggaa gcgcagcgtc ggcgagatca tccagcagct tgcaatcgag ttttccgccc 2040
   cgcgtgaggt cattgcgaaa gatgtcctcg cgcttctttc tgaactgaca gaaaagaacg 2100
   tcctgcacac atgacactcc cttcgccgcc gatgagcctt ctggctgaac tgacgcatcg 2160
   atgcccgctt tcctgcccct actgctccaa tccgcttgaa ctcgaacgca aggcggcaga 2220
   actcgacacg gccacctgga ctgccgtact ggagcaggcg gccgagcttg ggtgctccca 2280
   ggttcatttc tctggcggcg agcctatggc gcggcctgat ctggtcgaac tggtctccgt 2340
   cgcacggaga ctcaacctgt attccaactt gatcacgtcc ggcgtgttgc tggacgaacc 2400
   gaaactggaa gctctcgaca gggcggggct ggatcacatc cagctctctt tccaagacgt 2460
   gacggaggcg ggagccgagc gtatcggcgg tctcaaggga gcgcaggccc gcaaggttgc 2520
   ggcggcgcgg ctcatccgcg cgtccggcat tccgatgacg ctcaattttg tggtgcacag 2580
   ggaaaatgtc gcccgtatcc ccgagatgtt cgccctggcg cgggaactcg gagcggggcg 2640
   ggtggagatc gcgcataccc agtattatgg ctgggggctg aaaaaccgtg aggcgcttct 2700
   tcccagccgg gatcagctgg aggaatccac acgcgccgtg gaagcggagc gcgctaaggg 2760
   tggtttgtcc gttgattatg tgacgccgga ctatcatgca gaccggccca agccctgcat 2820
   ggggggatgg ggccagcgtt tcgtgaatgt cacaccttcg ggccgggtcc tgccgtgtca 2880
   tgcagccgaa atcattccgg atgtcgcatt cccgaatgtg caggatgtga ccctgtccga 2940
   aatctggaac atctcaccgc tgttcaacat gttccgcggg acggactgga tgccggagcc 3000
   ctgccgctcc tgcgagcgca aggagcgtga ctggggcggg tgtcgctgtc aggcgatggc 3060
   gctgacgggg aatgccgcga ataccgatcc cgtatgcagt ctctccccct atcacgatcg 3120
   ggtggagcag gccgtcgaga acaacatgca gccagaaagc acgttgttct acaggcgtta 3180
   tacgtaa 3187
<210> 69
   <211> 5474
   <212> DNA
   <213> Kluyvera intermedia
<400> 69
   atgtggaaaa aacctgcgtt tattgacctg cgtctgggct tggaagtgac tctgtacatc     60
   tccaaccgct aagacttctg cccgccgttc gtgcgggcat tttgttttct ctctttggtc    120
   aacgttcatg tttattaaag tcctcggttc agcggcgggc ggcggcttcc cgcagtggaa    180
   ctgtaactgc gccaattgca gcggcctgcg taacggcagt attcaggcgc aggcgcgcac    240
   ccaatcttca atcattgtca gcgacaacgg tgaagattgg gtgctgtgca acgcctcgcc    300
   ggacatcagc cagcaaattg cccacacgcc agaactgatc aaaaagggtg tgctgcgcgg    360
   cacggcgatt ggcagcatca ttctcaccga cagccaaatc gatcacacca ctggcttgtt    420
   gagcctgcgc gaaggctgtc cgcatcaggt gtggtgcacg ccggaagtgc atggcgatct    480
   caccagcggt ttcccgattt tcaccatgtt gcagcactgg aatggcggct tgcagcatca    540
   tgcgttaacg ccgctggagc cgttccgcgt cgatgtctgc cacagtttgc agttcaccgc    600
   gattccgatt ctcagcaacg cgccgccgta ttcgccgtat cgcgatcgcc ctgccggacc    660
   acaacgtggc gctgttcatg aaaccaccgg caatggccaa acgctgctct acgcgccggg    720
   actcggcgag ccggatgcgg tgatccatgc cgtggctgca aagagccgat tgcctgttat    780
   tgatggcacg gtgtggcagg acgacgagct gctcgccacc ggcgttggca aaaatactgg    840
   caaagcgatg ggccacttag cgctggccga agaacaaggt ttaatggcgt tgctggctgc    900
   gctgccggcc aaacgcaaga ttttgattca cattaacaac accaatccga tccttaatga    960
   gcaatccccg cagcgcgcct atttgacgca gcagggcatt gaagtgagtt gggatggcat   1020
   ggcgattcat ctgcaggagt ttgcatcatg atcatcactg aagcattatc gcccgccgcg   1080
   tttgagcagg cgctgcgcgg tttgcatcat gatcatcact gaagcattat cgcccgccgc   1140
   gtttgagcag gcgctgcgcg ataaaggcgc gttctaccac atacatcacc cgtatcacat   1200
   tgccatgcac aatggcgagg cgacgcgcga gcagattcag ggctgggtgg cgaaccgctt   1260
   ctattatcag accaacattc cgctgaaaga tgcggcgatc atggcgaatt gcccggatgc   1320
   ggcgacgcgg cgtaaatggg tgcagcgcat cctcgatcac gacggcagca acggtcacga   1380
   aggcgggatt gaagcctggc tgcagttggg tgaggcggtg gggctggaac gtgacgtgtt   1440
   gctgtcagag aagctggtgc tgccgggcgt gcgttttgcg gtggacgcct acgtgaactt 1500
   cgcgcgccgt gctaactggc aggaagcggc ctgtagctcg ctcaccgagc tgtttgcgcc 1560
   gcagattcat cagtcgcgtc tcgacagctg gccgcagcac tatccgtgga tcaaagagga 1620
   aggctatttc tacttccgca gccgtttgag ccaggcgaac cgcgatgtgg agcatgggct 1680
   ggcgctggcg cttgaggtgt ttacccgcgc cgaacagcag aaccgcatgc tggagatcct 1740
   gcagtttaaa ctcgatattc tctggaccat gctcgatgcc atgaccatgg cgtatgcgct 1800
   gcaacgtccg ccttatcata cggtcaccga tcaggtgtcc tggcacagca caagactggt 1860
   ataacaacat gaaagataac ggtcaccgat caggtgtcct ggcacagcac aagactggta 1920
   taacaacatg aaagataatg tgattccggc tttccgtcgc ggctaccgca tgcagtggga 1980
   agcggcgcag gatagccatg tggtgctcta tccagaaggc atggccaaac tgaacgagac 2040
   cgccgtggcg atcctcgaac tggtggatgg caaacaggat gtggcggcga tcgttgccac 2100
   gctggatgcg cgtttcccgg acgcgggcgg tgtcggcgac gacgtcaaag agttcctgca 2160
   atccgccatt gaacaaaaat ggatacagtg tcgtgaaccc gagtaaaagc gtgacgccac 2220
   cgctgtggct gctggcagaa ctgacctatc gctgcccgct gcagtgtcca tattgctcca 2280
   atccgctgga tttttcccag cagaagaaag agctgaccac cgaacagtgg attgaggtgt 2340
   ttcgtcaggc gcgcgccatg ggcagcgtgc agctcggttt ttccggcggc gaaccgctaa 2400
   cccgtaaaga cctgccggag ttgattcgcg ccgcgcgcga tctcggtttc tacaccaacc 2460
   tgatcacctc cggcatcggc ctgacggcga aaaaactcga cgcctttgcc gacgccggac 2520
   tcgatcatat ccagatcagc ttccaggcca gcgatgaaac gctgaacgcg gcgctggccg 2580
   ggtcaaaaaa ggccttccag cagaagctgg agatggcgaa agcggtgaaa gcgcacggct 2640
   atccgatggt gctgaatttc gtgctgcatc gccacaatat cgaccagatc gacaaaatca 2700
   tcgatctctg tatcgaactg gatgccgacg atgtcgagct ggcgacttgc caattctatg 2760
   gctgggcgca actcaatcgt gaaggattgc tgccgacgcg cgagcagatc gccaacgccg 2820
   aagcggtggt ggccgattat cgtcagcgca tgggcgccag cggcaatctc accaatcctg 2880
   ctgttcgtga cgccggatta ctacgaagag cggccgaaac cctgcatggg cggatgggga 2940
   tcgatcttcc tcagcgtcac gccgactgca ccgcgttgcc gtgccacagc gcgcgtcagc 3000
   tgccggttgc gttcccgtcg gtgctggagc gcacgctgga cgatatctgg tacaactcgt 3060
   ttggttttaa ccgctatcgc ggctttgact ggatgccgga accctgtcgc tcctgcgatg 3120
   aaaaagcgaa agactttggc ggctgtcgct gtcaggcctt tatgctgacc ggcgatgccg 3180
   ataacaccga tccggtgtgc agcaaatcgc cgcatcacgg caagatcctc gaagcacgac 3240
   gtgaagccaa ctgcagcgac atcaaaatcc agcagctgca gttccgcaat cgcagcaact 3300
   ccgaactgat cttttaagca gcgcgtcacc tgatgcacgc gcgccagctc acgctcgcca 3360
   acggcctgcg ctgccatctt tatcatcagc ccgacgcgcg tgaggcggcg gcgctgatgc 3420
   gcgtgcaggc cggcagtctg gatgaagcgg atcgctggcc gggtttggca catctgctgg 3480
   aacatctgct gttttgcggc agcgaagctt ttcacggcga cgatcgcctg atgccttggc 3540
   tgcagcagca gggcggccag gtgaacgcca ccacgcagtt gagccgcagc gcctatttct 3600
   ttcagctccc agctgcggcg ttgtcagcag gggtgctgcg gctgtgcaat atgttggcgt 3660
   caccgttgct gacggcgcag gccattcagc aggaaacggc ggtgattgag gctgagtatc 3720
   aactgctgca aaaccatgcc gacacgctga gtgaagctgc cgtgctggat aagtggcagg 3780
   gacgttttca gcgcttccgc gtcggcagtc ggcaggcctt tggtgacaat gtcacggagt 3840
   tgcagtccgc attacgcgac ttccatagcc gcctgtattg cgctgaaaac atggaacttt 3900
   ggctacaagg cccgcaatca ctggatgagc tcgaacagct ggcggcacgc ttcggcggca 3960
   gtttgctggc gggtggagaa tgcgctgcgg ctgttgaagc tacgctgttg cgaggcgatc 4020
   gtttactgct atcaggcggt gaagaaaatt tctggctgac gatgttggtc gcgggcgatg 4080
   agcagactgt gcgtgacaat gtcaccttat taaaagcatt ctggcaggac gaagcgccct 4140
   acagcttgct ggcgcagctg cgcgttgagg cgttatgcga aacctttgac gcgcattggt 4200
   tatggcaaga tgagcaacaa gcgttactgg cgctgcgctt tagcgccagg tgcatttccc 4260
   cggcgcaggc gcaacagatt gagcaacgcg tttggcagca tctggctgcg ttagctgaat 4320
   gtacggcgct tcagctgcgg cactacgcac agctcgcgca gcaggatttc gcgaccttga 4380
   ccttgtcgcc gctggaacag ttgcgcggcc gggcgttggg cttcgcgccg ggttgcgcgc 4440
   tgccagacaa ctttacggat tttgccgcag cgttgccaga ttgtccgcgc acgcgtctgc 4500
   tcacgcagca acagattgac ggcgcaccgc atgcacacgc agggcttcac cttgcagctg 4560
   gtcgagtggc tgccgccgtc gttggctgcg accaaacccg cagcgtttca cttctatcct 4620
   gcttcagccg ctgttccgct ccctcgtctc ccacccgttg cgcagccgtt gccgctgatt 4680
   gcaccggtaa aacaggtaga aacgctgctg ctgcgtccgg cgttttatca caccttgagg 4740
   aggaagacgc gcagtcgcgg cagcgccagt tacgcccgtt gctggcggaa ttgcgccacg 4800
   cgggtggcaa cggcagctgg caacagacgc atgccagctg gcagctactg ctcaatttgc 4860
   ctgcatccgc cgatcgcgcg ttgtttagcg tgcatcaggc attacaggcg ctgaacacgc 4920
   cgatgctggc gcaacccgca gcgaatactc cttcgattgt gattcgtcaa ttgctcgccg 4980
   cgttgcccac tcgcttgatc aagccattac ccgcgccaca atggctggcg gcatggtgcg 5040
   gcaccaatac cgcattaagc cagcgcgtgg cgcatctgct cagcgatttt acgccggatc 5100
   tggcgcgcga cacgccgccg gcacgactgc aacacggcat cgtgcccatc gcctgcgacg 5160
   ggcgcgatca ggcgctgctg ctatttatgc cgttaccgca ggccgacgat gccagcctgg 5220
   ccgctttacg cgtgctggcg ctgatgctgg aaacgcgctt tttccagcgg ttgcgcgtgg 5280
   atcagcaaat tggctatgtg gtgtcggcgc gctatcagcg ggtggcggat gtggatggct 5340
   tgatgctggc gctgcaatca ccggatatct cctggcgcgc gctgctgggt cattgcaaac 5400
   gctttatgcg ttgagatggt agccgagatc gcggcaatct cgccgcaaaa gctggcggca 5460
   tggcaggcaa gctt 5474
<210> 70
   <211> 5641
   <212> DNA
   <213> Erwinia amylovora
<400> 70
   atgcagtgga ctaaaccaac gtttatcgat atgcgtctgg ggctggaagt cacgttatat 60
   atatctaatc gttaacccct cgccttatca caatgtgaat aacgctggct aaaacggtga 120
   tgcctgtggt taagcccagg cattttttca ttccctttcc cggttaaccc atgaaaatca 180
   aagttctcgg atctgcggcc ggcggcggtt ttccgcaatg gaactgctat tgccctaatt 240
   gccagggcgt gcgtaacggt agcatccgcg ccacggcccg cacccagtct tccatcgcgg 300
   tcagcgataa cggcagcgac tgggtgcttt gcaacgcctc gcccgatatc tgtcatcaga 360
   ttgctgccaa ccccgaactt catccacagg gtaagctgcg cggttcggga attggctcca 420
   tcattcttac cgacagtcag atcgatcact gcaccggctt gctgaatctg cgagaaggtt 480
   gcccacatca tgtgtggtgc acagcagagg tgcacgcgga tctgaccagt ggttttccga 540
   tcttcaccat gctgcaacac tggaacggtg gactggtgca tcatgctatt cagctcgcgc 600
   agcctttttc cgtcgcggtg tgtccggcgt tgcggtttac tgctattccc atcctcagca 660
   acgccccacc ttactcaccg tatcgcgggc gtccgcttcc cggacacaat atcgcgctga 720
   tgatcgaaaa taccgccagc ggcagcaaac tgctgtatgc ccccggtttg ggcgaaccag 780
   atgcgcagct gttggatttg atgtcgcagg ccgactgcct gctggttgac ggtacgctgt 840
   ggcaggacaa cgagctggca aataccggcg ttgggcgcaa caccggccgc gatatgggcc 900
   acctggcgct ggatgaagat cgcgggctga tggcgctact gggcgatctg cccgcaccgc 960
   gcaagatcct gatccatatt aataatacta atccgatcct tgatgaatcc tccgccgagc 1020
   gtctggcgtt gagcgcacgc ggcatcgagg tcagctacga cggcatgagc atagaactat 1080
   gacccaaccg gcattgatga ctccacaaca gtttgaacag gcgctgcgcg ctcgcggcgc 1140
   ttactaccat attcatcacc cgtaccatat cgctatgcat aacggcgagg ctacgcgtga 1200
   gcagatccag ggctgggtgg cgaaccgcta ttattaccag acccgtattc ctttgaaaga 1260
   cgccgcgatt atggcgaact gccgggatgc gttaacccgg cgtaaatggg tacagcgtat 1320
   tctcgaccat gatggccagg gcgacagcga gggcggtatc gaagcctggc tgcggcttgg 1380
   tgaagccgtt ggcctggatc gtgatgtgct gcagtcagag cagcgggtgc tgcccggcgt 1440
   gcgctttgcc gttgatgcct acgtttcctt tgcccgccgt gccgtctggc aggaagccgc 1500
   ctgtagctca ctgactgaac tgtatgcccc tgaaatccat cagtcgcgtc tcaacagctg 1560
   gccgcagcac tacccgtgga tcgaggaaga gggctacggc tatttccgtg gtcgcctggg 1620
   ccaggcccgg cgcgatgttg aacatggttt acagctggcg cttgagtact gcaacagcgt 1680
   tgaaaaacag cagcgtatgc tggagatcct gcaatttaag ctggatattt tgtggagcat 1740
   gctggatgcc atgaccatgg cgtatacact taaccgtgcg ccttatcaca ccgtgaccga 1800
   tcaacccgtc tggcataaag gaaatctgct gtgaaatgtg accaacgcat tcccatattc 1860
   cgccgtggat atcgtctgca atgggaagag atgcagaatt gccatgtcat cctctatccc 1920
   gaagggatgg ctaaactcaa cgacagcgca acgatgatcc tcgaactggt ggatggtcaa 1980
   cgctcgctgg ctgatatcgc ccgtacgctg aatgtgcggt tcccggacgc gggtggcgtt 2040
   gatgatgacg tcaccgattt ctttgccgcc gcgcgtgaac agaagtggat aattttccgt 2100
   gaacccagct gaatcaccga tcaaaccgcc gctctggcta ctggcggagc tgacctaccg 2160
   ctgcccgtta caatgcccgt attgctccaa cccgcttgac tttgcacggc aggaaaaaga 2220
   gctgacgact gcccagtgga tcgacgtctt taagcaggcg cgggcgatgg gcgcggtaca 2280
   gattggcttt tcgggtggcg aaccgctggt gcgtaacgat ctgcccgagc tgatccgcag 2340
   cgcgcgcgat ctcggtttct ataccaacct gattacctcc gggatcggcc tgacgcagaa 2400
   gaagatcgac gccttcgccg aagcgggact ggatcatatt cagatcagct tccaggccag 2460
   cgatgaaacc ctgaatgcgg cgctggccgg atcgacaaag gcatttcaac aaaagctgga 2520
   gatggcgcgt gcggttaaag cgcacggcta tccaatggtg ctgaattttg tgctgcatcg 2580
   ccacaatatc gaccagcttg accgcattat cgagctgtgc atcgaactgg aagccgatga 2640
   tgtcgagctg gcaacctgcc agttttacgg ctgggcgcag ctcaaccgcg agggactgct 2700
   gcctacccgc gaccagctgg cacgcgccga ggcggtggtg caccactacc gcgagaagat 2760
   ggccgccagc ggtaatctgg ccaatctgct gttcgtcacc ccggactatt atgaagagcg 2820
   gcctaagggc tgcatgggcg gctggggcgc gatcttcctc agcgtcacgc cggaaggcat 2880
   ggcgctgccc tgtcacagcg cccgccagct accgatacct ttcccgtcgg tgctggagca 2940
   cagcctgcag gatatctggt ttaactcgtt tggctttaac cgttatcgcg gctttgactg 3000
   gatgccggag ccatgtcgat cctgtgatga aaaagagaaa gacttcggcg gctgccgctg 3060
   ccaggcgttt atgctgaccg gcaacgctga taacgccgac ccggtatgca gcaagtcaga 3120
   acatcacggc accatccttg ccgcgcgtga acaggccaac tgcagccaca ttcaggtgaa 3180
   ccagctacgg ttccgtaatc gcgcaaactc ccagcgggtt aacgcccagc tgatcttcaa 3240
   gggctgacgg atgcagccac agcgacgacg gctcgacaac ggtttgcgcg tggtgttgat 3300
   tagcgatgcg caggcagtac aggctgcagc gcttttccag gtggataccg gtagccatta 3360
   tgaaccggac agctggccgg ggctggctca tctgttggaa catttgctgt ttgccggcag 3420
   ttgcgcttat gcggatgacg agcggctgat ggcctggtta ccggccaggg gagggcggct 3480
   gaatgccacc acccagggca gcagcacggc cttttttttc gagtgcgatg ccgggctgct 3540
   ggctccgggg ctggctcgtt tgagcgatat gctgctggct ccgctgttgg cagaaaatgc 3600
   catccgccag gaagtggcta ccattgacgc cgagtgccgc ctgctggccg ggcagcagga 3660
   tacgttgtgc aatgccgcgc agagcatggc atttgccgca catccgtggc agcgttttca 3720
   tatcggtaac gccgcgagtt ttaccgggga ctggccggca ctgcggctgg cgttacagca 3780
   gtttcatcaa cgctattatc acgcggcgaa tatcacgctg tggttgcagg gaccgcagtc 3840
   gcccgaagcg ttatggcagc tggcccgaca gtacggcagt gcgttttctg ctgttggtgt 3900
   gccaccgcca gcgcttccgt tactgcatta ttcatcgcag cctgatatgg ctttgcaact 3960
   ggcgggatcg ccgcgtttgc gtctctcttt cctgctggac aggccgcgca gtaacgagct 4020
   gactttgctg cgccagctct tactggatga ggccgcaggt ggcctgatgg caacgctgcg 4080
   tgcgcaccat ctctgcgatg gcgcgcggct gctggtgcct tatcacagcg cgatgcaaac 4140
   actggtcagc gttgaactgg cgctgattga tgagcagcag gccgcagagg tggaaggttg 4200
   ggtgcatcac tggctgcaac gactgacggc actgacggcg cggcagcgcc agcactatat 4260
   gcggctcgct gactttcagt ttgccaggct ttcgccgatg gatcagttac gcgaacacgc 4320
   cgttggtttt gctccgccgc aagtacagca agacgactgg ccagggttct gtgcgcggct 4380
   gtcagtagcg cgtttagccc gcttgtggat cggcgcgcag tcgctggcgc agcagtgcag 4440
   cgttcagggc ttcaggctgc gctgcgctgc gcaaacgcgt gcgccggtaa ccgcgttaat 4500
   ccccgctgaa gcgctgactt ttttctgcga aagccagcct gacgctcagg cagcattacc 4560
   cgccgggcag gtcgcgctga accatcagcg ggcgggtaag ggccgtgcgg cgctgttgct 4620
   aagcccgctg gatgaacttc atgcgccctg gggagtcatc ctgcaatcgc gcctgagagc 4680
   gctggcagcc gactgtgccc ataagggcgg cgatctgagc gtcagctgcc agcagggtca 4740
   ttggctgatc cagctgtgcg gcagcccggc gttaatggtg cgcacgctgg caacgctgat 4800
   ccgccagctg tgtgagatat cccctgtgat gatcgcgctg ggcgagcggc aataccaacg 4860
   ccagcagcag gcgcagcgtg aggggattgc ggtacgcgcc ctgatcgatg cactgcccgc 4920
   gctgttgcgt tcatcggttt accaaccggc agggcggcgg ctgccgcgcc tggcgtggca 4980
   ggcggtcctc gatggcgggg acgatgcgct ccgccagtcg ttgtcgcagc tgcttagcgc 5040
   atttcccggc accatcaatc cgccgggccg gatgcagcca gagccgttag caccgcagcc 5100
   tgaatatcag gtggcgacca tcagccacga tgcgaccttg ttacgcttct gcccgctggt 5160
   ggaaaacagc acacagtgcc tggcggcctg gcagctgctg gcgctgatct atcaacctgc 5220
   atttttccaa cgtctgcgcg tggagcagaa tattggttat gtggtcagct gccgttttta 5280
   ccaggcggcg gggcggtcgg ggctgctgtt tgccctgcaa tctccccatc tgagcaccgg 5340
   cgagctttcg gcgcatatcg accgtttctt gccaggaatg gacgatgaac tggccgctct 5400
   cagcatggaa acagtgcgtg aaaagggggc ggcattgctg gcgcaacaaa ggctggcagc 5460
   gtctgatttc cagcaggagt gccggcagcg ctggctggct gcgcaacagt cggtacctca 5520
   gcccgatgag tacgttatcc aggggctgac cccggaacgc ctgtcagact accatcagcg 5580
   cctgctctcc gaccgccata acgcctggac gctggttggc atccctgcaa atcgcttctg 5640
   a 5641
<210> 71
   <211> 25
   <212> DNA
   <213> Artificial
<220>
   <223> Primer sequence
<400> 71
   gcgccatatg catcgacaat ccttt 25
<210> 72
   <211> 35
   <212> DNA
   <213> Artificial
<220>
   <223> Primer sequence
<400> 72
   gcgcgctcag gctaattgcg tgggctaact ttaag 35
<210> 73
   <211> 28
   <212> DNA
   <213> Artificial
<220>
   <223> Primer sequence
<400> 73
   gcgccatatg gctcctgcaa cggtaaat 28
<210> 74
   <211> 30
   <212> DNA
   <213> Artificial
<220>
   <223> Primer sequence
<400> 74
   ccggcatatg actgatctga aagcaagcag 30
<210> 75
   <211> 31
   <212> DNA
   <213> Artificial
<220>
   <223> Primer sequence
<400> 75
   ccgctcagct cattagtagc tgctggcgct c 31
<210> 76
   <211> 39
   <212> DNA
   <213> Artificial
<220>
   <223> Primer sequence
<400> 76
   gcaggctgag cttaacttta agaaggagat atacatatg 39
<210> 77
   <211> 35
   <212> DNA
   <213> Artificial
<220>
   <223> Primer sequence
<400> 77
   gcgcgctcag cctaattgcg tgggctaact ttaag 35
<210> 78
   <211> 37
   <212> DNA
   <213> Artificial
<220>
   <223> Primer sequence
<400> 78
   gcgcggtacc gcacatgtcg cggatgttca ggtgttc 37
<210> 79
   <211> 37
   <212> DNA
   <213> Artificial
<220>
   <223> Primer sequence
<400> 79
   gcgcggatcc gggcggagag tttggagaac ctcttca 37
<210> 80
   <211> 30
   <212> DNA
   <213> Artificial
<220>
   <223> Primer sequence
<400> 80
   gcgcggatcc acgcagcatc gggccgttct 30
<210> 81
   <211> 31
   <212> DNA
   <213> Artificial
<220>
   <223> Primer sequence
<400> 81
   gcgcggtacc aagcttcgct gccgcaaaac a 31
<210> 82
   <211> 42
   <212> DNA
   <213> Artificial
<220>
   <223> Linker sequence
<400> 82
   gcgcaagcat gcggatccgg taccaagctt gcatgcacac ta 42

## Description

### Field of the Invention

The present invention relates in general to the field of chemical technology and in particular to a process for preparing an active pharmaceutical ingredient (API) or intermediates thereof by using an enzyme. Particularly, the present invention relates to a preparation of HMG-CoA reductase inhibitors, known also as statins, wherein an aldose 1-dehydrogenase is provided for catalyzing a particular step in the synthesis. In certain embodiment, this invention relates to a process for preparing an intermediate by providing said enzyme. The invention further relates to an expression system effectively translating said enzyme. In addition, the invention relates to a specific use of such enzyme for preparing API or intermediate thereof, and in particular for preparing statin or interemediate thereof.

### Background of the Invention

Synthetic routes are routinely performed by carrying out chemical reactions in vitro. The chemistry can become complex and can require expensive reagents, multiple long steps, possibly with low yields because of low stereoselectivity. In specific cases, it is possible to employ a microorganism or its part that is capable of using a starting material as a substrate in its biochemical pathways that convert the starting material to a desired compound. With the aid of microorganisms or their parts, like for example enzymes, optionally together with synthetic process steps, it may be possible to put together complete synthesis pathways for preparing an API or an intermediate thereof, however, it is a challenging task.

For example, Patel et al., Enzyme Microb. Technol., vol. 14, 778-784 (1992) describe a stereoselective microbial/enzymatic oxidation of 7-oxybicyclo[2.2.1]heptane-2,3-dimethanol to the corresponding chiral lactol and lactone, by using horse liver alcohol dehydrogenase or by microorganisms oxidizing the compound. Further, Moreno-Horn et al., Journal of Molecular Catalysis B:Enzymes, vol. 49, 24-27 (2007) describe the oxidation of 1,4-alkanediols into γ-lactones via γ-lactols using Rhodococcus erythropolis as biocatalyst

A particular example of a complex synthesis route is the synthesis of inhibitors of the enzyme 3-hydroxy-3-methylglutaryl-coenzyme A reductase (HMG CoA reductase), which were found to be an excellent tool to reduce lipids and cholesterol blood levels in order to reduce mortality due to serious cardiovascular events in atherosclerosis triggered pathological processes and diseases. Among HMG CoA reductase inhibitors, a natural lovastatin is known, which has been superseded by semisynthetic pravastatin, simvastatin and later by completely synthetic atorvastatin, cerivastatin, rosuvastatin, fluvastatin, pitavastatin, bervastatin and dalvastatin. The clinically effective HMG CoA reductase inhibitors are also known as statins (characterised by INN name ending -statin).

All statins share a characteristic side chain consisting of respectively a heptenoic or heptanoic acid moiety (free acid, salt or lactone) connected to a statin backbone (Scheme 1). Biological activity of statins is linked to this structure and its stereochemistry. Normally, multiple chemical steps are required to prepare the heptenoic or heptanoic acid moiety. The construction of this side chain still represents a challenge for a chemist.

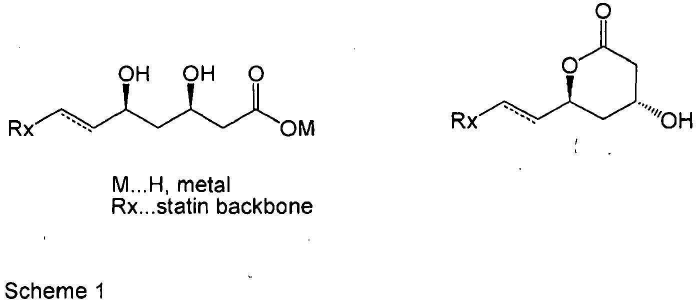

A first attempt to prepare a side chain and thus statin via lactol was disclosed in US 7414119. WO 2006/134482 disclosed the application of aldolases (in particular 2-deoxyribose-5-phosphate aldolase; DERA; EC class EC 4.1.2.4.) for the synthesis of lactols, which enabled direct coupling to obtain atorvastatin.

For further use and eventually yielding statin compounds lactol must be oxidised. In J. Am. Chem. Soc. 116 (1994), p. 8422-8423 and WO 2008/119810 the purified lactol was oxidised to lactone by Br₂/BaCO₃. WO 2006/134482 discloses a use of a catalytic dehydrogenation (e.g. Pt/C, Pd/C) for the same purpose.

It is an object of the present invention to provide a new process that generally allows oxidation or dehydrogenation in preparing API or intermediate thereof in reduced number of process steps, in shorter time, high yield and in an improved, more economic and simplified fashion. Specific aspects of the present invention can be applied for preparing statins or their intermediates.

### Summary of the Invention

To solve the aforementioned object, the present invention provides a process according to claim 1. Preferred embodiments are set forth in the subclaims. The present invention further provides a reaction system according to claim 10, a process for preparing a pharmaceutical composition according to claim 17, and uses according to claims 18 and 19.

Surprisingly it has been found that oxidation from certain lactol to lactone moieties typical for statin type compound synthesis is possible using particular enzymes. It is noted that oxidizing lactols with chemical reagents requires lactols to be first isolated, which is extremely burdensome and consumes large amounts of organic solvents. In addition, 4-hydroxy-lactols have to be first protected with the hydroxyl protection group, which adds to a number of reaction steps and to final impurity profile of an API. On the other hand, the oxidation or dehydrogenation of the compound of formula (II) performed with the enzyme catalyst as defined above according to the present invention is extremely favourable over the prior art chemical oxidation with Br₂/BaCO₃ or the chemical catalytic dehydrogenation (e.g. Pt/C, Pd/C). Chemical oxidants are not specific and thus need previous purification of lactol, otherwise too much reagent is consumed in oxidation of the side products or reagents, or even solvents. The purification of lactol is demanding and requires substantial amounts of a solvent for extraction, which is linked to the fact that lactol is normally hydrophilic and is hardly extracted to the organic solvent, such as for example ethylacetate. Also to note is that solvents used in the extraction need to be evaporated, which is not desired when working on the industrial scale. All aforementioned pitfalls of the chemical oxidation are solved by the process of the present invention. Circumventing the purification and evaporation steps by using the enzyme capable of catalyzing oxidation or dehydrogenation shortens significantly the process of preparing the compound of formula (I).

Although lactols such as compound (II) are non-natural type compounds, they have surprisingly been found to work as effective substrates for the enzymes disclosed herein. Based on this finding, use of the enzyme capable of catalyzing oxidation or dehydrogenation provides a valuable tool for generally preparing a synthetic API or synthetic intermediate thereof. Generally, the substrate within the use of the present invention for preparing a non-natural, synthetic API or intermediate thereof will therefore be different from naturally occurring ones of the enzyme capable of catalyzing oxidation or dehydrogenation, thereby excluding for example natural substrates selected from ethanol, methanol, acetaldehyde, acetic acid, naturally occurring sugars or amino acids, or sugar acids derived from sugars, including monosaccharides having a carboxylic group such as gluconic acid.

Aspects, advantageous features and preferred embodiments of the present invention summarized in the following items, respectively alone or in combination, contribute to solving the object of the invention.
(1) A process for preparing a compound of formula (I) 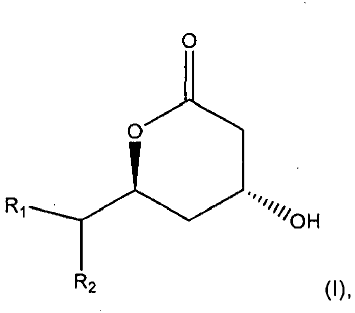 in which
   R₁ independently from R₂ denotes H, X, N₃, CN, NO₂, OH, (CH₂)ₙ-CH₃, O-(CH₂)n-CH_{3,} S-(CH₂)n-CH₃, NR³R⁴, OCO(CH₂)ₙCH₃, NR³CO(CH₂)ₙCH₃, CH₂-R⁵, optionally substituted mono- or bicyclic aryl, heterocyclic or alicyclic group; and
   R₂ independently from R₁ denotes H, (CH₂)ₘ-CH₃, or aryl;
   or both of R₁ and R₂ denote either X, OH or O((CH₂)ₙCH₃);
   or R₁ and R₂ together denote =O, =CH-R⁵,, wherein
   any one of CH₂ or CH₃ groups denoted above may optionally be further substituted by X, N₃, CN, NO₂, OH, (CH₂)n-CH3, aryl, O-(CH₂)n-CH_{3,} OCO(CH₂)ₙCH3. NR³R⁴, NR³CO(CH₂)ₙCH₃; or
   each CH₂ linking carbon atoms can be replaced by O, S or NR³; wherein
   R³ and R⁴ independently from each other, or together, denote H, (CH₂)ₘ-CH₃;
   R⁵ denotes optionally substituted mono- or bicyclic aryl, heterocyclic or alicyclic group,
   X denotes F, Cl, Br or I;
   n represents an integer from 0 to 10;
   m represents an integer from 0 to 3;
   or a pharmaceutically acceptable salt, or an ester, or a stereoisomer thereof, the process comprising bringing in contact a non-natural lactol compound of formula (II), 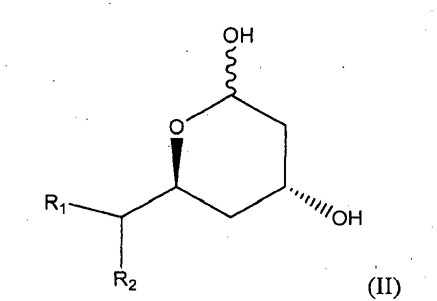 wherein R₁ and R₂ are defined as above, with an aldose 1-dehydrogenase.
(2) The process according to item 1, wherein
   R5 denotes a moiety selected from the formula (III), (IV), (V), (VI), (VII), (VIII) and (IX); 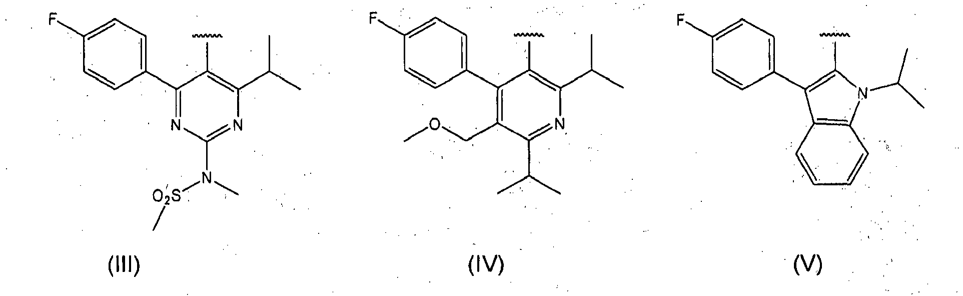 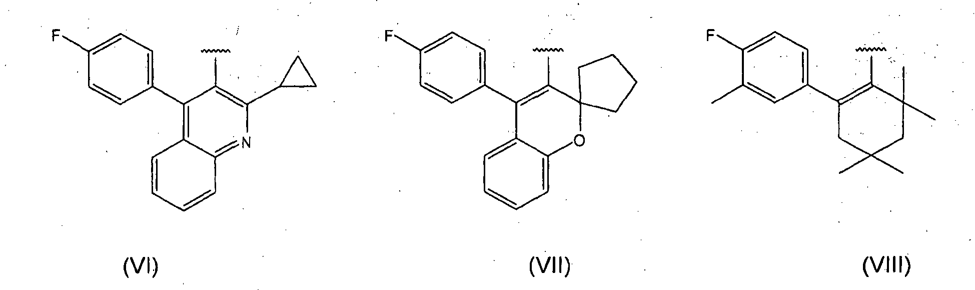 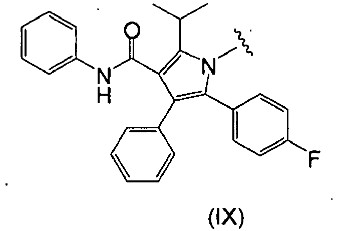
(3) The process for preparing a compound of formula (I) according to items 1 or 2, in which
   R₁ independently from R₂ denotes H, X, N₃, CN, NO₂, OH, (CH₂)ₙ-CH₃, O-(CH₂)ₙ-CH_{3,} S-(CH₂)ₙ-CH₃. NR³R⁴, OCO(CH₂)ₙCH₃, or NR³CO(CH₂)ₙCH₃, optionally substituted mono- or bicyclic aryl, heterocyclic or alicyclic group; and
   R₂ independently from R₁ denotes H, (CH₂)ₘ-CH₃, or aryl;
   or both of R₁ and R₂ denote X, OH or O(CH₂)ₙCH₃;
   or R₁ and R₂ together denote =O, wherein
   any one of CH₂ or CH₃ groups denoted above may optionally be further substituted by X, N₃, CN, NO₂, OH, (CH₂)n-CH3, aryl, O-(CH₂)ₙ-CH_{3,} OCO(CH₂)ₙCH₃, NR³R⁴, NR³CO(CH₂)ₙCH₃; or
   each CH₂ linking carbon atoms can be replaced by O, S or NR³; wherein
   R³ and R⁴ independently from each other, or together, denote H, (CH₂)ₘ-CH₃,;
   X denotes F, Cl, Br or I;
   n represents an integer from 0 to 10;
   m represents an integer from 0 to 3;
(4) The process for preparing a compound of formula (I) according to any one of the previous items, in which
   R₁ independently from R₂ denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, or NR³CO(CH₂)ₙCH₃; and
   R₂ independently from R₁ denotes H or (CH₂)ₘ-CH₃;
   or both of R₁ and R₂ denote X, OH or O(CH₂)ₙCH₃;
   or R₁ and R₂ together denote =O, wherein
   R³ and R⁴ independently from each other, or together, denote H, (CH₂)ₘ-CH₃, -;
   X denotes F, Cl, Br or I;
   n represents an integer from 0 to 10;
   m represents an integer from 0 to 3;
(5) The process according to any one of the previous items, wherein the enzyme capable of catalyzing oxidation or dehydrogenation is specific for oxidation of lactol hydroxy group.
(6) The process according to any one of the previous items, wherein the enzyme capable of catalyzing oxidation or dehydrogenation is aldose 1-dehydrogenase (EC 1.1.5.2).
(7) The process according to any one of the previous items, wherein the aldose 1-dehydrogenase is selected from a group of pyrroloquinoline quinine (PQQ) dependent dehydrogenases.
(8) The process according to any one of the previous items, wherein the enzyme is water soluble or membrane bound.
(9) The process according to any one of the previous items, wherein the aldose 1-dehydrogenase is selected from the group consisting of dehydrogenases encoded by dehydrogenase-encoding genes comprised withinany one of nucleotide sequences of SEQ ID NOS. 01, 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences SEQ ID NOS. 02, 04, 06, 08, 10, 24, 26, 28, 30, 32, 34, 36, 38, 40, 42, 44, 46, 48, 50, 52, 54, 56, 58, 60 and 62; or any dehydrogenase having a nucleotide sequence identity or an amino acid sequence identity respectively of at least 50 % to said sequences, preferably 70 % to, more preferably 90 % to said sequences, provided that the resulting sequence variants maintain dehydrogenase activity.
(10) The process according to any one of the previous items, wherein the aldose 1-dehydrogenase is Ylil aldose dehydrogenase from E. coli or Gcd membrane bound glucose dehydrogenase from E. coli.
(11) The process according to any one of the previous items, wherein 2-deoxyribose-5-phosphate aldolase (DERA, EC 4.1.2.4) enzyme is used for preparing the compound of formula (II).
(12) The process according to item 12, wherein 2-deoxyribose-5-phosphate aldolase (DERA, EC 4.1.2.4) enzyme is used for a synthetic step preceding, or alternatively simultaneously at least in an overlapping time period with, bringing in contact the compound of formula (II) with the enzyme capable of catalyzing oxidation or dehydrogenation.
(13) The process according to any one of the previous items, wherein the compound of formula (II) is brought in contact with the aldose 1-dehydrogenase without prior isolation or purification of the compound of formula (II).
(14) The process according to any one of the previous items, wherein the aldose 1-dehydrogenase, optionally also DERA enzyme independently, are comprised within living non-human whole cell, inactivated non-human whole cell, homogenized non-human whole cell, or non-human cell free extract; or are purified, immobilized and/or are in the form of an extracellularly expressed protein, preferably are within living non-human whole cell, inactivated non-human whole cell or homogenized non-human whole cell, more preferably are within living non-human whole cell or inactivate non-human whole cell, particularly are comprised within living non-human whole cell.
(15) The process according to any one of items 11 to 14, wherein the compound of formula (I) is prepared at least in part simultaneously with the preparation of the compound of formula (II).
   In the preferred embodiments as defined in items 11 to 14, the arrangement of having the compound (II) prepared by using 2-deoxyribose-5-phosphate aldolase (DERA) enzyme and allowing the product to be used, preferably to be simultaneously used at least in an overlapping time period, with a subsequent oxidation reaction by the enzyme capable of catalyzing oxidation or dehydrogenation is especially advantageous in terms of process efficiency and reduced time required for the process. When the processes proceed at least partially simultaneously, the prepared compound of formula (II) can get immediately consumed as a substrate in a subsequent oxidation reaction, which shifts the steady state equilibrium of the first reaction in a direction of the product.
(16) The process according to any one of the previous items, wherein electron acceptor is comprised in the process or added to the enzyme capable of catalyzing oxidation or dehydrogenation, preferably oxygen is used or added; and/or wherein cofactor(s) of the enzymes in order to become functional is comprised in the process or added, such as cofactors selected from the goup of FAD, NAD(P)⁺ and/or PQQ, and optionally further additives and auxiliary agents as disclosed herein.
   When according to this further preferred embodiment oxygen is added to the enzyme capable of catalyzing oxidation or dehydrogenation, or the process is run in the presence of oxygen, like for example under aerated conditions, preferably when air is bubbled to the dehydrogenation or oxidation reaction catalysed by the dehydrogenation or oxidation enzyme, the reaction becomes irreversible, which secures the obtained product and further enhances shifting of the steady state equilibrium of the first reaction, when the compounds of formula (II) and (I) are prepared simultaneously. The presence of oxygen, particularly the presence of dissolved oxygen above 5%, wherein 100% dissolved oxygen is understood as saturated solution of oxygen at given process conditions, is again a favourable process parameter that increases yield and reduces reaction times. In addition, allowing oxygen to be present might in a specific case promote proliferation of a microorganism used. Similar explanations apply to the use of cofactor(s) and optional further additives and auxiliary agents useful for the respective enzyme.
(17) A reaction system, or a one-pot process for preparing a compound of formula (I) 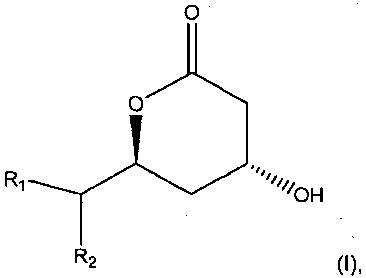 in which R₁ and R₂ are as defined in any one of items 1 to 4, or a pharmaceutically acceptable salt, ester or stereoisomer thereof,
   the reaction sytem being capable of or arranged for, or the one-pot process comprising reacting a compound of formula (X), 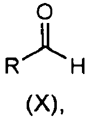 in which R denotes R₁-CH-R₂ moiety of formula (I), R₁ and R₂ being as defined in any one of tems 1 to 4,
   with acetaldehyde in the presence of 2-deoxyribose-5-phosphate aldolase (DERA) enzyme and an aldose 1-dehydrogenase, and optionally salifying, esterifying or stereoselectively resolving the product.
   Here, the aldose 1-dehydrogenase may preferably be represented by an enzyme as defined in any one of items 5 to 10.
   The term "reaction sytem" means a technical system, for example an *in* vitro-system, a reactor or a cultivation vessel or a fermentor.
   The terms "capable of" or "arranged for" means that the reaction system is suitable, or conditions and configurations are set that the defined reaction can take place.
(18) The system or process according to item 17, wherein a compound of formula (II) as defined in item 1 is generated as intermediate, which is not isolated.
(19) The system or process according to any one of items 17 or 18, wherein the aldose 1-dehydrogenase, optionally also DERA enzyme independently, are comprised within living non-human whole cell, inactivate non-human whole cell, homogenized non-human whole cell, or non-human cell free extract; or are purified, immobilized and/or are in the form of an extracellularly expressed protein, preferably are within living non-human whole cell, inactivated non-human whole cell or homogenized non-human whole cell, more preferably are within living non-human whole cell or inactivated non-human whole cell, particularly are comprised within living non-human whole cell.
(20) The system or process according to any of the items 17 to 19, wherein both said enzymes, i.e. DERA and the aldose 1-dehydrogenase, are expressed by a same cell.
(21) The process according to item 14, or the system or process according to items 19 or 20, wherein the cell is a bacteria, yeast, insect cell or a non-human mammalian cell, preferably is bacteria or yeast, more preferably is bacteria.
(22) The system or process according to item 21, wherein the bacteria is selected from the group of genera consisting of *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacfer, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans, Corynebacterium glutamicum, Escherichia coli, Bacillus licheniformis,* and *Lactobacillus lactis,* most preferably from *Escherichia coli, Gluconobacter oxydans, Acinetobacter calcoaceticus and Kluyvera intermedium,* particularly is *Escherichia coli.*
(23) The system or process according to item 21, wherein the yeast is selected from the group of genera consisting of Saccharomyces, Pichia, Shizosaccharomyces and Candida, preferably Saccharomyces.
(24) The system or process according to item 21, wherein non-human mammalian cell is Chinese hamster ovary cell or a hepatic cell, preferably is Chinese hamster ovary cell.
(25) The process according to any one of the previous items, further comprising subjecting said compound (I) to conditions sufficient to prepare a statin, or a pharmaceutically acceptable salt thereof, preferably lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin, or a pharmaceutically acceptable salt thereof.
(26) A process for preparing a pharmaceutical composition, the process comprising carrying out a process according to the process of item 25, and
   formulating said statin, or a pharmaceutically acceptable salt thereof, with at least one pharmaceutically acceptable excipient to obtain said pharmaceutical composition.
(27) Use of the reaction system according to item 17 for preparing a compound of formula (I), wherein the formula (I) is as defined in any one of items 1 to 3, and optionally for further preparation of a statin or a pharmaceutically acceptable salt thereof.
(28) Use of an aldose 1-dehydrogenase enzyme for preparing a compound of formula (I) 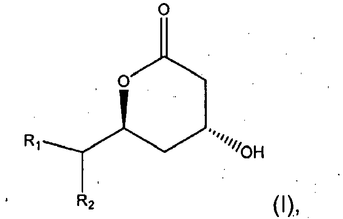 in which R₁ and R₂ have the same meaning as above,
   which compound is further converted into a statin or a pharmaceutically acceptable salt thereof.
(29) Use according to item 28, wherein the statin or a pharmaceutically acceptable salt thereof is selected from lovastatin, pravastatin, simvastatin, atorvastatin, cerivastatin, rosuvastatin, fluvastatin, pitavastatin, bervastatin, and dalvastatin, and pharmaceutically acceptable salts thereof.

### Detailed description of the Invention

Surprisingly we found a process for preparing a compound of formula (I) 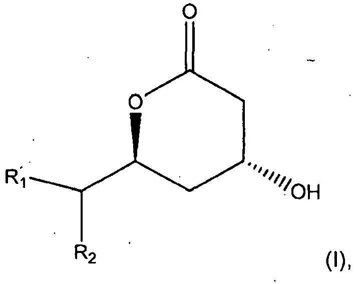 in which
R₁ independently from R₂ denotes H, X, N₃, CN, NO₂, OH, (CH₂)ₙ-CH₃, O-(CH₂)ₙ-CH_{3,} S-(CH₂)ₙ-CH₃, NR³R⁴, OCO(CH₂)ₙCH₃, NR³CO(CH₂)ₙCH₃, CH₂-R⁵, or optionally substituted mono- or bicyclic aryl, heterocyclic or alicyclic group; and
R₂ independently from R₁ denotes H, (CH₂)ₘ-CH₃, or aryl;
or both of R₁ and R₂ denote either X, OH or O((CH₂)ₙCH₃);
or R₁ and R₂ together denote =O, =CH-R₅,, wherein
any one of CH₂ or CH₃ groups denoted above may optionally be further substituted by X, N₃, CN, NO₂, OH, (CH₂)ₙ-CH₃, aryl, O-(CH₂)ₙ-CH_{3,} OCO(CH₂)ₙCH₃, NR³R⁴, NR³CO(CH₂)ₙCH₃; or each CH₂ linking carbon atoms can be replaced by O, S or NR³; wherein
R³ and R⁴ independently from each other, or together, denote H, (CH₂)ₘ-CH₃;
R⁵ denotes optionally substituted mono- or bicyclic aryl, heterocyclic or alicyclic group,
X denotes F, Cl, Br or I;
n represents an integer from 0 to 10;
m represents an integer from 0 to 3;
or a pharmaceutically acceptable salt, ester, or stereoisomer thereof,
wherein a non-natural lactol compound of formula (II), 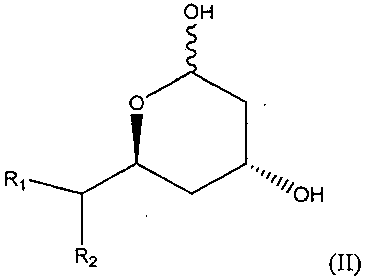 wherein R₁ and R₂ are defined as above, can be simply brought in contact with an aldose 1-dehydrogenase.

The term "mono- or bicyclic aryl group" as used herein refers to any mono- or bicyclic, 5-, 6- or 7-membered aromatic or heteroaromatic ring, such as for example pyrolyl, furanyl, tiophenyl, phenyl, imidazolyl, pyridinyl, piridazinyl, indolyl, kinolinyl ftaliminyl and benzimidazolyl.

The term "aryl" as used herein, if not stated otherwise with respect to particular embodiments, includes reference to an aromatic ring system comprising 6, 7, 8, 9, 10, 11, 12, 13, 14, 15 or 16 ring carbon atoms. Aryl can be phenyl but may also be a polycyclic ring system, having two or more rings, at least one of which is aromatic. This term includes phenyl, naphthyl, fluorenyl, azulenyl, indenyl, anthryl and the like.

The term "mono- or bicyclic heterocyclic group" as used herein refers to any mono- or bicyclic, 5-, 6- or 7-membered saturated or unsaturated ring, wherein at least one carbon in the ring is replaced by an atom selected from the group of oxygen, nitrogen and sulphur. The non-limiting examples of mono- or bicyclic heterocyclic group are oksazolyl, tiazolyl, isotiazolyl, morfolinyl.

The term "heterocycle" as used herein includes, if not stated otherwise with respect to particular embodiments, a saturated (e.g. heterocycloalkyl) or unsaturated (e.g. heteroaryl) heterocyclic ring moiety having 3, 4, 5, 6, 7, 8, 9, 10, 11, 12, 13, 14, 15 or 16 ring atoms, at least one of which is selected from nitrogen and oxygen. In particular, heterocyclyl includes a 3- to 10-membered ring or ring system and more particularly a 5- or 6-or 7-membered ring, which may be saturated or unsaturated; examples thereof include oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, β-carbolinyl, phenanthridinyl, acridinyl, perimidinyl, phenanthrolinyl, furazanyl, phenazinyl, phenothiazinyl, phenoxazinyl, ehromenyl, isochromanyl, chromanyl and the like.

More specifically, a saturated heterocyclic moiety may have 3, 4, 5, 6 or 7 ring carbon atoms and 1, 2, 3, 4 or 5 ring heteroatoms selected from nitrogen and oxygen. The group may be a polycyclic ring system but more often is monocyclic, for example including azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolinidinyl and the like. Furthermore, the "heteroaryl" may include an aromatic heterocyclic ring system having 5, 6, 7, 8, 9, 10, 11, 12, 13, 14, 15 or 16 ring atoms, at least one of which is selected from nitrogen and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, 2H-chromenyl, oxazolyl, isoxazolyl, thiazolyl, isoindolyl, indazolyl, purinyl, isoquinolinyl, quinazolinyl, pteridinyl and the like.

The term "mono- or bicyclic alicyclic group" as used herein refers to any mono- or bicyclic, 5-, 6- or 7-membered alicyclic ring.

The term "salifyed" or "a pharmaceutically acceptable salts" in a context of the compound of formula (I) or (II), API or statin, which can be optionally substituted, as used herein refers to the compound or statin in a form of a salt, such as potassium, sodium, calcium, magnesium, hydrochloride, hydrobromide, or the like, that is also substantially physiologically tolerated. The compound of formula (I) or (II), API or statin, can be salifyed or brought in the form of a salt by mixing the compound of formula (I) or (II), API or statin or intermediate thereof, with an acid or a base, optionally in an aqueous or organic solvent, or a mixture thereof. Preferably the solvent is afterwards removed.

The term "esterifying" or "esters" in a context of the compound of formula (I) or (II), API or statin, as used herein refers to the compound of formula (I) or (II), or statin, with at least one ester bond in their structure. Such ester bond or esterifying the compound can be achieved by coupling the compound of formula (I) or (II), API or statin or intermediate thereof, in the event the compound or statin contains hydroxyl group, with an carboxylic acid or a phosphate group containing compound. In the event that the compound of formula (I) or (II), API or statin or intermediate thereof, contain carboxylic or phosphate group, it can be achieved by coupling it with a hydroxylic group of another compound.

The term "stereoselectively resolved" is used herein to refer to any method known to the skilled person in the field of separating a mixture of stereoisomers, preparatory chemistry of stereospecific compounds, or analytics. The stereoisomers can be obtained for example by HPLC, wherein stereoselective column is used. Stereoselective columns are known in the art.

### Enzymes, Organisms

The aldose 1-dehydrogenease is an enzyme that catalyzes oxidation or dehydrogenation. The enzyme recognises and uses e.g. the lactol compound of formula (II) as a substrate. Combinations of enzymes, multiunit enzymes, wherein different units catalyse optionally different reactions, fused or joined enzymes, or enzymes coupled to another structural or a non-catalytic compound, unit, subunit or moiety, are also contemplated within the present invention as long as the requirement of being capable of catalyzing oxidation or dehydrogenation is fulfilled. The enzyme can be for example an enzyme found in the electron transfer chain of the prokaryote or eukaryote cells or in the biochemical pathways of alcohols, aldehydes or sugars in eukaryote or prokaryote cells. Enzymes that would normally act upon natural substrates were unexpectedly found to recognise and oxidise rather complex synthetic compounds, in particular convert lactols into lactones or possibly into esters. It is an important aspect of the present invention that the reactions, which are meant to be used for the an enzyme capable of catalyzing oxidation or dehydrogenation, do normally not occur in the nature, because the substrate is different from the natural occurring ones, like for example expempting from using the ethanol, methanol, acetaldehyde, acetic acid, naturally occurring sugars or naturally occurring amino acids, or acids obtained from the sugars, like for example gluconic acid. It was however surprisingly found that synthetic substrates as disclosed herein, even if being rather structurally complex, can yet be easily processed by using the enzyme capable of catalyzing oxidation or dehydrogenation in order to finally obtain API, particularly statin, or intermediates thereof, including e.g. a lactone compound of formula (I). Generally, enzyme can be chosen from oxydoreductases.

According to the enzyme nomenclature, the enzyme applicable in the present invention belongs primarily to EC 1.1 (oxidoreductases acting primarly on the CH-OH group of donors), more specifically to, but not limited to subclasses: EC 1.1.1 (with NAD⁺ or NADP⁺ as acceptor), EC 1.1.2 (with a cytochrome as acceptor), EC 1.1.3 (with oxygen as acceptor), EC 1.1.5 (with a quinine or flavine or similar compound as acceptor). Any of the oxidoreductases known in the art may be used for the reaction regardless of their sequence identity.

In the following, enzymes will be described in further detail, which are in principle applicable in the present invention - illustrative experimental examples will be described later, and if necessary suitable screeing and/or verification tests are also provided herein. Some of the particular enzymes had been described and optionally used prior to the invention, but in disctinctively other contexts or fields than the present invention. References, the disclosures of which are incorporated herein, will be cited and listed below.

Enzymes having activity of oxidation/dehydrogenation of sugars have been widely used in the industry. Typical examples of oxidative fermentations are traditional production of D-gluconate (gluconic acid), L-sorbose and others. These processes were developed as a practical industry based on empirically found properties of some microorganisms before the clarification of the molecular mechanisms of the responsible enzymes [Adachi, 2007].

Sugar oxidising enzymes had been used in food processing as additives, in dairy and the lactoperoxidase system for food preservation, in breadmaking, for producing dry egg powder, as antioxidants/preservatives (oxygen scavengers), for reducing alcohol wine, as glucose assays and fuel cells [Wong, 2008]. Sugar oxidising enzymes had been used also as amperometric biosensors, e.g. for measuring glucose concentration in blood [Igarashi, 2004], for detection of heavy metals [Lapenaite, 2003], for detection of formaldehyde in air [Acmann, 2008], for detection of phenolic compounds in flow injection analysis [Rose, 2001], as a ultrasensitive bienzyme sensor for adrenaline [Szeponik, 1997], for determination of xylose concentration [Smolander, 1992] etc.

A well known enzyme capable of oxidation of six-membered sugars is Glucose oxidase, Gox (EC 1.1.3.4), which is commercially available from Sigma as an extract from *Aspergillus niger.* This enzyme has a very narrow substrate specificity [Keilin, 1952]. It is produced naturally in some fungi and insects where its catalytic product, hydrogen peroxide, acts as an anti-bacterial and anti-fungal agent. Gox is generally regarded as safe, and Gox from *A*. *niger* is the basis of many industrial applications [Wong, 2008]. Gox-catalysed reaction has also been used in baking, dry egg powder production, wine production, gluconic acid production, etc. Its electrochemical activity makes it an important component in glucose sensors, especially in diabetics, as is also the case with PQQ dependent sugar dehydrogenase, and potentially in fuel cell applications. Glucose oxidase is capable of oxidising monosaccharides, nitroalkanes and hydroxyl compounds [Wilson, 1992]. Using the reaction rate of glucose as reference (100 %), only 2-deoxy-D-glucose (20-30 %), 4-O-methyl-D-glucose (15 %) and 6-deoxy-D-glucose (10 %) are oxidized by glucose oxidase from A. niger at a significant rate [Pazur, 1964; Leskovac, 2005]. The activities of glucose oxidase against other substrates are typically poor, with reaction rates lower than 2 % of glucose's [Keilin, 1948; Pazur, 1964; Leskovac, 2005].

In a preferred embodiment, the aldose 1-dehydrogenase is sugar dehydrogenase (EC 1.1). The terms of the current art describing such enzymes may be different to the one provided by this invention, however it will be understood herein that substrate specificity and capability of the enzyme to catalyse the oxidation/dehydrogenation of compound (II) or other compounds contemplated herein are independent of terminology found in the current art. One example which can be found is the terminology for an enzyme found in E. coli (Ylil, Adh, Asd) which is termed "soluble glucose dehydrogenase" by some authors, "aldose sugar dehydrogenase" or "soluble aldose dehydrogenase" by others. enzyme can be for example an enzyme found in the electron transfer chain of the prokaryote or eukaryote cells or in the biochemical pathways of alcohols, aldehydes or sugars in eukaryote or prokaryote cells. Enzymes that would normally act upon natural substrates were unexpectedly found to recognise and oxidise rather complex synthetic compounds, in particular convert lactols into lactones or possibly into esters. It is an important aspect of the present invention that the reactions, which are meant to be used for the an enzyme capable of catalyzing oxidation or dehydrogenation, do normally not occur in the nature, because the substrate is different from the natural occurring ones, like for example expempting from using the ethanol, methanol, acetaldehyde, acetic acid, naturally occurring sugars or naturally occurring amino acids, or acids obtained from the sugars, like for example gluconic acid. It was however surprisingly found that synthetic substrates as disclosed herein, even if being rather structurally complex, can yet be easily processed by using the enzyme capable of catalyzing oxidation or dehydrogenation in order to finally obtain API, particularly statin, or intermediates thereof, including e.g. a lactone compound of formula (I). Generally, enzyme can be chosen from oxydoreductases.

According to the enzyme nomenclature, the enzyme applicable in the present invention belongs primarily to EC 1.1 (oxidoreductases acting primarly on the CH-OH group of donors), more specifically to, but not limited to subclasses: EC 1.1.1 (with NAD⁺ or NADP⁺ as acceptor), EC 1.1.2 (with a cytochrome as acceptor), EC 1.1.3 (with oxygen as acceptor), EC 1.1.5 (with a quinine or flavine or similar compound as acceptor). Any of the oxidoreductases known in the art may be used for the reaction regardless of their sequence identity.

In the following, enzymes will be described in further detail, which are in principle applicable in the present invention - illustrative experimental examples will be described later, and if necessary suitable screeing and/or verification tests are also provided herein. Some of the particular enzymes had been described and optionally used prior to the invention, but in disctinctively other contexts or fields than the present invention. References will be cited and listed below.

Enzymes having activity of oxidation/dehydrogenation of sugars have been widely used in the industry. Typical examples of oxidative fermentations are traditional production of D-gluconate (gluconic acid), L-sorbose and others. These processes were developed as a practical industry based on empirically found properties of some microorganisms before the clarification of the molecular mechanisms of the responsible enzymes [Adachi, 2007].

Sugar oxidising enzymes had been used in food processing as additives, in dairy and the lactoperoxidase system for food preservation, in breadmaking, for producing dry egg powder, as antioxidants/preservatives (oxygen scavengers), for reducing alcohol wine, as glucose assays and fuel cells [Wong, 2008]. Sugar oxidising enzymes had been used also as amperometric biosensors, e.g. for measuring glucose concentration in blood [Igarashi, 2004], for detection of heavy metals [Lapenaite, 2003], for detection of formaldehyde in air [Acmann, 2008], for detection of phenolic compounds in flow injection analysis [Rose, 2001], as a ultrasensitive bienzyme sensor for adrenaline [Szeponik, 1997], for determination of xylose concentration [Smolander, 1992] etc.

A well known enzyme capable of oxidation of six-membered sugars is Glucose oxidase, Gox (EC 1.1.3.4), which is commercially available from Sigma as an extract from *Aspergillus niger.* This enzyme has a very narrow substrate specificity [Keilin, 1952]. It is produced naturally in some fungi and insects where its catalytic product, hydrogen peroxide, acts as an anti-bacterial and anti-fungal agent. Gox is generally regarded as safe, and Gox from A. *niger* is the basis of many industrial applications [Wong, 2008]. Gox-catalysed reaction has also been used in baking, dry egg powder production, wine production, gluconic acid production, etc. Its electrochemical activity makes it an important component in glucose sensors, especially in diabetics, as is also the case with PQQ dependent sugar dehydrogenase, and potentially in fuel cell applications. Glucose oxidase is capable of oxidising monosaccharides, nitroalkanes and hydroxyl compounds [Wilson, 1992]. Using the reaction rate of glucose as reference (100 %), only 2-deoxy-D-glucose (20-30 %), 4-*O-*methyl-D-glucose (15 %) and 6-deoxy-D-glucose (10 %) are oxidized by glucose oxidase from A. niger at a significant rate [Pazur, 1964; Leskovac, 2005]. The activities of glucose oxidase against other substrates are typically poor, with reaction rates lower than 2 % of glucose's [Keilin, 1948; Pazur, 1964; Leskovac, 2005].

The enzymes for use in the present invention is an aldose 1- dehydrogenase. The terms of the current art describing such enzymes may be different to the one provided by this invention, however it will be understood herein that substrate specificity and capability of the enzyme to catalyse the oxidation/dehydrogenation of compound (II) or other compounds contemplated herein are independent of terminology found in the current art. One example which can be found is the terminology for an enzyme found in E. coli (Ylil, Adh, Asd) which is termed "soluble glucose dehydrogenase" by some authors, "aldose sugar dehydrogenase" or "soluble aldose dehydrogenase" by others.

Another example is the terminology for the membrane bound glucose dehydrogenase found in E. Coli (mGDH, GCD, PQQMGDH) which is termed "PQQ dependant glucose dehydrogenase" by some authors or "membrane bound glucose dehydrogenase" or GCD by others.

The natural substrate for the sugar dehydrogenases (aldose dehydrogenases) are various sugars that get oxidized. The broad range of sugars that aldose dehydrogenase can act upon encompasses pentoses, hexoses, disaccharides and trisaccharides. The enzyme capable of catalyzing oxidation or dehydrogenation is specific for oxidation at position C1. The aldose 1-dehydrogenase enzyme oxidizes the aldehyde or cyclic hemiacetal to lactone.

In agreement to the above, sugar oxidoreductases are divided into classes, according to electron acceptors (in some cases these are the cofactors these enzymes use in order to become functional, i.e. FAD, NAD(P)⁺ or PQQ). In terms of substrate specificity, which may vary strongly between the subclasses of sugar oxidoreductases, the use of PQQ dependent dehydrogenases (EC 1.1.5) are preferred according to the present invention.

FAD- (flavoprotein dehydrogenases) and PQQ-dependent sugar dehydrogenases (quinoprotein dehydrogenases), EC 1.1.5, use flavin adenine dinucleotide (FAD) or pyrroloquinoline quinine (PQQ) cofactors respectively, and are located on the outer surface of the cytoplasmic membrane of bacteria, facing the periplasmic space with their active sites. These are often termed membrane sugar dehydrogenases. Alternatively, especially in the PQQ-dependent sugar dehydrogenases group, many enzymes are found in soluble form, located in the periplasmic space. There is no limitation in nature of the used sugar dehydrogenase in regard to solubility, however it may be preferred, relating to cloning, expression procedures and molecular tools available, that soluble periplasmic sugar dehydrogenases are selected, i.e. water-soluble ones. Construction of expression strain for efficient periplasmic expression of such enzyme is technically easier and thus preferable. On the other hand the membrane bound sugar dehydrogenases may be more challenging for efficient expression but may be preferred due to their more intimate connection to the respiratory chain, via the transfer of electrons from the PQQ to the ubiquinone pool. In the course of the present disclosure, examples are provided, e.g. using naturally occurring membrane bound sugar dehydrogenases as well as the overexpressed membrane bound sugar dehydrogenases for conversion of compound (II) to compound (I) in industrially useful yields.

### Respiratory Chain; Electron Acceptors

The electrons generated by the oxidation process are transferred from substrates via the enzyme cofactor (electron carrier) to the terminal ubiquinol oxidase with ubiquinone as a mediator in the respiratory chain of host organisms. The final acceptor of electrons is oxygen which is reduced to water by the respiratory chain oxidoreductases.

A respiratory chain is a series of oxidoreductive enzymes, having ability to transfer electrons from a reduced molecule in a cascade of finely tuned stepwise reactions, which are concluded by reduction of oxygen. Each step uses a difference in redox potential for useful work, e.g. transfer of protons across the cytoplasmic membrane, reduction of other molecules etc. Electron carriers have a major role in the respiratory chain as well as in overall cell's oxidoreductive processes.

Electrons can enter the respiratory chain at various levels. At the level of a NADH dehydrogenase which oxidizes NADH/NADPH (obtained by various oxidoreductive processes in the cell) with transfer of electrons to ubiquinone pool and release of protons to extracellular space. Alternatively oxidoreductases can transfer electrons directly to ubiquinon via enzyme bound cofactors (FAD,PQQ). The ubiquinoles are furher oxydised by terminal oxidoreductases such as Cytochomes, Nirate reductsases etc., whereby the electrons are coupled with intracellular protons to reduce oxygen (forming water) and ubiquinole bound protons are released into extracellular space. Any system which is capable of translocation of protons exploiting redox potential is ofted known as a proton pump. A cross membrane proton potential is thereby established and is the driving force for function of ATP synthases. These levels have successively more positive redox potentials, i.e. successively decreased potential differences relative to the terminal electron acceptor. Individual bacteria often simultaneously use multiple electron transport chains. Bacteria can use a number of different electron donors, a number of different dehydrogenases, different oxidases and reductases, and different electron acceptors. E.g., *E. coli* (when growing aerobically using glucose as an energy source) uses two different NADH dehydrogenases and two different quinol oxidases, for a total of four different electron transport chains operating simultaneously.

It is therefore clear that an oxidoreductase (for example sugar dehydrogenase) can only be functional when a electron acceptor is provided. In case of natural systems this is provided by the respiratory chain, alternatively artificial electron acceptors with appropriate redox potential compared to substrate/enzyme/cofactor cascade can be used. In the latter option the disadvantage is that the artificial electron acceptor has to be provided in rather large quantities, in other words normally in equimolar amount to the substrate being oxidized.

In this aspect, the acceptor of electrons generated by the enzyme capable of catalyzing oxidation or dehydrogenation may be provided in the reaction mixture in order to promote electron flow and the oxidation or dehydrogenation of compound (II). The acceptor may be selected from but is not limited to: dichlorophenolindophenol (DCPIP), phenazine methosulfate (PMS), potassium ferricyanide (PF), potassium ferrioxalate, *p*-benzoquinone, phenyl-*p*-benzoquinone, duroquinone, silicomolybdate, vitamin K3, diaminodurene (DAD), *N,N,N',N',*tetramethyl-*p*-phenylenediamine (TMDP). Electron acceptor may also be oxygen. A person skilled in art will recognize and can e.g. use compounds listed as Hill reagents, dyes that act as artificial electron acceptors, changing colors when reduced, and find many additional candidate acceptors from literature.

### PQQ Dehydrogenases

It has been shown that PQQ has the ability to complex divalent cations in solution, which is a prerequisite for the catalytic activity of the bacterial quinoprotein dehydrogenases [Mutzel, 1991; Itoh, 1998; Itoh, 2000]. In order to make bacterial quinoprotein dehydrogenase (regardless of its origin) active, besides PQQ also divalent ions (e.g. Mg2+, Ca2+) must be present to achieve successful reconstitution of the holo-form of the enzyme [James, 2003]. Complexed divalent ions have besides their structural role also a role in maintaining PQQ's active configuration [Anthony, 2001].

After screening PQQ dependant aldose dehydrogenases, we surprisingly found that all tested enzymes (e.g. Ylil aldose dehydrogenase from *E. coli,* Gcd membrane bound glucose dehydrogenase from *E*. *coli,* GDH from *Acinetobacter calcoaceticus* (soluble or membrane-bound form), GDH from *Gluconobacter oxidans,* GDH from Kluyvera intermedium) could oxidize compound (II) to compound (I) - (R1 = H, R2 = O-CO-CH₃) in the presence of an electron acceptor regardless of the acceptor being synthetic molecule or microorganism's respiratory chain. In fact all organisms tested, which contain PQQ dependent oxidoreductases, were found to successfully oxidize ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in our experiments. Several were tested successfully also for other compound (II) molecules which are exemplified below.

In this sense, the preferred aspect of this disclosure deals with PQQ dependent dehydrogenases (quinoproteins, EC 1.1.5), more specifically PQQ dependant sugar 1-dehydrogenases (EC 1.1.5.2).

For example Ylil is aldose sugar dehydrogenase from *E. coli,* which requires PQQ for its activity [Southall, 2006]. While *E. coli* lacks the ability to synthesize PQQ itself [Hommes, 1984; Matsushita, 1997], it shows positive chemotaxis effect towards PQQ, found in environment [de Jonge, 1998], and can use an externally supplied cofactor [Southall, 2006]. Ylil aldose sugar dehydrogenase is a soluble, periplasmic protein, containing N-terminal signal sequence necessary for translocation into the periplasm through the cytoplasmic membrane. Ylil aldose sugar dehydrogenase (Asd) fold contains six four stranded antiparallel β-sheets with PQQ-binding site lying on the surface of the protein in a shallow, solvent exposed cleft. Ylil protein is a monomer, each binding two calcium ions, one of them lying in the PQQ binding pocket, and the other compressed between two of the six strands that make up the propeller fold [Southall, 2006].

It had been shown [Southall, 2006] that D-glucose, D-galactose, D-fructose, D-arabinose, D-fucose, D-mannose, D-lyxose, D-xylose, D-ribose, xylitol, *myo*-inositol, L-sorbose, mannitol, 2-deoxy-D-glucose, glucosamine, N-acetylglucosamine, glucose 1-phosphate, glucose 6-phosphate, maltose, α-lactose, D-sucrose, D-cellobiose, melibiose and maltotriose are accepted natural substrates of Ylil aldose 1-dehydrogenase. None of the natural sugars with the stereochemistry at the C3 position, resembling compound (II), such as D-Allose, D-Altrose, D-Gulose or D-Idose, were tested by Suthall *et al.*

Besides Ylil aldose sugar dehydrogenase, *E. coli* contains also a membrane-bound glucose dehydrogenase (mGDH), which is also a quinoprotein involved within the respiratory chain in the periplasmic oxidation of alcohols and sugars [Yamada, 2003]. This enzyme, also termed GCD or mGDH or PQQGDH, occurs like Ylil in a form of apoenzyme, since *E. coli* lacks ability to synthesize pyrroloquinoline quinone (PQQ), the enyzme's prosthetic group. mGDH is a membrane-bound quinoprotein [Matsushita, 1993; Anthony, 1996; Goodwin, 1998] that catalyzes oxidation of D-glucose to D-gluconate on its C-terminal domain streaching into the periplasmic space. The electron transfer, mediated by PQQ is further driven by the N terminal, membrane integrated domain; the electron flow to the repiratory chain is channelled through ubiquinone pool to the ubiquinol oxidase [Van Schie, 1985; Matsushita, 1987; Yamada, 1993]. Active holo-form of the mGDH enzyme is obtained by the addition of PQQ and Mg2+ or Ca2+, or other bivalent metal ions. GCD is a monomeric protein that possesses N-terminal hydrophobic domain spanning the inner membrane [Yamada, 1993], and large C-terminal domain, located in the periplasmic space, containing binding sites for PQQ and Mg2+ or Ca2+ [Yamada, 1993; Cozier, 1999].

Surprisingly, mGDH enzyme is also able to catalyze oxidation of artificial substrate, a compound of formula (II). Cozier, *et al.* tested its activity towards D-allose, which was the only natural aldohexose with a similar stereochemistry on C-3 atom to that of compound (II) tested, and showed similar activity compared to D-glucose. It is noteworthy that D-allose differs from compound (II) in two additional OH-groups on C-2 and C-4, which makes the activity towards compound (II) equally surprising and unexpected.

Yet another example of PQQ dependant sugar dehydrogenase is *Acinetobacter calcoaceticus* GDH. At least two distinct quinoprotein glucose dehydrogenase from *Acinetobacter calcoaceticus* are known: the membrane-bound form (mGDH) and the soluble form (sGDH), which contains a 24-amino-acid N-terminal signal sequence needed for translocation through the cytoplasmic membrane into the periplasm. Both forms are different in all characteristics, e.g. substrate specificity, molecular size, kinetics, optimum pH, immunoreactivity.

The substrate specificity of sGDH is different from that of mGDH. sGDH oxidizes preferably D-glucose, maltose and lactose and less successfully D-fucose, D-xylose, D-galactose, while mGdh is less reactive with disaccharides; it oxidises preferably D-glucose, 6-deoxy-D-glucose, 2-deoxy-D-glucose, D-allose, D-fucose, 2-amino-D-glucose (glucosamine), 3-deoxy-D-glucose, D-melibiose, D-galactose, D-mannose, 3-O-methyl-D-glucose, D-xylose, L-arabinose, L-lyxose and D-ribose, yet less successfully maltose, and lactose [Cozier, 1999; Adachi, 2007].

The two possible reaction mechanisms for sGDH are: (A) The addition-elimination mechanism comprises general base-catalyzed proton abstraction followed by covalent addition of the substrate and subsequent elimination of the product; (B) Mechanism comprising general base catalyzed proton abstraction in concert with direct hydride transfer from substrate to PQQ, and tautomerization to PQQH₂ [Oubrie, 1999]. A similar mechanism is assumed to be the case for *E.coli* Ylil aldose dehydrogenase enzyme.
Like the previously described PQQ dependant dehydrogenases, both sGDH and mGDH require calcium or magnesium for dimerization and function [Olsthoorn, 1997]. The present structures confirm the presence of three calcium binding sites per monomer [Oubrie, 1999].

As exemplified by this invention the diversity of these enzymes in sence of structural properties, localization, mechanisms of cofactor binding and electron transfer etc. does not influence efficacy of said diverse enzymes in catalysing oxidation of compound (II) to compound (I)

Current industrial application of PQQ dependent sugar dehydrogenases includes D-gluconate production (*Gluconobacter oxydans*) in classic fermentation processes as well as production of various natural sugars. *Gluconobacter oxydans* organism is well known for its important ability to incompletely oxidize natural carbon substrates such as D-sorbitol (producing L-sorbose for vitamin C synthesis), glycerol (producing dihydroxyacetone), D-fructose, and D-glucose (producing gluconic acid, 5-keto-, 2-keto- and 2,5-diketogluconic acid) for the use in biotechnological applications [Gupta, 2001].

As derived from the above description, PQQ dependent dehydrogenases are found in *Acinetobacter calcoaceticus*, an industrial microorganism used in vinegar production.

A more recent attention given to PQQ dependent sugar dehydrogenases is directed to development of different amperometric biosensors, e.g. for measuring glucose concentration in blood [D'Costa, 1986; Igarashi, 2004; Heller, 2008], for detection of heavy metals [Lapenaite, 2003], for detection of formaldehyde in air [Acmann, 2008], for detection of phenolic compounds in flow injection analysis [Rose, 2001], as a ultrasensitive bienzyme sensor for adrenaline [Szeponik, 1997], for determination of xylose concentration [Smolander, 1992], etc. PQQ dependent sugar dehydrogenases may have found its use also in nanotechnology as biofuel cells [Gao, 2010]. Soluble PQQ dependent glucose dehydrogenases have become the major group of enzymes used in biosensor systems for self monitoring of blood glucose, because these enzymes, unlike glucose oxidase, are independent of oxygen presence [Heller, 2008].

In the current art no proceess using PQQ dependant aldose dehydrogenases for production of unnatural compounds, especially in connection to active pharmaceutical compounds or their intermediates, exists or has been contemplated, but as disclosed and provided by the present invention such enzymes have turned out to be feasible and accomplishable to provide an effective and easy synthesis principle for useful unnatural compounds. Therefore the present invention opens a new field for use of these enzymes in further oxidoreductive reactions, used for purpose of synthesis of unnatural compounds especially belonging to classes of synthetic APIs and their intermediate compounds.

### Selection of Enzymes Particularly Useful for the Present Invention

With the information and experimental guidance provided herein, the skilled person will become aware and derive how to select the enzyme capable of catalyzing oxidation or dehydrogenation in order to convert e.g. the compound of formula (II) to the compound of formula (I) based on its substrate specificity or promiscuity, operational pH, temperature and ionic strength window, a need of additional ions or cofactors, or the like. Substrates and reaction conditions are normally chosen to give the optimal activity of the enzyme. However, the substrates and conditions to provide the least inhibitory effect on the cell that hosts the enzyme, or deteriorate stability of the product, can be leveraged against the substrates and conditions by which the optimal activity is reached. In principle, the substrates allowing an enhanced, preferably the best activity of the enzyme are preferred, or vica versa the enzyme having an enhanced, preferably the best specificity towards a desired compound substrate are preferred. It will be immediately apparent to the skilled person that reaction conditions include in one aspect that the temperature, pH, solvent composition, agitation and length of the reaction allow accumulation of the desired product. In addition to satisfy the enzyme activity, the skilled person will know with the disclosure provided herein to adapt the conditions in terms of applying proper pH, temperature and reaction time to prevent the product, e.g. lactone or ester, to deteriorate. If needed, specific cofactors, co-substrates and/or salts can be added to the enzyme in order to either allow or improve its activity. Cofactors are salts or chemical compounds. Often, said species are already included in the solvent mixture, especially if the enzyme is comprised within living whole cell, inactivated whole cell, homogenized whole cell, or cell free extract. Nevertheless, the cofactors, co-substrates and/or salts can be further added to the enzyme, solvent or reaction mixture. Depending on the enzyme, cupric, ferric, nickel, selenium, zinc, magnesium, calcium, molybdenum, or manganese ions, or nicotinamid adenine dinucleotide (NAD), nicotinamid adenine dinucleotide phosphate (NADP+), lipoamide, ascorbic acid, flavin mononucleotide, flavin adenine dinucleotide (FAD), coenzyme Q, coenzyme F420, pyrroloquinoline quinine, coenzyme B, glutathione, heme, tetrahydrobiopterin, or the like can be added to the enzyme, to the solvent or medium or to the reaction mixture comprising the enzyme. For example, with aldose-1-dehydrogenase, or preferably Ylil or Gcd, calcium ions or magnesium ions and pyrroloquinoline quinine or similar electron acceptor is added to the reaction mixture, enzyme or solvent or medium. Specifically, suitable conditions are exemplified in the examples hereinafter.

In general any sugar 1-dehydrogenase known in the art can be used regardless of their sequence identity to the enzymes listed below, notably dehydrogenases. As noted, it is beneficial to choose an enzyme capable of catalyzing oxidation or dehydrogenation specifically at position C1. In the case of the aldose 1-dehydrogenase the enzyme oxidizes the aldehyde or cyclic hemiacetal to lactone. Special variants of the enzymes, like for example enzymes found in the termoresistant microorganism strains, are also contemplated within the present disclosure. The same applies to a modified or improved versions of the naturally occurring enzymes, whose amino acid sequence or structure has been changed to attain better substrate specificity, higher activity, activity over broader temperature or pH range, resistance to the presence of organic solvent or high ionic strength of the solvent, or the like.

We have surprisingly found that two distinct sugar dehydrogenases originating from taxonomically diverse micoroorganisms and having only 21,8% aminoacid sequence identity, performed equally successful in our experiments. Specifically, comparison was performed between SEQ ID NO. 02, representing amino acid sequence of GDH 01 aldose sugar dehydrogenase Ylil from *E. coli,* and SEQ ID NO. 06, representing amino acid sequence of GDH 02 glucose dehydrogenase GdhB from *A*. *calcoaceticus.* Sequence comparison algorithm was made with default settings in AlignX module, component of Vector NTI Advance 11.0 software (Invitrogen), using clustal W algoritm at default settings.

In addition, even structuraly highly distinct enzymes such as soluble aldose 1-dehydrogenase from *E. coli* with aminoacid sequence SEQ ID NO. 02 and the membrane bound glucose dehydrogenase from E. coli with aminoacid sequence SEQ ID NO. 04, were found to be equally successful in our experiments albeit at slightly different reaction conditions.

Owing to this surprising finding it is now reasonable to expect that proteins capable of converting compound (II) to compound (I) or similar reactions may be significantly diverse in ther aminoacid sequence. The yields of the reaction however may depend on each sugar dehydrogenase enzyme's substrate specificity.

Examples of suitable dehydrogenase enzyme include, but are not limited to enzymes in the sequence list, which are identified by their nucleotide sequences or respective codon optimized nucleotide sequences or amino acid sequences set forth in sequence listings.
GDH 01 is a dehydrogenase encoding gene comprised within nucleotide sequence of SEQ ID NO. 01 or an amino acid sequence of SEQ ID NO. 02.
GDH 02 is a dehydrogenase having a nucleotide sequence of SEQ ID NO. 03 or an amino acid sequence of SEQ ID NO. 04.
GDH 03 is a dehydrogenase having a nucleotide sequence of SEQ ID NO. 05 or an amino acid sequence of SEQ ID NO. 06.
GDH 04 is a dehydrogenase having a nucleotide sequence of SEQ ID NO. 07 or an amino acid sequence of SEQ ID NO. 08.
GDH 05 is a dehydrogenase having a nucleotide sequence of SEQ ID NO. 09 or an amino acid sequence of SEQ ID NO. 10.
GDH 06 is a dehydrogenase having a nucleotide sequence of SEQ ID NO. 23 or an amino acid sequence of SEQ ID NO. 24.
GDH 07 is a dehydrogenase having a nucleotide sequence of SEQ ID NO. 25 or an amino acid sequence of SEQ ID NO. 26.
GDH 08 is a dehydrogenase having a nucleotide sequence of SEQ ID NO. 27 or an amino acid sequence of SEQ ID NO. 28.
GDH 09 is a dehydrogenase having a nucleotide sequence of SEQ ID NO. 29 or an amino acid sequence of SEQ ID NO. 30.
GDH 10 is a dehydrogenase having a nucleotide sequence of SEQ ID NO. 31 or an amino acid sequence of SEQ ID NO. 32.
GDH 11 is a dehydrogenase having a nucleotide sequence of SEQ ID NO. 33 or an amino acid sequence of SEQ ID NO. 34.
GDH 12 is a dehydrogenase having a nucleotide sequence of SEQ ID NO. 35 or an amino acid sequence of SEQ ID NO. 36.
GDH 13 is a dehydrogenase having a nucleotide sequence of SEQ ID NO. 37 or an amino acid sequence of SEQ ID NO. 38.
GDH 14 is a dehydrogenase having a nucleotide sequence of SEQ ID NO. 39 or an amino acid sequence of SEQ ID NO. 40.
GDH 15 is a dehydrogenase having a nucleotide sequence of SEQ ID NO. 41 or an amino acid sequence of SEQ ID NO. 42.
GDH 16 is a dehydrogenase having a nucleotide sequence of SEQ ID NO. 43 or an amino acid sequence of SEQ ID NO. 44.
GDH 17 is a dehydrogenase having a nucleotide sequence of SEQ ID NO. 45 or an amino acid sequence of SEQ ID NO. 46.
GDH 18 is a dehydrogenase having a nucleotide sequence of SEQ ID NO. 47 or an amino acid sequence of SEQ ID NO. 48.
GDH 19 is a dehydrogenase having a nucleotide sequence of SEQ ID NO. 49 or an amino acid sequence of SEQ ID NO. 50.
GDH 20 is a dehydrogenase having a nucleotide sequence of SEQ ID NO. 51 or an amino acid sequence of SEQ ID NO. 52.
GDH 21 is a dehydrogenase having a nucleotide sequence of SEQ ID NO. 53 or an amino acid sequence of SEQ ID NO. 54.
GDH 22 is a dehydrogenase having a nucleotide sequence of SEQ ID NO. 55 or an amino acid sequence of SEQ ID NO. 56.
GDH 23 is a dehydrogenase having a nucleotide sequence of SEQ ID NO. 57 or an amino acid sequence of SEQ ID NO. 58.
GDH 24 is a dehydrogenase having a nucleotide sequence of SEQ ID NO. 59 or an amino acid sequence of SEQ ID NO. 60.
GDH 25 is a dehydrogenase having a nucleotide sequence of SEQ ID NO. 61 or an amino acid sequence of SEQ ID NO. 62.

Therefore a sugar dehydrogenase for use in the present invention may be any compound that has sugar 1- dehydrogenase activity toward compound (II). In one embodiment of the invention the sugar 1- dehydrogenase is a PQQ dependat sugar 1- dehydrogenase. Examples of sutable PQQ dependant sugar 1-dehydrogenase include but are not limited to: GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25, wherein each enzyme is identified by it's corresponding nucleotide sequence or respective codon optimized nucleotide sequence or aminoacid sequence as set forth in sequence listing above.

The present disclosure provides sugar dehydrogenases having an amino acid sequence identitiy of at least 50 % thereof; preferably at least 70 % thereof, to any of dehydrogenases selected from GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25. The amino acid sequence identities are determined by analysis with sequence comparison algorithm or by visual inspection. In one aspect, the sequence comparison IS made with default settings in AlignX module, component of Vector NTI Advance 11.0 software (Invitrogen), using clustal W algoritm at default settings.

A preferable sugar 1-dehydrogenase provided herein may be the sugar dehydrogenase originating from *Escherichia coli* identified as GDH01 in the above sequence listing and having corresponding nucleotide sequence SEQ ID NO. 01 and an amino acid sequence of SEQ ID NO. 02.

Equaly preferable sugar 1-dehydrogenase provided herein may be the sugar dehydrogenase originating from *Escherichia coli* identified as GDH02 in the above sequence listing and having corresponding nucleotide sequence SEQ ID NO. 03 and an amino acid sequence of SEQ ID NO. 04.

Another preferable sugar 1-dehydrogenase provided herein may be selected from the sugar dehydrogenase originating from *Acinetobacter calcoaceticus coli* identified as GDH03 in the above sequence listing and having corresponding nucleotide sequence SEQ ID NO. 05 and an amino acid sequence of SEQ ID NO. 06.

Yet another preferable sugar 1-dehydrogenase provided herein may be selected from the modified sugar dehydrogenase originating from *Acinetobacter calcoaceticus coli* identified as GDH04 in the above sequence listing and having corresponding nucleotide sequence SEQ ID NO. 07 and an amino acid sequence of SEQ ID No. 08.

The most preferable sugar 1-dehydrogenase provided herein may be the sugar dehydrogenase from originating from *Escherichia coli* identified as GDH02 in the above sequence listing and having corresponding nucleotide sequence SEQ ID NO. 03 and an amino acid sequence of SEQ ID NO. 04. The said sugar 1-dehydrogenase is also described in the art and within this disclosure as PQQ dependant sugar dehydrogenase, PQQ dependant glucose dehydrogenase, membrane bound glucose dehydrogenase, PQQ dependant aldose dehydrogenase, aldose dehydrogenase, aldose dehydrogenase quinoprotein or glucose dehydrogenase quinoprotein. This particular enzyme is encoded by gene *gcd* naturally occurring in *E. coli* and encodes a protein termed Gcd, mGDH or PQQGDH.

The present invention illustratively makes use of sugar dehydrogenases having an amino acid sequence identitiy of at least 21.8% thereof; 50 % thereof; preferably at least 70 % thereof, to the aminoacid sequence SEQ ID NO. 02.

Within the present methods it is possible to screen for enzymes/organisms capable of oxidizing lactols of formula (II) or, depending on the desired synthetic API or its precursor compound as product, another non-natural substrate. In one aspect, a person skilled in art will find additional candidate enzymes from literature, which could be applicable for the desired type of enzymatic conversion.

Oxidation/dehydrogenization activity towards compound (II) may be screened among different microorganisms and/or enzymes. The term "analysed material" as used herein refers to any microorganism and/or enzyme that can be used in screening method to screen for and identify microorganism and/or enzyme able to convert compound (II) to compound (I) as the living whole cell catalyst, resting whole cell catalyst, cell free lysate, partially purified or purified enzyme, immobilized enzyme or any other form of catalyst as provided by this invention of any microorganism regardless of it being native or genetically modified microorganism. For practical purposes it will be understood hereinafter that a term "analysed material" includes all preparations of candidate catalyst as described above. Several methods are provided in this disclosure that allow screening for and identification of oxidation/dehydrogenation activity towards compound (II) and thus method for screening and identifying organisms and/or enzymes useful to carry out the present invention.

To successfully perform said screening methods, "analysed material" may be obtained having regard to its cultivation properties. Cultivation may be performed to obtain biomass of "analysed material" in growth medium which satisfies the nutrient needs. Cultivation may be performed in liquid medium or on solid medium. Growth medium and conditions of cultivating may be chosen from but are not limited to Difco & BBL Manual, 2010 and to other protocols well known to person skilled in the art. Cultivated microorganisms may be prepared in different forms of catalyst as provided by this invention. In particular "analysed material" is brought in contact with compound (II) in such conditions that allow forming and accumulation of compound (I). These conditions include in one aspect that the "analysed material" is provided at sufficient load to be able to perform the oxidation/dehydrogenization, in another aspect that the substrate and electron acceptors are present in the reaction in an amount that displays minimal inhibition of the activity of the catalyst, in another aspect that the temperature, pH, solvent composition, agitation and length of reaction allow accumulation of desired product, in another aspect that said conditions do not have detrimental effect on product stability. Specifically such conditions may be defined as indicated by, or as modified or varied from, values or conditions disclosed in examples. "Analysed material" may be able to intrinsically provide all cofactors needed for activity towards compound (I) (naming PQQ), or "analysed material" possess the capability of converting compound (II) to compound (I) when PQQ is provided externally as described in this invention. In all screening methods provided herein to PQQ may preferebly be added in concentrations described and exemplified. Bivalent metal ions such as calcium or magnesium ion, provided in the form of a salt, such as CaCl₂ or MgCl₂ facilitate reconstitution of PWW to the apo-enzyme resulting in the active from of aldose dehydrogenase. These may preferably be added to the enzyme in concentration described and exemplified.

It will become apparent to a person skilled in the art that quantification of PQQ presence can be determined by the above method and as described elsewhere in the present specification. In this case the material used for the method has to be depleted of PQQ or contain no PQQ already by its nature.

One such method for screening of and identifying candidate catalysts is to bring into contact the "analysed material" with a compound (II). Converting of compound (II) to compound (I) should be performed at optimal reaction conditions as described above. Detection of substrates converting to product in presence of "analysed material" can be achieved by any of the well known chromatographic methods known in the art. The non-limiting examples include liquid HPLC, GC, TLC analysis etc. An exemplified but not limiting method for monitoring compound (I) and corresponding compound (II) is gas chromatography analysis (chromatographic column: DB-1 100 % dimethylpolysiloxane; temperature program: initial temperature: 50 °C, initial time: 5 min, temperature rate: 10 °C/min, final temperature: 215 °C, final time: 10 min; injector: split/splitless injector; carrier gas: helium, initial flow: 10 mL/min; detector: flame ionization detector (FID), detector temperature: 230 °C). The prerequisite for carrying out such method is a presence of electron acceptor in the reaction mixture. For "analysed material" where natural electron acceptor is present (such as respiratory chain) no additional components (apart from oxygene in the air) are needed to be able to observe formation of compound (I) from compound (II) by using said chromatographic methods. In case the electron acceptor capable to relieve PQQ of it's electron pair is not available (such in cell free lysate or cell membrane fraction), artificial electron acceptor such as DCPIP is needed. However the described method being analytical procedure and only small quantities of artificial electron acceptor being needed, the preferred way to carry out screening method with any kind of "analysed material" is in presence of artificial electron acceptor. Illustrative and preferred conditions for carrying out the above method are defined by values and conditions disclosed in examples.

Another screening method provided herein is a method performed in presence of alternative artificial electron acceptors with appropriate redox potential compared to a substrate/enzyme/cofactor cascade that can be used. In this sense, the present disclosure provides a screening method using artificial electron acceptor which changes its optically measurable property or properties (such as color, absorbance spectra, etc.) when reduced. Such artificial electron may be provided in the reaction mixture (a dye-linked system) in order to promote electron flow, hence being indicative of the oxidation or dehydrogenation of compound (II). The acceptor/indicator may be selected from but is not limited to: 2,6-dichlorophenol indophenol (DCPIP), phenazine methosulfate (PMS), potassium ferricyanide (PF), potassium ferrioxalate, p-benzoquinone, phenyl-p-benzoquinone, duroquinone, silicomolybdate, vitamin K3, diaminodurene (DAD), *N,N,N',N'*-tetramethyl-*p-*phenylenediamine (TMDP). A person skilled in the art will recognize compounds listed as Hill reagents, dyes that act as artificial electron acceptors, changing colors when reduced, and will find many additional candidate acceptors/indicators from literature. Preferably, 2,6-dichlorophenol indophenol (DCPIP) combined with phenazine methosulfate (PMS) may be used. An exemplified screening method contains following components in a reaction mixture: DCPIP combined with PMS as artificial electron acceptor, "analysed material" and compound (II). Oxidation/dehydrogenation activity of "analysed material" towards compound (II) is followed spectrophotometrically as reduction of absorbance of DCPIP which when oxidized is blue, turning color-less when reduced. When "analysed material" is capable of oxidation/dehydrogenization activity towards compound (II), electrons are transferred to artificial electron acceptor, which becomes reduced and thus reaction mixture turns color from blue to color-less.

One example of carrying out said method is to follow the following procedure: DCPIP and PMS are used in concentrations from about 0.01 mM to about 10mM for both said artificial electron acceptors.,in particular from about 0.05mM to about 5mM DCPIP combined with 0.01mM to about 2mM DCPIP. Preferably the amount of DCPIP in a screening method is provided in concentration from 0.1 mM to about 1 mM combined with PMS in concentration from 0.05mM to about 0.5 mM. Most preferably the DCPIP combined with PMS is provided in the amount which allows observation of reduction of absorbance in timeline that can be spectrophotometrically followed. The compound (II) may be dissolved in appropriate aqueous solution and used in a screening method in concentrations from about 0.5 mM to about 1M preferably from about 10 mM to about 500 mM, most preferably 20 mM to 200 mM. Compound (II) may be dissolved in distilled water or in suitable buffered solution. Suitable buffers for adjusting pH value are made with acids, bases, salts or mixtures thereof in particular phosphoric acid and sodium hydroxide may be used. The aqueous suspension, in which the screening method is performed, may be buffered to pH 5.5 to 9.0, preferably to 6.0 to 8.5, more preferably 6.0. to 8.0. "Analysed material" is added to reaction mixture in the said aqueous suspension (particularly in a concentration range from about 0.05 g/L to about 50 g/L), optionally in buffered solution (in particularly in phosphate buffer pH 6.0 to 8.5). Screening and identifying of catalysts capable of converting compound (II) to compound (I) can be observed spectrophotometrically following absorbance reduction in time line, may be at wavelength between 380 nm and 750 nm, perferably at wavelength between 450 nm and 650 nm, more preferably between 550 nm and 650 nm.

Also provided herein is an aldose dehydrogenase activity unit. The aldose dehydrogenase activity unit is defined as absolute value of reduction in absorbance unit per minute per wet weight of cultured microorganisms used for preparation of any "analysed material" (abs[mAU min⁻¹ mg⁻¹]). For comparative studies cell density of tested microorganisms may be quantified as wet weight in mg per mL of sample, protein concentrations and/or other indirect or direct methods for quantification well known to person skilled in the art.

Yet another screening method for identification of organisms capable of converting of compound (II) to compound (I), is the use of any known oxygen consumption measurement method known in the art. A nonlimiting example provided by this invention is the use measurement of the dissolved oxygen in the culture of the tested organism after addition of compound (II). More particularly the experimental setup may be composed of a stirred aerated vessel containing the liquid culture broth of the tested organism and a dissolved oxygen sensor. Upon addition of compound (II) one can observe increased oxygene consumption shown by a drop in dissolved oxygen values. The faster and the deeper the drop in dissolved oxygen values under standardized conditions, the higher oxidation rate of compound (II) is facilitated by the tested organism.

### PQQ.

Pyrroloquinoline quinine (4,5-dihydro-4,5-dioxo-1H-pyyrolo-[2,3-f]quinoline-2,7,9-tricarboxylic acid: PQQ) is a molecule needed for functioning when using quinoproteins. PQQ, a redox cofactor, which is water soluble and heat-stable, is considered as the third type of coenzyme, after nicotinamide and flavin in biological oxidoreductions and was discovered by Hauge, 1964. To that time unknown redox cofactor was also found by Anthony and Zatman in alcohol dehydrogenase and was named by them as methoxantin [Anthony, 1967]. Later, PQQ has been reported to occur in dehydrogenase, oxidases, oxygenases, hydratases, and decarboxylases. The role of these quinoproteins is to catalyze the primary oxidation step of non-phosporylated substrates, such as alcohols, aldehydes, or aldoses.
PQQ has been found in both prokaryotic (such as *Klebsiella pneumoniae, Acinetobacter calcoaceticus, Methylobacterium extorguens, Kluyvera intermedia, Gluconobacter oxydans, Pseudomonas aeruginosa, Erwinia amylovora, Rahnella aquatilis, Deinococcus radiodurans*) and eukaryotic organisms (such as *Polyporus versicolor, Rhus vernicifera*) [Goodwin, 1998; Hoelscher, 2006; Yang, 2010]

A generally accepted structure of PQQ is: 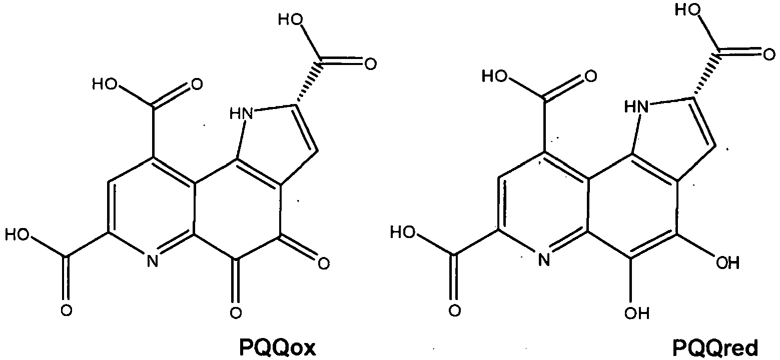 wherein the PQQox is the oxidized form of the cofactor and PQQred is the reduced from of the cofactor.

The number of genes involved in biosynthesis of PQQ varies between species, but in general it is known that for biosynthesis at least five or six genes are needed, usually clustered in the *pqq*ABCDE or *pqqABCDEF* operon. The number and organization of the genes is variable as it can be seen in following examples. In *Klebsiella pneumoniae,* the PQQ biosynthetic genes are clustered in the *pqqABCDEF* operon, while in *Pseudomonas aeruginosa* the *pqqF* is separated from the *pqqABCDE* operon. In *Acinetobacter calcoaceticus,* there is a *pqqABCDE* but no *pqqF* gene is known. A facultative methylotroph *Methylobacterium extorquens* AM1 contains a *pqqABC*/*DE* operon in which the *pqqC* and *pqqD* genes are fused, while the *pqqFG* genes form an operon with three others genes.

Although much is known about the enzymes that use PQQ as a cofactor, relatively little is known about its biosynthesis. However, backbone of PQQ is constructed from glutamate and tyrosine. Most probably these amino acids are encoded in the precursor peptide PqqA. The length of the small peptide varies between different organisms (from 23 amino acids in *K. pneumoniae* to 39 in *P. fluorescens,* respectively) and in all variants in the middle of the PqqA peptide motif Glu-X-X-X-Tyr is conserved. The PqqB protein might be involved in its transportation into the periplasm and thus is not directly required for PQQ biosynthesis. Residues of PqqC protein are highly conserved within PqqC proteins, which are responsible for catalyzing the final step in PQQ formation, from different bacteria. Although the alignment of protein sequences of PqqD proteins from different organisms shows strictly conserved residues, the function of PqqD is not fully resolved. In *Klebisella pneumoniae* it was shown that PqqE recognizes the PqqA, which links the C9 and C9a, afterwards it is accepted by PqqF which cuts out the linked amino acids. In the said organism it was shown that the next reaction (Schiff base) is spontaneous, following dioxygenation. The last cyclization and oxidation steps are catalysed by PqqC [Puehrunger, 2008].

When a comparison of PqqF and PqqG proteins derived from *Klebsiella pseudomonas* within a protein database was performed, it was purported that said proteins share similarity with a family divalent cation-containing endopeptidases that cleave small peptides [Meulenberg, 1992]. While the PqqF and PqqG proteins of *Methylobacterium extorquens* show some similarity to the two subunits of mitochondrial processing peptidases [Springer, 1996], the PqqF of *Klebsiella pneumoniae* is most closely related to the *Escherichia coli* peptidase pitrilysin encoded by *tldD* gene [Meulenberg, 1992]. It has been proposed and experimentally shown that PQQ gene clusters comprising only *pqqABCDE genes* and lacking *pqqF* may be used to provide compete PQQ biosynthetic maschinery in *E. coli.* (Kim C.H. et al., 2003, Yang X.-P. et al. 2010). The pitrilysin protease (encoded by *tldD* gene) is apparently complementig for the activity of pqqF gene found in some microorganisms.

While *E*. *coli* lacks the ability to synthesize PQQ itself [Hommes, 1984; Matsushita, 1997], it shows positive chemotaxis effect towards PQQ, found in environment [de Jonge, 1998], and can use an externally supplied cofactor [Southall, 2006]. Thus PQQ biosynthesis genes could be recombinantly expressed in *E. coli;* what is one of the aspects described in this invention.

In relation to the above, in general, there are at least three ways of providing PQQ to PQQ-dependent dehydrogenases *in situ:*
First, the PQQ can be added to the living or resting cells containing aldose dehydrogenase enzyme or to the cell free lysates or purified aldose dehydrogenase enzyme. The reconstitution of holo-enzyme form to the active apo-enzyme is almost instantaneous, which was shown in one aspect of our invention. Calcium, magnesium or other bivalent metal ions are added to the mixture in order to facilitate the coupling of the enzyme with the PQQ. This may be achieved by addition of salts such as MgCl₂ or CaCl₂ to the enzyme mixture. Further, there are yet some other possibilities of addition of PQQ which does not necessarily need to be purified in form of dietary complements, media components such as yeast extract etc. The fact that quinoproteins have a very high affinity towards PQQ [de Jonge, 1998] allows that equimolar quantities to the quinoproteins are used. In praxis this means concentrations at the nano molar to micro molar level. It will become apparent to a person skilled in the art that optimization of the amount of PQQ needed for optimal activity of the aldose dehydrogenase enzyme is easily performed .in order to reduce the cost of the process. As an non- limiting example, increasing amounts (starting from 0.1nM) of PQQ are added to the aldose dehydrogenase catalyst and the added amount is optimal when the activity of said catalyst no more increases with additional PQQ provided.

In this sense, the present disclosure provides a method of supplying the PQQ to the aldose dehydrogenase, more specifically to the living whole cell catalyst, resting or inactivated whole cell catalyst, cell free lysate or extract or any other form of catylst as provided herein in concentration from about 0.1nM to about 5mM. In particular from about 1nM to about 100uM of PQQ can be provided. More preferably the PQQ is provided in concentration from 100nM to about 5uM. Most preferably the PQQ is provided in the minimal amount which allows maximal activity of the said catalyst The PQQ can be obtained from any source and provided to the catalyst as solid matter or stock solution of PQQ.. In order to facilitate reconstitution of the aldose dehydrogenase by PQQ, calcium or magnesium ions are provided to the enzyme, preferably CaCl₂ or MgCl₂ in concentration from about 0.1mM to about 50mM, more preferably from about 1 mM to about 20mM. MgCl₂ is the preffered option howerer different enzymes may vary in their preference to a specific bivalent ion.
Second, option is, that the host organism for the production of appropriate dehydrogenase has intrinsic PQQ biosynthetic capability, in other words, contains functional genes for PQQ biosynthesis already integrated in its genetic material. Non-limiting examples are: *Klebsiella pneumoniae, Acinetobacter calcoaceticus, Methylobacterium extorquens, Kluyvera intermedia, Gluconobacter oxydans, Pseudomonas aeruginosa, Erwinia amylovora, Rahnella aquatilis, Deinococcus radiodurans* and others.
When using such microorganism as host for expression of homogenous or heterogeneous aldose dehydrogenases, the expressed enzymes are coupled with PQQ to form their active form. Some of the said microorganisms have in addition to PQQ production ability, active PQQ dependant aldose dehydrogenase present, which are capable of converting compound (II) to compound (I). In several aspects of our invention this approach proved to be highly effective and successful as exemplified below.

In this aspect, the present disclosure provides microorganisms with native ability to produce PQQ that can be used as hosts for homolohous or heterologous expression of PQQ dependant aldose dehydrogenases. Said microorganisms are preferably selected among bacteria, more preferably industrially culturable bacteria and particularly from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans.* In the most prefereble imbodiment *Klebsiella pneumoniae, Acinetobacter calcoaceticus, Pseudomonas aeruginosa, Erwinia amylovora, Gluconobacter oxydans* may be used.

In similar yet different embodiment the present disclosure provides microorganisms with natural capability to convert compound (II) to compound (I). No genetic modifications are needed with provided organisms in order to obtain a catalyst capable of performing the desired oxidation. Therefore this disclosure provides microorganisms for the presently disclosed purpose and use, selected among bacterial origin, more particularly from genera: *Klebsiella Enteorobacter, Acinetobacter, Rhizobioum. Methylobacterium, Kluyvera, Gluconobacter, Pseudomonas, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans,* most preferably from: *Gluconobacter oxydans, Acinetobacter calcoaceticus and Kluyvera intermedium.*

Described above are non-limiting examples of microorganisms with desired properties to carry out this invention. Further, methods are disclosed and provided which allow screening for and identification of such microorganisms.

The third option is especially applicable to microorganisms which do not have intrinsic capability of biosynthetis of PQQ, such as *Escherichia coli.* It is well known in the art that some microorganims such as *E. coli* and most of higher organisms have PQQ-dependent enzymes encoded in their genomes and expresses in certain conditions but lack biosynthesis of PQQ [Matshushita, 1997]. It is contemplated in the art that such microorganisms obtain the PQQ as an essential nutrient, or with other words, a vitamin. Ways to establish biosynthesis of PQQ in such organisms to be used for the present invention will be apparent to a person skilled in the art. Approaches of providing biosynthesis of PQQ to such organisms are described (see e.g. Goosen, 1988; Yoshida, 2001; Kim, 2003; Khairnar, 2003; Hoelscher, 2006; Yang, 2010). It can be established by cloning of PQQ biosynthesis gene cluster from microorganisms which do posses PQQ biosynthesis machinery to the plasmid vector or to the bacterial chromosome and then allowing expression of such genes in the host organisms. Non-limiting examples of microorganims suitable for this purpose include *Klebsiella pneumonia, Methylobacterium extorguens, Pseudomonas aeruginosa, Gluconobacter oxydans, Kluyvera intermedia, Erwinia amylovora* and others. A term "heterologous expression of PQQ gene cluster" will be immediately understood by a person skilled in the art, as a well established term describing the above procedures.

For use in the present invention any PQQ gene cluster may be used, providing that said gene cluster encodes functional proteins as described above with capability of biosynthesis of PQQ either alone or in concert with the host organism's enzymes.

In one embodiment, the pQQ gene cluster can be obtained from any living organism producing PQQ. In a more particular embodiment, the PQQ gene cluster can be obtained from any microorganisms selected among bacterial, more particularly from genera: *Klebsiella Enteorobacter, Acinetobacter, Rhizobioum, Methylobacterium,Kluyvera, Gluconobacter, Pseudomonas, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans,* most preferably from: *Gluconobacter oxydans, Acinetobacter calcoaceticus and Kluyvera intermedia.*

Examples of suitable PQQ gene clusters are included, but are not limited to nucleotide sequences of clusters or included genes in the sequence list, which are identified by their nucleotide sequences or amino acid sequences set forth in sequence listings. In general any of the PQQ clusters providing functional genes known in the art may be used for the reaction regardless of their sequence identity to the listed PQQ clusters, genes comprised within and proteins encoded by said genes.

PQQ 01 is a PQQ encoding gene cluster from *Gluconobacter oxydans* 621H comprised within nucleotide sequence of SEQ ID NO. 68 and allows expression of genes *pqqA, pqqB, pqqC, pqqD* and *pqqE* encoding proteins PqqA, PqqB, PqqC, PqqD and PqqE with aminoacid sequence SEQ ID NO. 12, 13, 14, 15, 16, respectively.

PQQ 02 is a PQQ encoding gene cluster from *Kluyvera intermedia* comprised within nucleotide sequence of SEQ ID NO. 69 and allows expression of genes *pqqA, pqqB, pqqC, pqqD* and *pqqE* encoding proteins PqqA, PqqB, PqqC, PqqD and PqqE with aminoacid sequence SEQ ID NO. 18, 19, 20, 21, 22, respectively.

PQQ 03 is a gene cluster *pqqABCDEF* from *Klebsiella pneumoniae* 324 having a nucleotide sequence of SEQ ID. NO 63. and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number CP000964 at NCBI genome database having location between 2602846 and 2599706.

PQQ 04 is a gene clusters *pqqABC*/*DE* and *pqqFG* from *Methylobacterium extorguens* AM1 having nucleotide sequences of SEQ ID. NO 64 and SEQ ID. NO 65, respectively and allows expression of genes *pqqA, pqqb, pqqC, pqqD, pqqE* and *pqqF* encoding proteins PqqA, PqqB, PqqC, PqqD, PqqE and PqqF. The above sequence is available as part of the genome sequence with accession number CP001510 at NCBI genome database having location between 1825235 and 1821763 (*pqqABC*/*DE*), 2401055 and 2403792 (*pqqrEF*).

PQQ 05 is a gene clusters *pqqABCDE* and *pqqF* from *Pseudomonas aeruginosa* PA7 having nucleotide sequences of SEQ ID. NO 66 and SEQ ID. NO 67, respectively and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number CP000744 at NCBI genome database having location between 3420385 and 3423578 (*pqqABCDE*), 3439512 and 3437221 (*pqqF*)*.*

PQQ 06 is a gene cluster *pqqABCDEF* from *Erwinia amylovora* ATCC 49946 having a nucleotide sequence of SEQ ID. NO 70. and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number FN666575 at NCBI genome database having location between 597604 and 600850.

A person skiled in art would also recognize additional candidate gene clusters providing for PQQ synthesis, in publicly available databases (GenBank, Swiss-Prot/TrEMBL, RCSB PDB, BRENDA, KEGG, MetaCyc) using well established data mining tools.

The method for measuring activity of PQQ dependant aldose dehydrogenase provided by the present invention can be used, as exemplified by the invention herein, to screen for and identify organisms capable of producing PQQ regardless of their origin (native or genetically modified), and in addition allows, if desired, a semi quantitative method for estimating the quantity of produced PQQ. A PQQ dependant aldose dehydrogenase in any form, preferably expressed in E. coli (or any other microorganism unable to produce PQQ), can be used for reconstitution of active holo-enzyme. A calibration curve obtained by measuring activity of said PQQ dependant aldose dehydrogenase, supplemented with various quantities of PQQ, is compared to the activity of said PQQ dependant aldose dehydrogenase which was supplemented with analysed sample. Whithin the linear range of the method, the more PQQ is present in the analysed sample, the more activity is observed.

In a particular embodiment, the PQQ gene clusters (or part thereof) are derived from *Kluyvera intermedia* or *Gluconobacter oxydans.* Particularly gene cluster from *Gluconobacter oxydans* 621H comprised within nucleotide sequence of SEQ ID NO. 68 may be used. Alternatively, a particular embodiment provides use of gene cluster from *Kluyvera intermedia* comprised within nucleotide sequence of SEQ ID NO. 69 The described gene cluster can be modified by methods known in the art, for example methods described in Sambrook and Russell, Molecular Cloning: A Laboratory Manual, 3'^" Ed., Cold Spring Harbor, NY 2001, in order to allow expression of genes encoded in said cluster in *E. coli.* Any of numerous strains of *E. coli* can be used: for example *E. coli* K12 strains such as JM109, DH5, DH10, HB101, MG4100 etc. or *E. coli* B strains such as BL21, Rossetta, Origami etc. Genes can be introduced into the said host strain by any genetic method known in the art, for example by transfection, transformation, electroporation, conjugal transfer and others. Said gene clusters may be maintained in the said host microorganism in any form known in the art, for example encoded in a autonomously replicating plasmid or integrated into host's genome. Expression of the genes encoded in said gene clusters can be obtained either by utilizing the activity of native promoters controlling the expression of said genes or by replacing the promoters by promoters which may be more suitable for expression in said host microorganism. Methods for making such modifications are well known in the art.

In one aspect the present disclosure provides gene cluster from *Gluconobacter oxydans* 621H comprised within nucleotide sequence of SEQ ID NO. 68, which is carried on a autonomously replicating plasmid comprised within the host *E. coli strain.* In this particular aspect the genes encoded on the cluster are expressed under control of their corresponding native promoters.

In one aspect the present disclosure provides gene cluster from gene cluster from *Kluyvera intermedia* comprised within nucleotide sequence of SEQ ID NO. 69, which is carried on a autonomously replicating plasmid comprised within the host *E. coli strain.* In this particular aspect the genes encoded on the cluster are expressed under control of their corresponding native promoters. In the same particular aspect the genes used for provision of PQQ synthesis in E. coli are pqqABCDE and function of pqqF is provided by intrinsic activity of *E*. *coli.*

According to our findings availability of PQQ during the heterologous expression of PQQ dependent dehydrogenases has little effect on their correct folding, transport, cleavage of leader sequence and other posttranslational modifications. This means that there is little difference in dehydrogenase activity regardless of when, i.e. at which time or during which period the PQQ is provided to the dehydrogenase, e.g. during or after the cultivation and induction of expression. Other parameters are more relevant when establishing enhanced or even maximal PQQ dependent aldose dehydrogenase activity in periplasmic space or in the cellular membrane, where optimal coupling to cell's native electrone acceptors (the respiratory chain) is allowed. One of these parameters are presence of appropriate leader sequence, directing the protein to the periplasm or to the membrane. Another such parameter is expression strength which can be controlled by temperature of cultivation, transcriptional promoter selected, codon usage in the PQQ dependent aldose dehydrogenase encoding gene, quantity of expression inducer etc. Yet another such parameter are intrinsic properties of selected PQQ dependent aldose dehydrogenase such as ability to fold correctly in heterologous host, toxicity to heterologous host, resistance to the host's degrading enzymes etc. All such parameters, which are useful for enhanced activity and optimization and methods to do so, will become apparent to persons skilled in the art.

Further Exemplified and Modified or Alternative Embodiments of the Present disclosure

Various further embodiments, modifications and alternatives to carry out the present disclosure will be become apparent from the above description.

Further exemplified, the present disclosure for example provides a particular process comprising the step of reacting a substrate (II) under dehydrogenase catalyzed oxidation conditions to form the corresponding lactone (I), wherein the dehydrogenase is selected in first embodiment from GDH 01 or GDH 02 or GDH 03 or GDH 04 or GDH 05, or any dehydrogenase having an amino acid sequence identity of at least 70 % to those, more preferably 90 % to those. In another embodiment the dehydrogenase is selected from GDH 06 or GDH 07 or GDH 08 or GDH 09 or GDH 10, or any dehydrogenase having an amino acid sequence identity of at least 70% to those, more preferably 90 % to those.
In another specific aspect, this disclosure relates to a method of constructing and providing appropriate synthetic biological pathways, such as exemplified with *E. coli* as a host microorganism, wherein DERA (deoxyribose 5-phosphate aldolase), PQQ dependant dehydrogenase and, optionally, PQQ biosynthetic pathway genes are expressed simultaniously. The respiratory chain of the host organism are established and provided also.

Gcd aldose dehydrogenase meets all preferred features and is thus most prefered enzyme used. The Gcd encompasses any aldose dehydrogenase having an amino acid sequence identity to at least 50 % of the Gcd described herein, preferably at least 70 %. The amino acid sequence identities are determined by the analysis with a sequence comparison algorithm or by a visual inspection. In one aspect the sequence comparison algorithm is made with AlignX algorithm of Vector NTI 9.0 (InforMax) with settings set to default.

A further special aspect, the present disclosure relates to a process of oxidation or dehydrogenation of compound (II) using an enzyme as described above, comprising the provision of microorganism or microorganism-derived material used as a living whole cell catalyst, a resting whole cell catalyst, a cell free lysate, a partially purified or purified enzyme, an immobilized enzyme or any other form of catalyst, wherein the enzyme capable of catalyzing oxidation or dehydrogenation reaction as described above is expressed in said microorganism naturally, i.e. it being the microorganism's natural property. In said aspect such organism when cultivated and used as catalyst in said reaction can convert compound (II) to corresponding lactone without the need for additional genetic modification of said microorganisms. Said microorganism can be selected from vide diversity of bacteria as exemplified below. An organism with described properties can be selected from bacteria, more particularly proteobacteria, actinomycetales, mixobacteriaceae. More particularly said microorganism may be selected from Gamma proteobacteriaceae. Most preferably organism in this sense is selected from the group of Enterobacteriaceae, Rihzobium, Gluconobacter and Acinetobacter.

Given the disclosure provided herein it will be apparent to a skilled person how to search for an organism having a capability of oxidation or dehydrogenation of compound (II). One such method would be to provide to a culture of studied microorganism an amount of compound (II) and look for activity.

In a further particular embodiment, the definition of the compound of formula (II) and thus also of the compound of formula (I) can be limited in that R5 denotes the moiety selected from the formulae (III), (IV), (V), (VI), (VII), (VIII) and (IX).

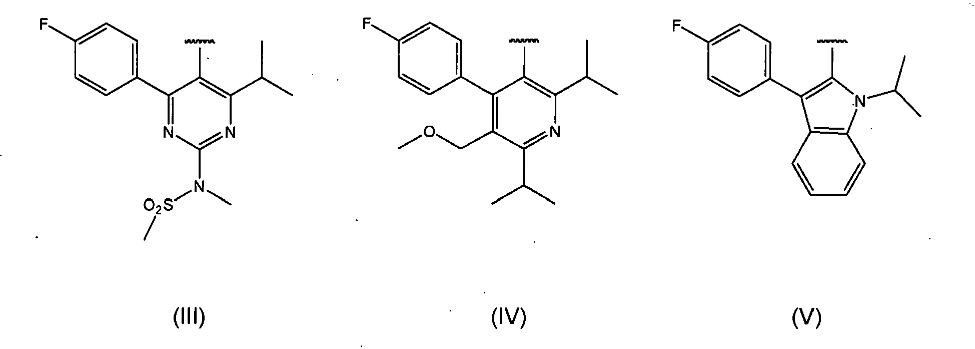

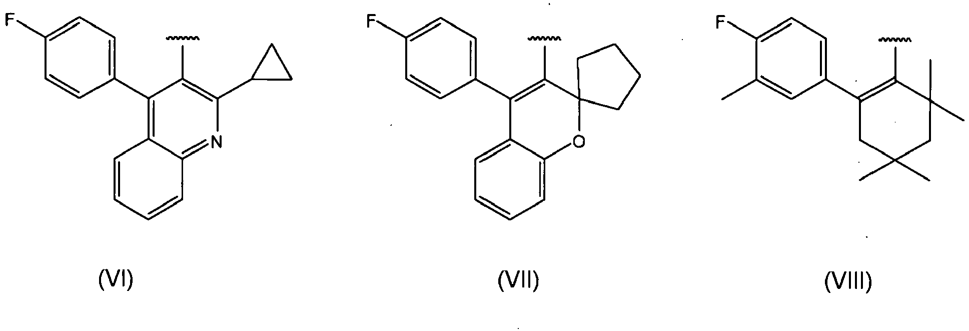

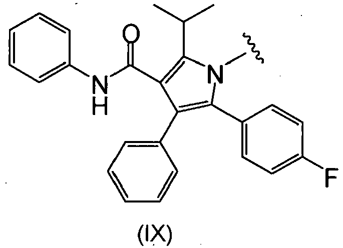

In yet another particular embodiment, the definition of formula (II) and thus also of formula (I) can be specified to the definition, wherein
R₁ independently from R₂ denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, or NR³CO(CH₂)ₙCH₃; and
R₂ independently from R₁ denotes H or (CH₂)ₘ-CH₃;
or both of R₁ and R₂ denote either X, OH or O(CH₂)ₙCH₃;
or R₁ and R₂ together denote =O, wherein R³ and R⁴ independently from each other, or together, denote H, (CH₂)ₘ-CH₃,
X denotes F, Cl, Br or I;
n represents an integer from 0 to 10;
m represents an integer from 0 to 3;

The compound of formula (I) obtained by the process of the present invention can be used as an intermediate for preparing a statin. The skilled person will know how to put the process step of obtaining said compound according to the present invention in the context of a statin synthesis. In principle, there are two basic ways to arrive at the statin. According to a first route, a lactone is prepared from the lactol and then coupled to the statin backbone. Alternatively, first the statin backbone containing the aldehyde side moiety is prepared, which is subsequently converted to lactol, for example by using 2-deoxyribose-5-phosphate aldolase (DERA) enzyme, and then oxidized to lactone. For the specific case of atorvastatin, as an example, one can refer to schemes 2 to 4 of the WO 2006134482.

In a specific embodiment an enzyme 2-deoxyribose-5-phosphate aldolase (DERA, EC 4.1.2.4) is used for preparing the compound of formula (II), which is subsequently converted to lactone by the enzyme capable of catalyzing oxidation or dehydrogenation. Multiple wild type, variants or mutant version of DERA enzyme are know in the art, including, but not limited to, J. Am. Chem. Soc. 116 (1994), p. 8422-8423, WO 2005/118794 or WO 2006/134482. In preferred embodiment, 2-deoxyribose-5-phosphate aldolase enzyme is used for a synthetic step just preceding the step of bringing in contact the compound of formula (II) with the enzyme capable of catalyzing oxidation or dehydrogenation.

In another preferred embodiment, DERA is used to prepare the compound of formula (II) at least in part simultaneously to conversion of said compound to the compound of the formula (I) by the enzyme capable of catalyzing oxidation or dehydrogenation. It will be immediately understood that the enzymes necessary to catalyse the reaction of preparing the compound of formula (II) and the reaction of oxidizing said compound to formula (I) can be used within the reaction mixture, or can be added to the reaction mixture, simultaneously or subsequently, at once, intermittently or continuously. The embodiment having the compound of formula (II), and thus the starting material for the reaction with the enzyme capable of catalyzing oxidation or dehydrogenation, prepared by DERA is advantageous, because this arrangement is well compatible and it allows using aqueous solvents in the preceding step and thus makes it unnecessary to purify the compound of formula (II) prior to offering it to the enzyme capable of catalyzing oxidation or dehydrogenation for conversion to lactone. The preferred embodiment is thus to bring the compound of formula (II) in contact with the enzyme capable of catalyzing oxidation or dehydrogenation without prior isolation or purification of said compound. In this event, the complete reaction mixture of the preceding step can be used for the subsequent reaction, which reduces the number of process steps and simplifies the process. With obviating a purification step yield is also increased. In addition, because of better enzyme specificity over chemical oxidants, to most extent only the compound of formula (II) gets oxidised to lactone, while the rest of the present compounds do not change, hence reducing a tendency of impurity generation and thus improving subsequent working-up or purification procedures. All of the substrate in any enzyme reaction according to the present invention can be added to the reaction at once or can be added continuously over longer period, or in one batch or intermittent batches.

Significant improvement can be achieved when the compound of formula (I) is prepared at least in part simultaneously to preparing the compound of formula (II). At the same time it solves multiple drawbacks of using the reaction with the DERA enzyme individually. For example, aldehydes used as a starting material in preparing the compound of formula (II) by using DERA enzyme tend to inactivate the DERA enzyme during the course of reaction and thus reduce enzyme's activity. In addition, the lactol of formula (II) that builds up in the reaction mixture is toxic to the living microorganism. Therefore, it is highly desired to shorten the reaction step with DERA and to consume the starting aldehyde and/or the lactol as soon as possible, which is achieved when both enzymatic reaction steps are performed at least in part simultaneously. Namely, when both reaction steps are performed at least in part simulateneously, preferably completely simultaneously, the toxic lactol immediately enters into the consequent reaction and is transformed to the non-toxic lactone. Moreover, since the second reaction step typically is not a rate limiting step, as confirmed in examples hereinafter, and proceeds faster than the first step with DERA, the steady state equilibrium of the first reaction shifts in a direction of the product. This leads to reduced time for completion of the first step and thus protects DERA from being inactivated. It also protects living cells from being disrupted by high concentrations of lactol.

Important aspect of present invention deals with the intrinsic capability of a microorganisms to transfer electrons produced by oxidation/dehydrogenation of (II), to oxygen (a terminal electron acceptor) via its respiratory chain. This drives the reaction of enzyme catalysed oxidation / dehydrogenation of compound (II) in a whole cell system. It will be immediately apparent that the capability of acting as an electron sink is a significant and beneficial property of whole cell systems as described hereby in the invention.

Use of whole cells therefore avoids use of additional electron acceptors such as DCIP and others described above. An additional benefit of using whole cell processes is ability to provide all aspects of described synthetic biological pathway, i.e. the DERA enzyme, PQQ and a PQQ dependant sugar dehydrogenase, in one organism. As productivity and yields of such process are industrially suitable as exemplified below, use of whole cells is preferred as costs can be controlled at significantly lower level compared to other approaches, e.g. free enzyme process, immobilized enzyme, cell free lysate etc. Also the possibility to perform both DERA and oxidation/ dehydrogenation step fully or partially simultaneously using one pot design leads to significant cost reductions when used in industrial scale.

In this sense of making use of whole cell system as electron sink, it is also advantageous to perform the process in the presence of oxygen, particularly where the oxygen is provided in quantity allowing at least 5%, preferably at least 15%, of dissolved oxygen at given process condition, wherein 100% dissolved oxygen is understood as saturated solution of oxygen at given process conditions and 0% is understood as oxygen free liquid and correlation between oxygen concentration and dissolved oxygen percent is linear between the 0% and 100% at said given process conditions. In this aspect process conditions are understood as liquid composition, temperature, pH, pressure, wherein the measurements in dynamic process are understood to be performed in a homogenous solid/liquid/gas multiphase system.

The presence of the oxygen in that amount makes the oxidation or dehydrogenation reaction irreversible, which secures the obtained lactone and further enhances shifting of the steady state equilibrium of the first reaction towards the product. This preferred embodiment thus increases yield and reduces time needed for the process.

The aldose 1-dehydrogenase, and/or the DERA enzyme, i.e. respectively alone or in combination and optionally independently, can be comprised within single or multiple living whole cell(s), inactivated whole cell(s), homogenized whole cell(s), or cell free extract(s); or are respectively purified, immobilized and/or are in the form of an extracellularly expressed protein. Preferably one of the two enzymes, yet more preferably both enzymes are comprised within same living whole cell, same inactivated whole cell or same homogenized whole cell, more preferably are within same living whole cell or same inactivated whole cell, particularly are comprised within same living whole cell, because having the enzyme in a common whole cell or at least in the common inactivated whole cell, does not demand much handling with the enzyme prior it being used in the process, which reduces costs. Moreover, having the enzyme comprised in a living whole cell enables simple removal of the enzyme by filtration, which alleviates final purification steps at the industrial scale. In addition, it allows a reuse of the enzyme comprised within the living cell in subsequent batches.
Another advantage of using the enzyme in a whole cell or at least in the inactivated whole cell is possibility of providing PQQ cofactor intrinsically as described in detail above.

As an advantageous option, a whole cell system capable of translating 2-deoxyribose-5-phosphate aldolase (DERA) enzyme and an enzyme capable of catalyzing oxidation or dehydrogenation can be arranged to overexpress both of the genes needed for said translation. Means for overexpression are known to the person skilled in the art, and are sometimes referred to elsewhere herein.

The present disclosure also provides; an expression system comprising one or more cell types, the respective cell type(s) being genetically engineered to express, in the totality of cell type(s), both the 2-deoxyribose-5-phosphate aldolase (DERA) enzyme and an enzyme capable of catalyzing oxidation or dehydrogenation.

An expression system can be made up of appropriate organisms or cells and optionally further factors and additives, wherein reference is made to the disclosure provided herein.

In one aspect, there is provided a method of constructing or providing synthetic biological pathway for use in the present disclosure, exemplified with *E. coli* as a host microorganism, wherein DERA (deoxyribose 5-phosphate aldolase), PQQ dependant dehydrogenase and, optionally, PQQ biosynthetic pathway genes are expressed simultaniously. Providing the respiratory chain of the host organism, said synthetic biological pathway has a capability of carrying out production of compound (I) from simple molecules such as compound (X), shown below, and acetaldehyde. This approach is advantageous since this approach joins previously separate steps of production of compound (II), purification of compound (II), and oxidation of compound (II) to compound (I). Additionaly, the cultivation of organisms carrying having said synthetic biological pathway is performed in one industrial fermentation process which immediately provides material capable of converting molecules such as compound (IX) and acetaldehyde into compound of formula (I).

Another embodiment of the present disclosure is obtaining the compound of formula (I), or salts, esters or stereoisomers thereof, in a one-pot process by reacting the starting materials for the DERA enzyme reaction in the presence of 2-deoxyribose-5-phosphate aldolase (DERA) enzyme and an enzyme capable of catalyzing oxidation or dehydrogenation, and optionally salifying, esterifying or stereoselectively resolving the product. This embodiment contemplates to start from the compound of formula (X) 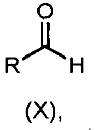 in which R denotes R₁-CH-R₂ moiety of formula (I), and R₁ and R₂ being as defined hereinabove; and subjecting said compound (X) to reaction with acetaldehyde in the presence of the two enzymes, namely aldolase (DERA) enzyme and the enzyme capable of catalyzing oxidation or dehydrogenation. This setup allows obtaining the compound of formula (I) in a single process step starting from relatively simple starting materials, e.g. acetaldehyde. The reaction is industrially suitable, as it proceeds to completion within few hours. It renders intermediate purification steps superfluous. In addition, it provides a possibility of having both enzymes added together - the product of the first enzymatic reaction forming a substrate of the second enzymatic reaction - preferably comprised within the same living whole cell, inactivated whole cell, homogenized whole cell, or cell free extract; or are purified, immobilized and/or are in the form of an extracellularly expressed protein, preferably are within the same living whole cell, inactivated whole cell or homogenized whole cell, more preferably are within the same living whole cell or inactivated whole cell, particularly are comprised within the same living whole cell.
This makes use of all the advantageous effects of combined enzymatic reactions, including, but not limited to the ones described herein. In one aspect, the total amount of substrates added to the mixture is such that the total amount of the substrate (X) added would be from about 20 mmol per liter of the reaction mixture to about 2 mol per liter of the reaction mixture, in particular from about 100 mmol per liter of the reaction mixture to about 1.5 mmol per liter of the reaction mixture, more particular from about 200 mmol per liter of the reaction mixture to about 700 mmol per liter of the reaction mixture. Acetaldehyde may be added by several means. In one aspect, acetaldehyde is added to the reaction mixture in one batch or more batches or alternatively continuously. Acetaldehyde may be premixed with the substrate of formula (X) and added to the reaction mixture. The total amount of acetaldehyde added to the reaction mixture is from about 0.1 to about 4 molar equivalents to the total amount of the acceptor substrate, in particular from about 2 to about 2.5 molar equivalents. In one aspect of the invention, the pH-value used for the reaction is from about 4 to about11. In one embodiment, the pH used for reaction is from about 5 to about 10. In another embodiment, the pH-value used for reaction is from about 5 to about 8. Specifically the pH-value will be maintained by a suitable buffer in a range from 5.2 to 7.5. Alternatively the pH-value as stated above may be controlled by, but not limited to, controlled addition of acid or base according to need as will be obvious to the person skilled in the art.

In one aspect the pH used for the reaction described by the present invention may be optimized so that the compromise between optimal enzyme activity and optimal substrate and/or product stability is taken. It is understood herein that optimal enzymatic activity for different enzymes described in this disclosure may not be identical to optimal conditions for substate/product stability. A person skilled in art may find it benneficial to sacrifice some enzyme activity by adjusting conditions to suite substrate and/or product stability (or vice versa) to obtain optimal product yields.

Specifically, aldolase enzyme, optionally at least in part together with the enzyme capable of catalyzing oxidation or dehydrogenation are prepared in an aqueous solution (particularly each in a concentration 0.1 g/L to 3 g/L), optionally in the presence of a salt (in particular NaCl in a concentration from 50 to 500 mM) optionally with addition of PQQ (particularly in concentration 250nM to 5uM) and CaCl₂, MgCl₂ or alternative Calcium or Magnesium salt) particularly in concentration from 0.1 to 20mM. The aqueous solution may contain organic solvents miscible with water (in particular dimethyl sulfoxide in a concentration from 2 to 15 % V/V), and may be buffered to pH 4 to 11. Some commonly used buffers can lower the yield of the aforementioned reaction that starts from the acetaldehyde by limiting the availability of aldolase-condensation intermediates, particularly first condensation reaction products as they may undergo a chemical reaction with a buffer. For example, bis-tris propane reacts with said intermediates ((S)-3-hydroxy-4,4-dimethoxybutanal) giving (*S*,*Z*)-2-(3-((1,3-dihydroxy-2-hydroxymethyl)propan-2-yl)(3-hydroxy-4,4-dimethoxybut-1-enyl)amino)propyl-amino)-2-(hydroxymethyl)propane-1,3-diol. Other buffers that may react similarly are bis-tris, tricin, tris, bicin or any other buffer having a primary, secondary or tertiary amino group. Thus suitable buffers for adjusting pH, if this adjustment is needed, are made with acids, bases, salts or mixtures thereof, in particular phosphoric acid and sodium hydroxide. In a particularly preferred embodiment, the buffer is a phosphate buffer. In particular, phosphate buffer, in a concentration 10 to 500 mM can be used. The aqueous solution can also be prepared by adding the aldolase enzyme, optionally at least in part together with the enzyme capable of catalyzing oxidation or dehydrogenation to water and maintaining the pH-value during the reaction by means of an automated addition of inorganic acids, bases, salts or mixtures thereof.

In one aspect, the temperature used for the reaction starting from acetaldehyde is from about 10 to about 70 °C. In one embodiment, the temperature used for the reaction is from about 20 to about 50 °C. In another embodiment, the temperature used for the reaction is from about 25 to about 40 °C.

In one aspect the temperature used for the reaction described herein may be optimized so that the compromise between optimal enzyme activity and optimal substrate and/or product stability is taken. It is understood herein that optimal enzymatic activity for different enzymes described herein may not be identical to optimal conditions for substate/product stability. A person skilled in art may find it beneficial to sacrifice some enzyme activity by adjusting conditions to suite substrate and/or product stability (or vice versa) to obtain optimal product yields.

After the completion of the reaction, either enzyme can be removed from the reaction mixture, for example by the addition of at least about 1 vol. of acetonitrile to 1 vol. of reaction mixture. Alternatively the enzyme can be removed by any salting out method known in the art. In one embodiment the salting out is performed with the addition of ammonium sulfate of at least 5 % m/V. In the embodiment, where the enzyme is comprised within living or inactivated cells, the enzyme may be removed by filtrating or centrifuging the reaction mixture.

In another embodiment the product is removed by liquid/liquid extraction to any of a number of water immiscible or poorly miscible solvents. The solvent may be selected from but is not limited to: methylene chloride, ethyl acetate, diethyl ether, propionyl acetate, methyl *t*-butyl ether (MTBE), nitromethane, pentane, hexane, heptane, 1,2-dichloroethane, chloroform, carbon tetrachloride, *n*-butanol, *n*-pentanol, benzene, toluene, *o*-, *m-, p*-xylene, cyclohexane, petroleum ether, triethylamine. Prior the liquid/liquid extraction with chosen organic solvent the pH of water solution of the product may be adjusted to values between 1 and 12, preferably between 2 and 8, more preferably between 3 and 5. Drying of water residues in organic phase after extraction completion may be performed with but is not limited to adding salts listed: sodium sulfate, magnesium sulfate (monohydrate), calcium sulfate, calcium chloride, copper sulfate.

In general, the aldolase enzyme and/or enzyme capable of catalyzing oxidation or dehydrogenation used can be prepared by any means known in the art, for example by methods of protein expression described in Sambrook et al. (1989) Molecular cloning: A laboratory Manual 2nd Edition, New York: Cold Spring Harbor Laboratory Press, Cold Spring Harbor. Gene coding aldolase enzyme and/or enzyme capable of catalyzing oxidation or dehydrogenation can be cloned into an expression vector and the enzyme be expressed in a suitable expression host. Modified versions of known aldolase enzyme or enzyme capable of catalyzing oxidation or dehydrogenation may be necessary or may result depending on cloning conditions.

### Cells and Organisms

One aspect of the present disclosure provides a process of oxidation or dehydrogenation of compound (II) or other compounds recited herein using a microorganism in any form described herein having enzyme capable of catalyzing oxidation or dehydrogenation reaction natively expressed. In said aspect such organism when cultivated and used as catalyst can convert compound (II) to corresponding lactone without the need for additional genetic modification of said microorganisms. Methods of identifying such organisms is exemplified in hereby invention. Non-limiting examples of such organism can be selected from vide diversity of bacteria, more particularly *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.*

In referred embodiments, and in order to practice embodiments of the present disclosure in its best configuration, a specially adapted expression system capable of translating 2-deoxyribose-5-phosphate aldolase (DERA) enzyme and an enzyme capable of catalyzing oxidation or dehydrogenation, and overexpressing both of the genes needed for said translation, is provided. The term "overexpressing" as used herein refers to the expression under control of a strong promoter, or wherein the gene is expressed at high levels (compared to w.t. expression control) and is accumulated intracellularly or extracellularly. The process of obtaining such a modified expression is known to a person skilled in the art. For example, cloning methods described in Sambrook et al. (1989) Molecular cloning: A laboratory Manual 2nd Edition, New York: Cold Spring Harbor Laboratory Press, Cold Spring Harbor, can be used. The genes for the enzymes can be for example cloned on the same or different vector and transformed into a cell. In an alternative, the expression system comprises separate cells, wherein first cell overexpresses the gene for aldolase enzyme and second cell overexpresses the gene for the enzyme capable of catalyzing oxidation or dehydrogenation. The present specification illustratively, without limitation facing common general knowledge, provides an example of making such expression system. The expression system is particularly suited for preparing statin or intermediate thereof.

The skilled person is aware of all the possible cell systems for either preparing or hosting of either DERA enzyme or the enzyme capable of catalyzing oxidation or dehydrogenation, either alone or in combination, and optionally independent from each other. In general, the cell system would be prokaryotic or eukaryotic. In a specific embodiment, the enzyme can be prepared synthetically. The cell for preparing or hosting either of the enzymes can be a bacteria, yeast, insect cell or a mammalian cell. Preferably the cell is bacteria or yeast and more preferably is bacteria, because bacteria or yeast cell are easier cultivated and grown. The bacteria can be selected from the group of genera consisting of Escherichia, Corynebacterium, Pseudomonas, Streptomyces, Rhodococcus, Bacillus and Lactobacillus, preferably from Escherichia and Lactobacillus, more preferably Escherichia, particularly is Escherichia Coli. In case of yeast, the cell can be selected from the group of genera consisting of Saccharomyces, Pichia, Shizosaccharomyces and Candida, preferably Saccharomyces. The examples of mammalian cells are Chinese hamster ovary cell or a hepatic cell, preferably is Chinese hamster ovary cell.

Another embodiment of the disclosure is a process for the preparation of compound (I), in particular an industrial fermentative process, wherein the process comprises the step of cultivation of a microorganism capable of oxidation of compound (II), wherein the said microorganism is brought in contact with compound (II).

Yet another embodiment of this disclosure is a process for the preparation of compound(I), in particular an industrial fermentative process, wherein the process comprises the step of cultivation of a microorganism, capable of oxidation of compound (II), wherein said microorganism is brought in contact with another microorganism having ability of enzymatic production of (II), particularly by catalysis of DERA and wherein substrates which allow production of compound (II) are provided to the reaction mixture.

A preferred embodiment of his disclosure is a process for the preparation of compound (I), in particular an industrial fermentative process, wherein the process comprises the step of cultivation of a microorganism, capable both of production as well as oxidation/dehydrogenation of compound (II) and wherein substrates which allow production of compound (II) are provided to the reaction mixture.

In particular embodiments, the process according to the present disclosure comprises the following steps:
Step a1) If not already known or provided, as disclosed elsewhere hererin, this step includes identification of a microorganism capable of oxidation/dehydrogenation of compound (II) and/or generation of genetically modified strain of a microorganism to obtain capability of oxidation/dehydrogenation of compound (II) as described in this invention. Particularly organisms having sugar 1-dehydrogenase activity are prefered.
Step a2 If not already known or provided, as disclosed elsewhere hererin, this step includes identification of a microorganism capable of production of compound (II) and/or generation of genetically modified strain of a microorganism to obtain capability of production of compound (II) as described in this invention or is known in the present art. Particularly organisms having aldolase catalytic ability are prefered.

It is particularly prefered that a microorganism is identified and/or genetically modified in order to obtain both properties described in step a1) and step a2). In this sense the disclosure specifically relates to a genetically modified strain of a microorganism wherein the genetic material of the strain comprises at least one over expressed gene coding for and enzyme capable of catalysing aldol condensation to form compound (II), more specifically a gene encoding DERA enzyme.
Procedures to identify and/or generate genetically modified microorganisms as described in step a1) and step a2) are exemplified in detail in this disclosure, however a skilled person will immediately find alternative procedures which may lead to the same desired properties of said microorganisms. For example, cloning methods described in Sambrook et al. (1989) Molecular cloning: A laboratory Manual 2nd Edition, New York: Cold Spring Harbor Laboratory Press, Cold Spring Harbor, can be used. The genes for the enzymes can be for example cloned on the same or different vector and transformed into a cell. In an alternative, the expression system comprises separate cells, wherein first cell overexpresses the gene for aldolase enzyme and second cell overexpresses the gene for the enzyme capable of catalyzing oxidation or dehydrogenation. The present specification illustratively, without limitation and taking into account common general knowledge, provides an example of making such modified microorganism. The microorganism is particularly suited for preparing statin or intermediate thereof.

The skilled person is aware of all the possible cell systems for either preparing or hosting of either DERA enzyme or the enzyme capable of catalyzing oxidation or dehydrogenation or, optionally, the PQQ biosynthetic genes, either alone or in combination, and optionally independent from each other. In general, the cell system would be prokaryotic or eukaryotic. In a specific embodiment, the enzyme can be prepared synthetically. The cell for preparing or hosting either of the enzymes can be a bacteria, yeast, insect cell or a mammalian cell. Preferably the cell is bacteria or yeast and more preferably is bacteria, because bacteria or yeast cell are easier cultivated and grown.

The bacteria can be selected from the group of genera consisting of *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans, Corynebacterium glutamicum, Escherichia coli, Bacillus licheniformis, Lactobacillus lactis,* most preferably from: *Escherichia coli, Gluconobacter oxydans, Acinetobacter calcoaceticus and Kluyvera intermedium.* In case of yeast, the cell can be selected from the group of genera consisting of *Saccharomyces, Pichia, Shizosaccharomyces* and *Candida,* preferably *Saccharomyces and Pichia.*

It is particularly preferred that a microorganism is identified and/or genetically modified in order to obtain both properties described in step a1) and step a2). In this sense the disclosure specifically relates to a genetically modified strain of a microorganism wherein the genetic material of the strain comprises at least one over expressed gene coding for and enzyme capable of catalysing aldol condensation to form compound (II), more specifically a gene encoding DERA aldolase enzyme.

Accordingly, the present disclosure provides an exemplified method of constructing synthetic biological pathway, examplified with *E. coli* as a host microorganism, wherein DERA (deoxyribose 5-phosphate aldolase), PQQ dependant dehydrogenase and optionally PQQ biosynthetic pathway genes are expressed simultaniously. Providing the respiratory chain of the host organism said synthetic biological pathway has a capability of carrying out production of compound (I) from simple molecules such as compound (X) and acetaldehyde.

One aspect described in this disclosure is to cultivate microorganisms described in step a1 and a2 simultaneously or independently.

### Step b) Preparation of seed medium

Cultivation of the microorganisms as described in the present disclosure can be carried out by methods known to a person skilled in art. Cultivation processes of various microorganisms are for example described in the handbook "Difco & BBL Manual, Manual of Microbiological Culture Media" (Zimbro M.J. et al., 2009, 2nd Edition. ISBN 0-9727207-1-5). Preferably the production of seed microorganism which can be used in the main fermentation process for the production of (I) starts from a colony of said microorganism. In this respect the process according to the present application comprises the preparation of frozen stock of described microorganism. This preparation of frozen stock may be carried out using method known in the state of art, such as using a liquid propagation medium. Preferably this frozen stock of microorganism is used to produce a vegetative seed medium by inoculation to a vegetative medium.

The seed medium may be transferred aseptically to a bioreactor. In principle the cultivating of seed microorganism can be carried out under the conditions (e.g. pH and temperature) as in the main fermentation process (described under step c).

### Step c) Main fermentation process

Preferably the main fermentation process using a microorganism as described in the present application is carried out in a bioreactor in particular under agitation and/or aeration.

Preferably, cultivation of a microorganism used for the process for the production of compound (I) as described in the present application is carried out under submerged aerobic conditions in aqueous nutrient medium (production medium), containing sources of assimilable carbon, nitrogen, phosphate and minerals. Additional compounds may be added to the production medium during or after the cultivation process in order to obtain appropriate enzymatic activities. These may include expression inducers , sources of cofactors and or compounds allowing maintanence of genetic elements (such as antibiotics).

Preferably the main fermentation process comprises the inoculation of production medium with seed microorganism obtained in step b) in particular by asepticall transfer into the reactor. It is preferred to employ the vegetative form of the microorganism for inoculation. The addition of nutrient medium (production medium) in the main fermentation process into the reactor can be carried out once or more batch-wise or in a continuous way. Addition of nutrient medium (production medium) can be carried out before and/or during the fermentation process.

The preferred sources of carbon in the nutrient media can selected from dextrin, glucose, soluble starch, glycerol, lactic acid, maltose, fructose, molasses and sucrose as exemplified below.

The preferred sources of nitrogen in the nutrient media are ammonia solution, yeast extract, soy peptone, soybean meal, bacterial peptone, casein hydrolysate, L-lysine, ammonium sulphate, corn steep liquor and other.

Inorganic/mineral salts such as calcium carbonate, sodium chloride, sodium or potassium phosphate, magnesium, manganese, zinc, iron and other salts may also be added to the medium.

Further known additives for fermentative process may be added in particular in the main fermentation process. To prevent excessively foaming of the culture medium anti-foaming agents could be added, such as silicone oil, fatty oil, plant oil and the like. Particularly a silicone-based anti-foaming agent may be added during the fermentation process to prevent excessively foaming of the culture medium. Expression inducers such as isopropyl β-D-1-tiogalaktopyranoside (IPTG), Arabinose, Tetracycline, indoleacrilate etc. may be added to the culture medium. Additionally, cofactors such as PQQ (pyrroloquinoline quinone), NAD(P), FAD may be added to improved the activity of involved enzymes. In a particular aspect IPTG and PQQ may be used.

The fermentative process could be performed in aerobic conditions with agitation and aeration of production medium. Agitation and aeration of the culture mixture may be accomplished in a variety of ways. The agitation of production medium may be provided by a propeller or similar mechanical device and varied to various extents according to fermentation conditions and scale. The aeration rate can be varied in the range of 0.5 to 2.5 VVM (gas volume flow per unit of liquid volume per minute (volume per volume per minute)) with respect to the working volume of the bioreactor.

The main fermentation process by the present process is carried out at a pH in the range of about 6.3 to 8.5 and temperature in the range of 18 to 37 °C. Preferably the pH is in the range of about 6.5 to 8.3 and the temperature is in the range of about 21 to 31 °C. Preferably, the cultures are incubated for 16 to about 300 hours, more preferably for about 30 to 70 hours.

It will be obvious to a person skilled in the art that different microorganisms may demand different growth conditions and that it is well described in the art how one can determine the optimal conditions for growth of specific organism. It will be also obvious to a person skilled in the art that different growth conditions may have a big effect on activity of the enzymes involved in the provided process. The present disclosure provides detailed examples of methods which can be used to optimize the growth conditions to obtain maximal activities and reaction rates of said enzymes.

Another embodiment of in this disclosure encompasses cultivation of microorganisms described in step a) simulaniously or independatly. Likewise it is also possible to conduct reactions for preparation of compound (II) and compound (I) separately or simultanuosly, successively or in a one pot manner.

Specifically, dehydrogenase/oxidase enzyme is prepared after step c) in an aqueous solution (particularly in a concentration range from 0.1 g/L to 300 g/L), optionally in the presence of salt (in particular NaCl in concentration range from 50 to 500 mM), diluted or concentrated to said concentration range. When supplemented with fresh medium or components of the medium allowing viability of the organism living whole cell catalyst is obtained. When prepared in buffered aqueous solution or in used medium, resting whole cell catalyst is obtained. The aqueous solution may be buffered to pH 4.0 to 11, preferably to pH 5.0. to 10.0, more preferably to 5.0 to pH 8.0. Most preferably the solution is buffered to about pH5.2 to obout pH 7,5. Suitable buffers can be prepared from: acids, bases, salts or mixtures thereof, and any other buffer system known in the art except those possessing primary, secondary or tertiary amino group. In particular, phosphate buffer, in concentration 10 to 500 mM may be used.

The aqueous solution can be prepared by adding the said dehydrogenase/oxidase enzyme to water and maintaing pH during the reaction by means of automated addition of inorganic or organic acids, bases, salts of mixtures thereof.

### Step d) Enzymatic reaction

Several options to carry out this invention are provided. It is possible to conduct reactions for preparation of compound (II) and compound (I) separately or simultaneously, successively or in a one pot manner. In one embodiment, these variations are:

### 1. Compound (II) is added into the culture of microorganism having compound (II) oxidation/dehydrogenation capability.

After completion of preceding step of cultivating a microorganism capable of oxidation/dehydrogenation of compound (II) in main fermentation step, compound (II) is brought into contact with said microorganism. Said microorganism may intrinsically contain all needed cofactors or external PQQ addition may be used. PQQ may be added in concentration from about 0.1 nM to about 5mM. In particular from about 1 nM to about 100uM of PQQ can be provided. More preferably the PQQ is provided in concentration from 100nM to about 10µM. Most preferably the PQQ is provided in the minimal amount which allows maximal activity of the said catalyst. Practically this is in the range of 250nM to 5µM final concentration of PQQ. To facilitate reconstitution of the enzyme capable of oxidation/dehydrogenation with the extemaly provided PQQ, magnesium or calcium ions may be added. Prefferably MgCl₂ or CaCl₂ are added in concentration from about 0.1mM to about 50mM, in particular from about 1 mM to about 20mM, Most prefferabli in concentration from about 2mM to about 20mM. Optionally an artificial electron acceptor is added in equimolar concentration to compound II as described in this invention. Preferably the process is preformed by using microorganisms capability of accepting electrons formed during dehydrogenation / oxidation reaction into its intrinsic respiratory chain. Compound (II) may be provided as partiallly or fully isolated compound (II) obtained from previous reaction mixture containing DERA aldolase under aldolase-catalysed aldol condensation conditions or from other sources (organic synthesis). Compound (II) may be added in by any means and rates as described within this invention in a final concentration from about 20mM to about 1M, preferably from about 50mM to about 700mM, most preferred concentrations are between 100mM and 500mM. During the process of dehydrogenation / ocxidation of compound (II) additional nutrients in order to support microorganisms viability may be added in similar way as described in step c). Practically it may be beneficial to provide additional carbon source to said reaction mixture as described within hereby invention, optionally independently or in addition also a nitrogen source. General reaction conditions which allow dehydrogenation/ oxidation of compound (II) in this specific aspect are defined as 'aldose dehydrogenation/oxidation conditions' as provided by this invention.

The compound (II) as substrate for oxidase/dehydrogenase to obtain compound (II) may be added to the reaction mixture in one batch or more batches. In one aspect, the total amount of substrate added to the mixture is such that the total amount of compound (II) added would be from about 20 mmol per liter of reaction mixture to about 1.5 mol per liter of reaction mixture, more particular from about 100 mmol per liter of reaction mixture to about 700 mmol per liter of reaction mixture. In preferred embodiment the substrates are added continuously to the reaction mixture by means of programmable pump at specific flow rate at any given time of the reaction. Optimally, the flow rate is determined as maximum flow rate where the substrate is not accumulating in the reaction mixture. In particular this allows minimal concentrations of undesired products. In another embodiment the inhibitory effect of substrate can be further minimized using correct addition strategy.

### 2. Sequential reaction using DERA aldolase and oxidation/dehydrogenase enzyme.

This aspect the disclosure provides a variant described as d1), however in this case the compound (II) is provided in situ directly in form of reaction mixture obtained by reacting DERA aldolase with compound (X) and acetaldehyde by methods known in the art. Preferably this approach uses the advantage of performing a one-pot reaction and thus significantly impacting the simplicity of the combined process. It is advantageous to use whole cell catalyst containing DERA aldolase in the preceeding step in accordance to 'aldolase-catalysed aldol condensation conditions"

### 3. Simultaneous reaction using DERA aldolase and oxidation/dehydrogenase enzyme within two separated catalysts.

Both catalysts are obtained by fermentation process simultaneously or independently (as described in step c) and catalysts are then transferred into a suitable vessel or reactor, preferably joined or left in same fermenter used for obtaining the catalyst. Another yet similar aspect of the process both enzymatic activities are present in a single microorganism which is preferably obtained as described in step c. In a specific embodiment a simultaneous process containing using DERA aldolase and enzyme capable of oxidation/dehydrogenation and thus providing compound (I). More perferably the acetyloxyacetaldehyde (CH₃CO₂CH₂CHO) as substrate for DERA aldolase to obtain compound (II) may be added to the reaction mixture continuously or alternatively the acetyloxyacetaldehyde (CH₃CO₂CH₂CHO) is added to the reaction mixture in one batch or more batches. In one aspect, the total amount of substrates added to the mixture is such that the total amount of acetyloxyacetaldehyde (CH₃CO₂CH₂CHO) added would be from about 20 mmol per liter of reaction mixture to about 1.5 mol per liter of reaction mixture, more particular from about 100 mmol per liter of reaction mixture to about 700 mmol per liter of reaction mixture. Acetaldehyde may be added by several means. In one aspect the acetaldehyde is added to the reaction mixture in one batch or more batches or alternatively continuously. Acetaldehyde may be premixed with acetyloxyacetaldehyde (CH₃CO₂CH₂CHO)and added to the reaction mixture. The total amount of acetaldehyde added to the reaction mixture is from about 0.1 to about 4 molar equivalents to total amount of acceptor substrate acetyloxyacetaldehyde (CH₃CO₂CH₂CHO), in particular from about 1 to about 3 molar equivalents, more preferably from about 2 to 2.5 molar equivalents. In particular, this allows minimal concentrations of undesired products. Optionally PQQ is added into the reaction mixture in concentrations from about 0.05 µM to about 10 mM, more preferably 0.1 uM to about 100uM. To facilitate reconstitution of the enzyme capable of oxidation/dehydrogenation with the externaly provided PQQ, magnesium or calcium ions may be added. Preferably MgCl₂ or CaCl₂ are added in concentration from about 0.1mM to about 50mM, in particular from about 1 mM to about 20mM, Most preferably in concentration from about 2mM to about 20mM. In preferred embodiment the substrates are added continuously to the reaction mixture by means of programmable pump at specific flow rate at any given time of the reaction. The flow rate is determined as maximum flow rate where the substrates are not accumulating in the reaction mixture. In particular this allows minimal concentrations of undesired products. In another embodiment the inhibitory effect of substrates can be further minimized using correct addition strategy. In one aspect, the temperature used for dehydrogenase/oxidase-catalysed reaction is from about 10 °C to about 70 °C. in one embodiment, the temperature used for dehydrogenase/oxidase reaction is from about 20 to about 50 °C. In one embodiment the temperature used for dehydrogenase/oxidase reaction is from about 25 °C to about 40 °C. The reaction is industrially suitable, as it proceeds to completion within few hours.

The term "aldose dehydrogenation/oxidation conditions" as used herein refers to any dehydrogenation/oxidation conditions known in the art that can be catalysed by any dehydogenase/oxidase enzyme, as described herein. In particular the dehydrogenase/oxidase activity conditions are such that allow forming and accumulation of desired product. These conditions include in one aspect that the dehydrogenase/oxidase is an active enzyme provided at sufficient load to be able to perform the dehydrogenation/oxidation. In another aspect, that the compound (II) as substrate is present in the reaction mixture in an amount that displays minimal inhibition of the activity of the aldolase. Preferably, that the temperature, pH, solvent composition, agitation and length of reaction allow accumulation of desired product and thus forming from compound (II) corresponding compound (I), in another aspect that said conditions do not have detrimental effect stability. Specifically those conditions are defined by values disclosed in examples. The aldose 1-dehydrogenase for use in the present invention may be any aldose 1-dehydrogenase enzyme that has dehydrogenase/oxidase activity towards compound of formula (II). In a prefered embodiment, the aldose 1-dehydrogenase enzymes include but are not limited to: GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25, wherein each enzyme is identified by it's corresponding nucleotide sequence or respective codon optimized nucleotide sequence or aminoacid sequence as set forth in sequence listing above. The dehydrogenase/oxidase catalyst described herein can be used in any biologically active form provided in this disclosure. Generally, catalyst able to obtain compound (I), will be provided in a suitable vessel or reactor, and the compound (II) will be added batch-wise or continuously, or provided by, at least in part simultaneous production of compound (II), preferably in same vessel under "aldolase-catalysed aldol condensation conditions".

The term "aldolase-catalysed aldol condensation conditions" as used herein refers to any aldol condensation conditions known in the art that can be catalysed by any aldolase, as described for example in WO2008/119810 A2. In particular the aldolase-catalysed aldol condensation conditions are such that allow forming and accumulation of desired product, more preferably that the substituted acetaldehyde R₁CO₂CH₂CHO, more particularly acetyloxyacetaldehyde (CH₃CO₂CH₂CHO) as substrate and acetaldehyde are present in the reaction mixture in an amount that displays minimal inhibition of the activity of the aldolase, in another aspect that the temperature, pH, solvent composition, agitation and length of reaction allow accumulation of desired product and thus forming corresponding lactole (compound II). The DERA aldolase described therein can be used in any biologically active form provided herein. The substrates for DERA aldolase, the compounds of formula (X) are selected according to the corresponding compound (II). These products having a masked aldehyde group are key intermediates in WO2008/119810 A2 and WO2009/092702 A2 allowing further steps in preparation of statins, in particular, substrates yielding a product with aldehyde group are preferred. The compound (X) may be in particular acetyloxyacetaldehyde (CH₃CO₂CH₂CHO).

### Optional step e): Separation and/or purification of compound (I)

Isolation and/or purification of compound (I), which was produced in the main fermentation process from the said medium, may be carried out in a further separation step.

In the first step of the isolation, whole cell catalyst present in reaction mixture may be removed by any known type of filtration/flocculation/sedimentation/centrifugation procedure or further steps of the isolation are performed on whole cell containing reaction mixture. Preferably it is the object to omit the step of separating solid particles from the reaction mixture and perform direct extraction step immediately after completion of the reaction as described further on.

In one embodiment, the isolation of compound (I) may be carried out by adsorbtion to an adsorbent capable of binding compound (I) at significant levels and releasing compound (I) upon elution conditions such as replacement of the medium by a more nonpolar compound. Adsorbent may be selected from but not limited to: silica gel, zeolites, activated carbon, Amberlite™ XAD™ adsorbent resins, Amberlite™ and Amberlite™ FP ion exchange resins etc.

Alternatively liquid-liquid extraction may be carried out using water miscible solvents such as acetonitrile or methanol and supplementing the mixture with high concentration of salts, optionally Sodium chloride in a so colled "salting out" extraction procedure. In this process separation of phases is observed and the compound (I) is preferentialy distributed in solvent rich phase.

In a preferred embodiment the extraction solvent for the liquid/liquid extraction is chosen from any a number of water immiscible or poorly miscible solvents. The solvent may be selected from but is not limited to: methylene chloride, diethyl ether, propionyl acetate, methyl *t*-butyl ether (MTBE), nitromethane, pentane, hexane, heptane, 1,2-dichloroethane, chloroform, carbon tetrachloride, *n*-butanol, *n*-pentanol, benzene, toluene, *o*-, *m-, p*-xylene, cyclohexane, petroleum ether, triethylamine. Prior the liquid/liquid extraction with chosen organic solvent the pH of water solution of the product may be adjusted to values between 1 and 9, preferably between 2 and 8, more preferably between 2 and 5. In addition, in a prefered embodiment, the ionic strength of the aqueous phase is increased prior to liquid/liquid extraction by addition of any if inorganic /organic acids, salts or bases. Non-limiting examples of such acids, salts or bases are: phosphoric acid, and salts thereof, sulphuric acid and salts thereof, citric acid and salts thereof, hydrochloric acid and salts thereof etc. The increased ionic strength of the aqueous phase increases the distribution coefficient of compound (II) between the aqueous and organic phase therefore increasing efficacy of the extraction process.

Drying of water residues in organic phase after extraction completion may be performed with but is not limited to adding salts listed: sodium sulfate, magnesium sulfate (monohydrate), calcium sulfate, calcium chloride, copper sulfate.

In most prefered embodiment of the purification process, the pH of the reaction mixture is first corrected to value from about 1 to 9, preferably from about 2 to 8, more preferably from about 3 to 6. Most preferably pH is corrected to about 5 using an acid compound such as phosphoric, sulphuric or hidricloric acid, etc. Sodium suphate, disodium hydrogen phosphate, sodium chloride etc is added in concentration from 50g/L to 300g/L, most preferably from 100 to 200 g/L. Ethyl acetate is added at least 1 time by the addition of at least 1 volume of ethyl acetate to 1 volume of reaction mixture, preferably at lest 3 times by the addition of 1 volume of ethyl acetate to 1 volume of reaction mixture. Most preferably the steps of adding ethyl acetate and separationg the extract are carried out until no more than 5% of compound (I) is present in aqueous phase. Ethyl acetate fractions are collected, dried with any of the drying salts known in the art, preferably CaCl₂ or MgSO₄ or other methods of water stripping known in the art. In one aspect the obtained ethylacetate extract can be evaporated to yield isolated compound (I) in a form of yellow - amber oul at room temperature or can alternatively proceede into further steps in order tu yield pharmaceutically usefull compounds, preferably statins.

### Optional step f): Further processing

After obtaining the compound of formula (I), the compound of formula (I) can be further transformed to an API, preferably statin, or a pharmaceutically acceptable salt thereof, by subjecting said compound (I) to conditions sufficient to prepare the API, preferably statin. Thus, in an embodiment, a statin or salt, ester or stereoisomer thereof is prepared by (i) bringing in contact the compound of formula (II) as defined hereinabove with an enzyme capable of catalyzing oxidation or dehydrogenation, to prepare a compound of formula (I) as defined hereinabove, (ii) subjecting said compound (I) to conditions sufficient to prepare a statin; and (iii) optionally salifying, esterifying or stereoselectively resolving the product. Again, the compound of formula (II) can be prepared by using 2-deoxyribose-5-phosphate aldolase (DERA, EC 4.1.2.4) enzyme. The reaction setup can be arranged to introduce both enzymes substantially simultaneously or subsequently, at once or continuously, in one batch or in intermittent batches. Preferably the compounds of formula (II) and (I) are prepared at least in part simultaneously, more preferably substantially simultaneously. It is advantageous to use enzymes for preparing compounds of formula (I) and/or (II) in the case of enzymes, because the product immediately contains the correct spacious orientation of the substituents and no further purification or separation is needed. In the event that other stereoisomers are needed, the process can be combined with methods of stereospecific chemistry known to the skilled person. In preferred embodiment, the statin prepared is lovastatin, pravastatin, simvastatin, atorvastatin, cerivastatin, rosuvastatin, fluvastatin, pitavastatin, bervastatin, or dalvastatin, more preferably atorvastatin, rosuvastatin or pitavastatin, particularly is rosuvastatin.

The term "conditions sufficient to produce an API, preferably statin" as used herein refers to those means described in the art, including those means described herein for conversion of the compound of formula (I) further to the API, preferably statin. In a specific embodiment, where statin is prepared, the skilled person would choose the chemical route by selecting the proper R1 and R2, or R. In the event that R1, R2 and R are chosen to represent the statin skeleton, the compound of formula (I) is already a statin molecule ro an "advanced" intermediate thereof. Nevertheless, modifying the statin like for example by opening the lactone ring, forming the salt or the ester or resolving desired stereoisomers from the mixture of stereoisomers is also possible. In an alternative, if the statin is to be prepared by first providing a lactone and then coupling it to the statin skeleton, a reaction scheme 2 can be followed. After obtaining the compound of formula (I), its hydroxyl group in position 4 can be protected with the protecting group P (formula (XI)), which can be any conventionally used protecting group, in particular is silyl protecting group. Afterwards, the compound can be brought in the form of an aldehyde or its hydrate (formulas (XII) and (XIII), respectively).

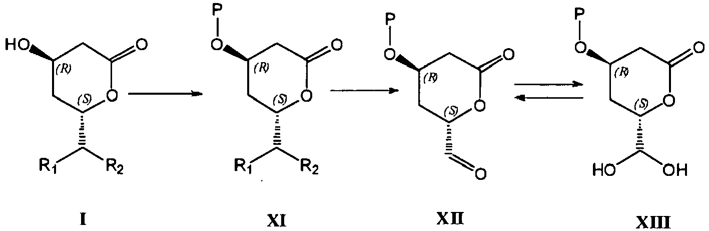

Compound of formula (XII), or hydrate thereof (XIII), obtained from I can be further used to prepare statin by reacting the compound of formula (XII), or hydrate thereof (XIII), under the condition of a Wittig coupling with a heterocylic or alicyclic derivative (statin skeleton) followed by hydrogenation when needed. In the specific embodiment related to preparing rosuvastatin, in the subsequent reaction step, (2*S*,4*R*)-4-(*P*-oxy)-6-oxo-tetrahydro-2*H*-pyran-2-carbaldehyde (XII) can be reacted under the conditions of a Wittig coupling (in the presence of a base) with a ((4-(4-fluorophenyl)-6-isopropyl-2-(*N-*methylmethylsulfonamido)pyrimidin-5-yl)methyl)triphenyl-phosphonium halide or any other ((4-(4-fluorophenyl)-6-isopropyl-2-(*N*-methylmethylsulfon-amido)pyrimidin-5-yl)methyl)phosphonium salt or alternatively di-*i*-propyl({4-(4-fluorophenyl)-6-isopropyl-2-[methyl(methylsulfonyl)amino]-5-pyrimidinyl}methylphosphonate or any other ({4-(4-fluorophenyl)-6-isopropyl-2-[methyl(methylsulfonyl)amino]-5-pyrimidinyl}methylphosphonate ester to give *N*-(5-((*E*)-2-((2*S*,4*R*)-4-(P-oxy)-6-oxo-tetrahydro-2*H*-pyran-2-yl)vinyl)-4-(4-fluoro-phenyl)-6-isopropylpyrimidin-2-yl)-*N*-methylmethanesulfonamide. As a base, lithium hexamethyldisilazane (LiHMDS), potassium hexamethyldisilazane (KHMDS), sodium hexamethyldisilazane (NaHMDS), lithium diisopropylamide (LDA), sodium hydride, butyllithium or Grignard reagents, preferably sodium hexamethyldisilazane may be used. When the source is the hydrate form XIII or a mixture of XII and the hydrate form XIII thereof, which is dissolved in ethers selected from THF, Et₂O. *i*-Pr₂O, *^{t}*BuMeO; hydrocarbons selected from: pentane, hexane, cyclohexane, methylcyclohexane, heptane; aromatic hydrocarbons selected from toluene or the chlorinated derivatives thereof; chlorinated hydrocarbons selected from: chloroform and dichloromethane or in mixtures of those solvents, water released from the hydrate should be removed prior to the addition to the formed ylide solution. The preferred solvents for the reaction are anhydrous toluene and dichloromethane. The reaction can be performed at temperatures between -80 °C and 90 °C preferably at 0 to 90 °C, more preferably at 80 - 90 °C. The reaction is accomplished in 1 - 12 hours. Isolation of the crude product with extraction can be performed with AcOEt, ethers or alkanes, preferably with *^{t}*BuMeO. The protecting group may be removed and the lactone opened to produce a rosuvastatin free acid or a salt thereof, optionally an amine, which may be converted to hemicalcium salt. The deprotection can be performed at temperatures between 0 °C to 80 °C. Preferably at 20 or 40 °C in a suitable solvent, preferably a solvent selected from alcohols, acetic acid, THF, acetonitrile, methyltetrahydrofuran, dioxane, CH₂Cl₂, more preferably in alcohols and a mixture of THF/AcOH. The usual deprotecting reagents may be used, such as tetra-n-butylammonium fluoride, ammonium fluoride, AcCl, FeCl₃, TMSCl/HF·2H₂O, chloroethylchloroformate (CEC), Ph₃PCH₂COMeBr. The opening of the lactone preferably takes place in a 4:1 to 2:1 mixture of THF/H₂O as well as in pure THF at temperatures between 20 °C to 60 °C with a suitable alkali such as NaOH, KOH, ammonia or amines. The hydrolysis is accomplished in 30 minutes (at 60 °C) to 2 hours (at 20 °C). After the hydrolysis step, evaporation of THF can be conducted at temperatures between 10 °C to 50 °C under the reduced pressure, and conversion to the calcium salt, preferably by the addition of Ca(OAC)₂.×H₂O, which can be added in one portion or dropwise in 5 to 60 minutes, can be performed at temperatures between 0 °C to 40 °C. After the addition of Ca(OAC)₂.×H₂O, the resulting suspension can be stirred at temperatures between 0 °C to 40 °C from 30 minutes to 2 hours. The details of such reaction are known in the art, including in, but not limited to, WO2008/119810.

The API, preferably statin, obtained by any of the aforementioned embodiments, can be formulated in a pharmaceutical formulation. The methods for preparing a pharmaceutical formulation with the API, preferably statin, are known to the person skilled in the art. Generally, one can chose among preparing formulations such as powder, granulate, tablet, capsule, suppository, solution, ointment, suspension, foam, patch, infusion, solution for injection, or the like. The formulation can be changed in order to modify specific aspects of the API like for example release, stability, efficacy or safety. Depending on the API, the skilled person knows how to select proper administration route for said API. Based on that, he can choose proper formulation. Then, the skilled person is in a position to select from the excipients needed for formulating the pharmaceutical formulation. Besides the API, which can be optionally combined with at least one another API, the skilled person can select from excipients and additives to formulate the pharmaceutical formulation. Suitable excipients may be, for example, binder, diluent, lubricant, disintegrant, filler, glidant, solvent, pH modifying agent, ionic strength modifying agent, surfactant, buffer agent, anti-oxidant, colorant, stabilizer, plasticizer, emulsifier, preservatives, viscosity-modifying agent, passifier, flavouring agent, without being limited thereto, which can be used alone or in combination. The method can involve, depending on the selected formulation, mixing, grinding, wet granulation, dry granulation, tabletting, dissolving, lyophilisation, filling into capsules, without being limited thereto.

### Application to APis and intermediates thereof

In further aspects, an enzyme capable of catalyzing oxidation or dehydrogenation can be generally used for preparing an API or intermediate thereof being compatible with the enzymatic system(s) disclosed herein.

This aspect preferably relates to synthetic API or intermediate thereof respectively categorisable as substituted or unsubstituted dideoxyaldose sugars, lactols (optionally containing multiple hydroxyl groups) and synthetic non-natural alcohols as possible substrates, and (optionally further hydroxylated) lactons or esters as possible products. From the present disclosure and its technical character, it will be understood that compounds naturally occurring or arising in the biochemical pathways of sugars (e.g. glycolysis, pentose phosphate pathway, glycogenesis), fatty acids, or cellular respiratory chain, would be exempted from being regarded as either substrate for the enzyme capable of catalyzing oxidation or dehydrogenation or the API or intermediate thereof according to the present disclosure. In the same manner, naturally occurring amino acids, vitamins or cofactors are also exempted from the definition of the API or intermediate thereof according to the present disclosure. The substrates or products of such pathways in the nature include methanol, ethanol, formaldehyde, acetaldehyde, methanoic, acetic acid, monosaccharide, disaccharide, trisaccharide, glucuronic acid, especially methanol, ethanol, formaldehyde, acetaldehyde, methanoic, acetic acid, monosaccharide, glucuronic acid, and particularly ethanol, acetic acid and monosaccharide. In a preferred embodiment, the enzyme capable of catalyzing oxidation or dehydrogenation is used for preparing the compound of formula (I), wherein the formula (I) is as defined hereinabove. In this aspect, it is the most efficient to use the enzyme to act upon the compound of formula (II), wherein the formula (II) is as defined hereinabove. Specific alternatives of the use will be immediately apparent to the skilled person when other embodiments, aspects, or preferred features of the disclosure hereinabove are taken into account.

To give an illustrative further example, a possible useful further definition of the substrate and thus starting compound useful for the preparation of appropriate APIs or intermediates thereof is given by the following formula XIV, hence leading to a corresponding product compound defined by formula XV. 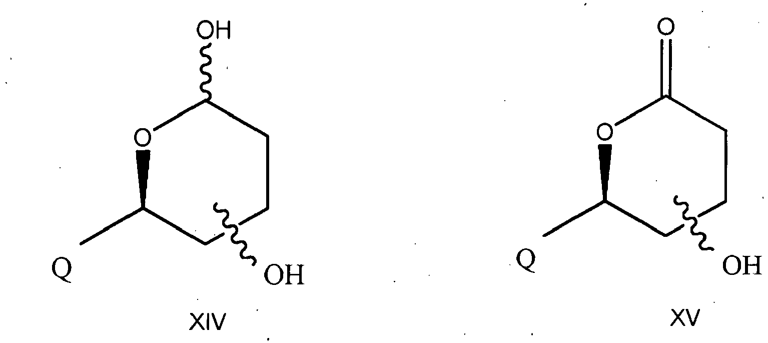 wherein Q is any desired structural moiety, for example selected from the groups of R₁, R₂ and R⁵ defined hereinabove, optionally with an intermediate linker molecule between R₁, R₂ or R⁵ and the lactol/lactone ring. As shown in the structural formulae, the non-lactol hydroxyl group not being oxidyzed can be positioned at any position of the lactol/lactone ring.

In a preferred embodiment, the enzyme capable of catalyzing oxidation or dehydrogenation is used for preparing an API or intermediate thereof simultaneously or subsequently with a DERA enzyme.

### Examples

### Example 1: Preparation of aldose dehydronenases comprised within the whole cell

Several aldose dehydrogenases have been prepared as described following examples:
First, a membrane bound glucose dehydrogenase, a pyrroloquinone quinone (PQQ) dependent dehydrogenase, encoded by gene *gcd* (*E. coli* GeneBank #JW0120, locus tag b0124) was prepared.

The genomic DNA from *E. coli* DH5α was isolated using Wizard Genomic DNA Purification Kit (Promega, Madison, WI, USA) according to manufacturer's instructions. Isolation of genomic DNA was made using overnight culture of *E. coli* grown on LB medium at 37 °C. Amplification of gene *gcd* was performed by PCR using oligonucleotide primers GCGCCATATGGCAATTAACAATACAGGCTCGCG and GCGCGCTCAGCGCAAGTCTTAC TTCACATCATCCGGCAG. Amplification was performed by *Pfx*50 DNA polymerase (Invitrogen, Calsbad, CA, USA) as follows: an initial denaturation at 94 °C for 10 min, followed by 30 cycles of 45 s at 94 °C, 45 s at 52 °C, and 150 s at 68 °C. Final elongation was performed 420 s at 68 °C. A 2.4-kb DNA fragment containing *gcd* (SEQ ID NO. 03) was separated by agarose gel electrophoresis and purified. The product was ligated into plasmid pGEM T-Easy (Promega, Madison, WI, USA) in a T4 ligase reaction. The plasmid construct was cleaved with restriction endonucleases *Nde*I and *Blp*I, the resulting fragments were separated on agarose gel electrophoresis and 2.4 kb fragment containing *gcd* was purified. An expression vector pET30a(+) (Novagen Inc., Madison, WI, USA) was cleaved using the same aforementioned restriction endonucleases and purified. The 2.4 kb fragment containing *gcd* gene was assembled with the cleaved expression vector in a T4 ligase reaction. *E. coli* JM109 cells were transformed with the obtained ligation reaction and kanamycin resistant colonies were cultured. Afterwards plasmid DNA was isolated. The resulting construct was designated pET30/Gcd and sequenced for confirmation of the gene sequence. The cloning procedure was performed to allow expression of protein having sequence (SEQ ID NO. 04) containing necessary signals for incorporation into the cellular membrane The membrane bound glucose dehydrogenase expressing organism was prepared by transforming BL21 (DE3) competent cells with the said plasmid.

Second, a water-soluble aldose dehydrogenase, a pyrroloquinoline quinone (PQQ) dependent dehydrogenase encoded by gene *yli*I (*E. coli* GeneBank # ECK0827, locus tag b0837) was prepared.

The genomic DNA from *E. coli* DH5α was isolated using Wizard Genomic DNA Purification Kit (Promega, Madison, WI, USA) according to manufacturer's instructions. Isolation of genomic DNA was made using overnight culture of *E. coli* grown on LB medium at 37 °C. Amplification of gene *yliI* was performed by PCR using oligonucleotide primers GCGCCATATGCATCGACAATCCTTT and GCGCGCTCAGGCTAATTGCGTGGGCTAA CTTTAAG Amplification was performed by *Pfx*50 DNA polymerase (Invitrogen, Calsbad, CA, USA) as follows: an initial denaturation at 94 °C for 10 min, followed by 30 cycles of 45 s at 94 °C, 45 s at 52 °C, and 150 s at 68 °C. Final elongation was performed 420 s at 68 °C. A 1.2-kb DNA fragment containing *yliI* (SEQ ID NO. 01) was separated by agarose gel electrophoresis and purified. The product was ligated into plasmid pGEM T-Easy (Promega, Madison, WI, USA) in a T4 ligase reaction. The plasmid construct was cleaved with restriction endonucleases *Nde*I and *Sal*I, the resulting fragments were separated on agarose gel electrophoresis and 1.2 kb fragment containing *yliI* was purified. An expression vector pET30a(+) (Novagen Inc., Madison, WI, USA) was cleaved using the same aforementioned restriction endonucleases and purified. The 1.2 kb fragment containing *yli*I gene was assembled with the cleaved expression vector in a T4 ligase reaction. *E. coli* JM109 cells were transformed with the obtained ligation reaction and kanamycin resistant colonies were cultured. Afterwards plasmid DNA was isolated. The resulting construct was designated pET30/Ylil and sequenced for confirmation of the gene sequence. The cloning procedure was performed to allow expression of protein having sequence (SEQ ID NO. 02) including leader sequence for YliI translocation to periplasm. The aldose dehydrogenase expressing organism was prepared by transforming BL21 (DE3) competent cells with said plasmid.

Another water-soluble aldose dehydrogenase, found in *Acinetobacter calcoaceficus,* was used for alternative oxidation studies. A nucleotide sequence encoding a gene and leader sequence for transportation to periplasm (PQQ GdhB, *A*. *calcoaceticus* GeneBank #X15871) was optimized for expression in *E. coli* and DNA was chemically synthesized (Geneart, Regensburg, Germany) (SEQ ID. NO. 05). *E. coli* JM109 cells were transformed with artificial plasmid bearing nucleotide sequence *gdhB* and kanamycin resistant colonies were cultured. Afterwards plasmid DNA was isolated and the construct was cleaved with restriction endonucleases *Nde*I and *Hin*dIII*,* the resulting fragments were separated on agarose gel electrophoresis and 1.5 kb fragment was purified (SEQ ID NO. 05). An expression vector pET30a(+) (Novagen Inc., Madison, WI, USA) was cleaved using the same aforementioned restriction endonucleases and purified. The 1.5 kb fragment containing *gdhB* gene was assembled in the cleaved expression vector in a T4 ligase reaction. *E. coli* JM109 cells were transformed with the obtained ligation reaction and kanamycin resistant colonies were cultured. Afterwards plasmid DNA was isolated. The resulting construct was designated pET30/GdhB and sequenced for confirmation of the gene sequence. The cloning procedure was performed with a target to allow expression of protein having sequence SEQ ID NO. 06. The aldose dehydrogenase from *A. calcoaceticus* expressing organism was prepared by transforming BL21 (DE3) competent cells with said plasmid.

Yet another water-soluble aldose dehydrogenase found in Acinetobacter calcoaceticus (PQQ GdhB, A. calcoaceticus GeneBank #X15871) with altered sequence and hence its increased thermal stability properties was used [Igarashi, 2003]. A nucleotide sequence encoding a gene and leader sequence for transportation to periplasm (PQQ GdhB, A. calcoaceticus GeneBank #X15871) was optimized for expression in E. coli and DNA was chemically synthesized (Geneart, Regensburg, Germany) (SEQ ID NO. 07). E. coli JM109 cells were transformed with artificial plasmid bearing nucleotide sequence gdhB_therm and ampicillin resistant colonies were cultured. Afterwards plasmid DNA was isolated and the construct was cleaved with restriction endonucleases NdeI and HindIII, the resulting fragments were separated on agarose gel electrophoresis and 1.5 kb fragment was purified (SEQ ID NO. 07). An expression vector pET30a(+) (Novagen Inc., Madison, WI, USA) was cleaved using the same aforementioned restriction endonucleases and purified. The 1.5 kb fragment containing gdhB_therm gene was assembled in the cleaved expression vector in a T4 ligase reaction. E. coli JM109 cells were transformed with the obtained ligation reaction and kanamycin resistant colonies were cultured. Afterwards plasmid DNA was isolated. The resulting construct was designated pET30/GdhB_therm and sequenced for confirmation of the gene sequence. The alternative aldose dehydrogenase expressing organism was prepared by transforming BL21 (DE3) competent cells with said plasmid. The cloning procedure was performed with a target to allow expression of protein having sequence SEQ ID NO. 08. SEQ ID NO. 08 which compared to SEQ ID NO 06 has altered sequence coding Ser residue at position 231 to Lys residue.

Where stated, *E. coli* BL21 (DE3) having an expression plasmid vector pET30a(+))·(Novagen Inc., Madison, WI, USA) was used as negative control. The control cells were prepared by transforming *E. coli* BL21 (DE3) competent cells with said plasmid and kanamycin resistant colonies were cultured.

The procedure of expressing the various enzymes in *E. coli* was undertaken as described in Procedure 1A. After expression, cells were harvested and whole cell catalyst was obtained as described in Procedure 1B. To obtain resting whole cell catalyst Procedure 1C was undertaken.

### Procedure 1A:

VD medium (30 mL; 10 g/L bacto yeast extract, 5 g/L glycerol, 5 g/L NaCl, 4 g/L NaH2PO4•2H2O, pH was adjusted with 1 M NaOH to pH=7.0) supplemented with kanamycin (25 µg/mL) was inoculated with a single colony E. coli BL21 DE(3) pET30/Gcd or E. coli BL21 DE(3) pET30/Ylil or BL21 DE(3) pET30/GdhB or BL21 DE(3) pET30/GdhB_therm from a freshly streaked VD agar plate and pre-cultured to late log phase (37 °C, 250 rpm, 8 h). The pre-culture cells (inoculum size 10 %, v/v) were then transferred to fresh VD medium (100 mL; 10 g/L bacto yeast extract, 5 g/L glycerol, 5 g/L NaCl, 4 g/L NaH2PO4•2H2O, pH was adjusted with 1 M NaOH to pH=7.0) supplemented with kanamycin (25 µg/mL). For the induction of protein expression 0.1 mM isopropyl-β-D-thiogalactopyranoside (IPTG, purchased by Sigma Aldrich, Germany) was added. The cells were cultured in a rotary shaker at 25 °C, 250 rpm for 16 h.

### Procedure 1B:

After expression (as described in Procedure 1A) cells were harvested by centrifugation (10 000 g, 5 min, 4 °C). Supernatant was discarded after centrifugation and pellet was resuspended in fresh VD medium (10 g/L bacto yeast extract, 5 g/L glycerol, 5 g/L NaCl, 4 g/L NaH2PO4•2H2O, pH was adjusted with 1 M NaOH to pH=6.0). Cells were resuspended in one tenth of initial culture volume.

### Procedure 1C

After discarding supernatant (with regard to Procedure 1A) the pellet was resuspended in phosphate buffer (50 mM KH2PO4, 150 mM NaCl, pH 6.0). Cells were resuspended in one tenth of initial culture volume.

### Example 2: Preparation of aldose dehydrogenase comprised within cell free lysate

To obtain cell free lysate after expression of *E. coli* YliI, *E. coli* GdhB, *E. coli Gcd* or *E. coli* GdhB_therm (as described in Example 1, more particularly after undertaking Procedure 1A) cells were harvested by centrifugation (10 000 g, 5 min, 4 °C). Supernatant was discarded after centrifugation and pellet was resuspended in phosphate buffer (50 mM KH₂PO₄, 150 mM NaCl, pH 7.0) in one tenth of initial volume. Cells were supplemented with 1 mg/mL lysozyme solution. Lysis was performed at 37 °C, 1h. After lysis cell debris were removed by sedimentation (10 min, 20 000 g, 4 °C) to obtain a clear aqueous solution. Aldose dehydrogenase comprised within a cell free extract was thus obtained.

Alternatively the pellet was resuspended in a lytic buffer (50 mM NaH2PO4, pH 7.0, 300 mM NaCl, 2 mM DTT) using 200 g of pellet per 1 L of said buffer. Cells were sonified (3 x 15 s) using Branson digital sonifier and cell debris were removed by sedimentation (10 min, 20 000 g, 4 °C) to obtain a clear aqueous solution. Aldose dehydrogenase comprised within a cell free extract was thus obtained.

In the same manner as above, cell free lysates were prepared from whole cell *Klyuvera intermedia* and *Gluconobacter oxydans* (cultivations and preparations of whole cell catalysts are described in Example 9 and Example 10, respectively).

Culture of *E. coli* BL21(DE3) pET30, used as negative control was treated by the same procedures.

### Example 3: Preparation of periplasmic cell fraction containing aldose dehydrogenase

To obtain specific release of periplasmic proteins after expression of *E. coli* YliI, *E.coli Gcd, E. coli* GdhB or *E. coli* GdhB_therm (as described in Example 1, more particularly after undertaking Procedure 1A) the following procedure was used. Cells from freshly expressed culture were pelleted by centrifugation and the growth medium was completely removed. The cell pellet was washed three times in 20 mM Tris-HCl (pH 7.5). The cell pellet was then centrifuged
(10 000 g, 5 min, 4 °C) before resuspended in one tenth of initial culture volume of hypertonic solution containing 20 mM Tris HCl (pH 7.5), 20 % sucrose, and 0.5 mM EDTA. The cell suspension was incubated on ice for 10 min. After centrifugation (10 min, 12 000 g, 4 °C) cell pellet was resuspended gently by pipetting into hypotonic solution (50 mM Tris-HCl pH 7.0) in the same volume as hypertonic solution. Cells were incubated on ice for additional 10 min. Cells were pelleted by centrifugation (10 min, 20 000 g, 4 °C) and the supernatant was removed and regarded as periplasmic fraction.

Culture of *E. coli* BL21(DE3) pET30, used as negative control was treated by the same procedure.

### Example 4: Preparation of membrane cell fraction containing aldose dehydrogenase

To obtain specific release of membrane bound proteins after expression of *E. coli* Gcd (as described in Example 1, more particularly after undertaking Procedure 1A) the following procedure was used. Cells from freshly expressed culture were pelleted by centrifugation and the growth medium was completely removed. The cell pellet was resuspended to initial volume in phosphate buffer (50 mM KH₂PO₄, 150 mM NaCl, pH 6.0) containing 0.05 % (v/v) Triton X-100 and 1 mg/mL lysozyme and incubated for 30 minutes at 37 °C. Cell debris was removed by centrifugation (30 min, 20 000 g, 4 °C) and the supernatant was separated and labeled as membrane fraction.

Culture of *E. coli* BL21(DE3) pET30, used as negative control was treated by the same procedure.

### Example 5: Preparation of deoxyribose-5-phosphate aldolase enzyme (DERA) comprised within the whole cell

The aldolase gene *deoC* (*E. coli* GeneBank #EG10221, locus tag b4381) comprised within whole cell catalyst can be obtained by a number of procedures, for example as described in WO2009/092702. Nevertheless, we provide a nonlimiting example of preparation of aldolase enzyme (DERA) comprised within the whole cell.

The genomic DNA from *E. coli* DH5α was isolated using Wizard Genomic DNA Purification Kit (Promega, Madison, WI, USA) according to manufacturer's instructions. Isolation of genomic DNA was made using overnight culture of *E. coli* grown on LB medium at 37 °C. Amplification of gene *deoC* was performed by PCR using oligonucleotide primers CCGGCATATGACTGATCTGAAAGCAAGCAG and CCGCTCAGCTCATTAGTAGCTGCTG GCGCTC Amplification was performed by *Pfx*50 DNA polymerase (Invitrogen, Calsbad, CA, USA) as follows: an initial denaturation at 95 °C for 10 min, followed by 30 cycles of 45 s at 94 °C, 45 s at 60 °C, and 60 s at 68 °C. Final elongation was performed 420 s at 68 °C. The resulting fragments were separated by agarose gel electrophoresis and purified. A 0.9-kb DNA fragment containing *deoC* (SEQ ID NO. 09) was separated by agarose gel electrophoresis and purified. The product was ligated into plasmid pGEM T-Easy (Promega, Madison, WI, USA) in a T4 ligase reaction. Thus obtained plasmid construct was cleaved with restriction endonucleases *Nde*I and *Blp*I, the resulting fragments were separated on agarose gel electrophoresis and 0.9 kb fragment containing *deoC* was purified. An expression vector pET30a(+) (Novagen Inc., Madison, WI, USA) was cleaved using the same aforementioned restriction endonucleases and purified. The 0.9-kb fragment containing *deoC* gene was assembled with the cleaved expression vector in a T4 ligase reaction. *E. coli* JM109 cells were transformed with the obtained ligation reaction and kanamycin resistant colonies were cultured. Afterwards plasmid DNA was isolated. The resulting construct was designated pET30/DeoC and sequenced for confirmation of the gene sequence. The cloning procedure was performed with a target to allow expression of protein having sequence SEQ ID NO. 10. The DERA aldolase expressing organism was prepared by transforming BL21 (DE3) competent cells with said plasmid.

Expression of *deoC*: VD medium (50 mL; 10 g/L bacto yeast extract, 5 g/L glycerol, 5 g/L NaCl, 4 g/L NaH₂PO₄·2H₂O, pH=7.0) supplemented with kanamycin (25 µg/mL) was inoculated with a single colony *E. coli* BL21 DE(3) pET30/DeoC from a freshly streaked plate and cultured overnight (37 °C, 250 rpm).

The procedure of expression of *E. coli* DeoC was undertaken as described in Procedure 1A, with a sole difference of IPTG inducer concentration being 0.5 mM. After expression cells were harvested and whole cell catalysts were obtained as described in Procedure 1B to obtain living whole cell catalysts. To obtain resting whole cell catalysts Procedure 1C was undertaken.

### Example 6: Preparation of aldolase enzyme (DERA) and quinoprotein glucose dehydrogenase enzyme comprised within a whole cell culture of a single microorganism

Two examples of a genetically modified organisms having both aldolase activity (DERA) and glucose dehydrogenase activity were constructed.

In the first case, the gene *yli*I*,* having additional ribosomal binding site (RBS) was added into the construct pET30/DeoC. The resulting construct is bearing two different coding sequences (one encoding DERA and the other encoding Yli) organized in a single operon and under transcriptional control of IPTG inducible promoter.

Specifically, the plasmid DNA from *E. coli* JM109 pET30/Ylil (construction is described in Example 1) was isolated using Wizard Plus SV Minipreps DNA Purification Kit (Promega, Madison, WI, USA) according to manufacturer's instructions. Isolation of plasmid DNA was made using overnight culture of *E. coli* grown on LB medium at 37 °C.

The isolated plasmid pET30/Ylil was used as a template in a PCR reaction to amplify sequence containing gene *yliI* and RBS derived from expression vector pET30a(+) upstream of the coding region. PCR reaction was performed using oligonucleotide primers GCAGGCTGAGCTTAACTTTAAGAAGGAGATATACATATG and GCGCGCTCAGCCTAAT TGCGTGGGCTAACTTTAAG Amplification of this fragment was performed by PCR using *Pfu* ULTRA II Fusion HS DNA 200 polymerase (Agilent, Santa Clara, CA, USA) as follows: an initial denaturation at 98 °C for 3 min, followed by 30 cycles of 20 s at 98 °C, 20 s at 55 °C, and 90 s at 72 °C. Final elongation was performed 180 s at 72 °C. The resulting fragments were separated by agarose gel electrophoresis and 1.2 kb fragment containing *yliI* and RBS site was purified. The product was transferred to plasmid pGEM T-Easy (Promega, Madison, WI, USA) and designated pGEM/RBS_ylil. The construct was cleaved with restriction endonuclease *Blp*I, then the resulting fragments were separated on agarose gel electrophoresis and 1.2 kb fragment containing *yliI* and RBS site was purified. The construct pET30a/DeoC (construction is described in Example 5) was cleaved using the aforementioned restriction endonuclease (*Blp*I) and purified. Shrimp Alkaline Phosphatase (SAP, purchased by Promega, Madison, WI, USA) was used to dephosphorylate the 5' phosphorylated ends of cleaved pET30a/DeoC according to manufacturer's instructions. Thus self-ligation of pET30a/DeoC was prevented. The fragments were assembled in a T4 ligase reaction. *E. coli* JM109 cells were transformed with the obtained ligation reactions and kanamycin resistant colonies were cultured and plasmid DNA was isolated. The resulting construct was designated pET30/DeoC_RBS_YliI and sequenced for confirmation of the gene sequences. Organisms expressing DERA aldolase and quinoprotein glucose dehydrogenases were prepared by transforming BL21 (DE3) competent cells with the desctibed plasmid.

In the second case, the gene *gcd,* having not only additional ribosomal binding site (RBS) but also additional T7 promoter sequence, was added into the construct pET30/DeoC. The resulting vector is bearing two different coding sequences (one encoding DERA and the other encoding Gcd) under transcriptional control of IPTG inducible promoters.

Specifically, the plasmid DNA from *E. coli* JM109 pET30/Gcd (construction is described in Example 1) was isolated using Wizard Plus SV Minipreps DNA Purification Kit (Promega, Madison, WI, USA) according to manufacturer's instructions. Isolation of plasmid DNA was made using overnight culture of *E. coli* grown on LB medium at 37 °C.

The isolated plasmid DNA was used as a template in a PCR reaction to amplify sequence containing gene *gcd* and RBS sequence derived from expression vector pET30a(+) upstream of the coding region. PCR reaction was performed using oligonucleotide primers GCTGGCTCAGCCTCGATCCCGCGAAATTAATA and GCGCGCTCAGCGCAA GTCTTACTTCACATCATCCGGCAG Amplification of this fragment was performed by PCR using *Pfu* ULTRA II Fusion HS DNA 200 polymerase (Agilent, Santa Clara, CA, USA) as follows: an initial denaturation at 98 °C for 3 min, followed by 30 cycles of 20 s at 98 °C, 20 s at 55 °C, and 90 s at 72 °C. Final elongation was performed 180 s at 72 °C. The resulting fragments were separated by agarose gel electrophoresis and 1.2 kb fragment containing *gcd* and RBS site was purified. The product was transferred to plasmid pGEM T-Easy (Promega, Madison, WI, USA) and designated pGEM/T7p_RBS_gcd. The construct was cleaved with restriction endonuclease *Blp*I, then the resulting fragments were separated on agarose gel electrophoresis and 2.4 kb fragment containing *gcd*, RBS and T7 promoter sequence site was purified.
The construct pET30a/DeoC (construction is described in Example 5) was cleaved using the same aforementioned restriction endonuclease (*Blp*I) and purified. Shrimp Alkaline Phosphatase (SAP, purchased by Promega, Madison, WI, USA) was used to dephosphorylate the 5' phosphorylated ends of cleaved pET30a/DeoC according to manufacturer's instructions. Thus self-ligation of pET30a/DeoC was prevented. The fragments were assembled in a T4 ligase reaction. *E. coli* JM109 cells were transformed with the obtained ligation reactions and kanamycin resistant colonies were cultured and plasmid DNA was isolated. The resulting constructs was designated pET30/DeoC_T7p_RBS_Gcd and sequenced for confirmation of the gene sequences. The organism expressing DERA aldolase and quinoprotein glucose dehydrogenase was prepared by transforming BL21 (DE3) competent cells with the described plasmid.

Unless stated otherwise the expression of the enzymes encoded by the strains obtained by the above procedure was performed as described in Procedure 1A.

### Example 7: Preparation of E. coli able to synthesize pyrroloquinone quinone (PQQ)

*Gluconobacter oxydans* (ATCC 621H) gene cluster *pqqABCDE* (*pqqA, pqqB, pqqC, pqqD, pqqE,* GeneBank #CP000009, approximate location in the genome 1080978...1084164), which is involved in pyrroloquinoline quinone (PQQ) biosynthesis was expressed in *E. coli.*

The genomic DNA from *Gluconobacter oxydans* (ATCC 621H) was isolated using Wizard Genomic DNA Purification Kit (Promega, Madison, WI, USA) according to manufacturer's instructions. Isolation of genomic DNA was made using culture of *G. oxydans* grown on mannitol medium (10 g/L bacto yeast extract, 3 g/L peptone, 5 g/L mannitol, pH 7.0) 48 h at 26 °C. The said isolated genome was used as template for amplification of gene cluster *pqqABCDE* and its own promoter. Amplification was performed by PCR using oligonucleotide primers GCGCGGTACCGCACATGTCGCGGATGTTCAGGTGTTC (SEQ ID NO. 80) and GCGCGGATCCGGGCGGAGAGTTTGGAGAACCTCTTCA (SEQ ID NO. 81) and using *Pfu* ULTRA II Fusion HS DNA 200 polymerase (Agilent, Santa Clara, CA, USA) as follows: an initial denaturation at 95 °C for 2 min, followed by 30 cycles of 20 s at 95 °C, 20 s at 65 °C, and 120 s at 72 °C. Final elongation was performed 180 s at 72 °C. A 3.4-kb DNA fragment of G. *oxydans* bearing the *pqqABCDE* operon and parts of the upstream and downstream sequences was separated by agarose gel electrophoresis and purified. The 3.4-kb product (SEQ ID NO. 11) was ligated to plasmid pGEM T-Easy (Promega, Madison, WI, USA). *E. coli* JM109 cells were transformed with the obtained ligation reaction and ampicillin resistant colonies were cultured and plasmid DNA was isolated. The resulting plasmid construct was designated pGEMpqqA-E and sequenced for confirmation of the gene sequences. The cloning procedure was performed with a target to allow expression of genes under control of their native promoters and thus production of proteins PqqA, PqqB, PqqC, PqqD and PqqE derived from *G. oxydans* (SEQ ID NO. 12, 13, 14, 15, 16, respectively).

Synthesis of PQQ in *E. coli:* (Procedure 7A): LB medium (30 mL; 20 g/L Bacto LB broth) supplemented with ampicillin (100 µg/mL) was inoculated with a single colony *E. coli* JM109 pGEM/*pqq*A-E from a freshly streaked plate and pre-cultured to late log phase (37 °C, 250 rpm, 8 h). The pre-culture cells were pelleted by centrifugation (10 000 g, 10 min) and washed three times with 50 mM phosphate buffer (pH 7.0). Pre-culture cells were resuspended in the same aforementioned buffer at the same volume as in the original pre-culture. The suspension (inoculum size 5 %, v/v) was then transferred to glucose minimal medium (100 mL, 5 g/L D-glucose, 2 g/L sodium citrate 10 g/L K₂HPO₄, 3.5 g/L (NH₄)₂SO₄, pH 7.0). The culture was grown at 37 °C, 250 rpm, 48 h. The cell culture was supplemented with 1 mg/mL lysozyme solution. Lysis was performed at 37 °C, 1h. After lysis cell debris were removed by sedimentation (10 min, 20 000 g, 4 °C) to obtain a clear aqueous solution. PQQ comprised within a cell free extract was thus obtained.

Method for confirming PQQ production in *E. coli* is described in Example 13.

### Example 8: Preparation of aldose dehydrogenase enzyme comprised within a whole cell culture of a single microorganism able to synthesize pyrroloquinoline quinone (PQQ)

The expression plasmid bearing gene *yli*I and gene cluster *pqq*A-E was constructed.

Construct pET30/Ylil (preparation is described in Example 1) was digested with restriction endonuclease *Sph*I*.* Shrimp alkaline phosphatase (SAP, purchased by Promega, Madison, WI, USA) was used to dephosphorylate the 5' phosphorylated ends of cleaved pET30a/Ylil according to manufacturer's instructions. Thus self-ligation of pET30a/Ylil was prevented. A double stranded linker GCGCAAGCATGCGGATCCGGTACCAAGCTTGCATGCACACTA (SEQ ID NO. 82) (ordered at Invitrogen, Calsbad, CA, USA) containing double restriction recognition sites *Sph*I and recognition sites for restriction endonucleases *Bam*HI, *Kpn*I and *Hin*dIII was following cleaving with *SphI* introduced into the *Sph*I site on the digested construct pET30/Ylil.

The cleaved linker and cleaved construct were assembled in a T4 ligase reaction. The introduction of the linker inserts additional restriction endonucleases recognition sites into the plasmid. The construct pGEM/pqqA-E (according to Example 7) was cleaved with restriction endonucleases *Bam*HI and *Kpn*I, then the resulting fragments were separated on agarose gel electrophoresis and 3.4 kb fragment containing gene operon *pqqABCDE* was purified. Construct pET30/YliI with linker was cleaved using the aforementioned restriction endonucleases and purified. The fragments (cleaved pET30/Ylil with linker and *pqq*A-E, respectively) were assembled in a T4 ligase reaction. *E. coli* JM109 cells were transformed with the obtained ligation reaction and kanamycin resistant colonies were cultured and plasmid DNA was isolated. The resulting plasmid construct was designated pET30/YliI+*pqq*A-E and sequenced for confirmation of the gene sequences.

The organism able to express aldose dehydrogenase and meanwhile synthesize pyrroloquinoline quinone was prepared by transforming BL21 (DE3) competent cells with said plasmid.

Expression of *E. coli* Ylil and comprised within a whole cell culture of a single microorganism able to produce PQQ was undertaken as described in Procedure 1A. Corresponding to Procedures 1B and 1C living whole cell catalyst and resting whole cell catalyst, respectively, were prepared. Cell free lysate was prepared following procedure described in Example 2.

Method for confirming oxidation capability and PQQ production in *E. coli* is described in Example 13.

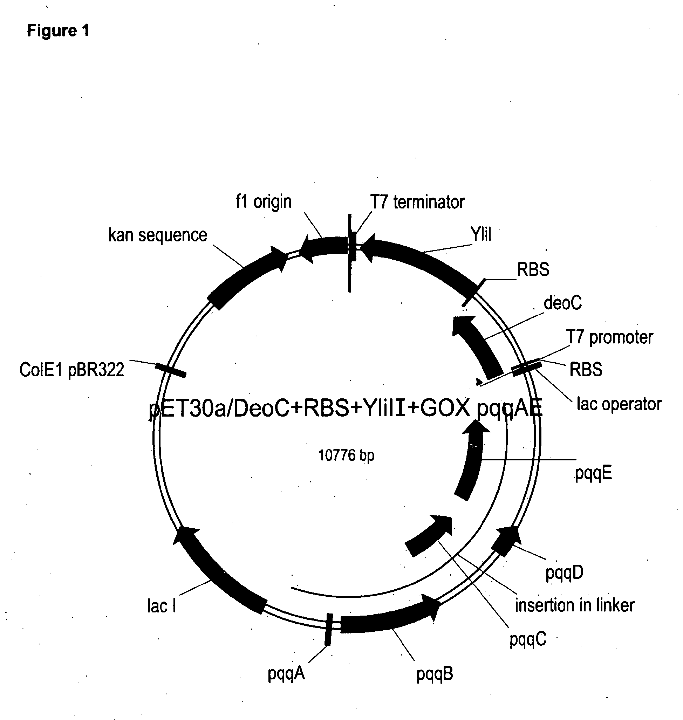

In addition a construct containing both DERA and aldose dehydrogenase Ylil, as well as PQQ biosynthetic cluster from Gluconobacter oxydans (ATCC 621H) was assembled. The procedure was performed in analogy to the above procedure, however the vector pET30/DeoC_RBS_Ylil (described in Example 6) was used as a starting point. The resulting plasmid was designated pET30/DeoC_RBS_Ylil+pqqA-E (Figure 1).

### Example 9: Preparation of Kluyvera intermedia whole cell catalyst

Cultivation of *Kluyvera intermedia* (ATCC 33421, formerly *Enterobacter intermedium*): VD medium (30 mL; 10 g/L bacto yeast extract, 5 g/L glycerol, 5 g/L NaCl, 4 g/L NaH₂PO₄·2H₂O, pH was adjusted with 1 M NaOH to pH=7.0) was inoculated with a single colony of *Kluyvera intermedia* from a freshly streaked VD agar plate and pre-cultured to late log phase (30 °C, 250 rpm, 8 h).

The pre-culture cells (inoculum size 10 %, v/v) were then transferred to fresh VD medium (100 mL; 10 g/L bacto yeast extract, 5 g/L glycerol, 5 g/L NaCl, 4 g/L NaH₂PO₄·2H₂O, pH was adjusted with 1 M NaOH to pH=7.0) .The cells were maintained at 30 °C, 250 rpm for 16h.

Preparation of whole cell catalysts (Procedure 1B): After cultivation cells were harvested by centrifugation (10 000 g, 5 min, 4 °C). Supernatant was discarded after centrifugation and pellet was resuspended in fresh VD medium (10 g/L bacto yeast extract, 5 g/L glycerol, 5 g/L NaCl, 4 g/L NaH₂PO₄·2H₂O, pH was adjusted with 1 M NaOH to pH=6.0). Cells were resuspended in one tenth of initial culture volume. The aldose dehydrogenase comprised within the living whole cell was thus obtained in fresh medium.

Alternatively (Procedure 1C), after discarding supernatant after centrifugation pellet was resuspended in phosphate buffer (50 mM KH₂PO₄, 150 mM NaCl, pH 6.0). Cells were resuspended in one tenth of initial culture volume. The aldose dehydrogenase comprised within the resting whole cell culture was thus obtained.

Cell free lysate was prepared by following the procedure described in Example 2.

### Example 10: Preparation of Gluconobacter oxydans whole cell catalyst

Cultivation of *Gluconobacter oxydans* (ATCC #621H): Mannitol medium (100 mL; 10 g/L bacto yeast extract, 3 g/L peptone, 5 g/L mannitol, pH was adjusted with 1 M NaOH to pH=7.0) was inoculated with a single colony *Gluconobacter oxydans* from a freshly streaked mannitol agar plate and cultured. The cells were maintained at 26 °C, 250 rpm for 48 h.

Preparation of whole cell catalysts (Procedure 1B): After cultivation cells were harvested by centrifugation (10 000 g, 5 min, 4 °C). Supernatant was discarded after centrifugation and pellet was resuspended in fresh VD medium (10 g/L bacto yeast extract, 5 g/L glycerol, 5 g/L NaCl, 4 g/L NaH₂PO₄·2H₂O, pH was adjusted with 1 M NaOH to pH=6.0). Cells were resuspended in one tenth of initial culture volume. The aldose dehydrogenase comprised within the living whole cell was thus obtained in fresh medium.

Alternatively (Procedure 1C), after discarding supernatant after centrifugation pellet was resuspended in phosphate buffer (50 mM KH₂PO₄, 150 mM NaCl, pH 6.0). Cells were resuspended in one tenth of initial culture volume. The aldose dehydrogenase comprised within the resting whole cell culture was thus obtained.

Cell free lysate was prepared following the procedure described in Example 2.

### Example 11: A method for determination of compound (II) oxidation/dehydrogenation activity using DCPIP as electron acceptor

This method is useful for determining aldose dehydrogenase activity (or activity of other dehydrogenases and/or oxidases). Accordingly the same method is used for screening and identifying enzymes and/or organisms capable of carrying out the reaction of oxidation/dehydrogenation of compound (II) resulting in compound (I).

Oxidation/dehydrogenation activity towards compound (II) can be measured using a living whole cell catalyst (regard to Procedure 1B), resting whole cell catalyst (regard to Procedure 1C), a lysate (preparation is described in Example 2), a periplasmic fraction (Example 3) or membrane fraction (Example 4) of any microorganism regardless of it being native or genetically modified microorganism. For practical purposes it will be understood hereinafter that a term "analyzed material" includes all preparations of catalysts as described in previous examples. The results obtained using different preparations are given separately in provided tables below.

For comparative studies cell density of tested microorganisms was quantified as wet weight in mg per mL of sample. The cells in a sample were separated from the broth by centrifugation (10 000 g, 10 min) and wet pellet was weighted. When lysate, periplasmic fraction or membrane fraction were used as a testing fraction, the data of wet cell weight of whole cell these fractions were derived from was taken into account.

1 mL of "analyzed material" was supplemented with 5 µM PQQ (Sigma Aldrich, Germany) and 10 mM MgCl₂ (Sigma Aldrich, Germany) and incubated at room temperature for 10 minutes. Prior to measurement pH value of "analyzed material" was adjusted to 8.0 with 1 M NaOH. Where stated otherwise (symbol "*" states for exception) the difference of measurement was pH value - more particularly, pH 6.0. Where stated, no additional, except intrinsic, PQQ was added into reaction mixture.

Screening method was performed in a 96-well microplate in order to screen for and identify "analyzed material" useful for converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in presence of artificial electron acceptor 2,6-dichlorobenzenone-indophenole (DCPIP) combined with phenazine methosulfate (PMS).

The reaction mixture (total volume was 200 µL) contained phosphate buffer pH 8.0 (or phosphate buffer pH 6.0, where stated), 100 mM substrate ((2S,4R)-4,6-dihydroxytetrahydro-2H-pyran-2-yl)methyl acetate), 1 mM DCPIP (Sigma Aldrich, Germany), 0.4 mM PMS (Sigma Aldrich, Germany). "Analyzed material" (50 µL) was added to the reaction mixture. Where needed (due to rapid completion of the reaction), the "analyzed material" was diluted in phosphate buffer pH 8.0 or phosphate buffer pH 6.0, where stated. All tested "analyzed materials" were made in triplicates. Useful range in which the assay is linear was found to be between 10 and 400 mAU/sec.

Method was performed with spectrophotometer Spectra Max Pro M2 (Molecular Devices, USA). Absorbance at 600 nm was measured every 15 seconds for 15 minutes at 28 °C. Results were collected and analyzed using software SoftMax Pro Data Acquisition & Analysis Software.

Activity of "analyzed material" and thus capability of carrying out the reaction of converting compound (II) to compound (I), specifically of converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate was defined as absolute value of reduction in absorbance unit per minute per wet weight of culture cells used for preparation of any "analyzed material" according to procedures 1C, 2, 3 or 4 present in the reaction mixture (abs[mAU min⁻¹ mg⁻¹]). Data are average values of three parallel measurements and are shown in a table below.

| | | **Resting whole cell catalyst (Procedure 1B)** | | **Lysate (Example 2)** | | **Periplasm fraction (Example 3)** | | **Membrane fraction (Example 4)** | |
|---|---|---|---|---|---|---|---|---|---|
| "analyzed material" | | without PQQ | with PQQ | without PQQ | with PQQ | without PQQ | with PQQ | without PQQ | with PQQ |
| 1* | *E. coli* BL21(DE3) pET30 (Example 1) | 90 | 270 | 72 | 90 | 75 | 130 | 99 | 355 |
| 2 | *E. coli* BL21(DE3) pET30/YliI (Example 1) | 88 | 1897 | 71 | 1913 | 72 | 1944 | 98 | 345 |
| 3* | *E. coli* BL21(DE3) pET30/Gcd (Example 1) | 87 | 2403 | 75 | 346 | 75 | 302 | 101 | 2707 |
| 4 | *E. coli* BL21(DE3) pET30/GdhB (Example 1) | 91 | 1703 | 88 | 1655 | 74 | 1622 | 97 | 353 |
| 5 | *E. coli* BL21(DE3) pET30/GdhB_therm (Example 1) | 88 | 1714 | 75 | 1687 | 72 | 1503 | 95 | 376 |
| 6* | *E. coli* BL21(DE3) pET30/DeoC (Example 5) | 89 | 275 | 78 | 135 | 77 | 112 | 96 | 345 |
| 7 | *E. coli* BL21(DE3) pET30/DeoC_RBS_Ylil (Example 6) | 92 | 1551 | 81 | 1581 | 75 | 1603 | 99 | 356 |
| 8* | *E. coli* BL21(DE3) pET30/DeoC_T7p_RBS_ Gcd (Example 6) | 89 | 1987 | 82 | 365 | 79 | 299 | 95 | 2104 |
| 9** | *E. coli* BL21(DE3) pET30/Ylil+pqqA-E (Example 8) | 301 | 1895 | 405 | 1902 | 455 | 1911 | 123 | 355 |
| 10 | *Kluyvera intermedia* (Example 9) | 1324 | 1330 | N.A. | N.A. | N.A. | N.A. | N.A. | N.A. |
| 11 | *Gluconobacter oxydans* (Example 10) | 1190 | 1205 | N.A. | N.A. | N.A. | N.A. | N.A. | N.A. |

| | | | | | | | | | |
|---|---|---|---|---|---|---|---|---|---|
| Legend: "*" - pH value of reactions was 6.0 "**"- no additional, except intrinsic, PQQ was added in column "without PQQ". | | | | | | | | | |

For negative controls, at least one component (except electron acceptor DCPIP combined with PMS) of reaction mixture was replaced with phosphate buffer pH 8.0 or pH 6.0 where appropriate. No discoloration was observed in all negative control wells even after prolonged incubation. When aldose dehydrogenase clear aqueous solution was replaced With phosphate buffer, no discoloration was observed. When any of the substrates (((2S,4R)-4,6-dihydroxytetrahydro-2H-pyran-2-yl)methyl acetate, glucose, galactose or glycerol) were replaced with phosphate buffer, no discoloration was observed. When PQQ was not provided to the reaction mixture by any means described in this invention (except in mixtures 9, 10 and 11 where PQQ is provided intrinsically), no discoloration was observed.

### Example 12: Determination of properties of aldose dehydrogenase contained in E. coli in a presence of DCPIP

a) For determination of activity of *E. coli* Ylil aldose dehydrogenases on other substrates and different concentrations, additional experiments were performed.
   50 µL of "analyzed material" (more particularly *E. coli* BL21. pET30/YliI reconstituted with 5, µL PQQ and 10 mM MgCl₂ and undertaking procedure 1B), 1 mM DCPIP, 0.4 mM PMS were placed in 96-well microtiter plate wells along with different concentrations (2.5-10 g/L) of substrates (((2S,4R)-4,6-dihydroxytetrahydro-2H-pyran-2-yl)methyl acetate, D-glucose and D-galactose). Final volume of reaction mixture was 200 µL, all components were dissolved in phosphate buffer pH 8.0.
   Discoloration of DCPIP was observed in these wells. The discoloration rate was about two times faster when ((2S,4R)-4,6-dihydroxytetrahydro-2H-pyran-2-yl)methyl acetate was used as substrate than with control wells containing D-glucose or D-galactose as a substrate.
   Discoloration was faster when 10 g/L of ((2S,4R)-4,6-dihydroxytetrahydro-2H-pyran-2-yl)methyl acetate used as substrate in contrast to 2.5 g/L said substrate. A slight substrate inhibition to the reaction rate was observed only when concentration of ((2S,4R)-4,6-dihydroxytetrahydro-2H-pyran-2-yl)methyl acetate in the reaction mixture was raised above 40 g/L.
   To estimate possible influence of the product ((2S,4R)-4-hydroxy-6-oxotetrahydro-2H-pyran-2-yl)methyl acetate on the reaction rate, various amounts of said product were added to the reaction mixture before the initiation of the reaction. Slight reduction in reaction rates were observed only when more than 35 g/L of ((2S,4R)-4-hydroxy-6-oxotetrahydro-2H-pyran-2-yl)methyl acetate was added in addition of 10 g/L of ((2S,4R)-4,6-dihydroxytetrahydro-2H-pyran-2-yl)methyl acetate.
   To investigate substrate specificity and pH optima of *E. coli* YliI aldose dehydrogenase the following experiments were performed:
   One reaction mixture (total volume was 200 µL) contained phosphate buffer pH 8.0, 50 mM substrate (((2S,4R)-4,6-dihydroxytetrahydro-2H-pyran-2-yl)methyl acetate, D-glucose or glycerol), 1 mM DCPIP, 0.4 mM PMS. 50 µL of "analyzed material" (described in detail further on), supplemented with 5 µM) PQQ (Sigma Aldrich, Germany) and 10 mM MgCl₂ was added to the mixture. The catalyst *E. coli* BL21 pET30/Ylil culture obtained either as resting whole cell catalyst (obtained undertaking Procedure 1C) or as lysate (as described in Example 2). The initial pH value of reaction mixture was 8.0. All tested solutions were made in triplicates.

   The second reaction mixture was prepared identically to the above with a sole difference: the pH value of assembled mixture being 6.0 (all compounds of reaction mixture were dissolved in phosphate buffer pH 6.0 and the initial pH value of reaction mixture was thus 6.0). All tested solutions were made in triplicates.
   Differentiation of aldose dehydrogenases and thus converting substrates at different optimums in said reaction mixtures was determined with spectrophotometer Spectra Max Pro M2 (Molecular Devices, USA). Absorbance at 600 nm was measured every 15 seconds for 15 minutes at 28 °C. Results were collected and analyzed using software SoftMax Pro Data Acquisition & Analysis Software. Experiment was performed as described in Example 11. In the table below are shown activities of "analyzed material" (abs[mAU min⁻¹ mg⁻¹]) 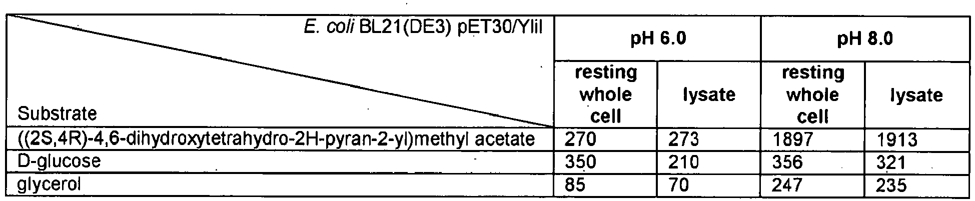
b) Further, the characteristics of structurally distinct, membrane bound glucose dehydrogenase from *E. coli,* the Gcd, were evaluated using the DCPIP method.

50 µL of "analyzed material" (more particularly *E. coli* BL21 pET30/Gcd reconstituted with 5 µL PQQ and 10 mM MgCl₂ and undertaking procedure 1B), 1 mM DCPIP, 0.4 mM PMS were placed in 96-well microtiter plate wells along with different concentrations (2.5-10 g/L) of substrates (((2S,4R)-4,6-dihydroxytetrahydro-2H-pyran-2-yl)methyl acetate, D-glucose and D-galactose). Final volume of reaction mixture was 200 µL, all components were dissolved in phosphate buffer pH 6.0.

Discoloration of DCPIP was observed in these wells. The discoloration rate was about two times slower when ((2S,4R)-4,6-dihydroxytetrahydro-2H-pyran-2-yl)methyl acetate was used as substrate than with control wells containing D-glucose or D-galactose as a substrate.

Discoloration was faster when 10 g/L of ((2S,4R)-4,6-dihydroxytetrahydro-2H-pyran-2-yl)methyl acetate used as substrate in contrast to 2.5 g/L said substrate. A slight substrate inhibition to the reaction rate was observed only when concentration of ((2S,4R)-4,6-dihydroxytetrahydro-2H-pyran-2-yl)methyl acetate in the reaction mixture was raised above 60 g/L.

To estimate possible influence of the product ((2S,4R)-4-hydroxy-6-oxotetrahydro-2H-pyran-2-yl)methyl acetate on the reaction rate, various amounts of said product were added to the reaction mixture before the initiation of the reaction. Slight reduction in reaction rates were observed only when more than 55 g/L of ((2S,4R)-4-hydroxy-6-oxotetrahydro-2H-pyran-2-yl)methyl acetate was added in addition of 10 g/L of ((2S,4R)-4,6-dihydroxytetrahydro-2H-pyran-2-yl)methyl acetate.

To investigate substrate specificity and pH optima of said *E. coli* membrane aldose dehydrogenase the above mentioned experiments were performed on the catalyst *E. coli* BL21 pET30/Gcd.

The results show that the overexpressed Gcd quinoprotein dehydrogenase in membrane is performing better at pH 6.0 when the substrate is ((2S,4R)-4,6-dihydroxytetrahydro-2H-pyran-2-yl)methyl acetate. Activity towards ((2S,4R)-4,6-dihydroxytetrahydro-2H-pyran-2-yl)methyl acetate is slightly slower than activity when glucose was used as substrate.

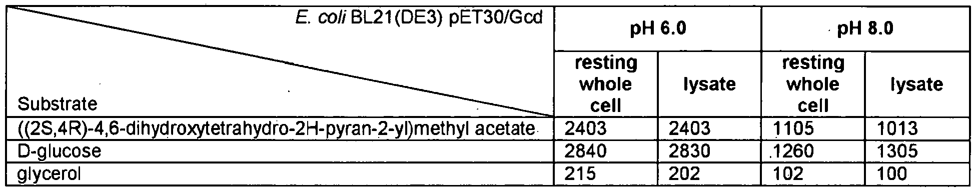

The results clearly show that distinct quinoprotein dehydrogenases, even if derived from the same organism (*E. coli*), may have completely different activity optima and other properties, which points out the need for individual characterization and optimization of reaction conditions for each individual enzyme.

### Example 13: Determination of whole cell catalysis capability in bioreactor using pO₂ sensor

Laboratory bioreactors Infors ISF100 with maximal volume of 2L were used for determination of whole cell catalysis capability of *E. coli* aldose dehydrogenases. The reactors were stirred, aerated, temperature and pH controlled as described below. When whole cell catalysts bearing holo aldose dehydrogenases were exposed to various substrates, unusual consumption of O₂ appeared. Using pO₂ sensor Hamilton OXYFERM FDA 225 PN 237452 the rate of pO₂ drop (oxygen consumption) in time after substrate was provided was found to correlate with the rate of whole cell catalysis capability.

Experiments were performed using whole cell catalyst *E. coli* BL21 pET30/Gcd (procedure of preparation in described in Example 1, Procedure 1A and moreover in Example 25).

Whole cell catalysts were dissolved in phosphate buffer (50 mM KH₂PO₄, 150 mM NaCl, pH 6.2) to concentrations of 5 g/L, 10 g/L and 20 g/L to the final volume (1L) in the bioreactor.

The initial process parameters before substrate was added were as follows: 37 °C, air flow rate 1.0 L/min (1.0 VVM), stirrer speed 1000 rpm, pH 6.2 and the dissolved oxygen concentration was kept at ≥ 80 % of saturation. 5 µM PQQ and 10 mM MgCl₂ were provided into bioreactor broth. Feeding solution were 12.5 % (v/v) ammonium hydroxide solution and the silicone antifoam compound synperonic antifoam (Sigma, A-5551) which were fed continuously.

Substrates (D-glucose and ((2S,4R)-4,6-dihydroxytetrahydro-2H-pyran-2-yl)methyl acetate) were added in one-shot in concentrations 0.25 g/L, 0.5 g/L and 1 g/L. When substrates were provided in the broth, oxygen consumption was followed in time.

The system described above is very complex in terms of mathematical description of dissolved oxygen levels over the time. On the one hand these are depending on the k_{La} (vessel properties, stirring, aeration, medium, temperature etc) and enzymatic kinetics oh the aldose dehydrogenase on the other hand. In addition the oxygen probe delay kinetics play an important role. We have therefore found empirically, that oxygen consumption in the phase after the pulse feed of the substrate till the minimum of the dissolved oxygen in this dynamic system is best correlated to quadratic equation. A good indicator of the activity potential of the studied biocatalyst is the slope of this quadratic equation, measured at specific time value (for example t=15 seconds). The slope was calculated from the first derivative of the quadratic equation. This calculation procedure, showed a good reproducibility as well as linearity of the method

| | Concentration of whole cell catalyst BL21 (DE3) pET30/Gcd | | |
|---|---|---|---|
| | 5 g/L | 10 g/L | 20 g/L |
| D-glucose | 45813 | 114771 | 230631 |
| 2S,4R)-4,6-dihydroxytetrahydro-2H-pyran-2-yl)methyl acetate | 38652 | 87425 | 163562 |

### Example 14: Reconstitution of holo-enzyme aldose dehydrogenase with PQQ from various sources and assay with DCPIP as electron acceptor

There are different possibilities of construction of holoenzyme with providing PQQ and appropriate divalent cations such as Mg²⁺ and Ca²⁺ to PQQ-dependent aldose dehydrogenases *in situ* and thus obtaining an active aldose dehydrogenase. For determination we used method with artificial electron acceptor (DCPIP combined with PMS) as described in Example 11.

To the whole cell catalysts *E. coli* BL21(DE3) pET30 or to whole cell catalysts *E. coli* BL21(DE3) pET30/Ylil, *E. coli* BL21(DE3) pET30/Gcd, *E. coli* BL21(DE3) pET30/GdhB, *E. coli* BL21(DE3) pET30/GdhB_therm, *E. coli* BL21(DE3) pET30/DeoC, *E. coli* BL21(DE3) pET30/DeoC_RBS_Ylil and pET30/DeoC_T7p_RBS_Gcd prepared according to Procedure 1B or 1C PQQ could be supplied as:
- Procedure 14A: 5 µM PQQ (Sigma Aldrich, Germany), 10 mM MgCl₂ (Sigma Aldrich, Germany) and phosphate buffer pH 6.0 (5 mL) was added to living whole cell catalysts or to resting whole cell catalysts (5 mL) and incubated 10 min at room temperature.
- Procedure 14B: 5 mL of supernatant of cultivated *E. coli* JM109 pGEM/*pqq*A-E (lysate was obtained as described in Procedure 7D) supplemented whole cell catalysts or resting cell catalysts (5 mL) in presence of 10 mM MgCl₂ and incubated 10 min at room temperature.
- Procedure 14C: 5 mL of supernatant of cultivated *Klyuvera intermedia* or *Gluconobacter oxydans* (cultivation was described in Example 10 and Example 11, respectively) supplemented whole cell catalysts or resting cell catalysts (5 mL). Supernatant was obtained after centrifugation of cultures (10 000 g, 5 min, 4 °C) and incubated 10 min at room temperature in presence of 10 mM MgCl₂ in suspension.

Living whole cell catalysts or resting whole cell catalysts *E. coli* BL21(DE3) pET30/Ylil+pqqA-E which have undertaken Procedures 1B or 1C (5 mL) were supplemented with phosphate buffer pH=6.0 and 10 mM MgCl₂ in same volume ratio and incubated 10 min at room temperature.

All reaction mixtures were tested in a 96-well microplate for their ability of converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in presence of artificial electron acceptor DCPIP undertaking procedure as described in Example 11.

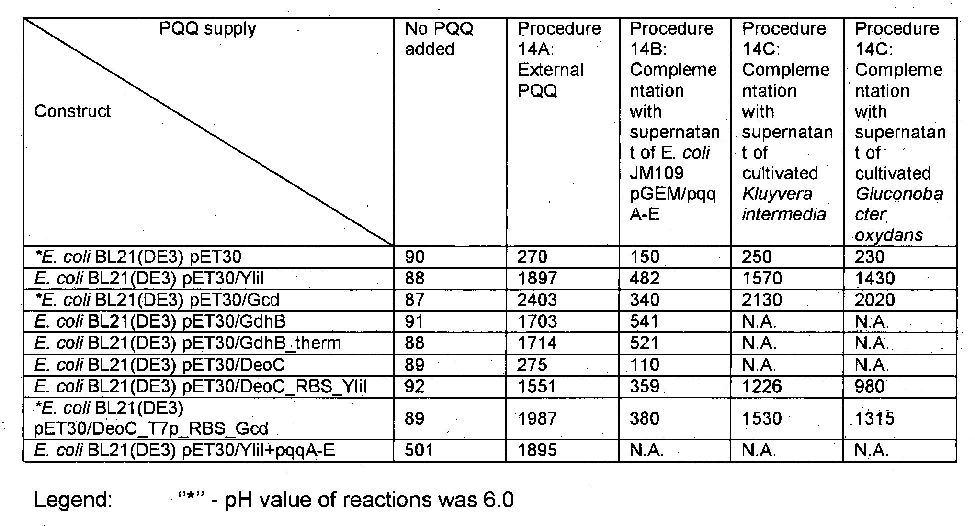

Addition of 5 µM PQQ and 10 mM MgCl₂ at the time of induction of expression of plasmids pET30, pET30/Ylil, pET30/Gcd, pET30/GdhB, pET30a/GdhB_therm in *E. coli* BL21(DE3) cells (following Procedure 1A) revealed similar results as addition immediately before the reaction.

### Example 15: Oxidation of lactol ((2S,4R)-4,6-dihydroxytetrahydro-2H-pyran-2-yl)methyl acetate with various aldose dehydrogenases comprised within living whole cell catalysts

Various living whole cell catalysts with aldose dehydrogenases (variety of them is described in Examples 1, 7, 8, 9 and 10, respectively) were tested for bioconversion of ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate.

Living whole cell catalysts *E. coli* BL21(DE3) pET30a, *E. coli* BL21(DE3) pET30a/Ylil, *E. coli* BL21(DE3) pET30a/Gcd, *E. coli* BL21(DE3) pET30a/GdhB, *E. coli* BL21(DE3) pET30a/GdhB_therm (preparation is described in Example 1) were prepared as described in Procedure 1B. 9 mL samples of aforementioned living whole cell catalysts were transferred to 100 mL Erlenmeyer flasks, where the reaction was performed. Reaction mixture was supplemented with 1 µM (10 µL of 1 mM pre-prepared stock of PQQ, Sigma) and 10 mM MgCl₂ (2.5 µL of 4 M pre-prepared stock of MgCl₂, Sigma).

Living whole cell catalyst *E. coli* BL21(DE3) pET30a/Ylil+pqqA-E (preparation is described in Example 7) were prepared as described in Procedure 1B. 9 mL samples of aforementioned cells were transferred to 100 mL Erlenmeyer flasks, where the reaction was performed.

Living whole cell catalysts *Kluyvera intermedia* (Example 10) and *Gluconobacter oxydans* (Example 11) were prepared as described in Procedure 1B. 9 mL samples of aforementioned cells were transferred to 100 mL Erlenmeyer flasks, where the reaction was performed.

((2*S*,4*R*)-4,6-dihydroxytetrahydro-2H-pyran-2-yl)methyl acetate was dissolved in phosphate buffer (50 mM KH₂PO₄, 150 mM NaCl, pH 6.0) to concentration of 2 mol/L. All samples of concentrated living whole cell catalyst were supplemented with 1 mL 2 mol/L ((2S,4R)-4,6-dihydroxytetrahydro-2H-pyran-2-yl)methyl acetate. Reaction was performed at 37 °C, 250 rpm for 6 hours on a rotary shaker. At time 0', 60', 120', 180', 240', 300' and 360', samples were taken. Each sample was diluted 50-times with acetonitrile for GC-MS analysis. Results at time 0', 180' and 360' are shown in the table below.

The major product of the reaction had identical retention time and ion distribution as ((2S,4R)-4-hydroxy-6-oxotetrahydro-2H-pyran-2-yl)methyl acetate obtained by chemical oxidation as described in the art.

After 360 min reaction mixture was extracted five times with equal volume of ethyl acetate and all organic fractions were collected and dried over rotavapor. Finally a yellow oil was obtained.

The structure of resulting product was confirmed by NMR and is identical to what is reported in the art. ¹H NMR (300 MHz, acetone-d₆) δ 4.88 (m, 1H), 4.45 (d, *J* = 3.0 Hz, 1H), 4.38 (hex, *J* = 3.0 Hz*,* 1H), 4.23 (dd, *J* = 3.5 Hz, *J* = 12.0 Hz, 1H), 4.16 (dd, *J* = 5.5 Hz, *J* = 12.1 Hz, 1H), 2.68 (dd, *J* = 4.3 Hz, *J* = 17.5 HZ, 1H), 2.51 (dddd, *J* = 0.8 Hz, *J* = 2.0 Hz, *J* = 3.3 Hz, *J* = 17.5 Hz, 1H), 2.03 (s, 3H), 1.91 (m, 2H), ¹³C NMR (75 MHz, acetone-d₆) δ 170.8, 169.7, 74.2, 66.5, 62.7, 39.1, 32.3, 20.6.

| | Time [min] | | | | | |
|---|---|---|---|---|---|---|
| | 0' | | 180' | | 360' | |
| Living whole cell catalyst | ((2S,4R)-4,6-dihydroxytet rahydro-2H-pyran-2-yl)methyl acetate | **((2*S*,4*R*)-4-hydroxy-6-oxotetrahy dro-2H-pyran-2-yl)methyl acetate** | ((2S,4R)-4,6-dihydroxytet rahydro-2H-pyran-2-yl)methyl acetate | **((2*S*,4*R*)-4-hydroxy-6-oxotetrahy dro-2H-pyran-2-yl)methyl acetate** | ((2S,4R)-4,6-dihydroxytet rahydro-2H-pyran-2-yl)methyl acetate | **((2*S*,4*R*)-4-hydroxy-6-oxotetrahy dro-2H-pyran-2-yl)methyl acetate** |
| *E. coli* BL21(DE3) pET30 | 38.20 g/L | 0.00 g/L | 19.17 g/L | 4.26 g/L | 10.12 g/L | 12.01 g/IL |
| *E. coli* BL21(DE3) pET30/Ylil | 37.880 g/L | 0.00 g/L | 13.01 g/L | 15.88 g/L | 1.65 g/L | 25.07 g/L |
| *E. coli* BL21(DE3) pET30/Gcd | 38.34 g/L | 0.00 g/L | 6.12 g/L | 21.74 g/L | 1.08 g/L | 28.65 g/L |
| *E. coli* BL21(DE3) pET30/GdhB | 38.10 g/L | 0.00 g/L | 12.88g/L | 14.01 g/L | 1.15 g/L | 23.15 g/L |
| *E. coli* BL21(DE3) pET30/GdhB_therm | 38.28 g/L | 0.00 g/L | 12.10 g/L | 14.52 g/L | 1.01 g/L | 24.95 g/L |
| *E. coli* BL21(DE3) pET30/Ylil+pqqA-E | 37.87g/L | 0.00 g/L | 16.55 g/L | 10.87 g/L | 6.15 g/L | 15.55 g/L |
| *Kluyvera intermedia* | 38.04 g/L | 0.00 g/L | 12.11 g/L | 13.85 g/L | 1.70 g/L | 23.45 g/L |
| *Gluconobacter oxydans* | 38.23 g/L | 0.00 g/L | 13.66 g/L | 13.12 g/L | 1.23 g/L | 25.56 g/L |

*E. coli* BL21(DE3) pET30 was used as negative control. Reaction was held at the same conditions as described above and at the end of the reaction small amount of ((2S.4R)-4-hydroxy-6-oxotetrahydro-2H-pyran-2-yl)methyl acetate was observed. However, all of the provided ((2*S*,4*R*)-4,6-dihydroxytetrahydro-2H-pyran-2-yl)methyl acetate did not remain untouched. The reason of conversion is activation of endogenous quinoprotein dehydrogenases (YliI and Gcd) present in *E*. *coli* forming holoenzyme when PQQ and MgCl₂ were provided.

### Example 16: Sequential reaction using DERA aldolase and aldose dehydrogenase (Ylil or Gcd) for the production ((2S.4R)-4-hydroxy-6-oxotetrahydro-2H-pyran-2-yl)methyl acetate from acetyloxyacetaldehyde and acetaldehyde

Living whole cell catalyst *E. coli* BL21(DE3) pET30/DeoC was prepared as described in Example 5 according to Procedure 1B. 5 mL of living whole cell catalyst was transferred to 100 mL Erlenmeyer flask.

Stock solution of acetyloxyacetaldehyde and acetaldehyde was prepared. 600.5 mg acetyloxyacetaldehyde (chemical synthesis of said compound was performed in our laboratory and revealed 85 % purity) and 467.2 mg acetaldehyde (purchased by Fluka, USA) were dissolved in ice cold phosphate buffer pH 6.0 to final volume 10 mL.

At time 0' 1 mL of said stock solution of acetyloxyacetaldehyde and acetaldehyde was added into reaction mixture. Reaction was performed at 37 °C, 250 rpm for 3 hours on a rotary shaker.

5 mL of living whole cell catalyst *E. coli* BL21(DE3) pET30/YliI (preparation is described in Example 1 according to Procedure 1B) supplemented with 1 µM PQQ (Sigma Aldrich. Germany) and 10 mM MgCl₂ (Sigma Aldrich. Germany) was added to the same Erlenmeyer flask after 3 hours. The oxidation reaction was left for additional 3 hours at 37 °C, 250 rpm on a rotary shaker.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art. After 360' 12.3 g/L of ((2*S*.4*R*)-4-hydroxy-6-oxotetrahydro-2H-pyran-2-yl)methyl acetate was observed which represents 64 % molar yield.

The same procedure as described above was performed using living whole cell catalyst *E*. *coli* BL21(DE3) pET30/Gcd (preparation is described in Example 1 according to Procedure 1B).After 360' od addition of 5ml of the living whole cell catalyst *E. coli* BL21(DE3) pET30/Gcd living whole cell catalyst to the reaction mixture with *E. coli* BL21(DE3) pET30/DeoC 14.45 g/L of ((2*S*.4*R*)-4-hydroxy-6-oxotetrahydro-2H-pyran-2-yl)methyl acetate was observed which represents 75 % molar yield.

### Example 17: Simultaneous reaction using DERA aldolase and aldose dehydrogenase (Gcd) for the production ((2S.4R)-4-hydroxy-6-oxotetrahydro-2H-pyran-2-yl)methyl acetate from acetyloxyacetaldehyde and acetaldehyde

Living whole cell catalysts *E*. *coli* BL21(DE3) pET30/Gcd and *E. coli* BL21(DE3) pET30/DeoC (preparation is described in Examples 1 and 5, respectively) were prepared as described in Procedure 1B. Both living whole cell catalysts were transferred to 100 mL Erlenmeyer flask in the same volume ratio (5 mL).

We set up two different reactions, where both living whole cell catalysts were present. First reaction mixture was supplemented with 5 µM PQQ (Sigma Aldrich, Germany) and 10 mM MgCl₂ (Sigma Aldrich, Germany), while the second one was used as a control where only apo aldose dehydrogenase enzyme was present.

Stock solution of acetyloxyacetaldehyde and acetaldehyde was prepared. 1.201 g acetyloxyacetaldehyde (chemical synthesis of said compound was performed in our laboratory and revealed 85 % purity) and 934.4 mg acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixture. Final concentrations of acetyloxyacetaldehyde and acetaldehyde were 100 mM and 210 mM, respectively. Reaction was performed at 37 °C, 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

In first reaction mixture, where holo dehydrogenase was present, after 360' 13.6 g/L of ((2*S*,4*R*)-4-hydroxy-6-oxotetrahydro-2H-pyran-2-yl)methyl acetate was observed which represents 71 % molar yield. After 360' only small amount of lactol ((2S,4R)-4,6-dihydroxytetrahydro-2H-pyran-2-yl)methyl acetate was observed, however substrates (acetaldehyde and acetyloxyacetaldehyde) and intermediate ((*S*)-(4-hydroxyoxtetan-2-yl)methyl acetate) were still present at the time.

At the same time, in the second reaction mixture, where only apo-dehydrogenase was present, and thus having only DERA aldolase active in the reaction produced only 9.55 g/L of lactol lactol ((2S,4R)-4,6-dihydroxytetrahydro-2H-pyran-2-yl)methyl acetate.

These indications show that the reaction step by the aldose dehydrogenase (Gcd) is faster than the step catalyzed by the aldolase enzyme (DERA) and that presence of aldose dehydrogenase shifts the steady state equilibrium of the DERA reaction step towards the product and simultaneous reaction with aldose dehydrogenase (Gcd) in fact improves overall rates toward the lactone (compound I).

### Example 18: Simultaneous reaction comprising DERA aldolase and aldose dehydrogenase (YliI or Gcd) within a living whole cell catatyst of a single microorganism for the production of ((2S,4R)-4-hydroxy-6-oxotetrahydro-2H-pyran-2-yl)methyl acetate from acetyloxyacetaldehyde and acetaldehyde

The below example provides a synthetic biological pathway provided within a living microorganism which is capable of producing highly enantiomerically pure ((2S,4R)-4-hydroxy-6-oxotetrahydro-2H-pyran-2-yl)methyl acetate from simple and inexpensive molecules: acetyloxyacetaldehyde and acetaldehyde.

Living whole cell catalysts *E. coli* BL21(DE3) pET30/DeoC_RBS_YliI and *E*. *coli* BL21(DE3) pET30/DeoC_T7p_RBS_Gcd (preparation is described in Example 6) was prepared as described in Procedure 1A. Said whole cell catalysts were separately concentrated by centrifugation (5 000 g, 10 min) and pellet was resuspended in one tenth of initial volume of the same supernatant. Exceeded supernatant was discarded. 10 mL of said concentrated living whole cell catalyst was transferred to 100 ml Erlenmeyer flask. To the cell broth were suplemented with 5 µM PQQ and 10 mM MgCl₂ and pH value of the broth was adjusted to 6.0 with ammonium solution.

Stock solution comprised of acetyloxyacetaldehyde and acetaldehyde was prepared. 1.201 g acetyloxyacetaldehyde (chemical synthesis of said compound was performed in our laboratory and revealed 85 % purity) and 934.4 g acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixtures. Final concentrations of acetyloxyacetaldehyde and acetaldehyde in reaction mixture were 100 mM and 210 mM, respectively. Reaction was performed at 37 °C, 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

After 360' 7.80 g/L of ((2*S*,4*R*)-4-hydroxy-6-oxotetrahydro-2H-pyran-2-yl)methyl acetate was observed which represents 41 % molar yield when *E. coli* BL21(DE3) pET30/DeoC_RBS_YliI were present as catalysts. At the same time, when reaction was performed in presence of *E*. *coli* BL21(DE3) pET30/DeoC_T7p_RBS_Gcd after 360' 13,12 g/L of ((2S,4R)-4-hydroxy-6-oxotetrahydro-2H-pyran-2-yl)methyl acetate was observed which represents 69 % molar yield.

### Example 19: Simultaneous reaction comprising DERA aldolase and living whole cell catalyst Kluyvera intermedia for the production ((2S,4R)-4-hydroxy-6-oxotetrahydro-2H-pyran-2-yl)methyl acetate from acetyloxyacetaldehyde and acetaldehyde

Living whole cell catalysts *E. coli* BL21(DE3) pET30/DeoC and *Kluyvera intermedia* (preparation is described in Examples 1 and 11, respectively) were prepared as described in Procedure 1B. Both living whole cell catalysts were transferred to 100 mL Erlenmayer flask in the same volume ratio (5 mL).

Stock solution of acetyloxyacetaldehyde and acetaldehyde was prepared. 1.201 g acetyloxyacetaldehyde (chemical synthesis of said compound was performed in our laboratory and revealed 85 % purity) and 934.4 mg acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixture. Final concentrations of acetyloxyacetaldehyde and acetaldehyde in reaction mixture were 100 mM and 210 mM, respectively. Reaction was performed at 37 °C, 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

After 360' 10.40 g/L of ((2*S*,4*R*)-4-hydroxy-6-oxotetrahydro-2H-pyran-2-yl)methyl acetate was observed which represents 54 % molar yield.

### Example 20: Simultaneous reaction using DERA aldolase and aldose dehydrogenase (Yli or Gcd) for the production of ((4R,6S)-6-(chloromethyl)-4-hydroxytetrahydro-2H-pyran-2-one)

Living whole cell catalysts *E*. *coli* BL21(DE3) pET30/YliI, *E. coli* BL21(DE3) pET30/Gcd and *E. coli* BL21(DE3) pET30/DeoC (preparation is described in Examples 1 and 5, respectively) were prepared as described in Procedure 1B. 6 mL of living whole cell catalyst *E. coli* BL21(DE3) pET30/DeoC and 3 mL of *E. coli* BL21(DE3) pET30/YliI or *E. coli* BL21(DE3) pET30/Gcd were transferred to 50 mL polystyrene conical tubes (BD Falcon, USA). Reaction mixture was supplemented with 1 µM PQQ (Sigma Aldrich, Germany) and 10 mM MgCl₂.

Stock solution of chloroacetaldehyde and acetaldehyde was prepared. 817 µL of chloroacetaldehyde solution (50 wt. % in H₂O, purchased by Aldrich, USA) and 500.6 mg acetaldehyde (purchased by Fluka. USA) were dissolved in ice cold phosphate buffer pH 6.0 to final volume 5 mL.

At time 0' 1 mL of said stock solution of chloroacetaldehyde and acetaldehyde was added into reaction mixture - total reaction volume was thus 10 mL and starting concentrations of chloroacetaldehyde and acetaldehyde in reaction mixture were 100 mM and 225 mM, respectively. Reaction was performed for 2 hours at 37 °C, 200 rpm of shaking in water bath.

The progress of the reaction was monitored with gas chromatography. After 120 min reaction mixture was extracted three times with equal volume of ethyl acetate and all organic fractions were collected and dried over rotavapor. 66 mg (36.2 % yield) of dried product (4*R*,6*S*)-6-(chloromethyl)-4-hydroxytetrahydro-2*H*-pyran-2-one remained after reaction combining *E*. *coli* BL21(DE3) pET30/Ylil and *E. coli* BL21(DE3) pET30/DeoC, and 72mg (39% yield) of dried product remained after reaction combining *E. coli* BL21(DE3) pET30/Gcd and *E*. *coli* BL21(DE3) pET30/DeoC. The products were analyzed with NMR. ¹H NMR (300 MHz, CDCl₃) δ 5.01 (m, 1H), 4.47 (m, 1H), 3.80 (m, 1H), 3.68 (m, 1H), 2.69 (d, *J* = 3.6 Hz, 2H), 2.09 (m, 1H), 1.97 (m, 1H). ¹³C NMR (75 MHz, CDCl₃) δ 170.2, 74.8, 62.5, 46.6, 38.5, 32.8.

### Example 21: Simultaneous reaction using DERA aldolase and aldose dehydrogenase (Ylil or Gcd) for the production of ((4R,6S)-6-(dimethoxymethyl)-4-hydroxytetrahydro-2H-pyran-2-one)

Living whole cell catalysts E. coli BL21(DE3) pET30/Ylil, E. coli BL21(DE3) pET30/Gcd and E. coli BL21(DE3) pET30/DeoC (preparation is described in Examples 1 and 5, respectively) were prepared as described in Procedure 1B. 6 mL of living whole cell catalyst E. coli BL21(DE3) pET30/DeoC and 3 mL of E. coli BL21(DE3) pET30/YliI or E. coli BL21(DE3) pET30/Gcd were transferred to 50 mL polystyrene conical tubes (BD Falcon, USA). Reaction mixture was supplemented with 1 µM PQQ (Sigma Aldrich, Germany) and 10 mM MgCl2.

Stock solution of dimethoxyacetaldehyde and acetaldehyde was prepared. 868 µL of 2,2-dimethoxyacetaldehyde solution (60 wt. % in H₂O. purchased by Fluka. USA) and 500.6 mg acetaldehyde (purchased by Fluka, USA) were dissolved in ice cold phosphate buffer pH 6.0 to final volume 5 mL.

At time 0' 1 mL of said stock solution of dimethoxyacetaldehyde and acetaldehyde was added into reaction mixture - total reaction volume was thus 10 mL and starting concentrations of dimethoxyacetaldehyde and acetaldehyde in reaction mixture were 100 mM and 225 mM, respectively. Reaction was performed for 2 hours at 37 °C, 200 rpm of shaking in water bath.

The progress of the reaction was monitored with gas chromatography. After 120 min reaction mixture was extracted three times with equal volume of ethyl acetate and all organic fractions were collected and dried over rotavapor. 53 mg (25.3 % yield) of dried product (4*R*,6*S*)-6-(dimethoxymethyl)-4-hydroxytetrahydro-2*H*-pyran-2-one remained after reaction combining E. coli BL21(DE3) pET30/Ylil and E. coli BL21(DE3) pET30/DeoC, and 67mg (31.9% yield) of dried product remained after reaction combining E. coli BL21(DE3) pET30/Gcd and E. coli BL21(DE3) pET30/DeoC. The products were analyzed with NMR. ¹H NMR (300 MHz, CDCl₃) δ 4.74 (quint, *J* = 3.5 Hz, 1H), 4.43 (m, 2H), 3.49 (s, 3H), 3.47 (s, 3H), 3.10 (br s, 1H), 2.72 (dd, *J* = 3.5 Hz, *J* = 17.8 Hz, 2H), 2.62 (m, 2H),1.99 (m, 2H).

### Example 22: Simultaneous reaction using DERA aldolase and aldose dehydrogenase (Ylil or Gcd) for the production of ((4R,6S)-6-(benzyloxymethyl)-4-hydroxytetrahydro-2H-pyran-2-one)

Living whole cell catalysts E. coli BL21(DE3) pET30/Ylil, E. coli BL21(DE3) pET30/Gcd and E. coli BL21(DE3) pET30/DeoC (preparation is described in Examples 1 and 5, respectively) were prepared as described in Procedure 1B. 6 mL of living whole cell catalyst E. coli BL21(DE3) pET30/DeoC and 3 mL of E. coli BL21(DE3) pET30/Ylil or E. coli BL21(DE3) pET30/Gcd were transferred to 50 mL polystyrene conical tubes (BD Falcon, USA). Reaction mixture was supplemented with 1 µM PQQ (Sigma Aldrich, Germany) and 10 mM MgCl2.

Stock solution of benzyloxyacetaldehyde and acetaldehyde was prepared. 774.1 mg of benzyloxyacetaldehyde (purchased by Fluka, USA) and 500.6 mg acetaldehyde (purchased by Fluka, USA) were dissolved in ice cold phosphate buffer pH 6.0 to final volume 5 mL.

At time 0' 1 mL of said stock solution of benzyloxyacetaldehyde and acetaldehyde was added into reaction mixture - total reaction volume was thus 10 mL and starting concentrations of benzyloxyacetaldehyde and acetaldehyde in reaction mixture were 100 mM and 225 mM, respectively. Reaction was performed for 2 hours at 37 °C. 200 rpm of shaking in water bath.

The progress of the reaction was monitored with gas chromatography. After 120 min reaction mixture was extracted three times with equal volume of ethyl acetate and all organic fractions were collected and dried over rotavapor. 51 mg (19.6 % yield) of dried product (4*R*,6*S*)-6-(benzyloxymethyl)-4-hydroxytetrahydro-2*H*-pyran-2-one remained after reaction combining E. coli BL21(DE3) pET30/Ylil and E. coli BL21(DE3) pET30/DeoC, and 56 mg (21.5% yield) of dried product remained after reaction combining E. coli BL21(DE3) pET30/Gcd and E. coli BL21(DE3) pET30/DeoC. The crude products were dissolved in dichloromethane and reacted with TBDMSCI and imidazole for 24 h. Solution was concentrated and purified by chromatography to give TBDMSCI protected compound ((4*R*,6*S*)-6-(benzyloxymethyl)-4-hydroxytetrahydro-2*H*-pyran-2-one), which was analyzed with NMR. ¹H NMR (500 MHz, CDCl₃) δ 7.36 (m, 5H), 5.17 (s, 2H), 4.93 (quint, *J* = 4.7 Hz, 1 H), 4.38 (dd, *J* = 3.4 Hz, *J* = 11.8 Hz, 1H), 4.35 (quint, *J* = 3.3 Hz, 1H), 4.27 (dd, *J* = 4.7 Hz, *J* = 11.8 Hz, 1H), 2.58 (d, *J* = 3.3 Hz, 2H), 1.85 (m, 2H), 0.88 (s, 9H), 0.09 (s, 3H), 0.08 (s, 3H), ¹³C NMR (125 MHz, CDCl₃) δ 154.8, 134.8, 128.7, 128.6, 128.4, 73.2, 70.0, 68.8, 63.2, 39.0, 32.2, 25.6, 17.8, - 5.0.

### Example 23: Simultaneous reaction using DERA aldolase and aldose dehydrogenase (Ylil or Gcd) for the production of ((4R,6R)-4-hydroxy-6-methyltetrahydro-2H-pyran-2-one)

Living whole cell catalysts E. coli BL21(DE3) pET30/Ylil, E. coli BL21(DE3) pET30/Gcd and E. coli BL21(DE3) pET30/DeoC (preparation is described in Examples 1 and 5, respectively) were prepared as described in Procedure 1B. 6 mL of living whole cell catalyst E. coli BL21(DE3) pET30/DeoC and 3 mL of E. coli BL21(DE3) pET30/Ylil or E. coli BL21(DE3) pET30/Gcd were transferred to 50 mL polystyrene conical tubes (BD Falcon, USA). Reaction mixture was supplemented with 1 µM PQQ (Sigma Aldrich, Germany) and 10 mM MgCl2.

Stock solution of acetaldehyde was prepared. 445 mg of acetaldehyde (purchased by Fluka, USA) was dissolved in ice cold phosphate buffer pH 6.0 to final volume 5 mL.

At time 0' 1 mL of said stock solution of acetaldehyde was added into reaction mixture - total reaction volume was thus 10 mL and its final concentration in reaction mixture was 200 mM. Reaction was performed for 2 hours at 37 °C, 200 rpm of shaking in water bath.

The progress of the reaction was monitored with gas chromatography. After 120 min reaction mixture was extracted three times with equal volume of ethyl acetate and all organic fractions were collected and dried over rotavapor. 99 mg (34.1 % yield) of dried product (4*R*,6*R*)-4-hydroxy-6-methyltetrahydro-2*H*-pyran-2-one remained after reaction combining E. coli BL21(DE3) pET30/Ylil and E. coli BL21(DE3) pET30/DeoC, and 110 mg (37.9.% yield) of dried product remained after reaction combining E. coli BL21(DE3) pET30/Gcd and E. coli BL21(DE3) pET30/DeoC. The products were analyzed with NMR. ¹H NMR (300 MHz, acetone-d₆) δ 4.76 (dq, *J*_{d} = 11.2 Hz, *J*_{q} = 3.2 Hz, 1H), 4.41-4.15 (m, 2H), 2.63 (dd, *J* = 4.3 Hz, *J* = 17.0 Hz, 1H), 2.46 (ddd, *J* = 1.7 Hz, *J* = 3.3 Hz, *J* = 17.0 Hz, 1H), 1.92 (m, 1H), 1.71 (dd, *J* = 3.0 Hz, *J* = 14.3 Hz, 1H), 1.29 (d, *J* = 6.4 Hz, 3H). ¹³C NMR (75 MHz, acetone-d₆) δ 170.6, 72.7, 63.1, 39.1, 38.2, 21.8.

### Example 24: High cell density production of living whole cell catalysts and one pot sequential production of ((2S,4R)-4-hydroxy-6-oxotetrahydro-2H-pyran-2-yl)methyl acetate from acetyloxyacetaldehyde and acetaldehyde

The high cell density culture of living whole cell catalysts *E. coli* BL21(DE3) pET30/Ylil+pqqA-E and *E. coli* BL21(DE3) pET30a/DeoC (preparation is described in Example 7 and Example 4, respectively) were prepared in "fed batch" bioprocess using laboratory bioreactors Infors ISF100 with maximal volume of 2L. The reactors were stirred, aerated, temperature and pH controlled as described bellow. After consumption of initial substrates provided in the medium ammonia arid glucose are fed continuously to the process as nitrogen and carbon source, respectively.

The composition and preparation of the media was as follows:
13.3 g/L KH2PO4, 1.7 g/L citric acid, 60 mg/L Fe(III)citrate, 40 g/L D-glucose, 8 mg/L Zn(CH3COO)2 2H2O, 1 g/L (NH4)2HPO4, 2.7 g/L MgSO4 7H20 and 10 mL/L mineral solution.

Mineral solution was pre-prepared as follows:
1.5 g/L MnCl2 4H20, 0.3 g/L H3BO3, 0.25 g/L NaMoO4 2H2O, 0.25 g/L CoCl2 6H20, 0.15 g/L CuCl2 2H20, 0.84 g/L EDTA, 1 g/L Na2PO4 2H20

To prevent precipitation, the initial medium was prepared according to a special protocol: KH2PO4, Fe(III)citrate, mineral solution, Zn(CH3COO)2 2H2O and (NH4)2HPO4 were sequentially added as solutions to about half of the final volume. After autoclaving (20 min at 121 °C). sterile solutions of glucose, MgSO4 7H2O and kanamycin (25 mg/mL) were added after prior adjustment of the pH to 6.8 with 12.5 % (v/v) ammonium hydroxide solution. Sterile distilled water was added to adjust the final volume (1L) in the bioreactor. The above said solutions were sterilized separately by filtration (0.2 µm).

Feeding solutions were 12.5 % (v/v) ammonium hydroxide solution, the silicone antifoam compound synperonic antifoam (Sigma, A-5551)and 50 % (w/v) glucose.

Inoculums for both cultures were provided as 50mL of shake flask culture in exponential growth phase. VD medium (50 mL; 10 g/L Bacto yeast extract, 5 g/L glycerol, 5 g/L NaCl, 4 g/L NaH2PO4*2H2O. pH was adjusted with 1 M NaOH to 7.0) was inoculated with a single colony of said whole cell catalyst from a freshly streaked VD agar plate and pre-cultured to late exp. phase (37 °C, 250 rpm. 8h).

The initial process parameters at inoculation were as follows: 25 °C, air flow rate 1.5 L/min (1.5 VVM), stirrer speed 800 rpm, pH 6.8. During cultivation, the dissolved oxygen concentration was kept at ≥ 20 % of saturation by a pO2/agitation rate control loop and a pO2/air flow ratio control loop. towards the end of bioprocess approaching to 0 % and bioreactor capabilities reaching maximum (stirrer speed 2000 rpm, aeration 3L/min).

The pH was kept at 6.8 during the whole process using a pH sensor controlled external pump which provides pulses of Ammonia solution to the bioreactor whenever the pH drops below 6.8.

After depletion of glucose present in the initial medium, a distinctive rise in pO₂ and pH level is observed (about 10-12h into the process). At this time feeding with 50 % (w/v) glucose started and was manually regulated as approximation as exponential curve (feeding started with 0.1 mL/min and ended with 0.6 mL/min in 24 h period. The process can be controlled by substrate supply; when glucose concentration is kept at dynamic zero, the glucose solution flow controls respiration rate of the culture. Technical limitations in regards to oxygen supply and heat transfer can be successfully overcome this way.

Induction for expression of protein Ylil in the culture of *E. coli* BL21(DE3) pET30/Ylil+pqqA-E. was performed by adding 0.05 mM IPTG (Sigma Aldrich, Germany) 6 hours after start of the feeding phase.

Induction for expression of protein DeoC in the culture of *coli* BL21(DE3)pET30a/DeoC, was performed by adding 0.1 mM IPTG (Sigma Aldrich, Germany) 6 hours after start of the feeding phase.

The overall length of the process is 34 - 42h and wet weight of biomass at level between 200 and 240g/L is obtained.

The high density culture of *E. coli* BL21(DE3) pET30/Ylil+pqqA-E was cooled down to 15 °C and kept in the reactor with light steering and aeration (400 rpm, 0.5 L/min) until used for the reaction (5h).

The high density culture of *E*. *coli* BL21(DE3) pET30a/DeoC was kept in the bioreactor and stirred with 800 rpm. Temperature was raised to 37 °C and 56.6 g of acetyloxyacetaldehyde and 120 mL of acetaldehyde (45.4 g) diluted in water were added with programmable pump to the reaction mixture. The whole quantity of acetyloxyacetaldehyde was added in with the constant flow rate in 30 minutes. Acetaldehyde was added continuously in 3 hours time span as described in the table below:

| | | | | | | | | | | | | | | | | | | |
|---|---|---|---|---|---|---|---|---|---|---|---|---|---|---|---|---|---|---|
| Time [min] | 0 | 5 | 10 | 15 | 20 | 25 | 30 | 35 | 45 | 60 | 75 | 90 | 105 | 120 | 135 | 150 | 165 | 180 |
| Volume of added acetaldehyde [mL] | 0 | 15 | 27 | 38 | 47 | 54 | 60 | 65 | 73 | 82 | 90 | 96 | 102 | 106 | 110 | 114 | 117 | 120 |
| Flow [mL/min] | 3.000 | 2.400 | 2.200 | 1.800 | 1.400 | 1.200 | 1.000 | 0.800 | 0.600 | 0.533 | 0.400 | 0.400 | 0.267 | 0.267 | 0.267 | 0.200 | 0.200 | 0 |

During the reaction the pH was kept at 5.8 using 12.5 % (v/v) ammonium hydroxide solution and bioreactor's sensor dependent pH correction function. After 3h or the reaction, some of the reaction mixture was removed from the reactor leaving 1L of the reaction mixture steering with 800 rpm at 37 °C. 0.5 L of high density culture of *E. coli* BL21(DE3) pET30/Ylil+pqqA-E. preheated to 37 °C was added to the reaction mixture and aeration (1L/min) was provided. The reaction mixture was left for 6 hours and during that time. 0.1mL/min of glycerol was added to the mixture and pH was maintained at 5.8 using 12.5 % (v/v) ammonium hydroxide solution.

After the 6 hours the reaction was stopped pH lowered to5 using 5 M HCl solution and the whole volume of reaction mixture was transferred to simple glass vessel and mixed with 1.5 L of ethyl acetate to perform a "whole broth" extraction process. The organic phase was collected and another 1.5L of ethyl acetate were added to the aqueous phase. The whole procedure was repeated 5 times. The collected organic phase fractions were joined, 200 g of anhydrous sodium sulphate was added (in order to bind the ethyl acetate dissolved water) and filtered off. The solvent was then removed by low pressure evaporation at 37 °C. The remaining substance (82.6g) was yellow to amber oil with consistency of honey at RT. Subsequent analysis using ¹H NMR and GC-MS confirmed the structure of ((2S,4R)-4-hydroxy-6-oxotetrahydro-2H-pyran-2-yl)methyl acetate and the chromatographic purity was 52%. Overall molar yield of the reaction was estimated at 42.5%.

### Example 25: High cell density production of living whole cell catalysts and one pot sequential production of ((2S,4R)-hydroxy-6-oxotetrahydro-2H-pyran-2-yl)methyl acetate from acetyloxyacetaldehyde and acetaldehyde

The high cell density culture of living whole cell catalysts *E. coli* BL21(DE3) pET30/Gcd and *E. coli* BL21(DE3) pET30a/DeoC (contruction is described in Example 1 and Example 5, respectively) were prepared in a "fed batch" bioprocess using laboratory bioreactors Infors ISF-100 with maximal volume of 2 L. The reactors were stirred, aerated, temperature and pH controlled as described below. After consumption of initial substrates provided in the medium, ammonia and glucose are fed continuously to the process as nitrogen and carbon source, respectively.

The composition and preparation of the media was as follows:
13.3 g/L KH₂PO₄, 1.7 g/L citric acid, 60 mg/L Fe(III)citrate, 40 g/L D-glucose, 8 mg/L Zn(CH₃COO)₂·2H₂O, 1 g/L (NH₄)₂HPO₄, 2.7 g/L MgSO₄·7H₂O and 10 mL/L mineral solution.

Mineral solution was pre-prepared as follows:
1.5 g/L MnCl₂·4H₂0, 0.3 g/L H₃BO₃, 0.25 g/L NaMoO₄·2H₂O, 0.25 g/L CoCl₂·6H₂O, 0.15 g/L CuCl₂·2H₂O, 0.84 g/L EDTA, 1 g/L Na₂PO₄·2H₂O.

To prevent precipitation, the initial medium was prepared according to a special protocol: KH₂PO₄, Fe(III)citrate, mineral solution, Zn(CH₃COO)₂·2H₂O and (NH₄)₂HPO₄ were sequentially added as solutions to about half of the final volume. After autoclaving (20 min at 121 °C), sterile solutions of glucose, MgSO₄·7H₂O and kanamycin (25 mg/mL) were added after prior adjustment of the pH to 6.8 with 12.5 % (v/v) ammonium hydroxide solution. Sterile distilled water was added to adjust the final volume (1 L) in the bioreactor. The above said solutions were sterilized separately by filtration (0.2 µm).

Feeding solutions were 12.5 % (v/v) ammonium hydroxide solution, the silicone antifoam compound synperonic antifoam (Sigma, A-5551)and 50 % (w/v) glucose.

Inoculums for both cultures were provided as 50 mL of shake flask culture in exponential growth phase. VD medium (50 mL; 10 g/L Bacto yeast extract, 5 g/L glycerol, 5 g/L NaCl, 4 g/L NaH₂PO₄·2H₂O. pH was adjusted with 1 M NaOH to 7.0) was inoculated with a single colony of said whole cell catalyst from a freshly streaked VD agar plate and pre-cultured to late exp. phase (37 °C, 250 rpm, 8 h).

The initial process parameters at inoculation were as follows: 25 °C, air flow rate 1.5 L/min (1.5 VVM), stirrer speed 800 rpm, pH 6.8. During cultivation, the dissolved oxygen concentration was kept at ≥ 20 % of saturation by a pO2/agitation rate control loop and a pO2/air flow ratio control loop, towards the end of bioprocess approaching to 0 % and bioreactor capabilities reaching maximum (stirrer speed 2000 rpm, aeration 3 L/min).

The pH was kept at 6.8 during the whole process using a pH sensor controlled external pump which provides pulses of ammonia solution to the bioreactor whenever the pH drops below 6.8.

After depletion of glucose present in the initial medium, a distinctive rise in pO₂ and pH level is observed (about 10 - 12 h into the process). At this time feeding with 50 % (w/v) glucose started and was manually regulated as approximation as exponential curve (feeding started with 0.1 mL/min and ended with 0.6 mL/min in 24 h period. The process can be controlled by substrate supply; when glucose concentration is kept at dynamic zero, the glucose solution flow controls respiration rate of the culture. Technical limitations in regards to oxygen supply and heat transfer can be successfully overcome this way.

Induction for expression of protein Gcd in the culture of *E*. *coli* BL21(DE3) pET30/Gcd was performed by adding 0.1 mM IPTG (Sigma Aldrich, Germany) 6 hours after start of the feeding phase.

Induction for expression of protein DeoC in the culture of *coli* BL21(DE3) pET30a/DeoC, was performed by adding 0.2 mM IPTG (Sigma Aldrich, Germany) 6 hours after start of the feeding phase.

The overall length of the process is 34 - 42 h and wet weight of biomass at level between 150 and 200 g/L is obtained.

The high density culture of *E. coli* BL21(DE3) pET30/Gcd was cooled down to 15 °C and kept in the reactor with light steering and aeration (400 rpm, 0.5 Umin) until used for the reaction (5 h).

592 mL of the high density culture of *E. coli* BL21(DE3) pET30a/DeoC was kept in the bioreactor and stirred with 1300 rpm. Temperature was raised to 37 °C and 39.30 g of acetyloxyacetaldehyde and 100 mL of acetaldehyde (48.46 g) diluted in water was prepared. The whole quantity of acetyloxyacetaldehyde was added in with the constant flow rate in 27 minutes. Acetaldehyde solution was added continuously with programmable pump to the reaction mixture in 90 min time span as described in the table below:

| | | | | |
|---|---|---|---|---|
| Time [min] | 0 | 30 | 60 | 90 |
| Volume of added acetaldehyde solution [mL] | 0.00 | 52.50 | 71.75 | 77.00 |
| Flow [mL/min] | 1.750 | 0.642 | 0.175 | 0.000 |

During the reaction the pH was kept at 6.2 using 12.5 % (v/v) ammonium hydroxide solution and bioreactor's sensor dependent pH correction function. After 30 min of the reaction, additional 70 mL of high density culture of *E*. *coli* BL21(DE3) pET30a/DeoC was added. After 2 h or the reaction, 735 mL of the reaction mixture remaining was steered with 1400 rpm at 37 °C. The GC-FID analysis showed the concentration of 75,6/L of ((2S,4R)-4,6-dihydroxytetrahydro-2H-pyran-2-yl)methyl acetate present at the conclusion of the reaction.

183 mL of high density culture of *E. coli* BL21(DE3) pET30/Gcd, preheated to 37 °C, was added to the reaction mixture and aeration (1.4 Umin) was provided. MgCl₂ and PQQ were added to their final concentration 10 mM and 2 µM, respectively, and the reaction mixture was left stirring for 10 min in order to achieve full reconstitution of the Gcd enzyme with PQQ. The reaction mixture was left for 3 hours and during that time pH was maintained at 6.2 using 12.5 % (v/v) ammonium hydroxide solution. After 65 min of the beginning of the second step reaction, steering speed was lowered to 1200 rpm and aeration flow rate to 1.2 Umin. Again, after 137 min of the beginning of the second step reaction, steering speed was lowered to 1000 rpm and aeration flow rate to 1.0 Umin. The GC-FID analysis showed the concentration of 58,6g/L of ((2S,4R)-4-hydroxy-6-oxotetrahydro-2H-pyran-2-yl)methyl acetate predent at the conclusion of the reaction. The yield of the conversion of ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in this step, calculated from the GC-FID results was higher than 95%.

After 3 h the reaction was stopped, pH lowered to 4.0 using 5 M phosphoric acid solution and the whole volume of reaction mixture was transferred to simple glass vessel, in which 200 g/L of Na₂SO₄ wase added, pH again corrected to 4.0 with 5 M phosphoric acid and mixed with 920 mL of ethyl acetate (1 : 1) to perform a "whole broth" extraction process. The organic phase was collected and another 920 mL of ethyl acetate was added to the aqueous phase. The whole procedure was repeated 5 times. The collected organic phase fractions were joined, ∼150 g of anhydrous magnesium sulphate was added (in order to remove the water from ethyl acetate phase) and filtered off. The solvent was then removed by low pressure evaporation at 40 °C. The remaining substance (51.1 g), yellow to amber oil with consistency of honey at RT was analyzed using 1H NMR and GC-MS confirmed the structure of ((2S,4R)-4-hydroxy-6-oxotetrahydro-2H-pyran-2-yl)methyl acetate and the chromatographic purity (GC-FID) was 78,6 %. Overall molar yield of the two sequential enzymatic reactions was calculated to be 81.6 %.

### Example 26: High cell density production of living whole cell catalysts and one pot simultaneous production of ((2S,4R)-4-hydroxy-6-oxotetrahydro-2H-pyran-2-yl)methyl acetate from acetyloxyacetaldehyde and acetaldehyde

The high cell density culture of living whole cell catalysts *E. coli* BL21(DE3) pET30/DeoC_T7p_RBS_Gcd (preparation is described in Example 6) was prepared in "fed batch" bioprocess using laboratory bioreactors Infors ISF-100 with maximal volume of 2 L. The reactors were stirred, aerated, temperature and pH controlled as described bellow. After consumption of initial substrates provided in the medium, ammonia and glucose are fed continuously to the process as nitrogen and carbon source, respectively.

The composition and preparation of the media was as follows:
13.3 g/L KH₂PO₄, 1.7 g/L citric acid, 60 mg/L Fe(III)citrate, 40 g/L D-glucose, 8 mg/L Zn(CH₃COO)₂·2H₂O, 1 g/L (NH₄)₂HPO₄, 2.7 g/L MgSO₄·7H₂O and 10 mL/L mineral solution.

Mineral solution was pre-prepared as follows:
1.5 g/L MnCl₂·4H₂0, 0.3 g/L H₃BO₃, 0.25 g/L NaMoO₄·2H₂O, 0.25 g/L CoCl₂·6H₂O, 0.15 g/L CuCl₂·2H₂O, 0.84 g/L EDTA, 1 g/L Na₂PO₄·2H₂O.

To prevent precipitation, the initial medium was prepared according to a special protocol: KH₂PO₄, Fe(III)citrate, mineral solution, Zn(CH₃COO)₂·2H₂O and (NH₄)₂HPO₄ were sequentially added as solutions to about half of the final volume. After autoclaving (20 min at 121 °C), sterile solutions of glucose, MgSO₄·7H₂O and kanamycin (25 mg/mL) were added after prior adjustment of the pH to 6.8 with 12.5 % (v/v) ammonium hydroxide solution. Sterile distilled water was added to adjust the final volume (1 L) in the bioreactor. The above said solutions were sterilized separately by filtration (0.2 µm).

Feeding solutions were 12.5 % (v/v) ammonium hydroxide solution, the silicone antifoam compound synperonic antifoam (Sigma, A-5551)and 50 % (w/v) glucose.

Inoculums for both cultures were provided as 50 mL of shake flask culture in exponential growth phase. VD medium (50 mL; 10 g/L Bacto yeast extract, 5 g/L glycerol, 5 g/L NaCl, 4 g/L NaH₂PO₄·2H₂O. pH was adjusted with 1 M NaOH to 7.0) was inoculated with a single colony of said whole cell catalyst from a freshly streaked VD agar plate and pre-cultured to late exp. phase (37 °C, 250 rpm, 8 h).

The initial process parameters at inoculation were as follows: 25 °C, air flow rate 1.5 L/min (1.5 VVM), stirrer speed 800 rpm, pH 6.8. During cultivation, the dissolved oxygen concentration was kept at ≥ 20 % of saturation by a pO2/agitation rate control loop and a pO2/air flow ratio control loop, towards the end of bioprocess approaching to 0 % and bioreactor capabilities reaching maximum (stirrer speed 2000 rpm, aeration 3 L/min).

The pH was kept at 6.8 during the whole process using a pH sensor controlled external pump which provides pulses of ammonia solution to the bioreactor whenever the pH drops below 6.8.

After depletion of glucose present in the initial medium, a distinctive rise in pO₂ and pH level is observed (about 10 - 12 h into the process). At this time feeding with 50 % (w/v) glucose started and was manually regulated as approximation as exponential curve (feeding started with 0.1 mL/min and ended with 0.6 mL/min in 24 h period. The process can be controlled by substrate supply; when glucose concentration is kept at dynamic zero, the glucose solution flow controls respiration rate of the culture. Technical limitations in regards to oxygen supply and heat transfer can be successfully overcome this way.

Induction for expression of proteins DERA and Gcd in the culture of *E. coli* BL21(DE3) pET30/DeoC+Gcd, was performed by adding 0.1 mM IPTG (Sigma Aldrich, Germany) 6 hours after start of the feeding phase.

The overall length of the process is 34 - 42 h and wet weight of biomass at level between 150 and 200 g/L is obtained.

690 mL of the high density culture of *E. coli* BL21(DE3) pET30a/DeoC+Gcd was kept in the bioreactor and stirred with 1300 rpm. Temperature was raised to 37 °C, aeration (1.0 L/min) was provided, and 32.67 g of acetyloxyacetaldehyde and 100 mL of acetaldehyde (37.00 g) diluted in water was prepared. MgCl₂ and PQQ were added to their final concentration 10 mM and5 µM, respectively, and the reaction mixture was left stirring for 10 min in order to achieve full reconstitution of the Gcd enzyme with PQQ. The whole quantity of acetyloxyacetaldehyde was added in with the constant flow rate in 35 min. Acetaldehyde solution was added continuously with programmable pump to the reaction mixture in 60 min time span as described in the table below:

| | | | |
|---|---|---|---|
| Time [min] | 0 | 30 | 60 |
| Volume of added acetaldehyde solution [mL] | 0.00 | 60.00 | 80.00 |
| Flow [mL/min] | 2.000 | 0.667 | 0.000 |

During the reaction the pH was kept at 6.2 using 12.5 % (v/v) ammonium hydroxide solution and bioreactor's sensor dependent pH correction function. The reaction mixture was left for 3.5 h and during that time pH was maintained at 6.2 using 12.5 % (v/v) ammonium hydroxide solution. The GC-FID analysis showed the concentration of 46,1g/L of ((2S,4R)-4-hydroxy-6-oxotetrahydro-2H-pyran-2-yl)methyl acetate predent at the conclusion of the reaction.

After 3.5 h the reaction was stopped, pH lowered to 4.0 using 5 M phosphoric acid solution and the whole volume of reaction mixture was transferred to simple glass vessel, in which 200 g/L of Na₂SO₄ were added, pH again corrected to 4.0 with 5 M phosphoric acid and mixed with 800 mL of ethyl acetate (1 : 1) to perform a "whole broth" extraction process. The organic phase was collected and another 800 mL of ethyl acetate was added to the aqueous phase. The whole procedure was repeated 5 times. The collected organic phase fractions were joined, ∼150 g of anhydrous magnesium sulphate was added (in order to remove the water from ethyl acetate phase) and filtered off. The solvent was then removed by low pressure evaporation at 40 °C. The remaining substance (34.2 g), yellow to amber oil with consistency of honey at RT was analysed using 1H NMR and GC-MS confirmed the structure of ((2S,4R)-4-hydroxy-6-oxotetrahydro-2H-pyran-2-yl)methyl acetate and the chromatographic purity (GC-FID) was 76.3 %. Overall molar yield of the two simultaneous enzymatic reactions was calculated to be 59.9 %.

### REFERENCES

1. Achmann, S. et al. Direct detection of formaldehyde in air by a novel NAD+- and glutathione-independent formaldehyde dehydrogenase-based biosensor. Talanta 75, 786-91(2008).
2. Adachi, O. et al. Biooxidation with PQQ-and FAD-Dependent Dehydrogenases. Modern Biooxidation: Enzymes, Reactions and Applications, Wiley-VCH, Weinheim 1-41(2007).
3. Adachi, O. et al. New quinoproteins in oxidative fermentation. Biochimica et Biophysica Acta (BBA)-Proteins & Proteomics 1647, 10-17(2003).
4. Ameyama, M. et al. Method of enzymatic determination of pyrroloquinoline quinone. Analytical biochemistry 151, 263-7(1985).
5. Anthony C, Zatman LJ (1967). "The microbial oxidation of methanol. The prosthetic group of the alcohol dehydrogenase of Pseudomonas sp. M27: a new oxidoreductase prosthetic group". Biochem J 104 (3): 960-9. PMID 6049934. PMID 6049934
6. Anthony, C. Pyrroloquinoline quinone (PQQ) and quinoprotein enzymes. Antioxidants and Redox Signaling 3, 757-774(2001).
7. Anthony, C. The quinoprotein dehydrogenases for methanol and glucose. Archives of biochemistry and biophysics 428, 2-9(2004).
8. Cline, A. & Hu, A. Enzymatic characterization and comparison of three sugar dehydrogenases from a pseudomonad. Journal of Biological Chemistry 240, 4493(1965).
9. Cozier, G.E., Salleh, R.A. & Anthony, C. Characterization of the membrane quinoprotein glucose dehydrogenase from Escherichia coli and characterization of a site-directed mutant in which histidine-262 has been changed to tyrosine. Biochemical Journal 340, 639(1999).
10. D'Costa, E. J., I. J. Higgins & A. P. F. Turner. 1986. Quinoprotein glucose dehydrogenase and its application in an amperometric glucose sensor. Biosensors. 2 71-87
11. Duine, J.A. Quinoproteins: enzymes containing the quinonoid cofactor pyrroloquinoline quinone, topaquinone or tryptophan-tryptophan quinone. European Journal of Biochemistry 200, 271-284(1991).
12. Durand, F. et al. Designing a highly active soluble PQQ-glucose dehydrogenase for efficient glucose biosensors and biofuel cells. Biochemical and biophysical research communications 402, 750-4(2010).
13. Gao, F.,Viry, L., Maugey, M., Poulin, P., Mano, N., Engineering hybrid nanotube wires for high-power biofuel cells, Nat.Commun.1 (2010), doi:10.1038/ncomms1000
14. Goodwin P.M, Anthony C., "The biochemistry, physisiology and genetics of PQQ and PQQ-containing enzymes," Adv. Microb. Physiol. (1998) 40:1-80
15. Goosen N. et al., "Acinetobacter calcoaceticus Genes Involved in Biosynthesis of the Coenzyme Pyrrolo-Quinoline-Quinone: Nucleotide Sequence and Expression in Escherichia coli K-12," J Bacteriol. (1989) 171:447-455
16. Gupta, A. et al. Gluconobacter oxydans: its biotechnological applications. Journal of molecular microbiology and biotechnology 3, 445-56(2001).
17. Hauge JG (1964). "Glucose dehydrogenase of bacterium anitratum: an enzyme with a novel prosthetic group". J Biol Chem 239: 3630-9. PMID 14257587.PMID 14257587
18. Heller, A. and Feldman, B., Electrochemical glucose sensors and their applications in diabetes management, Chem. Rev. 108 (2008), pp. 2482-2505. Full Text via CrossRef | View Record in Scopus | Cited By in Scopus (77)
19. Hoelscher T., Goerisch H., "Knockout and Overexpression of Pyrroloquinoline Quinone Biosynthetic Genes in Gluconobacter oxydans 621H," J Bacteriology (2006) 188;21:7668-7676
20. Hommes, R. W. J., Postma, P. W., Neijssel, 0. M., Tempest, D. W.,Dokter, P. & Duine, J.A. (1984). Evidence for a glucose dehydrogenase apo-enzyme in several strains of Escherichia cofi.FEMS Microbiol Lett 24,329-333.
21. Igarashi, S. et al. Molecular engineering of PQQGDH and its applications. Archives of biochemistry and biophysics 428, 52-63(2004).
22. Igarashi, S., Hirokawa, T. & Sode, K. Engineering PQQ glucose dehydrogenase with improved substrate specificity. Site-directed mutagenesis studies on the active center of PQQ glucose dehydrogenase. Biomolecular engineering 21, 81-9(2004).
23. Jonge, R.D., Mattos, M.D. & Stock, J. Pyrroloquinoline quinone, a chemotactic attractant for Escherichia coli. Journal of 178, 1224-1226(1996).
24. Keilin, D. & Hartree, E.F. Properties of glucose oxidase (notatin): Addendum. Sedimentation and diffusion of glucose oxidase (notatin). The Biochemical journal 42, 221-9(1948).
25. Keilin, D. & Hartree, E.F. Specificity of glucose oxidase (notatin). The Biochemical journal 50, 331-41(1952).
26. Keilin, D. & Hartree, E.F. The use of glucose oxidase (notatin) for the determination of glucose in biological material and for the study of glucose-producing systems by manometric methods. The Biochemical journal 42, 230-8(1948).
27. Khairnar N.P. et al., "Pyrroloquinoline-quinone synthesized in Escherichia coli by pyrroloquinoline-quinone synthase of Deinococcus radiodurans plays a role beyond mineral phosphate solubilization," Biochemical and Biophysical Research Communications (2003) 312:303-308
28. Kim C.H. et al., "Cloning and Expression of Pyrroloquinoline Quinone (PQQ) Genes from a Phosphate-Solubilizing Bacterium Enterobacter intermedium," Current Microbiology (2003) 47:457-461
29. Kujawa, M. et al. Properties of pyranose dehydrogenase purified from the litter-degrading fungus Agaricus xanthoderma. The FEBS journal 274, 879-94(2007).
30. Lapėnaitė, I, Kurtinaitienė, B. & Pliuškys, L. Application of PQQ-GDH Based Polymeric Layers in Design of Biosensors for Detection of Heavy Metals. (2003)
31. Lapenaite, I., Ramanaviciene, A. & Ramanavicius, A. Current trends in enzymatic determination of glycerol. Critical Reviews in Analytical Chemistry 36,13-25(2006).
32. Leskovac V, Trivic S, Wohlfahrt G, Kandrac J, Pericin D (2005)Glucose oxidase from Aspergillus niger. the mechanism of action with molecular oxygen, quinones, and one-electron acceptors. Int J Biochem 37:731-750
33. Lidstrom, M.E. Genetics of bacterial quinoproteins. Methods in enzymology. San Diego CA 258, 217-227(1995).
34. Linton, J., Woodard, S. & Gouldney, D. The consequence of stimulating glucose dehydrogenase activity by the addition of PQQ on metabolite production by Agrobacterium radiobacter NCIB 11883. Applied Microbiology and Biotechnology 25, 357-361 (1987).
35. Magnusson, O.T. et al. The structure of a biosynthetic intermediate of pyrroloquinoline quinone (PQQ) and elucidation of the final step of PQQ biosynthesis. Journal of the American Chemical Society 126, 5342-3(2004).
36. Martin, E.J.S. Assay for D-allose using a NAD cofactor coupled D-allose dehydrogenase. US Patent 5,567,605 (1996).
37. Matsushita, K. et al. Escherichia coli is unable to produce pyrroloquinoline quinone (PQQ). Microbiology (Reading, England) 143 (Pt 1, 3149-56(1997).
38. Meulenberg J.J., "nucleotide sequence and structure of the Klebsiella pneumoniae pqq operon," Mol. Gen. Genet. (1992) 232:284-294
39. Mitchell, R. & Duke, F. Kinetics and equilibrium constants of the gluconic acid-gluconolactone equilibrium. Ann. NYAcad. Sci 172, 129-138(1970).
40. Olsthoorn, a J. & Duine, J. a On the mechanism and specificity of soluble, quinoprotein glucose dehydrogenase in the oxidation of aldose sugars. Biochemistry 37, 13854-61 (1998).
41. Olsthoorn, a J., Otsuki, T. & Duine, J. a Ca2+ and its substitutes have two different binding sites and roles in soluble, quinoprotein (pyrroloquinoline-quinone-containing) glucose dehydrogenase. European journal of biochemistry / FEBS 247, 659-65(1997).
42. Oubrie, a & Dijkstra, B.W. Structural requirements of pyrroloquinoline quinone dependent enzymatic reactions. Protein science : a publication of the Protein Society 9, 1265-73(2000).
43. Oubrie, a et al. Structure and mechanism of soluble quinoprotein glucose dehydrogenase. The EMBO journal 18, 5187-94(1999).
44. Oubrie, a Structure and mechanism of soluble glucose dehydrogenase and other PQQ-dependent enzymes. Biochimica et Biophysica Acta (BBA)-Proteins & Proteomics 1647, 143-151(2003).
45. Pazur JH, Kleppe K (1964) The oxidation of glucose and related compounds by glucose oxidase from Aspergillus niger. Biochemistry 3:578-583
46. Puehringer S., Metlitzky M. et al., "The pyrroloquinoline quinine biosynthesis pathway revisited: A structural approach,", BMC Biochemistry (2008) 9:8
47. Rose, a, Scheller, F. & Wollenberger, U. Quinoprotein glucose dehydrogenase modified thick-film electrodes for the amperometric detection of phenolic compounds in flow injection analysis. Fresenius' journal of 369, 145-52(2001).
48. Schie, B.J. van et al. Energy transduction by electron transfer via a pyrrolo-quinoline quinone-dependent glucose dehydrogenase in Escherichia coli, Pseudomonas aeruginosa, and Acinetobacter calcoaceticus (var. Iwoffi). Journal of bacteriology 163, 493-9(1985).
49. Schmid, R. & Urlacher, V.B. Modern biooxidation: enzymes, reactions and applications. Engineering (Vch Verlagsgesellschaft Mbh: 2007).
50. Sierks, M.R. et al. Active site similarities of glucose dehydrogenase, glucose oxidase, and glucoamylase probed by deoxygenated substrates. Biochemistry 31, 8972-7(1992).
51. Smolander, M., Livio, H.-L., Rasanen, L., Mediated amperometric determination of xylose and glucose with an immobilized aldose dehydrogenase electrode, Biosensors and Bioelectronics, Volume 7, Issue 9, 1992, Pages 637-643.
52. Sode, K. et al. Effect of PQQ glucose dehydrogenase overexpression in Escherichia coli on sugar-dependent respiration. Journal of biotechnology 43, 41-4(1995).
53. Sode, K. et al. Thermostable chimeric PQQ glucose dehydrogenase. FEBS letters 364, 325-7(1995).
54. Sode, K., Ootera, T., Shirahane, M., Witarto, A.B., Igarashi, S., Yoshida, H., Increasing the thermal stability of the water-soluble pyrroloquinoline quinone glucose dehydrogenase by single amino acid replacement, Enzyme Microb. Technol. 26 (2000) 491-496.
55. Southall, S.M. et al. Soluble aldose sugar dehydrogenase from Escherichia coli: a highly exposed active site conferring broad substrate specificity. The Journal of biological chemistry 281, 30650-9(2006).
56. Springer A.L. et al., "Characterisation and nucleotide sequence of pqqE and pqqF in Methylobacterium extorquens AM1," J Bacteriol. (1996) 178:2154-2157
57. Szeponik, J. et al. Ultrasensitive bienzyme sensor for adrenaline. Biosensors and 12, 947-52(1997).
58. Tanaka, S. et al. Increasing stability of water-soluble PQQ glucose dehydrogenase by increasing hydrophobic interaction at dimeric interface. BMC biochemistry 6, 1 (2005).
59. Volc, J. et al. Pyranose 2-dehydrogenase, a novel sugar oxidoreductase from the basidiomycete fungus Agaricus bisporus. Archives of microbiology 167, 119-25(1997).
60. Wilson, G.S. et al. Progress toward the Development of an Implantable Sensor for Glucose. 1617, 1613-1617(1992).
61. Wong, C.M., Wong, K.H. & Chen, X.D. Glucose oxidase: natural occurrence, function, properties and industrial applications. Applied microbiology and biotechnology 78, 927-38(2008).
62. Yamada, M. et al. Escherichia coli PQQ-containing quinoprotein glucose dehydrogenase: its structure comparison with other quinoproteins. Biochimica et Biophysica Acta (BBA)-Proteins & Proteomics 1647, 185-192(2003).
63. Yang X.-P. et al., "Pyrroloquinoline quinine biosynthesis in Escherichia coli through expression of the Gluconobacter oxydans pqqABCDE gene cluster," J Ind Microbiol Biotechnol (2010) 37:575-580
64. Yoshida H. et al., "Secretion of water soluble pyrroloquinoline quinine glucose dehydrogenase by recombinant Pichia pastoris," Enzy Microbial Tech (2002) 30:312-318
65. Zheng, Y.J. & Bruice, T.C. Conformation of coenzyme pyrroloquinoline quinone and role of Ca2+ in the catalytic mechanism of quinoprotein methanol dehydrogenase. Proceedings of the Rational Academy of Sciences of the United States of America 94, 11881-6(1997).
66. Gijsen, H. Unprecedented asymmetric aldol reactions with three aldehyde substrates catalyzed by 2-deoxyribose-5-phosphate aldolase. Journal of the American Chemical 8422-8423(1994)

Patent references cited:
WO2009156083, JP2009232872, EP2251420, US2009148874, MX2007000560, JP2006314322, JP2006217811, WO 2006/134482 WO2008/119810, WO 2005/118794, WO 2006/134482, WO2009/092702

## Claims

1. A process for preparing a compound of formula (I) 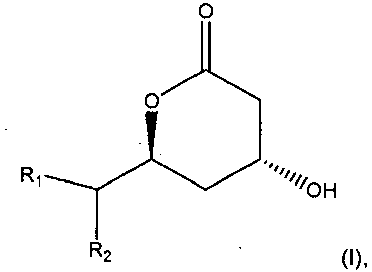 in which
R₁ independently from R₂ denotes H, X, N₃, CN, NO₂, OH, (CH₂)ₙ-CH₃, 0-(CH₂)ₙ-CH₃, S-(CH₂)ₙ-CH₃, NR₃R₄, OCO(CH₂)ₙCH₃, NR₃CO(CH₂)ₙCH₃, CH2-R₅, optionally substituted mono- or bicyclic aryl, heterocyclic or alicyclic group; and
R₂ independently from R₁ denotes H, (CH₂)ₘ-CH₃, or aryl;
or both of R₁ and R₂ denote either X, OH or O((CH₂)ₙCH₃);
or R₁ and R₂ together denote =O, =CH-R₅, wherein
any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR₃R₄, NR₃CO(CH₂)ₙCH₃; or
each CH₂ linking carbon atoms can be replaced by O, S or NR₃; wherein
R₃ and R₄ independently from each other, or together, denote H, (CH₂)ₘ-CH₃;
R₅ denotes optionally substituted mono- or bicyclic aryl, heterocyclic or alicyclic group,
X denotes F, Cl, Br or I;
n represents an integer from 0 to 10;
m represents an integer from 0 to 3;
or a pharmaceutically acceptable salt, or a stereoisomer thereof,
the process comprising bringing in contact a non-natural lactol compound of formula (II), 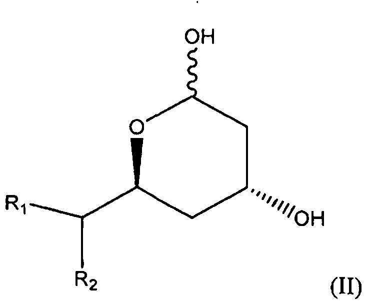 wherein R₁ and R₂ are defined as above, with an aldose 1-dehydrogenase.

2. The process according to claim 1, wherein
R₅ denotes a moiety selected from the formula (III), (IV), (V), (VI), (VII), (VIII) and (IX);

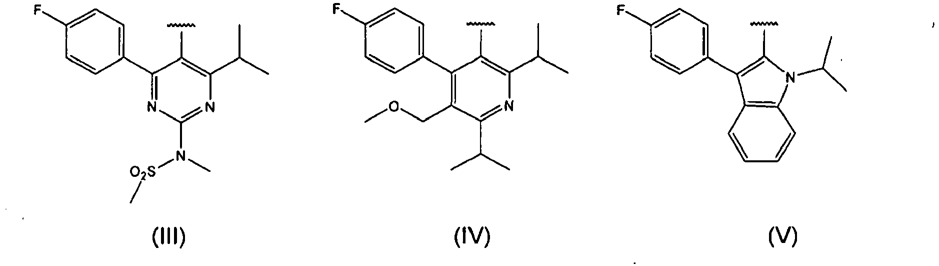

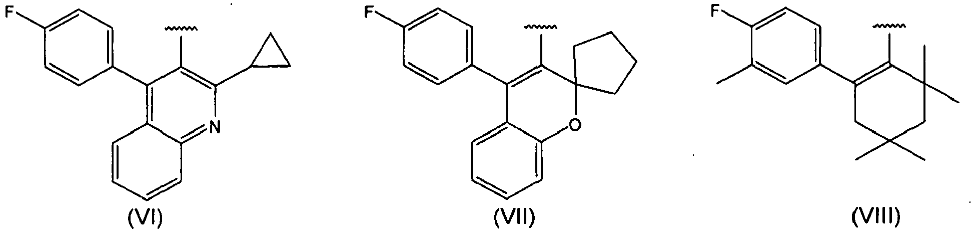

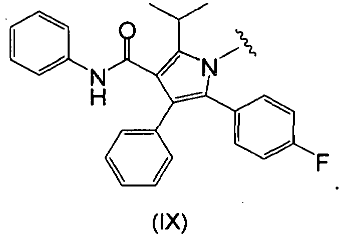

3. The process for preparing a compound of formula (I) according to claim 1, in which
R₁ independently from R₂ denotes H, X, N₃, CN, NO₂, OH, (CH₂)ₙ-CH₃, O-(CH₂)ₙ-CH₃, S-(CH₂)ₙ-CH₃, NR₃R₄, OCO(CH₂)ₙCH₃, or NR₃CO(CH₂)ₙCH₃, optionally substituted mono- or bicyclic aryl, heterocyclic or alicyclic group; and
R₂ independently from R₁ denotes H, (CH₂)ₘ-CH₃, or aryl;
or both of R₁ and R₂ denote X, OH or O(CH₂)ₙCH₃;
or R₁ and R₂ together denote =O, wherein
any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR₃R₄, NR₃CO(CH₂)ₙCH₃; or
each CH₂ linking carbon atoms can be replaced by O, S or NR₃; wherein
R₃ and R₄ independently from each other, or together, denote H, (CH₂)ₘ-CH₃;
X denotes F, Cl, Br or I;
n represents an integer from 0 to 10;
m represents an integer from 0 to 3.

4. The process according to any one of the previous claims, wherein the aldose 1-dehydrogenase is a pyrroloquinoline quinine (PQQ) dependent dehydrogenase.

5. The process according to any one of the previous claims, wherein the aldose dehydrogenase is Ylil aldose dehydrogenase from E. coli or mGDH glucose dehydrogenase from E. coli.

6. The process according to any one of the previous claims, wherein the aldose 1-dehydrogenase is selected from the group consisting of dehydrogenases encoded by dehydrogenase-encoding genes comprised within SEQ ID NO. 01 or is selected from the group consisting of dehydrogenases encoded by dehydrogenase-encoding genes selected from any one of nucleotide sequences of SEQ ID NOS. 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences SEQ ID NOS. 02, 04, 06, 08, 10, 24, 26, 28, 30, 32, 34, 36, 38, 40, 42, 44, 46, 48, 50, 52, 54, 56, 58, 60 and 62;
or any dehydrogenase having a nucleotide sequence identity or an amino acid sequence identity respectively of at least 50 % to said sequences, provided that the resulting sequence variants maintain dehydrogenase activity.

7. The process according to any one of the previous claims, wherein 2-deoxyribose-5-phosphate aldolase (DERA, EC 4.1.2.4) enzyme is used for preparing the compound of formula (II).

8. The process according to claim 7, wherein the 2-deoxyribose-5-phosphate aldolase enzyme is used for a synthetic step preceding, or alternatively simultaneously at least in an overlapping time period with, bringing in contact the compound of formula (II) with the enzyme capable of catalyzing oxidation or dehydrogenation.

9. The process according to any one of the previous claims, wherein the aldose 1-dehydrogenase, optionally also the DERA enzyme independently, are comprised within living non-human whole cell, inactivated non-human whole cell, homogenized non-human whole cell, or non-human cell free extract; or are purified, immobilized and/or are in the form of an extracellularly expressed protein.

10. A reaction system comprising a 2-deoxyribose-5-phosphate aldolase (DERA) enzyme and an aldose 1-dehydrogenase, and the reaction system being capable of, or being arranged for, converting a compound of formula (IX), 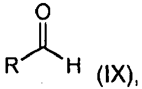 in which R denotes R₁-CH-R₂ moiety of formula (I), R₁ and R₂ being as defined in any one of claims 1 to 4, with acetaldehyde into a compound of formula (I) 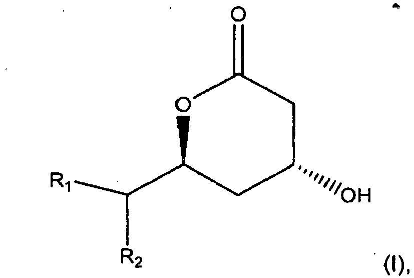 in which R₁ and R₂ are as defined above.

11. The process according to claim 7 or the reaction system according to claim 10, wherein both said enzymes, i.e. DERA and the aldose 1-dehydrogenase, are expressed by one or more cells, wherein the type of cell is selected from the group consisting of bacteria, yeast, insect cell and non-human mammalian cells.

12. The process according to claim 7 or the reaction system according to claim 10, further providing for the presence of Pyrroloquinoline quinine (PQQ).

13. The process or the reaction system according to claim 12, wherein providing for the presence of Pyrroloquinoline quinine (PQQ) is accomplished by a measure selected from the group consisting of:
(i) PQQ is added from externally;
(ii) a host non-human organism is used which, beyond providing for the presence of dehydrogenase activity, further has intrinsic PQQ biosynthetic capability; and
(iii) a microorganism is used, which does not have intrinsic capability of biosynthetis of PQQ, but which is genetically engineered to express PQQ-synthesis related gene cluster.

14. The process or the reaction system according to claim 13, wherein the microorganism used according to measure (iii) is genetically engineered to provide for an expression of a PQQ-synthesis encoding gene comprised within, or constituted by, any one of the nucleotide sequences of SEQ ID NOS. 11, 17, 63, 64, 65, 66, 67, 68, 69, 70; or expression of a PQQ-synthesis gene encoding any one of the aminoacid sequence of SEQ ID NOS. 12, 13, 14, 15, 16, 18, 19, 20, 21 and 22;
or is genetically engineered to provide for expression of a PQQ-synthesis encoding gene having a nucleotide sequence identity or an amino acid sequence identity respectively of at least 50 % to said sequences, provided that the resulting sequence variants maintain activity to produce PQQ.

15. The process according to any one of the previous claims, further comprising subjecting said compound (I) to conditions sufficient to prepare a statin or a pharmaceutically acceptable salt thereof, optionally salifying, esterifying or stereoselectively resolving the statin product.

16. The process according to claim 15, wherein the statin is selected from the group consisting of lovastatin, pravastatin, simvastatin, atorvastatin, cerivastatin, rosuvastatin, fluvastatin, pitavastatin, bervastatin, and dalvastatin, and pharmaceutically acceptable salts thereof.

17. A process for preparing a pharmaceutical composition, the process comprising carrying out a process according to the process of claim 15 or 16, and
formulating said statin, or a pharmaceutically acceptable salt thereof, with at least one pharmaceutically acceptable excipient to obtain said pharmaceutical composition.

18. Use of the reaction system according to any one of claims 10 to 14 for preparing a compound of formula (I), wherein the formula (I) is as defined in any one of claims 1 to 3, and optionally for further preparation of a statin or a pharmaceutically acceptable salt thereof.

19. Use of an aldose 1-dehydrogenase enzyme for preparing a compound of formula (I) 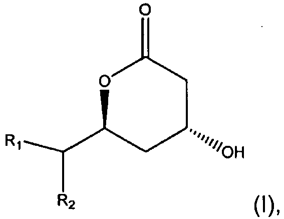 in which R₁ and R₂ have the same meaning as above,
which compound-is further converted into a statin or a pharmaceutically acceptable salt thereof.

## Patentansprüche

1. Ein Verfahren zur Herstellung einer Verbindung der Formel (I), 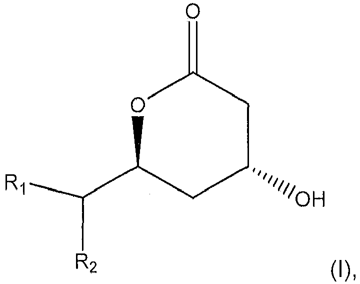 wobei
R₁ unabhängig von R₂ für H, X, N₃, CN, NO₂, OH, (CH₂)ₙ-CH₃, O-(CH₂)ₙ-CH₃, S-(CH₂)ₙ-CH₃, NR₃R₄, OCO(CH₂)ₙCH₃, NR₃CO(CH₂)ₙCH₃, CH₂-R₅, optional substituierte mono- oder bicyclische Aryl-, heterocyclische oder alicyclische Gruppen steht; und
R₂ unabhängig von R₁ für H, (CH₂)ₘ-CH₃, oder Aryl steht;
oder beide, R₁ und R₂ entweder für X, OH oder O((CH₂)ₙCH₃) stehen;
oder R₁ und R₂ zusammen für =O, =CH-R₅ stehen, wobei
jede der vorstehend genannten CH₂ oder CH₃ Gruppen optional weiter substituiert sein kann durch X, N₃, CN, NO₂, OH, (CH₂)ₙ-CH₃, Aryl, 0-(CH₂)ₙ-CH₃, OCO(CH₂)ₙCH₃, NR₃R₄, NR₃CO(CH₂)ₙCH₃; oder
jedes CH₂, das Kohlenstoffatome verbindet, durch O, S oder NR₃ ersetzt sein kann; wobei
R₃ und R₄ unabhängig voneinander, oder gemeinsam, für H, (CH₂)ₘ-CH₃ stehen;
R₅ für optional substituierte mono- oder bicyclische Aryl-, oder heterocyclische oder alicyclische Gruppen steht,
X für F, Cl, Br oder I steht;
n eine ganze Zahl von 0 bis 10 darstellt;
m eine ganze Zahl von 0 bis 3 darstellt;
oder ein pharmazeutisch akzeptables Salz oder ein Stereoisomer davon,
wobei das Verfahren das in Kontakt bringen einer nicht natürlichen Lactolverbindung der Formel (II) 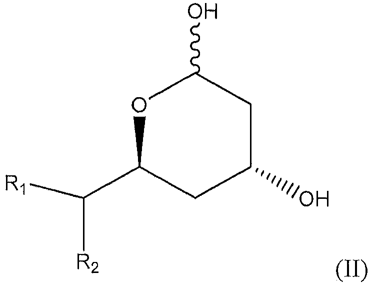 wobei R₁ und R₂ wie oben definiert sind,
mit einer Aldose-1-Dehydrogenase umfasst.

2. Das Verfahren gemäß Anspruch 1, wobei
R₅ für einen Rest, ausgewählt aus einer der Formeln (III), (IV), (V), (VI), (VII), (VIII) und (IX); 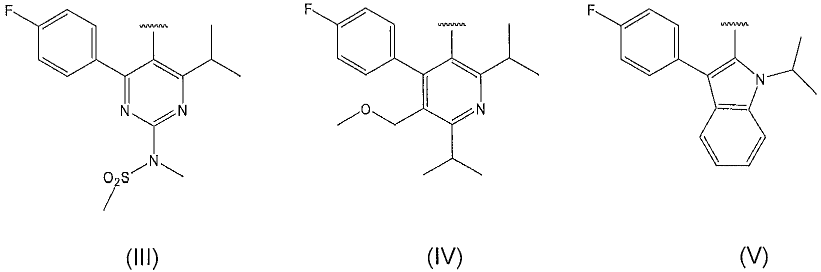 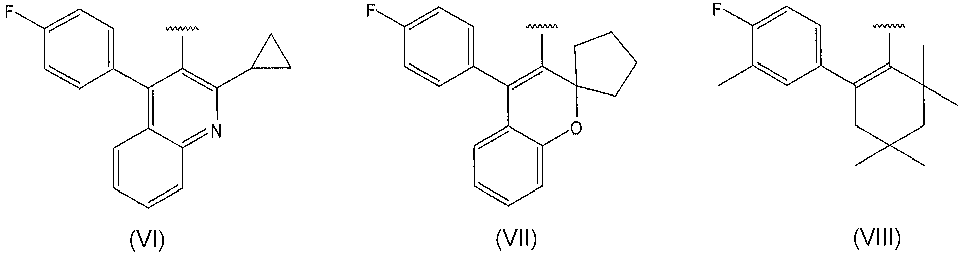 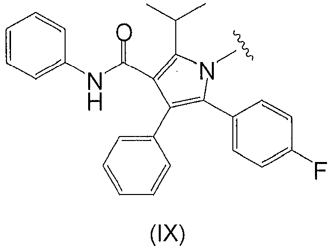 steht.

3. Das Verfahren zur Herstellung einer Verbindung der Formel (I) gemäß Anspruch 1, wobei
R₁ unabhängig von R₂ für H, X, N₃, CN, NO₂, OH, (CH₂)ₙ-CH₃, O-(CH₂)ₙ-CH₃, S-(CH₂)ₙ-CH₃, NR₃R₄, OCO(CH₂)ₙCH₃, oder NR₃CO(CH₂)ₙCH₃ oder optional substituierte mono- oder bicyclische Aryl-, heterocyclische oder alicyclische Gruppen steht; und
R₂ unabhängig von R₁ für H, (CH₂)ₘ-CH₃, oder Aryl steht;
oder beide, R₁ und R₂ für X, OH oder O(CH₂)ₙCH₃ stehen;
oder R₁ und R₂ zusammen für =O stehen, wobei
jede der vorstehend genannten CH₂ oder CH₃ Gruppen optional weiter substituiert sein kann durch X, N₃, CN, NO₂, OH, (CH₂)ₙ-CH₃, aryl, O-(CH₂)ₙ-CH₃, OCO(CH₂)ₙCH₃, NR₃R₄, NR₃CO(CH₂)ₙCH₃; oder
jedes CH₂, das Kohlenstoffatome verbindet, durch O, S oder NR₃ ersetzt sein kann; wobei
R₃ und R₄ unabhängig voneinander, oder gemeinsam, für H, (CH₂)ₘ-CH₃ stehen; X für F, Cl, Br oder I steht;
n eine ganze Zahl von 0 bis 10 darstellt;
m eine ganze Zahl von 0 bis 3 darstellt.

4. Das Verfahren nach einem der vorhergehenden Ansprüche, wobei die Aldose-1-Dehydrogenase eine Pyrrolochinolin-Chinin (PQQ) -abhängige Dehydrogenase ist.

5. Das Verfahren nach einem der vorhergehenden Ansprüche, wobei die Aldose-Dehydrogenase Ylil-Aldose-Dehydrogenase aus E. coli oder mGDH-Glucose-Dehydrogenase aus E. coli ist.

6. Das Verfahren nach einem der vorhergehenden Ansprüche, wobei die Aldose-1-Dehydrogenase ausgewählt ist aus der Gruppe bestehend aus Dehydrogenasen, die von Dehydrogenase-kodierenden Genen gemäß SEQ ID NO. 01 kodiert werden, oder ausgewählt ist aus der Gruppe bestehend aus Dehydrogenasen, die von Dehydrogenase-kodierenden Genen gemäß einer der Nukleotidsequenzen der SEQ ID NO. 03, 05, 07, 09, 23, 25, 27, 29, 31, 33, 35, 37, 39, 41, 43, 45, 47, 49, 51, 53, 55, 57, 59 und 61 kodiert werden, oder
ausgewählt ist aus der Gruppe bestehend aus Dehydrogenasen, die durch eine der Aminosäuresequenzen gemäß SEQ ID NO. 02, 04, 06, 08, 10, 24, 26, 28, 30, 32, 34, 36, 38, 40, 42, 44, 46, 48, 50, 52, 54, 56, 58, 60 und 62 bestimmt ist; oder irgendeine Dehydrogenase mit einer Nukleotidsequenzidentität bzw. einer Aminosäuresequenzidentität von mindestens 50% zu den genannten Sequenzen, vorausgesetzt, dass die resultierenden Sequenzvarianten die Dehydrogenaseaktivität beibehält.

7. Das Verfahren nach einem der vorhergehenden Ansprüche, wobei zur Herstellung der Verbindung der Formel (II) das Enzym 2-Desoxyribose-5-phosphat-Aldolase (DERA, EC 4.1.2.4) verwendet wird.

8. Das Verfahren nach Anspruch 7, wobei das 2-Desoxyribose-5-phosphat-Aldolase-Enzym für einen Syntheseschritt vor, oder alternativ gleichzeitig, mit dem in Kontakt bringen mit der Verbindung der Formel (II) mit dem Enzym, welches die Oxidation oder Dehydrierung katalysieren kann, verwendet wird.

9. Das Verfahren nach einem der vorhergehenden Ansprüche, wobei die Aldose-1-Dehydrogenase, optional und unabhängig auch das DERA-Enzym, in lebenden nicht-menschlichen ganzen Zellen, in inaktivierten nicht-menschlichen ganzen Zellen, in homogenisierten nicht-menschlichen ganzen Zellen oder in nicht-menschlichem zellfreien Extrakt beinhaltet sind; oder gereinigt, immobilisiert und/oder in Form eines extrazellulär exprimierten Proteins vorliegen.

10. Ein Reaktionssystem umfassend ein 2-Desoxyribose-5-phosphat-Aldolase (DERA) - Enzym und eine Aldose-1-Dehydrogenase, und das Reaktionssystem in der Lage ist, oder dazu vorgesehen ist, eine Verbindung der Formel (IX) 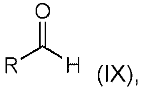 wobei R für eine R₁-CH-R₂ -Einheit der Formel (I) steht, R₁ und R₂ wie in einem der Ansprüche 1 bis 4 definiert sind, mit Acetaldehyd zu einer Verbindung der Formel (I) umzuwandeln, 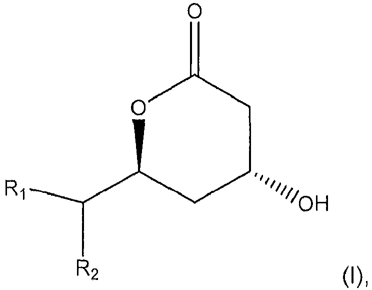 wobei R₁ und R₂ wie oben definiert sind.

11. Das Verfahren nach Anspruch 7 oder das Reaktionssystem nach Anspruch 10, wobei beide Enzyme, d.h. DERA und die Aldose-1-Dehydrogenase, von einer oder mehreren Zellen exprimiert werden, wobei der Zelltyp ausgewählt ist aus der Gruppe bestehend aus Bakterien, Hefe, Insektenzellen und nicht-menschliche Säugetierzellen.

12. Das Verfahren nach Anspruch 7 oder das Reaktionssystem nach Anspruch 10, **dadurch gekennzeichnet, dass** Pyrrolochinolin-Chinin (PQQ) vorhanden ist.

13. Das Verfahren oder das Reaktionssystem nach Anspruch 12, **dadurch gekennzeichnet, dass** das Vorliegen von Pyrrolochinolin-Chinin (PQQ) durch eine Maßnahme sichergestellt wird, die ausgewählt wird aus der Gruppe bestehend aus:
(i) Hinzufügen von PQQ von außen;
(ii) Verwenden eines nicht-menschlichen Wirts-Organismus, der über die Bereitstellung der Dehydrogenase-Aktivität hinaus eine intrinsische PQQ-Biosynthesefähigkeit aufweist; und
(iii) Verwenden eines Mikroorganismus, der keine intrinsische Fähigkeit zur Biosynthese von PQQ besitzt, aber genetisch manipuliert ist, um einen mit der PQQ-Synthese verwandten Gencluster zu exprimieren.

14. Das Verfahren oder das Reaktionssystem nach Anspruch 13, wobei der gemäß Maßnahme (iii) verwendete Mikroorganismus gentechnisch verändert ist, um eine Expression eines PQQ-Synthese-kodierenden Gens bereitzustellen, das in einer der Nukleotide-Sequenzen gemäß SEQ ID NO. 11, 17, 63, 64, 65, 66, 67, 68, 69, 70 enthalten ist oder daraus aufgebaut ist; oder
eine Expression eines PQQ-Synthesegens bereitzustellen, das für eine der Aminosäuresequenzen der SEQ ID NO: 12, 13, 14, 15, 16, 18, 19, 20, 21 und 22 kodiert; oder genetisch verändert ist, um für die Expression eines PQQ-Synthese-kodierenden Gens mit einer Nukleotidsequenzidentität oder einer Aminosäuresequenzidentität von mindestens 50% zu den Sequenzen bereitzustellen, vorausgesetzt, dass die resultierenden Sequenzvarianten die Aktivität erhalten, um PQQ zu produzieren.

15. Das Verfahren nach einem der vorangehenden Ansprüche, ferner umfassend, dass die Verbindung (I) Bedingungen unterworfen wird, die ausreichen, um ein Statin oder ein pharmazeutisch akzeptables Salz davon herzustellen, optional um es in ein Salz zu verwandeln, es zu verestern oder es stereoselektiv aufzulösen.

16. Das Verfahren nach Anspruch 15, wobei das Statin ausgewählt ist aus der Gruppe bestehend aus Lovastatin, Pravastatin, Simvastatin, Atorvastatin, Cerivastatin, Rosuvastatin, Fluvastatin, Pitavastatin, Bervastatin und Dalvastatin und pharmazeutisch akzeptabler Salze davon.

17. Ein Verfahren zur Herstellung einer pharmazeutischen Zusammensetzung, wobei das Verfahren das Durchführen eines Verfahrens nach Anspruch 15 oder 16 und das Formulieren des Statins oder eines pharmazeutisch akzeptablen Salzes davon mit mindestens einem pharmazeutisch akzeptablen Exzipienten umfasst, um die pharmazeutische Zusammensetzung zu erhalten.

18. Verwendung des Reaktionssystems nach einem der Ansprüche 10 bis 14 zur Herstellung einer Verbindung der Formel (I), wobei die Formel (I) wie in einem der Ansprüche 1 bis 3 definiert ist, und optional zur weiteren Herstellung eines Statins oder eines pharmazeutisch akzeptablen Salzes davon.

19. Verwendung eines Aldose-1-Dehydrogenase-Enzyms zur Herstellung einer Verbindung der Formel (I) 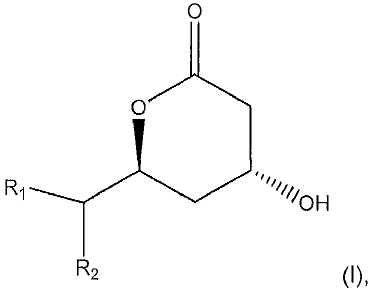 wobei R₁ und R₂ wie oben definiert sind,
wobei die Verbindung weiter in ein Statin oder ein pharmazeutisch akzeptables Salz davon umgewandelt wird.

## Revendications

1. Procédé de préparation d'un composé de formule (I) 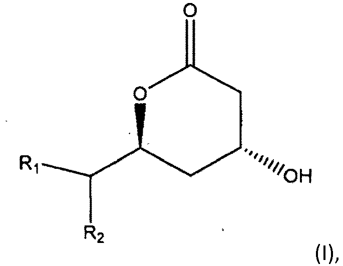 dans laquelle
R₁ indépendamment de R₂ désigne H, X, N₃, CN, NO₂, OH, (CH₂)ₙ-CH₃, O-(CH₂)n-CH₃, S-(CH₂)n-CH₃, NR₃R₄, OCO(CH₂)ₙCH₃, NR₃CO(CH₂)ₙCH₃, CH₂-R₅, un aryle mono- ou bicyclique éventuellement substitué, un groupe hétérocyclique ou alicyclique ; et
R₂ indépendamment de R₁ désigne H, (CH₂)ₘ-CH₃, ou un aryle ;
ou à la fois R₁ et R₂ désignent soit X, OH ou O((CH₂)ₙCH₃) ;
ou R₁ et R₂ désignent conjointement =O, =CH-R₅, dans laquelle l'un quelconque des groupes CH₂ ou CH₃ désignés ci-dessus peut éventuellement être en outre substitué par X, N₃, CN, NO₂, OH, (CH₂)ₙ-CH₃, un aryle, 0-(CH₂)ₙ-CH₃, OCO(CH₂)ₙCH₃, NR₃R₄, NR₃CO(CH₂)ₙCH₃ ; ou
chaque CH₂ liant des atomes de carbone peut être remplacé par O, S ou NR₃ ; dans laquelle
R₃ et R₄ indépendamment l'un de l'autre, ou conjointement, désignent H, (CH₂)ₘ-CH₃ ;
R₅ désigne un aryle mono- ou bicyclique éventuellement substitué, un groupe hétérocyclique ou alicyclique,
X désigne F, Cl, Br ou I ;
n représente un entier de 0 à 10 ;
m représente un entier de 0 à 3 ;
ou un sel pharmaceutiquement acceptable, ou un stéréoisomère de celui-ci,
le procédé comprenant la mise en contact d'un composé lactol non naturel de formule (II), 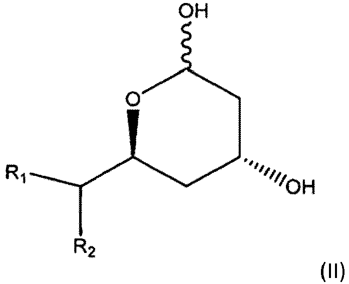 dans lequel R₁ et R₂ sont tels que définis ci-dessus, avec une aldose 1-déshydrogénase.

2. Procédé selon la revendication 1, dans lequel
R₅ désigne une partie sélectionnée parmi l'une des formules (III), (IV), (V), (VI), (VII), (VIII) et (IX) ;

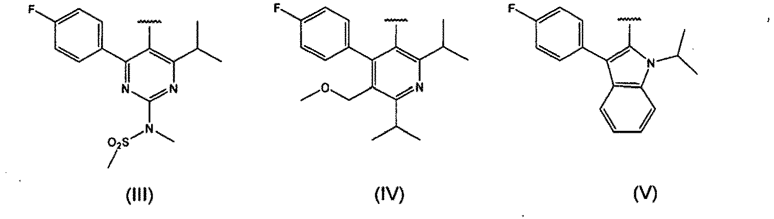

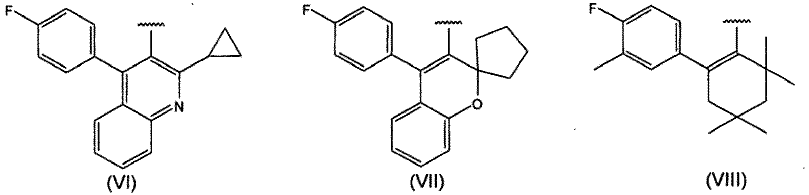

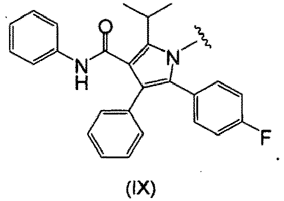

3. Procédé de préparation d'un composé de formule (I) selon la revendication 1, dans lequel
R₁ indépendamment de R₂ désigne H, X, N₃, CN, NO₂, OH, (CH₂)ₙ-CH₃, O-(CH₂)ₙ-CH₃, S-(CH₂)ₙ-CH₃, NR₃R₄, OCO(CH₂)ₙCH₃, ou NR₃CO(CH₂)ₙCH₃, un aryle mono- ou bicyclique éventuellement substitué, un groupe hétérocyclique ou alicyclique ; et
R₂ indépendamment de R₁ désigne H, (CH₂)ₘ-CH₃, ou un aryle ;
ou à la fois R₁ et R₂ désignent soit X, OH ou O(CH₂)ₙCH₃ ;
ou R₁ et R₂ désignent conjointement =O, dans lequel
l'un quelconque de groupes CH₂ ou CH₃ désignés ci-dessus peut éventuellement être en outre substitué par X, N₃, CN, NO₂, OH, (CH₂)ₙ-CH₃, un aryle, 0-(CH₂)ₙ-CH₃, OCO(CH₂)ₙCH₃, NR₃R₄, NR₃CO(CH₂)ₙCH₃ ; ou
chaque CH₂ liant des atomes de carbone peut être remplacé par O, S ou NR₃ ; dans lequel
R₃ et R₄ indépendamment l'un de l'autre, ou conjointement, désignent H, (CH₂)ₘ-CH₃;
X désigne F, Cl, Br ou I ;
n représente un entier de 0 à 10 ;
m représente un entier de 0 à 3.

4. Procédé selon l'une quelconque des revendications précédentes, dans lequel l'aldose 1-déshydrogénase est une déshydrogénase dépendante de la pyrroloquinoline quinine (PQQ).

5. Procédé selon l'une quelconque des revendications précédentes, dans lequel l'aldose déshydrogénase est l'aldose déshydrogénase Ylil de E. coli ou la glucose déshydrogénase mGDH de E. coli.

6. Procédé selon l'une quelconque des revendications précédentes, dans lequel l'aldose 1-déshydrogénase est sélectionnée dans le groupe constitué des déshydrogénases codées par les gènes codant les déshydrogénases compris dans SEQ ID NO. 01 ou est sélectionnée dans le groupe constitué des déshydrogénases codées par les gènes codant les déshydrogénases sélectionnés dans l'une quelconque des séquences nucléotidiques SEQ ID NO. 03, 05, 07, 09, 23, 25, 27, 29, 31, 33, 35, 37, 39, 41, 43, 45, 47, 49, 51, 53, 55, 57, 59 et 61 ; ou les déshydrogénases définies par l'une quelconque des séquences d'acides aminés SEQ ID NO. 02, 04, 06, 08, 10, 24, 26, 28, 30, 32, 34, 36, 38, 40, 42, 44, 46, 48, 50, 52, 54, 56, 58, 60, et 62 ;
ou n'importe quelle déshydrogénase ayant une identité de séquence nucléotidique ou une identité de séquence d'acides aminés respectivement d'au moins 50% avec lesdites séquences, sous réserve que les variantes de séquences résultantes conservent une activité de déshydrogénase.

7. Procédé selon l'une quelconque des revendications précédentes, dans lequel l'enzyme aldolase 2-désoxyribose-5-phosphate (DERA, EC 4.1.2.4) est utilisée pour préparer le composé de formule (II).

8. Procédé selon la revendication 7, dans lequel l'enzyme aldolase 2-désoxyribose-5-phosphate est utilisée dans une étape de synthèse précédant, ou alternativement simultanément au moins dans une période de chevauchement à, la mise en contact du composé de formule (II) avec l'enzyme capable de catalyser l'oxydation ou la déshydrogénation.

9. Procédé selon l'une quelconque des revendications précédentes, dans lequel l'aldolase 1-déshydrogénase, éventuellement l'enzyme DERA également sont indépendamment comprises dans une cellule entière non humaine vivante, une cellule entière non humaine inactivée, une cellule entière non humaine homogénéisée, ou un extrait sans cellule non humain ; ou sont purifiées, immobilisées et/ou sont sous la forme de protéine exprimée de manière extracellulaire.

10. Système réactionnel comprenant une enzyme aldolase 2-désoxyribose-5-phosphate (DERA) et une aldose 1-déshydrogénase, et le système réactionnel étant capable de, ou étant agencé pour, convertir un composé de formule (IX), 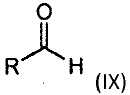 dans laquelle R désigne une partie R₁-CH-R₂ de formule (I), R₁ et R₂ étant tels que définis dans l'une quelconque des revendications 1 à 4, avec un acétaldéhyde en un composé de formule (I) 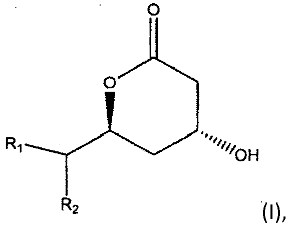 dans laquelle R₁ et R₂ sont tels que définis ci-dessus.

11. Procédé selon la revendication 7 ou système réactionnel selon la revendication 10, dans lequel chacune desdites enzymes, c'est à dire DERA et l'aldose 1-déshydrogénase, sont exprimées par une ou plusieurs cellules, dans lequel le type de cellule est sélectionné dans le groupe constitué des bactéries, des levures, des cellules d'insecte et des cellules de mammifères non humaines.

12. Procédé selon la revendication 7 ou système réactionnel selon la revendication 10, fournissant en outre la présence de Pyrroloquinoline quinine (PQQ).

13. Procédé ou système réactionnel selon la revendication 12, dans lequel la fourniture de la présence de Pyrroloquinoline quinine (PQQ) est accomplie par une action sélectionnée dans le groupe constitué de :
(i) la PQQ est ajouté de l'extérieur ;
(ii) un organisme hôte non humain est utilisé qui, au-delà de la fourniture de la présence de l'activité déshydrogénase, a en outre une capacité de biosynthèse de PQQ intrinsèque ; et
(iii) un microorganisme est utilisé, qui n'a pas la capacité intrinsèque de biosynthèse de PQQ, mais qui est génétiquement modifié pour exprimer le groupe de gènes en lien avec la synthèse de PQQ.

14. Procédé ou système réactionnel selon la revendication 13, dans lequel le microorganisme utilisé selon l'action (iii) est génétiquement modifié pour fournir une expression d'un gène codant pour la synthèse de PQQ compris dans, ou constitué par, l'une quelconque des séquences nucléotidiques de SEQ ID NO. 11, 17, 63, 64, 65, 66, 67, 68, 69, 70 ; ou l'expression d'un gène de synthèse de PQQ codant pour l'une quelconque des séquences d'acides aminés de SEQ ID NO. 12, 13, 14, 15, 16, 18, 19, 20, 21 et 22 ;
ou est génétiquement modifié pour fournir l'expression d'un gène codant pour la synthèse de PQQ ayant une identité de séquence nucléotidique ou une identité de séquence d'acides aminés respectivement d'au moins 50% avec lesdites séquences, sous réserve que les variantes de séquences résultantes conservent une activité pour produire PQQ.

15. Procédé selon l'une quelconque des revendications précédentes, comprenant en outre la soumission dudit composé (I) à des conditions suffisantes pour préparer une statine ou un sel pharmaceutiquement acceptable de celle-ci, éventuellement pour salifier, estérifier ou résoudre de manière stéréochimique le produit de statine.

16. Procédé selon la revendication 15, dans lequel la statine est sélectionnée dans le groupe constitué de la lovastatine, la pravastatine, la simvastatine, l'atorvastatine, la cérivastatine, la rosuvastatine, la fluvastatine, la pitavastatine, la bervastatine et la dalvastatine, et leurs sels pharmaceutiquement acceptables.

17. Procédé de préparation d'une composition pharmaceutique, le procédé comprenant la mise en oeuvre d'un procédé selon le procédé de la revendication 15 ou 16, et la formulation de ladite statine, ou d'un sel pharmaceutiquement acceptable de celle-ci, avec au moins un excipient pharmaceutiquement acceptable pour obtenir ladite composition pharmaceutique.

18. Utilisation du système réactionnel selon l'une quelconque des revendications 10 à 14 pour préparer un composé de formule (I), dans laquelle la formule (I) est telle que définie dans l'une quelconque des revendications 1 à 3, et éventuellement pour la préparation ultérieure d'une statine ou d'un sel pharmaceutiquement acceptable de celle-ci.

19. Utilisation d'une aldose 1-déshydrogénase pour préparer un composé de formule (I) 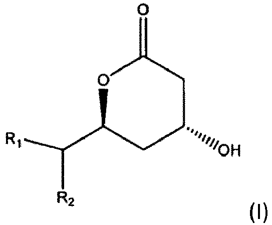 dans laquelle R₁ et R₂ ont la même signification que ci-dessus,
lequel composé est en outre converti en une statine ou un sel pharmaceutiquement acceptable de celle-ci.
